# EUROPEAN PATENT SPECIFICATION

(11) **EP 2 712 624 B1**
(45) Date of publication and mention of the grant of the patent: **06.11.2019**
(21) Application number: 13195822.5
(22) Date of filing: 15.11.2006
(51) Int. Cl.: A61K 39/00, C12N 7/00, A61K 39/12, A61K 39/155, A61K 39/265, A61K 39/02, C07K 14/005

(54) **COMBINATION VACCINE COMPRISING AN ATTENUATED BOVINE VIRAL DIARRHEA VIRUS**
KOMBINATIONSIMPFSTOFF MIT EINEM ATTENUIERTEN RINDERVIREN-DIARRHÖE-VIRUS
VACCIN COMBINÉ COMPRENANT UN VIRUS ATTÉNUÉ DE LA DIARRHÉE VIRALE BOVINE

(30) Priority: 15.11.2005 US 736705 P
(43) Date of publication of application: 02.04.2014
(62) Divisional of application: 06850760.7
(73) Proprietor: Boehringer Ingelheim Vetmedica GmbH, 55216 Ingelheim am Rhein (DE)
(72) Inventor: Elbers, Knut, 55216 Ingelheim am Rhein (DE); Knittel, Jeffrey P, 55216 Ingelheim am Rhein (DE); Meyers, Gregor, 55216 Ingelheim am Rhein (DE); Tremblay, Gregor, 55216 Ingelheim am Rhein (DE); Jones, Craig, 55216 Ingelheim am Rhein (DE); Lischewski, Axel, 55216 Ingelheim am Rhein (DE)
(74) Representative: Hoffmann Eitle

(56) References cited:
- EP-A1- 1 104 676
- EP-A2- 1 013 757
- WO-A1-2005/089793
- WO-A1-2005/111201
- WO-A2-03/023041
- US-A1- 2004 146 854
- US-A1- 2005 002 966
- MAYER D ET AL: "Attenuation of classical swine fever virus by deletion of the viral N<pro> gene", VACCINE, ELSEVIER LTD, GB, vol. 22, no. 3-4, 2 January 2004 (2004-01-02), pages 317-328, XP004479351, ISSN: 0264-410X, DOI: 10.1016/J.VACCINE.2003.08.006
- SCHUNICHT OLIVER C ET AL: "Comparison of a multivalent viral vaccine program versus a univalent viral vaccine program on animal health, feedlot performance, and carcass characteristics of feedlot calves", CANADIAN VETERINARY JOURNAL - REVUE VETERINAIRE CANADIENNE, OTTAWA, CA, vol. 44, no. 1, 1 January 2003 (2003-01-01), pages 43-50, XP009146489, ISSN: 0008-5286
- PETERS A R ET AL: "Duration of immunity of a quadrivalent vaccine against respiratory diseases caused by BHV-1, PI3V, BVDV, and BRSV in experimentally infected calves", PREVENTIVE VETERINARY MEDICINE, ELSEVIER, AMSTERDAM, NL, vol. 66, no. 1-4, 15 December 2004 (2004-12-15), pages 63-77, XP004660337, ISSN: 0167-5877, DOI: DOI:10.1016/J.PREVETMED.2004.08.001
- PATEL J R: "EVALUATION OF A QUADRIVALENT INACTIVATED VACCINE FOR THE PROTECTION OF CATTLE AGAINST DISEASES DUE TO COMMON VIRAL INFECTIONS", JOURNAL OF THE SOUTH AFRICAN VETERINARY MEDICAL ASSOCIATION, PRETORIA, SA, vol. 75, no. 3, 1 September 2004 (2004-09-01), pages 137-146, XP009050707, ISSN: 0038-2809
- DONKERSGOED VAN J ET AL: "COMPARATIVE SEROLOGICAL RESPONSES IN CALVES TO EIGHT COMMERCIAL VACCINES AGAINST INFECTIOUS BOVINE RHINOTRACHEITIS, PARAINFLUENZA-3, BOVINE RESPIRATORY SYNCYTIAL, AND BOVINE VIRAL DIARRHEA VIRUSES", CANADIAN VETERINARY JOURNAL - REVUE VETERINAIRE CANADIENNE, OTTAWA, CA, vol. 32, no. 12, 1 December 1991 (1991-12-01), pages 727-733, XP008024765, ISSN: 0008-5286
- FULTON R W ET AL: "Bovine viral diarrhea virus types 1 and 2 antibody response in calves receiving modified live virus or inactivated vaccines", VACCINE, ELSEVIER LTD, GB, vol. 19, no. 2-3, 15 September 2000 (2000-09-15), pages 264-274, XP004228836, ISSN: 0264-410X, DOI: DOI:10.1016/S0264-410X(00)00168-7
- FULTON R W ET AL: "Antibody responses by cattle after vaccination with commercial viral vaccines containing bovine herpesvirus-1, bovine viral diarrhea virus, parainfluenza-3 virus, and bovine respiratory syncytial virus immunogens and subsequent revaccination at day 140", VACCINE, ELSEVIER LTD, GB, vol. 13, no. 8, 1 June 1995 (1995-06-01), pages 725-733, XP004057520, ISSN: 0264-410X, DOI: DOI:10.1016/0264-410X(94)00072-U
- "Material safety data sheet: CattleMaster 4 +VL5", INTERNET CITATION, 13 August 2003 (2003-08-13), XP002297702, Retrieved from the Internet: URL:http://www.cattlemastergold.com/pahima ges/msds_us/c4.pdf [retrieved on 2004-09-26]

### SEQUENCE LISTING

<110> Boehringer Ingelheim Vetmedica GmbH
<120> Attenuated Bovine Viral Diarrhea Virus (BVDV)
<130> Case 1-1947/EP/2
<160> 39
<170> PatentIn version 3.1
<210> 1
   <211> 12332
   <212> DNA
   <213> Wildtyp BVDV: XIKE-A
<400> 1
   gtatacgaga ttagctaaag aactcgtata tggattggac gtcaacaaat ttttaattgg     60
   caacgtaggg aaccttcccc tcagcgaagg ccgaaaagag gctagccatg cccttagtag    120
   gactagcaaa agtaggggac tagcggtagc agtgagttcg ttggatggcc gaacccctga    180
   gtacagggga gtcgtcaatg gttcgacact ccattagtcg aggagtctcg agatgccatg    240
   tggacgaggg catgcccacg gcacatctta acccatgcgg gggttgcatg ggtgaaagcg    300
   ctattcgtgg cgttatggac acagcctgat agggtgtagc agagacctgc tattccgcta    360
   gtaaaaactc tgctgtacat ggcacatgga gttgttttca aatgaacttt tatacaaaac    420
   atataaacaa aaaccagcag gcgtcgtgga acctgtttac gacgtcaacg ggcgcccact    480
   gtttggagag agcagtgact tgcacccgca gtcaacacta aaactaccac accaacgagg    540
   cagcgccaac atcctgacca atgctaggtc cctaccgcgg aaaggtgact gccggagagg    600
   taatgtgtat ggaccggtga gtggcatcta tatcaaacca ggaccgatct actaccagga    660
   ttatgtgggc cccgtctatc atagagcccc actggaacta tgtagggagg caagtatgtg    720
   cgaaacaact aggagagttg gcagagtgac cggtagtgat gggaaattat atcatatcta    780
   catctgcata gatgggtgta tcctcctgaa gagggcgact aggaaccaac cagaagtcct    840
   gaaatgggta tacaacagat taaattgtcc tttatgggtc accagctgct ccgatgaagg    900
   gagcaagggt gctacaagta agaagcagcc taagccagat aggatagaaa aaggtaagat    960
   gaaaatagcc ccaaaagaga cagaaaaaga ttgcaaaacc agaccccccg acgcgactat   1020
   agtagtagaa ggggttaagt accaggtgaa gaaaaaagga aaggtaaggg gaaaaaatac   1080
   tcaagatggg ttatatcaca acaagaataa gccccctgaa tcaagaaaaa aattggaaaa   1140
   ggcactgctg gcttgggcca tcttagcagc ggtcctgctt cagctggtaa caggagagaa   1200
   tatcacccag tggaacttga tggacaacgg caccgaggga atacagcaag cgatgttcct   1260
   aagaggggtg aacaggagtc tacatggaat ttggccagag aaaatttgca ccggagtacc   1320
   aactcactta gcaacagact atgagcttaa agagatagtg gggatgatgg acgcgagtga    1380
   gaagaccaac tacacgtgtt gcaggttgca aagacatgag tggaataaac atggttggtg 1440
   taactggttt catatagaac cgtggatatg gttgatgaac aaaacccaaa acaacctgac 1500
   agaagggcaa ccgcttaggg agtgtgctgt gacttgtagg tatgacaagg aaacagaatt 1560
   gaacatcgtg acacaggcta gggacagacc tacaactctg acaggttgca agaaaggcaa 1620
   gaatttctct ttcgcaggtg ttatactgga tgggccctgt aactttaaag tatcggttga 1680
   agatgtgctg ttcaaggagc acgattgcgg caacatgctg caagagaccg cgatacagct 1740
   actcgatggg gcaaccaaca ccattgaggg agcaagggta gggacggcca agttgacaac 1800
   ctggttaggg aagcaattag ggatccttgg taagaagttg gagaacaaaa gcaaagcatg 1860
   gtttggtgca catgcagcaa gtccatactg cggagtggag aggaagatcg gttacgtatg 1920
   gtatacaaaa aactgcactc cagcttgcct tccaagaaac actagaataa taggccccgg 1980
   gaaatttgat accaacgccg aagatggaaa aatactccat gagatggggg ggcacctctc 2040
   agaatttgtc ctattgtcct tggtggttct gtctgacttt gccccggaaa ccgcgagcgt 2100
   catctacttg gttctacatt ttgcgatccc gcaaagccac gttgatgtag acacatgcga 2160
   caagaaccag ctgaatttaa cggtagcaac cacagtagca gaggtcatac cagggacagt 2220
   gtggaaccta gggaagtatg tctgcataag accagactgg tggccatatg agacgacgac 2280
   agtcttcgtc atagaggaag cagggcaagt aatcaaattg atgctaaggg ccatcagaga 2340
   cttaactagg atatggaatg ctgccactac cacagctttc ttaatctttt tagtaaaagc 2400
   actgagggga caactaatcc aagggctatt gtggctgatg ctaataacag gagcacaggg 2460
   cttccctgaa tgcaaagagg gcttccaata tgccatatct aaagacagga aaatggggtt 2520
   attggggcca gagagcttaa ctacaacatg gcacctcccc accaaaaaaa tagtggattc 2580
   catggtgcat gtatggtgtg aaggaaaaga cttgaaaata ttaaaaatgt gcacaaagga 2640
   agagaggtat ctagtggctg tgcacgagag agccttatca accagtgccg agtttatgca 2700
   gatcagtgat gggacaatag gcccagacgt gatagatatg cctgatgact ttgagtttgg 2760
   actctgccct tgtgactcaa aaccagtgat aaagggcaaa tttaatgcca gcttactgaa 2820
   tggaccagct ttccagatgg tatgcccaca ggggtggact ggtacaatag aatgcaccct 2880
   agcgaaccaa gacaccttgg acacaactgt cattaggaca tatagaagaa ctaccccatt 2940
   tcagcggaga aaatggtgta cctatgaaaa aataataggg gaagatatct atgaatgcat 3000
   tctaggtgga aactggacat gcataaccgg tgaccatagc aggttgaaag acggacctat 3060
   caagaagtgt aagtggtgtg gccatgactt cgtcaactca gaggggctac cacactaccc 3120
   aataggcaag tgcatgctca tcaacgagag tgggtacagg tatgtagatg acacctcttg 3180
   cgataggggt ggtgtagcca tagttccatc tggcaccgta aagtgtagaa taggtaacgt 3240
   cacggtgcaa gttatcgcta ctaacaatga tctgggaccc atgccttgca gcccagctga 3300
   agtgatagca agtgaaggac cagtggaaaa gactgcatgc acattcaact attcaaggac 3360
   tctacctaat aagtattatg agccaaggga ccggtacttc caacaataca tgttaaaagg 3420
   ggagtggcaa tattggttcg acctggattc tgtagaccac cacaaagact acttctcaga 3480
   gttcataatc atagcagtgg tcgccttgtt gggtggtaag tacgtactgt ggctcttgat 3540
   aacatacaca atactgtctg agcagatggc tatgggtgct ggagtgaata ctgaagagat 3600
   agtcatgata ggcaatttgc tgacagacag tgatattgag gttgtggttt atttccttct 3660
   tctgtactta atagttaaag aggaactggc gaggaaatgg attatactgg tataccacat 3720
   ccttgtagcc aaccctatga aaacaattgg ggtcgtctta ctaatgctag ggggagtggt 3780
   gaaggccagc agaatcaatg ctgatgacca aagtgctatg gacccatgct ttcttctcgt 3840
   gacaggcgta gtggctgttt tgatgatcgc tagaagagaa cctgccacat taccactgat 3900
   tgtagcattg ctagcaataa gaacatcagg attcctactg cccgctagca ttgatgtaac 3960
   tgtagcagta gtattaattg tacttttgtt ggctagctac ataacagact actttagata 4020
   taaaaagtgg cttcaactct tatttagtct gatagctggt atctttatta taaggagctt 4080
   aaaacatatc aaccagatgg aggtaccaga aatatctatg ccaagttgga gacctctagc 4140
   tctggtcctt ttctatataa catctacagc aataaccact aattgggaca ttgacttagc 4200
   aggcttcctg ctgcaatggg cgccagcagt gatcatgatg gctaccatgt gggcagactt 4260
   tttgactctg atcatagtcc tgcccagtta cgagttatct aagctttact tcctaaagaa 4320
   cgtcaggaca gacgtggaaa agaactggct cggcaaagtg aaatacagac agatcagttc 4380
   agtttatgac atctgtgaca gtgaggaagc agtgtaccta tttccatcaa ggcataagag 4440
   tggaagcagg ccagatttca tattaccttt tttgaaagcc gtgttaataa gctgcatcag 4500
   cagccaatgg caagtggttt acatttctta cctaatactg gaaattacat actatatgca 4560
   caggaaaatc atagatgagg tgtcaggagg agcaaatttt ctatcaagac tcatagcagc 4620
   catcatagaa ttaaattggg ccatagatga tgaggaatgt aaaggactga agaaactgta 4680
   tctcttgtca gggagagcga agaatttgat agttaaacat aaggtaagaa atgaagccgt 4740
   ccacagatgg tttggtgagg aggaaatata cggggcaccc aaggtgatca ctatcataaa 4800
   agctagtacc ctaagtaaaa acaggcactg cataatctgc acgatctgtg aagggaaaga 4860
   atggaatgga gccaactgcc caaagtgtgg aagacaagga aagcccataa catgtggaat 4920
   gacactcgca gactttgagg agaaacatta caaaaagata tttataagag aagaatcttc 4980
   ttgtcctgtg ccttttgatc cttcttgcca ttgtaattat tttcgccacg atgggccttt 5040
   caggaaagag tataagggtt acgtccaata cacagccaga ggacaactct ttctgaggaa 5100
   cctaccaatt ctagcgacga agatgaagct attaatggtg ggaaacctcg gcgcagaaat 5160
   tggcgacctg gaacatctag gatgggtact gagagggcca gccgtgtgca aaaaaattac 5220
   caaccatgag aagtgccacg taaacatcat ggataagcta actgcatttt ttggaatcat 5280
   gcctagaggc acgaccccta gggcacctgt gaggttcccc acagcactac taaaagtgag 5340
   aagggggcta gagacgggat gggcttacac gcaccaagga gggatcagct cggtagacca 5400
   tgtcacagcc ggaaaggatt tactagtgtg tgacagtatg ggcaggacca gggttgtctg 5460
   tcatagtaac aataagatga ctgatgagac tgagtatggc atcaagaccg actcagggtg 5520
   tcccgaaggt gcgaggtgtt acgtgctaaa cccagaagct gttaacattt ctggcacaaa 5580
   aggagctatg gtacacctcc agaaaacggg gggggagttc acatgtgtca ctgcctcagg 5640
   gaccccggct ttcttcgatc tgaaaaatct aaaaggctgg tccgggctac caatttttga 5700
   agcatccagt ggcagggtgg ttggtagggt gaaagtcggc aagaatgagg attccaagcc 5760
   caccaaacta atgagcggaa tccagacagt gtctaagaac cagacagacc tagcggacat 5820
   cgtaaaaaaa ttgactagta tgaacagagg agagttcaaa cagataacat tagccactgg 5880
   ggcaggaaaa actacggaac tgccaaggtc cgtcatagag gagataggga ggcacaaaag 5940
   ggtcttagtc ctgataccat tgagagcagc agcagagtca gtgtatcagt atatgagagt 6000
   gaagtaccca agtatatctt tcaatttgag aataggagat atgaaggaag gtgacatggc 6060
   cactggtatc acctacgcct catatgggta cttttgtcag cttcctcagc ccaaactgag 6120
   agctgccatg gtagagtact catatatatt cttagatgag taccactgtg ctacacccga 6180
   gcaattagca ataattggaa agatacacag gtttgctgaa aatcttagag tggtagcaat 6240
   gacagcaacc ccagctggaa cggtcacaac gactggtcag aaacacccta tagaggagtt 6300
   catagcccca gaggtgatga aaggtgaaga tctaggtagt gaatacttgg atattgcagg 6360
   gttgaagata ccgactgaag agatgaaagg caacatgctc gtgttcgcgc caactaggaa 6420
   catggcagta gaaacagcta agaaattgaa ggctaaggga tacaactctg gatactatta 6480
   cagtggggaa aacccagaga acttgagggt ggtaacctcg caatccccgt atgtggtagt 6540
   agccaccaat gccatagagt caggtgtgac attaccagac ttagacacag ttgtagacac 6600
   tggactaaag tgtgagaaga gggtgaggat ttcttcaaaa atgcccttca ttgtaacagg 6660
   acttaagaga atggcagtca caatcggaga gcaagcccag cgcaggggta gagtaggaag 6720
   agtcaagcca ggtaggtact ataggagtca agaaacagct tcagggtcaa aagattacca 6780
   ttacgaccta ctgcaagccc agaggtacgg aatagaagat ggaattaatg taacaaagtc 6840
   attcagggag atgaactatg attggagcct ttacgaagag gacagcttga tgataactca 6900
   actcgaggtc cttaacaacc tccttatatc agaagacctg cctgccgcag tgaagaacat 6960
   catggcccgg accgatcacc cagaacccat acaactggcc tataacagtt atgaaaacca 7020
   aattccagtg ctgttcccaa agatcaaaaa tggtgaggtg acagacagtt atgagaatta 7080
   cacatatctc aatgcaagaa aattaggaga ggacgtgccg gcatatgtgt acgccacaga 7140
   ggatgaggat ctagcagtgg atcttctggg tatggattgg ccggacccag gcaaccaaca 7200
   ggtggtagag acagggaggg cattaaaaca agtaactggc ttatccacag cagaaaacgc 7260
   cctcttgata gccctattcg gctacgtcgg gtaccagaca ctttcaaaaa ggcacatacc 7320
   catgattact gacatctata cacttgaaga ccacaggctt gaggacacaa cccacctcca 7380
   gtttgcccca aacgctataa ggaccgacgg caaggactca gagttgaagg aattagctgt 7440
   gggagacctt gataaatatg tggacgcact ggtagactac tccaaacaag ggatgaaatt 7500
   catcaaagtc caagctgaaa aggtcagaga ctcccagtct acgaaggaag gcttgcaaac 7560
   cattaaggag tatgtggata agtttataca atcactaaca gagaataagg aggagatcat 7620
   caggtatgga ctatggggag ttcacacggc actctacaaa agcttggcag cgagactggg 7680
   gcatgaaaca gcttttgcaa ctttagtggt aaaatggttg gcttttgggg gcgaaacggt 7740
   atctgctcac atcaagcaag tagcagttga tctagtagta tattatatca tcaacaaacc 7800
   atcttttcct ggagatacag agacccaaca agaggggagg aagtttgtgg ctagtctttt 7860
   tatatctgca ctagcaacat acacatataa aacctggaat tacaacaatc tgcaacgggt 7920
   tgtcgaacct gccttagctt acctcccata tgctacaagt gccttgaagt tgttcacacc 7980
   cacaagatta gagagtgtgg tcatactcag ttctacaatt tacaagacat acctctctat 8040
   aaggaagggt aagagtgacg gcttgttagg tacaggcata agtgcagcca tggagatctt 8100
   aaaccaaaac ccaatctcag taggtatatc tgtgatgctg ggggtaggtg ccatcgccgc 8160
   ccataatgca atagaatcta gtgaacagaa aagaactttg ctgatgaagg tctttgtaaa 8220
   aaacttctta gaccaagcag caacagatga gctagtcaaa gagaaccctg aaaaaataat 8280
   catggctcta tttgaagcag tccagaccat aggaaacccc ctaagactca tctaccatct 8340
   gtacggggtg tactataagg ggtgggaagc aaaagaactc gcagagaaaa ctgctggccg 8400
   caacttattc acattgatca tgtttgaggc ctttgagctt ttaggtatgg actcagaagg 8460
   aaagataaga aacttgtcag gcaactacat actggactta atcttcaact tgcataataa 8520
   attaaacaag gggctcaaaa aactagtcct tgggtgggct cctgcacctt tgagctgtga 8580
   ttggacacca agtgatgaga gaataagcct acctcataac aactacttaa gggtagaaac 8640
   caggtgtcct tgtggctatg agatgaaggc aataaaaaat gttgctggta aattgacaaa 8700
   agttgaagaa aaggggtcct tcctatgcag gaatagatta gggagaggac ctccaaactt 8760
   caaagtaaca aagttctatg atgataactt gatagaagtc aagccagtag ctaggctaga 8820
   aggccaggtg gacctctatt acaagggagt aacagctaag ttagactaca acaatgggaa 8880
   agtactgtta gctaccaaca agtgggaggt ggaccacgct ttcctgacca gactagtaaa 8940
   gaagcacaca gggataggtt ttaaaggtgc atatttgggt gaccgaccag accatcaaga 9000
   tcttgtcgat agagattgtg caactataac gaagaactca gtacagttcc taaaaatgaa 9060
   gaagggttgc gctttcacat atgacctaac aatctctaac cttgtcaggc ttattgaact 9120
   agtccataag aataatttac aagaaagaga gatccctacc gtgacagtaa ctacttggct 9180
   tgcatattct tttgtcaatg aagacctggg gactatcaag cctgtattgg gggagaaagt 9240
   catcccagaa ccccccgagg agttgagtct ccaacccacc gtgagactag tcaccactga 9300
   aacagcaata accataacag gggaggctga agtgatgacg acagggatca caccagtggt 9360
   agagatgaaa gaagaacctc agctggacca ccagtcaact accctaaagg tagggttgaa 9420
   ggaaggggaa tatccagggc caggagttaa ccctaaccat ttagcagagg tgatagatga 9480
   gaaagatgac aggccttttg tcctaatcat cggtaacaaa ggttctacct cgaacagagc 9540
   aagaacggcc aagaatatac ggctgtacaa aggaaacaac ccaagagaga tcagggatct 9600
   gatgagccaa ggaagaatat tgacggttgc tctaaaagag ttggacccgg aattaaaaga 9660
   attagtagat tacaagggga cctttctcaa tagggaagct ttagaagccc taagcttagg 9720
   taagccaatc aagaggaaaa ccacaacagc aatgatcagg aggttaatag agccagaggt 9780
   tgaggaggaa ctaccagatt ggttccaagc ggaagaaccc ctatttttgg aagcaaaaat 9840
   acagaatgac ttataccacc taattggcag tgtagatagt ataaaaagca aagcaaagga 9900
   attaggggcc acagataaca caaagatagt gaaggaagtt ggggctagga cctatacgat 9960
   gaaattgagc agctggagca cacaagttac aaaaaaacag atgagtctag cccctctctt 10020
   tgaagagctg ttattaaagt gccctccatg tagtaaaatt tcaaagggac atatggtgtc 10080
   agcataccaa ctggctcaag gaaactggga acccctcggg tgtggggtct atatgggaac 10140
   cataccagct aggcgtctca agatccaccc ttatgaggct taccttaaac tcaaagagct 10200
   ggtggaagtt gaatcttcga gggccactgc aaaagaatcc atcataagag aacataacac 10260
   ctggatcctg cggaaggtga gacatgaagg gaacctaaga accaaatcaa tgatcaaccc 10320
   tgggaaaata tcagatcagc tatgcagaga tggacacaaa agaaacatat ataataagat 10380
   cataggctca acaatggcct ctgctggtat taggctggag aaactgccag tagtccgagc 10440
   ccaaactgac acaaccagtt tccaccaagc cataagagaa aaaattgata aaacagaaaa 10500
   caagcagacc cctgaattgc atgaagaact aatgaaggtc ttcgactgct taaagatccc 10560
   agagctgaag gaatcgtatg atgaagtttc atgggaacaa ttagaagccg ggataaaccg 10620
   taagggtgca gcaggctatc tagagagcaa gaacataggg gaagtcctag acacagagaa 10680
   acacatagta gagcagctga tcaaggatct gaggaagggg aagaagatta ggtactatga 10740
   aacagccatc cccaagaatg agaagagaga cgtcagcgac gactgggaag ccggagagtt 10800
   cgttgatgaa aagaaaccaa gagtaatcca gtacccggac gccaaggtga gactggccat  10860
   tacaaaagtg atgtacaaat gggtaaagca aaaaccagtg gtgatacccg gctatgaagg  10920
   taaaacacct ctatttgaca tattcaacaa agtgaagaag gaatgggatt cattccagga  10980
   ccccgtagca gtgagctttg acaccaaagc gtgggataca caagtcacca gtagagacct  11040
   aatgttgata aaggatatcc agaaatatta tttcaagaga agtatacaca aatttttaga  11100
   tacaataaca gaacacatgg tggaggtacc tgtcattaca gcagacggtg aagtttacat  11160
   aaggaatggt cagaggggta gtggccaacc cgacacaagt gctggtaata gtatgttgaa  11220
   tgtcctaacc atgatatatg ctttctgtaa aagtacaggc ataccttaca ggggattcag  11280
   cagagtggca agaatccatg tgtgtggtga tgatggcttt ttgataacag agagaggact  11340
   gggactgaaa ttctctgaga agggtatgca gatattacat gaggccggga agccccagaa  11400
   aataactgaa ggggacaaaa tgaaagtggc atacagattc gaggacatag agttttgttc  11460
   ccatactccc gtgccagtca gatgggcaga taacaccagt agttacatgg cagggaggag  11520
   cacagccact atactagcta agatggcaac caggctggat tccagcggag agaggggtag  11580
   cacagcttat gagaaggccg tagccttcag cttccttttg atgtactcat ggaatcccgt  11640
   agttagaagg atctgcttac tggtgttgtc acagtttcca gaaatatccc catccaaaaa  11700
   cacaatatac tactaccaag gggatcccat agctgcgtac agagaagtga tagggaaaca  11760
   gctgtgtgaa ctgaaaagaa caggatttga gaagctggct ggtctgaatt tgagtatgac  11820
   cactctaggc atctggacaa aacatactag taaaagacta atccaagcct gtgtagaaat  11880
   aggtaagaga gaaggtacct ggttagttaa tgctgacaga ctgattgcag gaaagactgg  11940
   gaagttttac atcccaagca ctggtgtcac tctgttggga aaacactatg aggaaattaa  12000
   cttaaagcaa aaggcggcac aaccgccgat agagggggtt gacagatata agttgggccc  12060
   catagttaat gttatcttga gaaggctgag ggtgatgctg atgacagttg ccagcggaag  12120
   ctggtgaatc cgtccggagc gtcgtgccct cactcaaggt ttttaattgt aaatattgta  12180
   aatagacagc taagatattt attgtagttg gatagtaatg cagtgatagt aaatacccca  12240
   atttaacact acctccaatg cactaagcac tttagctgtg tgaggttaac tcgacgtcca  12300
   cggttggact agggaagacc tctaacagcc cc                                12332
<210> 2
   <211> 11840
   <212> DNA
   <213> Artificial Sequence: Mutated BVDV: XIKE- A-NdN
<400> 2
   gtatacgaga ttagctaaag aactcgtata tggattggac gtcaacaaat ttttaattgg 60
   caacgtaggg aaccttcccc tcagcgaagg ccgaaaagag gctagccatg cccttagtag 120
   gactagcaaa agtaggggac tagcggtagc agtgagttcg ttggatggcc gaacccctga 180
   gtacagggga gtcgtcaatg gttcgacact ccattagtcg aggagtctcg agatgccatg 240
   tggacgaggg catgcccacg gcacatctta acccatgcgg gggttgcatg ggtgaaagcg 300
   ctattcgtgg cgttatggac acagcctgat agggtgtagc agagacctgc tattccgcta 360
   gtaaaaactc tgctgtacat ggcacatgga gttgttttcc gatgaaggga gcaagggtgc 420
   tacaagtaag aagcagccta agccagatag gatagaaaaa ggtaagatga aaatagcccc 480
   aaaagagaca gaaaaagatt gcaaaaccag accccccgac gcgactatag tagtagaagg 540
   ggttaagtac caggtgaaga aaaaaggaaa ggtaagggga aaaaatactc aagatgggtt 600
   atatcacaac aagaataagc cccctgaatc aagaaaaaaa ttggaaaagg cactgctggc 660
   ttgggccatc ttagcagcgg tcctgcttca gctggtaaca ggagagaata tcacccagtg 720
   gaacttgatg gacaacggca ccgagggaat acagcaagcg atgttcctaa gaggggtgaa 780
   caggagtcta catggaattt ggccagagaa aatttgcacc ggagtaccaa ctcacttagc 840
   aacagactat gagcttaaag agatagtggg gatgatggac gcgagtgaga agaccaacta 900
   cacgtgttgc aggttgcaaa gacatgagtg gaataaacat ggttggtgta actggtttca 960
   tatagaaccg tggatatggt tgatgaacaa aacccaaaac aacctgacag aagggcaacc 1020
   gcttagggag tgtgctgtga cttgtaggta tgacaaggaa acagaattga acatcgtgac 1080
   acaggctagg gacagaccta caactctgac aggttgcaag aaaggcaaga atttctcttt 1140
   cgcaggtgtt atactggatg ggccctgtaa ctttaaagta tcggttgaag atgtgctgtt 1200
   caaggagcac gattgcggca acatgctgca agagaccgcg atacagctac tcgatggggc 1260
   aaccaacacc attgagggag caagggtagg gacggccaag ttgacaacct ggttagggaa 1320
   gcaattaggg atccttggta agaagttgga gaacaaaagc aaagcatggt ttggtgcaca 1380
   tgcagcaagt ccatactgcg gagtggagag gaagatcggt tacgtatggt atacaaaaaa 1440
   ctgcactcca gcttgccttc caagaaacac tagaataata ggccccggga aatttgatac 1500
   caacgccgaa gatggaaaaa tactccatga gatggggggg cacctctcag aatttgtcct 1560
   attgtccttg gtggttctgt ctgactttgc cccggaaacc gcgagcgtca tctacttggt 1620
   tctacatttt gcgatcccgc aaagccacgt tgatgtagac acatgcgaca agaaccagct 1680
   gaatttaacg gtagcaacca cagtagcaga ggtcatacca gggacagtgt ggaacctagg 1740
   gaagtatgtc tgcataagac cagactggtg gccatatgag acgacgacag tcttcgtcat 1800
   agaggaagca gggcaagtaa tcaaattgat gctaagggcc atcagagact taactaggat 1860
   atggaatgct gccactacca cagctttctt aatcttttta gtaaaagcac tgaggggaca 1920
   actaatccaa gggctattgt ggctgatgct aataacagga gcacagggct tccctgaatg 1980
   caaagagggc ttccaatatg ccatatctaa agacaggaaa atggggttat tggggccaga 2040
   gagcttaact acaacatggc acctccccac caaaaaaata gtggattcca tggtgcatgt 2100
   atggtgtgaa ggaaaagact tgaaaatatt aaaaatgtgc acaaaggaag agaggtatct 2160
   agtggctgtg cacgagagag ccttatcaac cagtgccgag tttatgcaga tcagtgatgg 2220
   gacaataggc ccagacgtga tagatatgcc tgatgacttt gagtttggac tctgcccttg 2280
   tgactcaaaa ccagtgataa agggcaaatt taatgccagc ttactgaatg gaccagcttt 2340
   ccagatggta tgcccacagg ggtggactgg tacaatagaa tgcaccctag cgaaccaaga 2400
   caccttggac acaactgtca ttaggacata tagaagaact accccatttc agcggagaaa 2460
   atggtgtacc tatgaaaaaa taatagggga agatatctat gaatgcattc taggtggaaa 2520
   ctggacatgc ataaccggtg accatagcag gttgaaagac ggacctatca agaagtgtaa 2580
   gtggtgtggc catgacttcg tcaactcaga ggggctacca cactacccaa taggcaagtg 2640
   catgctcatc aacgagagtg ggtacaggta tgtagatgac acctcttgcg ataggggtgg 2700
   tgtagccata gttccatctg gcaccgtaaa gtgtagaata ggtaacgtca cggtgcaagt 2760
   tatcgctact aacaatgatc tgggacccat gccttgcagc ccagctgaag tgatagcaag 2820
   tgaaggacca gtggaaaaga ctgcatgcac attcaactat tcaaggactc tacctaataa 2880
   gtattatgag ccaagggacc ggtacttcca acaatacatg ttaaaagggg agtggcaata 2940
   ttggttcgac ctggattctg tagaccacca caaagactac ttctcagagt tcataatcat 3000
   agcagtggtc gccttgttgg gtggtaagta cgtactgtgg ctcttgataa catacacaat 3060
   actgtctgag cagatggcta tgggtgctgg agtgaatact gaagagatag tcatgatagg 3120
   caatttgctg acagacagtg atattgaggt tgtggtttat ttccttcttc tgtacttaat 3180
   agttaaagag gaactggcga ggaaatggat tatactggta taccacatcc ttgtagccaa 3240
   ccctatgaaa acaattgggg tcgtcttact aatgctaggg ggagtggtga aggccagcag 3300
   aatcaatgct gatgaccaaa gtgctatgga cccatgcttt cttctcgtga caggcgtagt 3360
   ggctgttttg atgatcgcta gaagagaacc tgccacatta ccactgattg tagcattgct 3420
   agcaataaga acatcaggat tcctactgcc cgctagcatt gatgtaactg tagcagtagt 3480
   attaattgta cttttgttgg ctagctacat aacagactac tttagatata aaaagtggct 3540
   tcaactctta tttagtctga tagctggtat ctttattata aggagcttaa aacatatcaa 3600
   ccagatggag gtaccagaaa tatctatgcc aagttggaga cctctagctc tggtcctttt 3660
   ctatataaca tctacagcaa taaccactaa ttgggacatt gacttagcag gcttcctgct 3720
   gcaatgggcg ccagcagtga tcatgatggc taccatgtgg gcagactttt tgactctgat 3780
   catagtcctg cccagttacg agttatctaa gctttacttc ctaaagaacg tcaggacaga 3840
   cgtggaaaag aactggctcg gcaaagtgaa atacagacag atcagttcag tttatgacat 3900
   ctgtgacagt gaggaagcag tgtacctatt tccatcaagg cataagagtg gaagcaggcc 3960
   agatttcata ttaccttttt tgaaagccgt gttaataagc tgcatcagca gccaatggca 4020
   agtggtttac atttcttacc taatactgga aattacatac tatatgcaca ggaaaatcat 4080
   agatgaggtg tcaggaggag caaattttct atcaagactc atagcagcca tcatagaatt 4140
   aaattgggcc atagatgatg aggaatgtaa aggactgaag aaactgtatc tcttgtcagg 4200
   gagagcgaag aatttgatag ttaaacataa ggtaagaaat gaagccgtcc acagatggtt 4260
   tggtgaggag gaaatatacg gggcacccaa ggtgatcact atcataaaag ctagtaccct 4320
   aagtaaaaac aggcactgca taatctgcac gatctgtgaa gggaaagaat ggaatggagc 4380
   caactgccca aagtgtggaa gacaaggaaa gcccataaca tgtggaatga cactcgcaga 4440
   ctttgaggag aaacattaca aaaagatatt tataagagaa gaatcttctt gtcctgtgcc 4500
   ttttgatcct tcttgccatt gtaattattt tcgccacgat gggcctttca ggaaagagta 4560
   taagggttac gtccaataca cagccagagg acaactcttt ctgaggaacc taccaattct 4620
   agcgacgaag atgaagctat taatggtggg aaacctcggc gcagaaattg gcgacctgga 4680
   acatctagga tgggtactga gagggccagc cgtgtgcaaa aaaattacca accatgagaa 4740
   gtgccacgta aacatcatgg ataagctaac tgcatttttt ggaatcatgc ctagaggcac 4800
   gacccctagg gcacctgtga ggttccccac agcactacta aaagtgagaa gggggctaga 4860
   gacgggatgg gcttacacgc accaaggagg gatcagctcg gtagaccatg tcacagccgg 4920
   aaaggattta ctagtgtgtg acagtatggg caggaccagg gttgtctgtc atagtaacaa 4980
   taagatgact gatgagactg agtatggcat caagaccgac tcagggtgtc ccgaaggtgc 5040
   gaggtgttac gtgctaaacc cagaagctgt taacatttct ggcacaaaag gagctatggt 5100
   acacctccag aaaacggggg gggagttcac atgtgtcact gcctcaggga ccccggcttt 5160
   cttcgatctg aaaaatctaa aaggctggtc cgggctacca atttttgaag catccagtgg 5220
   cagggtggtt ggtagggtga aagtcggcaa gaatgaggat tccaagccca ccaaactaat 5280
   gagcggaatc cagacagtgt ctaagaacca gacagaccta gcggacatcg taaaaaaatt 5340
   gactagtatg aacagaggag agttcaaaca gataacatta gccactgggg caggaaaaac 5400
   tacggaactg ccaaggtccg tcatagagga gatagggagg cacaaaaggg tcttagtcct 5460
   gataccattg agagcagcag cagagtcagt gtatcagtat atgagagtga agtacccaag 5520
   tatatctttc aatttgagaa taggagatat gaaggaaggt gacatggcca ctggtatcac 5580
   ctacgcctca tatgggtact tttgtcagct tcctcagccc aaactgagag ctgccatggt 5640
   agagtactca tatatattct tagatgagta ccactgtgct acacccgagc aattagcaat 5700
   aattggaaag atacacaggt ttgctgaaaa tcttagagtg gtagcaatga cagcaacccc 5760
   agctggaacg gtcacaacga ctggtcagaa acaccctata gaggagttca tagccccaga 5820
   ggtgatgaaa ggtgaagatc taggtagtga atacttggat attgcagggt tgaagatacc 5880
   gactgaagag atgaaaggca acatgctcgt gttcgcgcca actaggaaca tggcagtaga 5940
   aacagctaag aaattgaagg ctaagggata caactctgga tactattaca gtggggaaaa 6000
   cccagagaac ttgagggtgg taacctcgca atccccgtat gtggtagtag ccaccaatgc 6060
   catagagtca ggtgtgacat taccagactt agacacagtt gtagacactg gactaaagtg 6120
   tgagaagagg gtgaggattt cttcaaaaat gcccttcatt gtaacaggac ttaagagaat 6180
   ggcagtcaca atcggagagc aagcccagcg caggggtaga gtaggaagag tcaagccagg 6240
   taggtactat aggagtcaag aaacagcttc agggtcaaaa gattaccatt acgacctact 6300
   gcaagcccag aggtacggaa tagaagatgg aattaatgta acaaagtcat tcagggagat 6360
   gaactatgat tggagccttt acgaagagga cagcttgatg ataactcaac tcgaggtcct 6420
   taacaacctc cttatatcag aagacctgcc tgccgcagtg aagaacatca tggcccggac 6480
   cgatcaccca gaacccatac aactggccta taacagttat gaaaaccaaa ttccagtgct 6540
   gttcccaaag atcaaaaatg gtgaggtgac agacagttat gagaattaca catatctcaa 6600
   tgcaagaaaa ttaggagagg acgtgccggc atatgtgtac gccacagagg atgaggatct 6660
   agcagtggat cttctgggta tggattggcc ggacccaggc aaccaacagg tggtagagac 6720
   agggagggca ttaaaacaag taactggctt atccacagca gaaaacgccc tcttgatagc 6780
   cctattcggc tacgtcgggt accagacact ttcaaaaagg cacataccca tgattactga 6840
   catctataca cttgaagacc acaggcttga ggacacaacc cacctccagt ttgccccaaa 6900
   cgctataagg accgacggca aggactcaga gttgaaggaa ttagctgtgg gagaccttga 6960
   taaatatgtg gacgcactgg tagactactc caaacaaggg atgaaattca tcaaagtcca 7020
   agctgaaaag gtcagagact cccagtctac gaaggaaggc ttgcaaacca ttaaggagta 7080
   tgtggataag tttatacaat cactaacaga gaataaggag gagatcatca ggtatggact 7140
   atggggagtt cacacggcac tctacaaaag cttggcagcg agactggggc atgaaacagc 7200
   ttttgcaact ttagtggtaa aatggttggc ttttgggggc gaaacggtat ctgctcacat 7260
   caagcaagta gcagttgatc tagtagtata ttatatcatc aacaaaccat cttttcctgg 7320
   agatacagag acccaacaag aggggaggaa gtttgtggct agtcttttta tatctgcact 7380
   agcaacatac acatataaaa cctggaatta caacaatctg caacgggttg tcgaacctgc 7440
   cttagcttac ctcccatatg ctacaagtgc cttgaagttg ttcacaccca caagattaga 7500
   gagtgtggtc atactcagtt ctacaattta caagacatac ctctctataa ggaagggtaa 7560
   gagtgacggc ttgttaggta caggcataag tgcagccatg gagatcttaa accaaaaccc 7620
   aatctcagta ggtatatctg tgatgctggg ggtaggtgcc atcgccgccc ataatgcaat 7680
   agaatctagt gaacagaaaa gaactttgct gatgaaggtc tttgtaaaaa acttcttaga 7740
   ccaagcagca acagatgagc tagtcaaaga gaaccctgaa aaaataatca tggctctatt 7800
   tgaagcagtc cagaccatag gaaaccccct aagactcatc taccatctgt acggggtgta 7860
   ctataagggg tgggaagcaa aagaactcgc agagaaaact gctggccgca acttattcac 7920
   attgatcatg tttgaggcct ttgagctttt aggtatggac tcagaaggaa agataagaaa 7980
   cttgtcaggc aactacatac tggacttaat cttcaacttg cataataaat taaacaaggg 8040
   gctcaaaaaa ctagtccttg ggtgggctcc tgcacctttg agctgtgatt ggacaccaag 8100
   tgatgagaga ataagcctac ctcataacaa ctacttaagg gtagaaacca ggtgtccttg 8160
   tggctatgag atgaaggcaa taaaaaatgt tgctggtaaa ttgacaaaag ttgaagaaaa 8220
   ggggtccttc ctatgcagga atagattagg gagaggacct ccaaacttca aagtaacaaa 8280
   gttctatgat gataacttga tagaagtcaa gccagtagct aggctagaag gccaggtgga 8340
   cctctattac aagggagtaa cagctaagtt agactacaac aatgggaaag tactgttagc 8400
   taccaacaag tgggaggtgg accacgcttt cctgaccaga ctagtaaaga agcacacagg 8460
   gataggtttt aaaggtgcat atttgggtga ccgaccagac catcaagatc ttgtcgatag 8520
   agattgtgca actataacga agaactcagt acagttccta aaaatgaaga agggttgcgc 8580
   tttcacatat gacctaacaa tctctaacct tgtcaggctt attgaactag tccataagaa 8640
   taatttacaa gaaagagaga tccctaccgt gacagtaact acttggcttg catattcttt 8700
   tgtcaatgaa gacctgggga ctatcaagcc tgtattgggg gagaaagtca tcccagaacc 8760
   ccccgaggag ttgagtctcc aacccaccgt gagactagtc accactgaaa cagcaataac 8820
   cataacaggg gaggctgaag tgatgacgac agggatcaca ccagtggtag agatgaaaga 8880
   agaacctcag ctggaccacc agtcaactac cctaaaggta gggttgaagg aaggggaata 8940
   tccagggcca ggagttaacc ctaaccattt agcagaggtg atagatgaga aagatgacag 9000
   gccttttgtc ctaatcatcg gtaacaaagg ttctacctcg aacagagcaa gaacggccaa 9060
   gaatatacgg ctgtacaaag gaaacaaccc aagagagatc agggatctga tgagccaagg 9120
   aagaatattg acggttgctc taaaagagtt ggacccggaa ttaaaagaat tagtagatta 9180
   caaggggacc tttctcaata gggaagcttt agaagcccta agcttaggta agccaatcaa 9240
   gaggaaaacc acaacagcaa tgatcaggag gttaatagag ccagaggttg aggaggaact 9300
   accagattgg ttccaagcgg aagaacccct atttttggaa gcaaaaatac agaatgactt 9360
   ataccaccta attggcagtg tagatagtat aaaaagcaaa gcaaaggaat taggggccac 9420
   agataacaca aagatagtga aggaagttgg ggctaggacc tatacgatga aattgagcag 9480
   ctggagcaca caagttacaa aaaaacagat gagtctagcc cctctctttg aagagctgtt 9540
   attaaagtgc cctccatgta gtaaaatttc aaagggacat atggtgtcag cataccaact 9600
   ggctcaagga aactgggaac ccctcgggtg tggggtctat atgggaacca taccagctag 9660
   gcgtctcaag atccaccctt atgaggctta ccttaaactc aaagagctgg tggaagttga 9720
   atcttcgagg gccactgcaa aagaatccat cataagagaa cataacacct ggatcctgcg 9780
   gaaggtgaga catgaaggga acctaagaac caaatcaatg atcaaccctg ggaaaatatc 9840
   agatcagcta tgcagagatg gacacaaaag aaacatatat aataagatca taggctcaac 9900
   aatggcctct gctggtatta ggctggagaa actgccagta gtccgagccc aaactgacac 9960
   aaccagtttc caccaagcca taagagaaaa aattgataaa acagaaaaca agcagacccc 10020
   tgaattgcat gaagaactaa tgaaggtctt cgactgctta aagatcccag agctgaagga 10080
   atcgtatgat gaagtttcat gggaacaatt agaagccggg ataaaccgta agggtgcagc 10140
   aggctatcta gagagcaaga acatagggga agtcctagac acagagaaac acatagtaga 10200
   gcagctgatc aaggatctga ggaaggggaa gaagattagg tactatgaaa cagccatccc 10260
   caagaatgag aagagagacg tcagcgacga ctgggaagcc ggagagttcg ttgatgaaaa 10320
   gaaaccaaga gtaatccagt acccggacgc caaggtgaga ctggccatta caaaagtgat 10380
   gtacaaatgg gtaaagcaaa aaccagtggt gatacccggc tatgaaggta aaacacctct 10440
   atttgacata ttcaacaaag tgaagaagga atgggattca ttccaggacc ccgtagcagt 10500
   gagctttgac accaaagcgt gggatacaca agtcaccagt agagacctaa tgttgataaa 10560
   ggatatccag aaatattatt tcaagagaag tatacacaaa tttttagata caataacaga 10620
   acacatggtg gaggtacctg tcattacagc agacggtgaa gtttacataa ggaatggtca 10680
   gaggggtagt ggccaacccg acacaagtgc tggtaatagt atgttgaatg tcctaaccat 10740
   gatatatgct ttctgtaaaa gtacaggcat accttacagg ggattcagca gagtggcaag 10800
   aatccatgtg tgtggtgatg atggcttttt gataacagag agaggactgg gactgaaatt 10860
   ctctgagaag ggtatgcaga tattacatga ggccgggaag ccccagaaaa taactgaagg 10920
   ggacaaaatg aaagtggcat acagattcga ggacatagag ttttgttccc atactcccgt 10980
   gccagtcaga tgggcagata acaccagtag ttacatggca gggaggagca cagccactat 11040
   actagctaag atggcaacca ggctggattc cagcggagag aggggtagca cagcttatga 11100
   gaaggccgta gccttcagct tccttttgat gtactcatgg aatcccgtag ttagaaggat 11160
   ctgcttactg gtgttgtcac agtttccaga aatatcccca tccaaaaaca caatatacta 11220
   ctaccaaggg gatcccatag ctgcgtacag agaagtgata gggaaacagc tgtgtgaact 11280
   gaaaagaaca ggatttgaga agctggctgg tctgaatttg agtatgacca ctctaggcat 11340
   ctggacaaaa catactagta aaagactaat ccaagcctgt gtagaaatag gtaagagaga 11400
   aggtacctgg ttagttaatg ctgacagact gattgcagga aagactggga agttttacat 11460
   cccaagcact ggtgtcactc tgttgggaaa acactatgag gaaattaact taaagcaaaa 11520
   ggcggcacaa ccgccgatag agggggttga cagatataag ttgggcccca tagttaatgt 11580
   tatcttgaga aggctgaggg tgatgctgat gacagttgcc agcggaagct ggtgaatccg 11640
   tccggagcgt cgtgccctca ctcaaggttt ttaattgtaa atattgtaaa tagacagcta 11700
   agatatttat tgtagttgga tagtaatgca gtgatagtaa ataccccaat ttaacactac 11760
   ctccaatgca ctaagcactt tagctgtgtg aggttaactc gacgtccacg gttggactag 11820
   ggaagacctc taacagcccc 11840
<210> 3
   <211> 12329
   <212> DNA
   <213> Artificial Sequence: Mutated BVDV: XIKE-B
<400> 3
   gtatacgaga ttagctaaag aactcgtata tggattggac gtcaacaaat ttttaattgg 60
   caacgtaggg aaccttcccc tcagcgaagg ccgaaaagag gctagccatg cccttagtag 120
   gactagcaaa agtaggggac tagcggtagc agtgagttcg ttggatggcc gaacccctga 180
   gtacagggga gtcgtcaatg gttcgacact ccattagtcg aggagtctcg agatgccatg 240
   tggacgaggg catgcccacg gcacatctta acccatgcgg gggttgcatg ggtgaaagcg 300
   ctattcgtgg cgttatggac acagcctgat agggtgtagc agagacctgc tattccgcta 360
   gtaaaaactc tgctgtacat ggcacatgga gttgttttca aatgaacttt tatacaaaac 420
   atataaacaa aaaccagcag gcgtcgtgga acctgtttac gacgtcaacg ggcgcccact 480
   gtttggagag agcagtgact tgcacccgca gtcaacacta aaactaccac accaacgagg 540
   cagcgccaac atcctgacca atgctaggtc cctaccgcgg aaaggtgact gccggagagg 600
   taatgtgtat ggaccggtga gtggcatcta tatcaaacca ggaccgatct actaccagga 660
   ttatgtgggc cccgtctatc atagagcccc actggaacta tgtagggagg caagtatgtg 720
   cgaaacaact aggagagttg gcagagtgac cggtagtgat gggaaattat atcatatcta 780
   catctgcata gatgggtgta tcctcctgaa gagggcgact aggaaccaac cagaagtcct 840
   gaaatgggta tacaacagat taaattgtcc tttatgggtc accagctgct ccgatgaagg 900
   gagcaagggt gctacaagta agaagcagcc taagccagat aggatagaaa aaggtaagat 960
   gaaaatagcc ccaaaagaga cagaaaaaga ttgcaaaacc agaccccccg acgcgactat 1020
   agtagtagaa ggggttaagt accaggtgaa gaaaaaagga aaggtaaggg gaaaaaatac 1080
   tcaagatggg ttatatcaca acaagaataa gccccctgaa tcaagaaaaa aattggaaaa 1140
   ggcactgctg gcttgggcca tcttagcagc ggtcctgctt cagctggtaa caggagagaa 1200
   tatcacccag tggaacttga tggacaacgg caccgaggga atacagcaag cgatgttcct 1260
   aagaggggtg aacaggagtc tacatggaat ttggccagag aaaatttgca ccggagtacc 1320
   aactcactta gcaacagact atgagcttaa agagatagtg gggatgatgg acgcgagtga 1380
   gaagaccaac tacacgtgtt gcaggttgca aagacatgag tggaataaag gttggtgtaa 1440
   ctggtttcat atagaaccgt ggatatggtt gatgaacaaa acccaaaaca acctgacaga 1500
   agggcaaccg cttagggagt gtgctgtgac ttgtaggtat gacaaggaaa cagaattgaa 1560
   catcgtgaca caggctaggg acagacctac aactctgaca ggttgcaaga aaggcaagaa 1620
   tttctctttc gcaggtgtta tactggatgg gccctgtaac tttaaagtat cggttgaaga 1680
   tgtgctgttc aaggagcacg attgcggcaa catgctgcaa gagaccgcga tacagctact 1740
   cgatggggca accaacacca ttgagggagc aagggtaggg acggccaagt tgacaacctg 1800
   gttagggaag caattaggga tccttggtaa gaagttggag aacaaaagca aagcatggtt 1860
   tggtgcacat gcagcaagtc catactgcgg agtggagagg aagatcggtt acgtatggta 1920
   tacaaaaaac tgcactccag cttgccttcc aagaaacact agaataatag gccccgggaa 1980
   atttgatacc aacgccgaag atggaaaaat actccatgag atgggggggc acctctcaga 2040
   atttgtccta ttgtccttgg tggttctgtc tgactttgcc ccggaaaccg cgagcgtcat 2100
   ctacttggtt ctacattttg cgatcccgca aagccacgtt gatgtagaca catgcgacaa 2160
   gaaccagctg aatttaacgg tagcaaccac agtagcagag gtcataccag ggacagtgtg 2220
   gaacctaggg aagtatgtct gcataagacc agactggtgg ccatatgaga cgacgacagt 2280
   cttcgtcata gaggaagcag ggcaagtaat caaattgatg ctaagggcca tcagagactt 2340
   aactaggata tggaatgctg ccactaccac agctttctta atctttttag taaaagcact 2400
   gaggggacaa ctaatccaag ggctattgtg gctgatgcta ataacaggag cacagggctt 2460
   ccctgaatgc aaagagggct tccaatatgc catatctaaa gacaggaaaa tggggttatt 2520
   ggggccagag agcttaacta caacatggca cctccccacc aaaaaaatag tggattccat 2580
   ggtgcatgta tggtgtgaag gaaaagactt gaaaatatta aaaatgtgca caaaggaaga 2640
   gaggtatcta gtggctgtgc acgagagagc cttatcaacc agtgccgagt ttatgcagat 2700
   cagtgatggg acaataggcc cagacgtgat agatatgcct gatgactttg agtttggact 2760
   ctgcccttgt gactcaaaac cagtgataaa gggcaaattt aatgccagct tactgaatgg 2820
   accagctttc cagatggtat gcccacaggg gtggactggt acaatagaat gcaccctagc 2880
   gaaccaagac accttggaca caactgtcat taggacatat agaagaacta ccccatttca 2940
   gcggagaaaa tggtgtacct atgaaaaaat aataggggaa gatatctatg aatgcattct 3000
   aggtggaaac tggacatgca taaccggtga ccatagcagg ttgaaagacg gacctatcaa 3060
   gaagtgtaag tggtgtggcc atgacttcgt caactcagag gggctaccac actacccaat 3120
   aggcaagtgc atgctcatca acgagagtgg gtacaggtat gtagatgaca cctcttgcga 3180
   taggggtggt gtagccatag ttccatctgg caccgtaaag tgtagaatag gtaacgtcac 3240
   ggtgcaagtt atcgctacta acaatgatct gggacccatg ccttgcagcc cagctgaagt 3300
   gatagcaagt gaaggaccag tggaaaagac tgcatgcaca ttcaactatt caaggactct 3360
   acctaataag tattatgagc caagggaccg gtacttccaa caatacatgt taaaagggga 3420
   gtggcaatat tggttcgacc tggattctgt agaccaccac aaagactact tctcagagtt 3480
   cataatcata gcagtggtcg ccttgttggg tggtaagtac gtactgtggc tcttgataac 3540
   atacacaata ctgtctgagc agatggctat gggtgctgga gtgaatactg aagagatagt 3600
   catgataggc aatttgctga cagacagtga tattgaggtt gtggtttatt tccttcttct 3660
   gtacttaata gttaaagagg aactggcgag gaaatggatt atactggtat accacatcct 3720
   tgtagccaac cctatgaaaa caattggggt cgtcttacta atgctagggg gagtggtgaa 3780
   ggccagcaga atcaatgctg atgaccaaag tgctatggac ccatgctttc ttctcgtgac 3840
   aggcgtagtg gctgttttga tgatcgctag aagagaacct gccacattac cactgattgt 3900
   agcattgcta gcaataagaa catcaggatt cctactgccc gctagcattg atgtaactgt 3960
   agcagtagta ttaattgtac ttttgttggc tagctacata acagactact ttagatataa 4020
   aaagtggctt caactcttat ttagtctgat agctggtatc tttattataa ggagcttaaa 4080
   acatatcaac cagatggagg taccagaaat atctatgcca agttggagac ctctagctct 4140
   ggtccttttc tatataacat ctacagcaat aaccactaat tgggacattg acttagcagg 4200
   cttcctgctg caatgggcgc cagcagtgat catgatggct accatgtggg cagacttttt 4260
   gactctgatc atagtcctgc ccagttacga gttatctaag ctttacttcc taaagaacgt 4320
   caggacagac gtggaaaaga actggctcgg caaagtgaaa tacagacaga tcagttcagt 4380
   ttatgacatc tgtgacagtg aggaagcagt gtacctattt ccatcaaggc ataagagtgg 4440
   aagcaggcca gatttcatat tacctttttt gaaagccgtg ttaataagct gcatcagcag 4500
   ccaatggcaa gtggtttaca tttcttacct aatactggaa attacatact atatgcacag 4560
   gaaaatcata gatgaggtgt caggaggagc aaattttcta tcaagactca tagcagccat 4620
   catagaatta aattgggcca tagatgatga ggaatgtaaa ggactgaaga aactgtatct 4680
   cttgtcaggg agagcgaaga atttgatagt taaacataag gtaagaaatg aagccgtcca 4740
   cagatggttt ggtgaggagg aaatatacgg ggcacccaag gtgatcacta tcataaaagc 4800
   tagtacccta agtaaaaaca ggcactgcat aatctgcacg atctgtgaag ggaaagaatg 4860
   gaatggagcc aactgcccaa agtgtggaag acaaggaaag cccataacat gtggaatgac 4920
   actcgcagac tttgaggaga aacattacaa aaagatattt ataagagaag aatcttcttg 4980
   tcctgtgcct tttgatcctt cttgccattg taattatttt cgccacgatg ggcctttcag 5040
   gaaagagtat aagggttacg tccaatacac agccagagga caactctttc tgaggaacct 5100
   accaattcta gcgacgaaga tgaagctatt aatggtggga aacctcggcg cagaaattgg 5160
   cgacctggaa catctaggat gggtactgag agggccagcc gtgtgcaaaa aaattaccaa 5220
   ccatgagaag tgccacgtaa acatcatgga taagctaact gcattttttg gaatcatgcc 5280
   tagaggcacg acccctaggg cacctgtgag gttccccaca gcactactaa aagtgagaag 5340
   ggggctagag acgggatggg cttacacgca ccaaggaggg atcagctcgg tagaccatgt 5400
   cacagccgga aaggatttac tagtgtgtga cagtatgggc aggaccaggg ttgtctgtca 5460
   tagtaacaat aagatgactg atgagactga gtatggcatc aagaccgact cagggtgtcc 5520
   cgaaggtgcg aggtgttacg tgctaaaccc agaagctgtt aacatttctg gcacaaaagg 5580
   agctatggta cacctccaga aaacgggggg ggagttcaca tgtgtcactg cctcagggac 5640
   cccggctttc ttcgatctga aaaatctaaa aggctggtcc gggctaccaa tttttgaagc 5700
   atccagtggc agggtggttg gtagggtgaa agtcggcaag aatgaggatt ccaagcccac 5760
   caaactaatg agcggaatcc agacagtgtc taagaaccag acagacctag cggacatcgt 5820
   aaaaaaattg actagtatga acagaggaga gttcaaacag ataacattag ccactggggc 5880
   aggaaaaact acggaactgc caaggtccgt catagaggag atagggaggc acaaaagggt 5940
   cttagtcctg ataccattga gagcagcagc agagtcagtg tatcagtata tgagagtgaa 6000
   gtacccaagt atatctttca atttgagaat aggagatatg aaggaaggtg acatggccac 6060
   tggtatcacc tacgcctcat atgggtactt ttgtcagctt cctcagccca aactgagagc 6120
   tgccatggta gagtactcat atatattctt agatgagtac cactgtgcta cacccgagca 6180
   attagcaata attggaaaga tacacaggtt tgctgaaaat cttagagtgg tagcaatgac 6240
   agcaacccca gctggaacgg tcacaacgac tggtcagaaa caccctatag aggagttcat 6300
   agccccagag gtgatgaaag gtgaagatct aggtagtgaa tacttggata ttgcagggtt 6360
   gaagataccg actgaagaga tgaaaggcaa catgctcgtg ttcgcgccaa ctaggaacat 6420
   ggcagtagaa acagctaaga aattgaaggc taagggatac aactctggat actattacag 6480
   tggggaaaac ccagagaact tgagggtggt aacctcgcaa tccccgtatg tggtagtagc 6540
   caccaatgcc atagagtcag gtgtgacatt accagactta gacacagttg tagacactgg 6600
   actaaagtgt gagaagaggg tgaggatttc ttcaaaaatg cccttcattg taacaggact 6660
   taagagaatg gcagtcacaa tcggagagca agcccagcgc aggggtagag taggaagagt 6720
   caagccaggt aggtactata ggagtcaaga aacagcttca gggtcaaaag attaccatta 6780
   cgacctactg caagcccaga ggtacggaat agaagatgga attaatgtaa caaagtcatt 6840
   cagggagatg aactatgatt ggagccttta cgaagaggac agcttgatga taactcaact 6900
   cgaggtcctt aacaacctcc ttatatcaga agacctgcct gccgcagtga agaacatcat 6960
   ggcccggacc gatcacccag aacccataca actggcctat aacagttatg aaaaccaaat 7020
   tccagtgctg ttcccaaaga tcaaaaatgg tgaggtgaca gacagttatg agaattacac 7080
   atatctcaat gcaagaaaat taggagagga cgtgccggca tatgtgtacg ccacagagga 7140
   tgaggatcta gcagtggatc ttctgggtat ggattggccg gacccaggca accaacaggt 7200
   ggtagagaca gggagggcat taaaacaagt aactggctta tccacagcag aaaacgccct 7260
   cttgatagcc ctattcggct acgtcgggta ccagacactt tcaaaaaggc acatacccat 7320
   gattactgac atctatacac ttgaagacca caggcttgag gacacaaccc acctccagtt 7380
   tgccccaaac gctataagga ccgacggcaa ggactcagag ttgaaggaat tagctgtggg 7440
   agaccttgat aaatatgtgg acgcactggt agactactcc aaacaaggga tgaaattcat 7500
   caaagtccaa gctgaaaagg tcagagactc ccagtctacg aaggaaggct tgcaaaccat 7560
   taaggagtat gtggataagt ttatacaatc actaacagag aataaggagg agatcatcag 7620
   gtatggacta tggggagttc acacggcact ctacaaaagc ttggcagcga gactggggca 7680
   tgaaacagct tttgcaactt tagtggtaaa atggttggct tttgggggcg aaacggtatc 7740
   tgctcacatc aagcaagtag cagttgatct agtagtatat tatatcatca acaaaccatc 7800
   ttttcctgga gatacagaga cccaacaaga ggggaggaag tttgtggcta gtctttttat 7860
   atctgcacta gcaacataca catataaaac ctggaattac aacaatctgc aacgggttgt 7920
   cgaacctgcc ttagcttacc tcccatatgc tacaagtgcc ttgaagttgt tcacacccac 7980
   aagattagag agtgtggtca tactcagttc tacaatttac aagacatacc tctctataag 8040
   gaagggtaag agtgacggct tgttaggtac aggcataagt gcagccatgg agatcttaaa 8100
   ccaaaaccca atctcagtag gtatatctgt gatgctgggg gtaggtgcca tcgccgccca 8160
   taatgcaata gaatctagtg aacagaaaag aactttgctg atgaaggtct ttgtaaaaaa 8220
   cttcttagac caagcagcaa cagatgagct agtcaaagag aaccctgaaa aaataatcat 8280
   ggctctattt gaagcagtcc agaccatagg aaacccccta agactcatct accatctgta 8340
   cggggtgtac tataaggggt gggaagcaaa agaactcgca gagaaaactg ctggccgcaa 8400
   cttattcaca ttgatcatgt ttgaggcctt tgagctttta ggtatggact cagaaggaaa 8460
   gataagaaac ttgtcaggca actacatact ggacttaatc ttcaacttgc ataataaatt 8520
   aaacaagggg ctcaaaaaac tagtccttgg gtgggctcct gcacctttga gctgtgattg 8580
   gacaccaagt gatgagagaa taagcctacc tcataacaac tacttaaggg tagaaaccag 8640
   gtgtccttgt ggctatgaga tgaaggcaat aaaaaatgtt gctggtaaat tgacaaaagt 8700
   tgaagaaaag gggtccttcc tatgcaggaa tagattaggg agaggacctc caaacttcaa 8760
   agtaacaaag ttctatgatg ataacttgat agaagtcaag ccagtagcta ggctagaagg 8820
   ccaggtggac ctctattaca agggagtaac agctaagtta gactacaaca atgggaaagt 8880
   actgttagct accaacaagt gggaggtgga ccacgctttc ctgaccagac tagtaaagaa 8940
   gcacacaggg ataggtttta aaggtgcata tttgggtgac cgaccagacc atcaagatct 9000
   tgtcgataga gattgtgcaa ctataacgaa gaactcagta cagttcctaa aaatgaagaa 9060
   gggttgcgct ttcacatatg acctaacaat ctctaacctt gtcaggctta ttgaactagt 9120
   ccataagaat aatttacaag aaagagagat ccctaccgtg acagtaacta cttggcttgc 9180
   atattctttt gtcaatgaag acctggggac tatcaagcct gtattggggg agaaagtcat 9240
   cccagaaccc cccgaggagt tgagtctcca acccaccgtg agactagtca ccactgaaac 9300
   agcaataacc ataacagggg aggctgaagt gatgacgaca gggatcacac cagtggtaga 9360
   gatgaaagaa gaacctcagc tggaccacca gtcaactacc ctaaaggtag ggttgaagga 9420
   aggggaatat ccagggccag gagttaaccc taaccattta gcagaggtga tagatgagaa 9480
   agatgacagg ccttttgtcc taatcatcgg taacaaaggt tctacctcga acagagcaag 9540
   aacggccaag aatatacggc tgtacaaagg aaacaaccca agagagatca gggatctgat 9600
   gagccaagga agaatattga cggttgctct aaaagagttg gacccggaat taaaagaatt 9660
   agtagattac aaggggacct ttctcaatag ggaagcttta gaagccctaa gcttaggtaa 9720
   gccaatcaag aggaaaacca caacagcaat gatcaggagg ttaatagagc cagaggttga 9780
   ggaggaacta ccagattggt tccaagcgga agaaccccta tttttggaag caaaaataca 9840
   gaatgactta taccacctaa ttggcagtgt agatagtata aaaagcaaag caaaggaatt 9900
   aggggccaca gataacacaa agatagtgaa ggaagttggg gctaggacct atacgatgaa 9960
   attgagcagc tggagcacac aagttacaaa aaaacagatg agtctagccc ctctctttga 10020
   agagctgtta ttaaagtgcc ctccatgtag taaaatttca aagggacata tggtgtcagc 10080
   ataccaactg gctcaaggaa actgggaacc cctcgggtgt ggggtctata tgggaaccat 10140
   accagctagg cgtctcaaga tccaccctta tgaggcttac cttaaactca aagagctggt 10200
   ggaagttgaa tcttcgaggg ccactgcaaa agaatccatc ataagagaac ataacacctg 10260
   gatcctgcgg aaggtgagac atgaagggaa cctaagaacc aaatcaatga tcaaccctgg 10320
   gaaaatatca gatcagctat gcagagatgg acacaaaaga aacatatata ataagatcat 10380
   aggctcaaca atggcctctg ctggtattag gctggagaaa ctgccagtag tccgagccca 10440
   aactgacaca accagtttcc accaagccat aagagaaaaa attgataaaa cagaaaacaa 10500
   gcagacccct gaattgcatg aagaactaat gaaggtcttc gactgcttaa agatcccaga 10560
   gctgaaggaa tcgtatgatg aagtttcatg ggaacaatta gaagccggga taaaccgtaa 10620
   gggtgcagca ggctatctag agagcaagaa cataggggaa gtcctagaca cagagaaaca 10680
   catagtagag cagctgatca aggatctgag gaaggggaag aagattaggt actatgaaac 10740
   agccatcccc aagaatgaga agagagacgt cagcgacgac tgggaagccg gagagttcgt  10800
   tgatgaaaag aaaccaagag taatccagta cccggacgcc aaggtgagac tggccattac  10860
   aaaagtgatg tacaaatggg taaagcaaaa accagtggtg atacccggct atgaaggtaa  10920
   aacacctcta tttgacatat tcaacaaagt gaagaaggaa tgggattcat tccaggaccc  10980
   cgtagcagtg agctttgaca ccaaagcgtg ggatacacaa gtcaccagta gagacctaat  11040
   gttgataaag gatatccaga aatattattt caagagaagt atacacaaat ttttagatac  11100
   aataacagaa cacatggtgg aggtacctgt cattacagca gacggtgaag tttacataag  11160
   gaatggtcag aggggtagtg gccaacccga cacaagtgct ggtaatagta tgttgaatgt  11220
   cctaaccatg atatatgctt tctgtaaaag tacaggcata ccttacaggg gattcagcag  11280
   agtggcaaga atccatgtgt gtggtgatga tggctttttg ataacagaga gaggactggg  11340
   actgaaattc tctgagaagg gtatgcagat attacatgag gccgggaagc cccagaaaat  11400
   aactgaaggg gacaaaatga aagtggcata cagattcgag gacatagagt tttgttccca  11460
   tactcccgtg ccagtcagat gggcagataa caccagtagt tacatggcag ggaggagcac  11520
   agccactata ctagctaaga tggcaaccag gctggattcc agcggagaga ggggtagcac  11580
   agcttatgag aaggccgtag ccttcagctt ccttttgatg tactcatgga atcccgtagt  11640
   tagaaggatc tgcttactgg tgttgtcaca gtttccagaa atatccccat ccaaaaacac  11700
   aatatactac taccaagggg atcccatagc tgcgtacaga gaagtgatag ggaaacagct  11760
   gtgtgaactg aaaagaacag gatttgagaa gctggctggt ctgaatttga gtatgaccac  11820
   tctaggcatc tggacaaaac atactagtaa aagactaatc caagcctgtg tagaaatagg  11880
   taagagagaa ggtacctggt tagttaatgc tgacagactg attgcaggaa agactgggaa  11940
   gttttacatc ccaagcactg gtgtcactct gttgggaaaa cactatgagg aaattaactt  12000
   aaagcaaaag gcggcacaac cgccgataga gggggttgac agatataagt tgggccccat  12060
   agttaatgtt atcttgagaa ggctgagggt gatgctgatg acagttgcca gcggaagctg  12120
   gtgaatccgt ccggagcgtc gtgccctcac tcaaggtttt taattgtaaa tattgtaaat  12180
   agacagctaa gatatttatt gtagttggat agtaatgcag tgatagtaaa taccccaatt  12240
   taacactacc tccaatgcac taagcacttt agctgtgtga ggttaactcg acgtccacgg  12300
   ttggactagg gaagacctct aacagcccc                                     12329
<210> 4
   <211> 11837
   <212> DNA
   <213> Artificial Sequence: Mutated BVDV: XIKE-B-NdN
<400> 4
   gtatacgaga ttagctaaag aactcgtata tggattggac gtcaacaaat ttttaattgg 60
   caacgtaggg aaccttcccc tcagcgaagg ccgaaaagag gctagccatg cccttagtag 120
   gactagcaaa agtaggggac tagcggtagc agtgagttcg ttggatggcc gaacccctga 180
   gtacagggga gtcgtcaatg gttcgacact ccattagtcg aggagtctcg agatgccatg 240
   tggacgaggg catgcccacg gcacatctta acccatgcgg gggttgcatg ggtgaaagcg 300
   ctattcgtgg cgttatggac acagcctgat agggtgtagc agagacctgc tattccgcta 360
   gtaaaaactc tgctgtacat ggcacatgga gttgttttcc gatgaaggga gcaagggtgc 420
   tacaagtaag aagcagccta agccagatag gatagaaaaa ggtaagatga aaatagcccc 480
   aaaagagaca gaaaaagatt gcaaaaccag accccccgac gcgactatag tagtagaagg 540
   ggttaagtac caggtgaaga aaaaaggaaa ggtaagggga aaaaatactc aagatgggtt 600
   atatcacaac aagaataagc cccctgaatc aagaaaaaaa ttggaaaagg cactgctggc 660
   ttgggccatc ttagcagcgg tcctgcttca gctggtaaca ggagagaata tcacccagtg 720
   gaacttgatg gacaacggca ccgagggaat acagcaagcg atgttcctaa gaggggtgaa 780
   caggagtcta catggaattt ggccagagaa aatttgcacc ggagtaccaa ctcacttagc 840
   aacagactat gagcttaaag agatagtggg gatgatggac gcgagtgaga agaccaacta 900
   cacgtgttgc aggttgcaaa gacatgagtg gaataaaggt tggtgtaact ggtttcatat 960
   agaaccgtgg atatggttga tgaacaaaac ccaaaacaac ctgacagaag ggcaaccgct 1020
   tagggagtgt gctgtgactt gtaggtatga caaggaaaca gaattgaaca tcgtgacaca 1080
   ggctagggac agacctacaa ctctgacagg ttgcaagaaa ggcaagaatt tctctttcgc 1140
   aggtgttata ctggatgggc cctgtaactt taaagtatcg gttgaagatg tgctgttcaa 1200
   ggagcacgat tgcggcaaca tgctgcaaga gaccgcgata cagctactcg atggggcaac 1260
   caacaccatt gagggagcaa gggtagggac ggccaagttg acaacctggt tagggaagca 1320
   attagggatc cttggtaaga agttggagaa caaaagcaaa gcatggtttg gtgcacatgc 1380
   agcaagtcca tactgcggag tggagaggaa gatcggttac gtatggtata caaaaaactg 1440
   cactccagct tgccttccaa gaaacactag aataataggc cccgggaaat ttgataccaa 1500
   cgccgaagat ggaaaaatac tccatgagat gggggggcac ctctcagaat ttgtcctatt 1560
   gtccttggtg gttctgtctg actttgcccc ggaaaccgcg agcgtcatct acttggttct 1620
   acattttgcg atcccgcaaa gccacgttga tgtagacaca tgcgacaaga accagctgaa 1680
   tttaacggta gcaaccacag tagcagaggt cataccaggg acagtgtgga acctagggaa 1740
   gtatgtctgc ataagaccag actggtggcc atatgagacg acgacagtct tcgtcataga 1800
   ggaagcaggg caagtaatca aattgatgct aagggccatc agagacttaa ctaggatatg 1860
   gaatgctgcc actaccacag ctttcttaat ctttttagta aaagcactga ggggacaact 1920
   aatccaaggg ctattgtggc tgatgctaat aacaggagca cagggcttcc ctgaatgcaa 1980
   agagggcttc caatatgcca tatctaaaga caggaaaatg gggttattgg ggccagagag 2040
   cttaactaca acatggcacc tccccaccaa aaaaatagtg gattccatgg tgcatgtatg 2100
   gtgtgaagga aaagacttga aaatattaaa aatgtgcaca aaggaagaga ggtatctagt 2160
   ggctgtgcac gagagagcct tatcaaccag tgccgagttt atgcagatca gtgatgggac 2220
   aataggccca gacgtgatag atatgcctga tgactttgag tttggactct gcccttgtga 2280
   ctcaaaacca gtgataaagg gcaaatttaa tgccagctta ctgaatggac cagctttcca 2340
   gatggtatgc ccacaggggt ggactggtac aatagaatgc accctagcga accaagacac 2400
   cttggacaca actgtcatta ggacatatag aagaactacc ccatttcagc ggagaaaatg 2460
   gtgtacctat gaaaaaataa taggggaaga tatctatgaa tgcattctag gtggaaactg 2520
   gacatgcata accggtgacc atagcaggtt gaaagacgga cctatcaaga agtgtaagtg 2580
   gtgtggccat gacttcgtca actcagaggg gctaccacac tacccaatag gcaagtgcat 2640
   gctcatcaac gagagtgggt acaggtatgt agatgacacc tcttgcgata ggggtggtgt 2700
   agccatagtt ccatctggca ccgtaaagtg tagaataggt aacgtcacgg tgcaagttat 2760
   cgctactaac aatgatctgg gacccatgcc ttgcagccca gctgaagtga tagcaagtga 2820
   aggaccagtg gaaaagactg catgcacatt caactattca aggactctac ctaataagta 2880
   ttatgagcca agggaccggt acttccaaca atacatgtta aaaggggagt ggcaatattg 2940
   gttcgacctg gattctgtag accaccacaa agactacttc tcagagttca taatcatagc 3000
   agtggtcgcc ttgttgggtg gtaagtacgt actgtggctc ttgataacat acacaatact 3060
   gtctgagcag atggctatgg gtgctggagt gaatactgaa gagatagtca tgataggcaa 3120
   tttgctgaca gacagtgata ttgaggttgt ggtttatttc cttcttctgt acttaatagt 3180
   taaagaggaa ctggcgagga aatggattat actggtatac cacatccttg tagccaaccc 3240
   tatgaaaaca attggggtcg tcttactaat gctaggggga gtggtgaagg ccagcagaat 3300
   caatgctgat gaccaaagtg ctatggaccc atgctttctt ctcgtgacag gcgtagtggc 3360
   tgttttgatg atcgctagaa gagaacctgc cacattacca ctgattgtag cattgctagc 3420
   aataagaaca tcaggattcc tactgcccgc tagcattgat gtaactgtag cagtagtatt 3480
   aattgtactt ttgttggcta gctacataac agactacttt agatataaaa agtggcttca 3540
   actcttattt agtctgatag ctggtatctt tattataagg agcttaaaac atatcaacca 3600
   gatggaggta ccagaaatat ctatgccaag ttggagacct ctagctctgg tccttttcta 3660
   tataacatct acagcaataa ccactaattg ggacattgac ttagcaggct tcctgctgca 3720
   atgggcgcca gcagtgatca tgatggctac catgtgggca gactttttga ctctgatcat 3780
   agtcctgccc agttacgagt tatctaagct ttacttccta aagaacgtca ggacagacgt 3840
   ggaaaagaac tggctcggca aagtgaaata cagacagatc agttcagttt atgacatctg 3900
   tgacagtgag gaagcagtgt acctatttcc atcaaggcat aagagtggaa gcaggccaga 3960
   tttcatatta ccttttttga aagccgtgtt aataagctgc atcagcagcc aatggcaagt 4020
   ggtttacatt tcttacctaa tactggaaat tacatactat atgcacagga aaatcataga 4080
   tgaggtgtca ggaggagcaa attttctatc aagactcata gcagccatca tagaattaaa 4140
   ttgggccata gatgatgagg aatgtaaagg actgaagaaa ctgtatctct tgtcagggag 4200
   agcgaagaat ttgatagtta aacataaggt aagaaatgaa gccgtccaca gatggtttgg 4260
   tgaggaggaa atatacgggg cacccaaggt gatcactatc ataaaagcta gtaccctaag 4320
   taaaaacagg cactgcataa tctgcacgat ctgtgaaggg aaagaatgga atggagccaa 4380
   ctgcccaaag tgtggaagac aaggaaagcc cataacatgt ggaatgacac tcgcagactt 4440
   tgaggagaaa cattacaaaa agatatttat aagagaagaa tcttcttgtc ctgtgccttt 4500
   tgatccttct tgccattgta attattttcg ccacgatggg cctttcagga aagagtataa 4560
   gggttacgtc caatacacag ccagaggaca actctttctg aggaacctac caattctagc 4620
   gacgaagatg aagctattaa tggtgggaaa cctcggcgca gaaattggcg acctggaaca 4680
   tctaggatgg gtactgagag ggccagccgt gtgcaaaaaa attaccaacc atgagaagtg 4740
   ccacgtaaac atcatggata agctaactgc attttttgga atcatgccta gaggcacgac 4800
   ccctagggca cctgtgaggt tccccacagc actactaaaa gtgagaaggg ggctagagac 4860
   gggatgggct tacacgcacc aaggagggat cagctcggta gaccatgtca cagccggaaa 4920
   ggatttacta gtgtgtgaca gtatgggcag gaccagggtt gtctgtcata gtaacaataa 4980
   gatgactgat gagactgagt atggcatcaa gaccgactca gggtgtcccg aaggtgcgag 5040
   gtgttacgtg ctaaacccag aagctgttaa catttctggc acaaaaggag ctatggtaca 5100
   cctccagaaa acgggggggg agttcacatg tgtcactgcc tcagggaccc cggctttctt 5160
   cgatctgaaa aatctaaaag gctggtccgg gctaccaatt tttgaagcat ccagtggcag 5220
   ggtggttggt agggtgaaag tcggcaagaa tgaggattcc aagcccacca aactaatgag 5280
   cggaatccag acagtgtcta agaaccagac agacctagcg gacatcgtaa aaaaattgac 5340
   tagtatgaac agaggagagt tcaaacagat aacattagcc actggggcag gaaaaactac 5400
   ggaactgcca aggtccgtca tagaggagat agggaggcac aaaagggtct tagtcctgat 5460
   accattgaga gcagcagcag agtcagtgta tcagtatatg agagtgaagt acccaagtat 5520
   atctttcaat ttgagaatag gagatatgaa ggaaggtgac atggccactg gtatcaccta 5580
   cgcctcatat gggtactttt gtcagcttcc tcagcccaaa ctgagagctg ccatggtaga 5640
   gtactcatat atattcttag atgagtacca ctgtgctaca cccgagcaat tagcaataat 5700
   tggaaagata cacaggtttg ctgaaaatct tagagtggta gcaatgacag caaccccagc 5760
   tggaacggtc acaacgactg gtcagaaaca ccctatagag gagttcatag ccccagaggt 5820
   gatgaaaggt gaagatctag gtagtgaata cttggatatt gcagggttga agataccgac 5880
   tgaagagatg aaaggcaaca tgctcgtgtt cgcgccaact aggaacatgg cagtagaaac 5940
   agctaagaaa ttgaaggcta agggatacaa ctctggatac tattacagtg gggaaaaccc 6000
   agagaacttg agggtggtaa cctcgcaatc cccgtatgtg gtagtagcca ccaatgccat 6060
   agagtcaggt gtgacattac cagacttaga cacagttgta gacactggac taaagtgtga 6120
   gaagagggtg aggatttctt caaaaatgcc cttcattgta acaggactta agagaatggc 6180
   agtcacaatc ggagagcaag cccagcgcag gggtagagta ggaagagtca agccaggtag 6240
   gtactatagg agtcaagaaa cagcttcagg gtcaaaagat taccattacg acctactgca 6300
   agcccagagg tacggaatag aagatggaat taatgtaaca aagtcattca gggagatgaa 6360
   ctatgattgg agcctttacg aagaggacag cttgatgata actcaactcg aggtccttaa 6420
   caacctcctt atatcagaag acctgcctgc cgcagtgaag aacatcatgg cccggaccga 6480
   tcacccagaa cccatacaac tggcctataa cagttatgaa aaccaaattc cagtgctgtt 6540
   cccaaagatc aaaaatggtg aggtgacaga cagttatgag aattacacat atctcaatgc 6600
   aagaaaatta ggagaggacg tgccggcata tgtgtacgcc acagaggatg aggatctagc 6660
   agtggatctt ctgggtatgg attggccgga cccaggcaac caacaggtgg tagagacagg 6720
   gagggcatta aaacaagtaa ctggcttatc cacagcagaa aacgccctct tgatagccct 6780
   attcggctac gtcgggtacc agacactttc aaaaaggcac atacccatga ttactgacat 6840
   ctatacactt gaagaccaca ggcttgagga cacaacccac ctccagtttg ccccaaacgc 6900
   tataaggacc gacggcaagg actcagagtt gaaggaatta gctgtgggag accttgataa 6960
   atatgtggac gcactggtag actactccaa acaagggatg aaattcatca aagtccaagc 7020
   tgaaaaggtc agagactccc agtctacgaa ggaaggcttg caaaccatta aggagtatgt 7080
   ggataagttt atacaatcac taacagagaa taaggaggag atcatcaggt atggactatg 7140
   gggagttcac acggcactct acaaaagctt ggcagcgaga ctggggcatg aaacagcttt 7200
   tgcaacttta gtggtaaaat ggttggcttt tgggggcgaa acggtatctg ctcacatcaa 7260
   gcaagtagca gttgatctag tagtatatta tatcatcaac aaaccatctt ttcctggaga 7320
   tacagagacc caacaagagg ggaggaagtt tgtggctagt ctttttatat ctgcactagc 7380
   aacatacaca tataaaacct ggaattacaa caatctgcaa cgggttgtcg aacctgcctt 7440
   agcttacctc ccatatgcta caagtgcctt gaagttgttc acacccacaa gattagagag 7500
   tgtggtcata ctcagttcta caatttacaa gacatacctc tctataagga agggtaagag 7560
   tgacggcttg ttaggtacag gcataagtgc agccatggag atcttaaacc aaaacccaat 7620
   ctcagtaggt atatctgtga tgctgggggt aggtgccatc gccgcccata atgcaataga 7680
   atctagtgaa cagaaaagaa ctttgctgat gaaggtcttt gtaaaaaact tcttagacca 7740
   agcagcaaca gatgagctag tcaaagagaa ccctgaaaaa ataatcatgg ctctatttga 7800
   agcagtccag accataggaa accccctaag actcatctac catctgtacg gggtgtacta 7860
   taaggggtgg gaagcaaaag aactcgcaga gaaaactgct ggccgcaact tattcacatt 7920
   gatcatgttt gaggcctttg agcttttagg tatggactca gaaggaaaga taagaaactt 7980
   gtcaggcaac tacatactgg acttaatctt caacttgcat aataaattaa acaaggggct 8040
   caaaaaacta gtccttgggt gggctcctgc acctttgagc tgtgattgga caccaagtga 8100
   tgagagaata agcctacctc ataacaacta cttaagggta gaaaccaggt gtccttgtgg 8160
   ctatgagatg aaggcaataa aaaatgttgc tggtaaattg acaaaagttg aagaaaaggg 8220
   gtccttccta tgcaggaata gattagggag aggacctcca aacttcaaag taacaaagtt 8280
   ctatgatgat aacttgatag aagtcaagcc agtagctagg ctagaaggcc aggtggacct 8340
   ctattacaag ggagtaacag ctaagttaga ctacaacaat gggaaagtac tgttagctac 8400
   caacaagtgg gaggtggacc acgctttcct gaccagacta gtaaagaagc acacagggat 8460
   aggttttaaa ggtgcatatt tgggtgaccg accagaccat caagatcttg tcgatagaga 8520
   ttgtgcaact ataacgaaga actcagtaca gttcctaaaa atgaagaagg gttgcgcttt 8580
   cacatatgac ctaacaatct ctaaccttgt caggcttatt gaactagtcc ataagaataa 8640
   tttacaagaa agagagatcc ctaccgtgac agtaactact tggcttgcat attcttttgt 8700
   caatgaagac ctggggacta tcaagcctgt attgggggag aaagtcatcc cagaaccccc 8760
   cgaggagttg agtctccaac ccaccgtgag actagtcacc actgaaacag caataaccat 8820
   aacaggggag gctgaagtga tgacgacagg gatcacacca gtggtagaga tgaaagaaga 8880
   acctcagctg gaccaccagt caactaccct aaaggtaggg ttgaaggaag gggaatatcc 8940
   agggccagga gttaacccta accatttagc agaggtgata gatgagaaag atgacaggcc 9000
   ttttgtccta atcatcggta acaaaggttc tacctcgaac agagcaagaa cggccaagaa 9060
   tatacggctg tacaaaggaa acaacccaag agagatcagg gatctgatga gccaaggaag 9120
   aatattgacg gttgctctaa aagagttgga cccggaatta aaagaattag tagattacaa 9180
   ggggaccttt ctcaataggg aagctttaga agccctaagc ttaggtaagc caatcaagag 9240
   gaaaaccaca acagcaatga tcaggaggtt aatagagcca gaggttgagg aggaactacc 9300
   agattggttc caagcggaag aacccctatt tttggaagca aaaatacaga atgacttata 9360
   ccacctaatt ggcagtgtag atagtataaa aagcaaagca aaggaattag gggccacaga 9420
   taacacaaag atagtgaagg aagttggggc taggacctat acgatgaaat tgagcagctg 9480
   gagcacacaa gttacaaaaa aacagatgag tctagcccct ctctttgaag agctgttatt 9540
   aaagtgccct ccatgtagta aaatttcaaa gggacatatg gtgtcagcat accaactggc 9600
   tcaaggaaac tgggaacccc tcgggtgtgg ggtctatatg ggaaccatac cagctaggcg 9660
   tctcaagatc cacccttatg aggcttacct taaactcaaa gagctggtgg aagttgaatc 9720
   ttcgagggcc actgcaaaag aatccatcat aagagaacat aacacctgga tcctgcggaa 9780
   ggtgagacat gaagggaacc taagaaccaa atcaatgatc aaccctggga aaatatcaga 9840
   tcagctatgc agagatggac acaaaagaaa catatataat aagatcatag gctcaacaat 9900
   ggcctctgct ggtattaggc tggagaaact gccagtagtc cgagcccaaa ctgacacaac 9960
   cagtttccac caagccataa gagaaaaaat tgataaaaca gaaaacaagc agacccctga 10020
   attgcatgaa gaactaatga aggtcttcga ctgcttaaag atcccagagc tgaaggaatc 10080
   gtatgatgaa gtttcatggg aacaattaga agccgggata aaccgtaagg gtgcagcagg 10140
   ctatctagag agcaagaaca taggggaagt cctagacaca gagaaacaca tagtagagca 10200
   gctgatcaag gatctgagga aggggaagaa gattaggtac tatgaaacag ccatccccaa 10260
   gaatgagaag agagacgtca gcgacgactg ggaagccgga gagttcgttg atgaaaagaa 10320
   accaagagta atccagtacc cggacgccaa ggtgagactg gccattacaa aagtgatgta 10380
   caaatgggta aagcaaaaac cagtggtgat acccggctat gaaggtaaaa cacctctatt 10440
   tgacatattc aacaaagtga agaaggaatg ggattcattc caggaccccg tagcagtgag 10500
   ctttgacacc aaagcgtggg atacacaagt caccagtaga gacctaatgt tgataaagga 10560
   tatccagaaa tattatttca agagaagtat acacaaattt ttagatacaa taacagaaca 10620
   catggtggag gtacctgtca ttacagcaga cggtgaagtt tacataagga atggtcagag 10680
   gggtagtggc caacccgaca caagtgctgg taatagtatg ttgaatgtcc taaccatgat 10740
   atatgctttc tgtaaaagta caggcatacc ttacagggga ttcagcagag tggcaagaat 10800
   ccatgtgtgt ggtgatgatg gctttttgat aacagagaga ggactgggac tgaaattctc 10860
   tgagaagggt atgcagatat tacatgaggc cgggaagccc cagaaaataa ctgaagggga 10920
   caaaatgaaa gtggcataca gattcgagga catagagttt tgttcccata ctcccgtgcc 10980
   agtcagatgg gcagataaca ccagtagtta catggcaggg aggagcacag ccactatact 11040
   agctaagatg gcaaccaggc tggattccag cggagagagg ggtagcacag cttatgagaa 11100
   ggccgtagcc ttcagcttcc ttttgatgta ctcatggaat cccgtagtta gaaggatctg 11160
   cttactggtg ttgtcacagt ttccagaaat atccccatcc aaaaacacaa tatactacta 11220
   ccaaggggat cccatagctg cgtacagaga agtgataggg aaacagctgt gtgaactgaa 11280
   aagaacagga tttgagaagc tggctggtct gaatttgagt atgaccactc taggcatctg 11340
   gacaaaacat actagtaaaa gactaatcca agcctgtgta gaaataggta agagagaagg 11400
   tacctggtta gttaatgctg acagactgat tgcaggaaag actgggaagt tttacatccc  11460
   aagcactggt gtcactctgt tgggaaaaca ctatgaggaa attaacttaa agcaaaaggc  11520
   ggcacaaccg ccgatagagg gggttgacag atataagttg ggccccatag ttaatgttat  11580
   cttgagaagg ctgagggtga tgctgatgac agttgccagc ggaagctggt gaatccgtcc  11640
   ggagcgtcgt gccctcactc aaggttttta attgtaaata ttgtaaatag acagctaaga  11700
   tatttattgt agttggatag taatgcagtg atagtaaata ccccaattta acactacctc  11760
   caatgcacta agcactttag ctgtgtgagg ttaactcgac gtccacggtt ggactaggga  11820
   agacctctaa cagcccc                                                 11837
<210> 5
   <211> 3913
   <212> PRT
   <213> Wildtyp BVDV: XIKE-A
<400> 5
   Met Glu Leu Phe Ser Asn Glu Leu Leu Tyr Lys Thr Tyr Lys Gln Lys
   1               5                   10                  15
   Pro Ala Gly Val Val Glu Pro Val Tyr Asp Val Asn Gly Arg Pro Leu
               20                  25                  30
   Phe Gly Glu Ser Ser Asp Leu His Pro Gln Ser Thr Leu Lys Leu Pro
           35                  40                  45
   His Gln Arg Gly Ser Ala Asn Ile Leu Thr Asn Ala Arg Ser Leu Pro
       50                  55                  60
   Arg Lys Gly Asp Cys Arg Arg Gly Asn Val Tyr Gly Pro Val Ser Gly
   65                  70                  75                  80
   Ile Tyr Ile Lys Pro Gly Pro Ile Tyr Tyr Gln Asp Tyr Val Gly Pro
                   85                  90                  95
   Val Tyr His Arg Ala Pro Leu Glu Leu Cys Arg Glu Ala Ser Met Cys
               100                 105                 110
   Glu Thr Thr Arg Arg Val Gly Arg Val Thr Gly Ser Asp Gly Lys Leu
           115                 120                 125
   Tyr His Ile Tyr Ile Cys Ile Asp Gly Cys Ile Leu Leu Lys Arg Ala
       130                 135                 140
   Thr Arg Asn Gln Pro Glu Val Leu Lys Trp Val Tyr Asn Arg Leu Asn
   145                 150                 155                 160
   Cys Pro Leu Trp Val Thr Ser Cys Ser Asp Glu Gly Ser Lys Gly Ala
                   165                 170                 175
   Thr Ser Lys Lys Gln Pro Lys Pro Asp Arg Ile Glu Lys Gly Lys Met
               180                 185                 190
   Lys Ile Ala Pro Lys Glu Thr Glu Lys Asp Cys Lys Thr Arg Pro Pro
           195                 200                 205
   Asp Ala Thr Ile Val Val Glu Gly Val Lys Tyr Gln Val Lys Lys Lys
       210                 215                 220
   Gly Lys Val Arg Gly Lys Asn Thr Gln Asp Gly Leu Tyr His Asn Lys
   225                 230                 235                 240
   Asn Lys Pro Pro Glu Ser Arg Lys Lys Leu Glu Lys Ala Leu Leu Ala
                   245                 250                 255
   Trp Ala Ile Leu Ala Ala Val Leu Leu Gln Leu Val Thr Gly Glu Asn
               260                 265                 270
   Ile Thr Gln Trp Asn Leu Met Asp Asn Gly Thr Glu Gly Ile Gln Gln
           275                 280                 285
   Ala Met Phe Leu Arg Gly Val Asn Arg Ser Leu His Gly Ile Trp Pro
       290                 295                 300
   Glu Lys Ile Cys Thr Gly Val Pro Thr His Leu Ala Thr Asp Tyr Glu
   305                 310                 315                 320
   Leu Lys Glu Ile Val Gly Met Met Asp Ala Ser Glu Lys Thr Asn Tyr
                   325                 330                 335
   Thr Cys Cys Arg Leu Gln Arg His Glu Trp Asn Lys His Gly Trp Cys
               340                 345                 350
   Asn Trp Phe His Ile Glu Pro Trp Ile Trp Leu Met Asn Lys Thr Gln
           355                 360                 365
   Asn Asn Leu Thr Glu Gly Gln Pro Leu Arg Glu Cys Ala Val Thr Cys
       370                 375                 380
   Arg Tyr Asp Lys Glu Thr Glu Leu Asn Ile Val Thr Gln Ala Arg Asp
   385                 390                 395                 400
   Arg Pro Thr Thr Leu Thr Gly Cys Lys Lys Gly Lys Asn Phe Ser Phe
   405 410 415
   Ala Gly Val Ile Leu Asp Gly Pro Cys Asn Phe Lys Val Ser Val Glu
   420 425 430
   Asp Val Leu Phe Lys Glu His Asp Cys Gly Asn Met Leu Gln Glu Thr
   435 440 445
   Ala Ile Gln Leu Leu Asp Gly Ala Thr Asn Thr Ile Glu Gly Ala Arg
   450 455 460
   Val Gly Thr Ala Lys Leu Thr Thr Trp Leu Gly Lys Gln Leu Gly Ile
   465 470 475 480
   Leu Gly Lys Lys Leu Glu Asn Lys Ser Lys Ala Trp Phe Gly Ala His
   485 490 495
   Ala Ala Ser Pro Tyr Cys Gly Val Glu Arg Lys Ile Gly Tyr Val Trp
   500 505 510
   Tyr Thr Lys Asn Cys Thr Pro Ala Cys Leu Pro Arg Asn Thr Arg Ile
   515 520 525
   Ile Gly Pro Gly Lys Phe Asp Thr Asn Ala Glu Asp Gly Lys Ile Leu
   530 535 540
   His Glu Met Gly Gly His Leu Ser Glu Phe Val Leu Leu Ser Leu Val
   545 550 555 560
   Val Leu Ser Asp Phe Ala Pro Glu Thr Ala Ser Val Ile Tyr Leu Val
   565 570 575
   Leu His Phe Ala Ile Pro Gln Ser His Val Asp Val Asp Thr Cys Asp
   580 585 590
   Lys Asn Gln Leu Asn Leu Thr Val Ala Thr Thr Val Ala Glu Val Ile
   595 600 605
   Pro Gly Thr Val Trp Asn Leu Gly Lys Tyr Val Cys Ile Arg Pro Asp
   610 615 620
   Trp Trp Pro Tyr Glu Thr Thr Thr Val Phe Val Ile Glu Glu Ala Gly
   625 630 635 640
   Gln Val Ile Lys Leu Met Leu Arg Ala Ile Arg Asp Leu Thr Arg Ile
   645 650 655
   Trp Asn Ala Ala Thr Thr Thr Ala Phe Leu Ile Phe Leu Val Lys Ala
               660                 665                 670
   Leu Arg Gly Gln Leu Ile Gln Gly Leu Leu Trp Leu Met Leu Ile Thr
           675                 680                 685
   Gly Ala Gln Gly Phe Pro Glu Cys Lys Glu Gly Phe Gln Tyr Ala Ile
       690                 695                 700
   Ser Lys Asp Arg Lys Met Gly Leu Leu Gly Pro Glu Ser Leu Thr Thr
   705                 710                 715                 720
   Thr Trp His Leu Pro Thr Lys Lys Ile Val Asp Ser Met Val His Val
                   725                 730                 735
   Trp Cys Glu Gly Lys Asp Leu Lys Ile Leu Lys Met Cys Thr Lys Glu
               740                 745                 750
   Glu Arg Tyr Leu Val Ala Val His Glu Arg Ala Leu Ser Thr Ser Ala
           755                 760                 765
   Glu Phe Met Gln Ile Ser Asp Gly Thr Ile Gly Pro Asp Val Ile Asp
       770                 775                 780
   Met Pro Asp Asp Phe Glu Phe Gly Leu Cys Pro Cys Asp Ser Lys Pro
   785                 790                 795                 800
   Val Ile Lys Gly Lys Phe Asn Ala Ser Leu Leu Asn Gly Pro Ala Phe
                   805                 810                 815
   Gln Met Val Cys Pro Gln Gly Trp Thr Gly Thr Ile Glu Cys Thr Leu
               820                 825                 830
   Ala Asn Gln Asp Thr Leu Asp Thr Thr Val Ile Arg Thr Tyr Arg Arg
           835                 840                 845
   Thr Thr Pro Phe Gln Arg Arg Lys Trp Cys Thr Tyr Glu Lys Ile Ile
       850                 855                 860
   Gly Glu Asp Ile Tyr Glu Cys Ile Leu Gly Gly Asn Trp Thr Cys Ile
   865                 870                 875                 880
   Thr Gly Asp His Ser Arg Leu Lys Asp Gly Pro Ile Lys Lys Cys Lys
                   885                 890                 895
   Trp Cys Gly His Asp Phe Val Asn Ser Glu Gly Leu Pro His Tyr Pro
               900                 905                 910
   Ile Gly Lys Cys Met Leu Ile Asn Glu Ser Gly Tyr Arg Tyr Val Asp
           915                 920                 925
   Asp Thr Ser Cys Asp Arg Gly Gly Val Ala Ile Val Pro Ser Gly Thr
       930                 935                 940
   Val Lys Cys Arg Ile Gly Asn Val Thr Val Gln Val Ile Ala Thr Asn
   945                 950                 955                 960
   Asn Asp Leu Gly Pro Met Pro Cys Ser Pro Ala Glu Val Ile Ala Ser
                   965                 970                 975
   Glu Gly Pro Val Glu Lys Thr Ala Cys Thr Phe Asn Tyr Ser Arg Thr
               980                 985                 990
   Leu Pro Asn Lys Tyr Tyr Glu Pro  Arg Asp Arg Tyr Phe  Gln Gln Tyr
           995                 1000                 1005
   Met Leu  Lys Gly Glu Trp Gln  Tyr Trp Phe Asp Leu  Asp Ser Val
       1010                 1015                 1020
   Asp His  His Lys Asp Tyr Phe  Ser Glu Phe Ile Ile  Ile Ala Val
       1025                 1030                 1035
   Val Ala  Leu Leu Gly Gly Lys  Tyr Val Leu Trp Leu  Leu Ile Thr
       1040                 1045                 1050
   Tyr Thr  Ile Leu Ser Glu Gln  Met Ala Met Gly Ala  Gly Val Asn
       1055                 1060                 1065
   Thr Glu  Glu Ile Val Met Ile  Gly Asn Leu Leu Thr  Asp Ser Asp
       1070                 1075                 1080
   Ile Glu  Val Val Val Tyr Phe  Leu Leu Leu Tyr Leu  Ile Val Lys
       1085                 1090                 1095
   Glu Glu  Leu Ala Arg Lys Trp  Ile Ile Leu Val Tyr  His Ile Leu
       1100                 1105                 1110
   Val Ala  Asn Pro Met Lys Thr  Ile Gly Val Val Leu  Leu Met Leu
       1115                 1120                 1125
   Gly Gly  Val Val Lys Ala Ser  Arg Ile Asn Ala Asp  Asp Gln Ser
       1130                 1135                 1140
   Ala Met  Asp Pro Cys Phe Leu  Leu Val Thr Gly Val  Val Ala Val
       1145                 1150                 1155
   Leu Met  Ile Ala Arg Arg Glu  Pro Ala Thr Leu Pro  Leu Ile Val
       1160                 1165                 1170
   Ala Leu  Leu Ala Ile Arg Thr  Ser Gly Phe Leu Leu  Pro Ala Ser
       1175                 1180                 1185
   Ile Asp  Val Thr Val Ala Val  Val Leu Ile Val Leu  Leu Leu Ala
       1190                 1195                 1200
   Ser Tyr  Ile Thr Asp Tyr Phe  Arg Tyr Lys Lys Trp  Leu Gln Leu
       1205                 1210                 1215
   Leu Phe  Ser Leu Ile Ala Gly  Ile Phe Ile Ile Arg  Ser Leu Lys
       1220                 1225                 1230
   His Ile  Asn Gln Met Glu Val  Pro Glu Ile Ser Met  Pro Ser Trp
       1235                 1240                 1245
   Arg Pro  Leu Ala Leu Val Leu  Phe Tyr Ile Thr Ser  Thr Ala Ile
       1250                 1255                 1260
   Thr Thr  Asn Trp Asp Ile Asp  Leu Ala Gly Phe Leu  Leu Gln Trp
       1265                 1270                 1275
   Ala Pro  Ala Val Ile Met Met  Ala Thr Met Trp Ala  Asp Phe Leu
       1280                 1285                 1290
   Thr Leu  Ile Ile Val Leu Pro  Ser Tyr Glu Leu Ser  Lys Leu Tyr
       1295                 1300                 1305
   Phe Leu  Lys Asn Val Arg Thr  Asp Val Glu Lys Asn  Trp Leu Gly
       1310                 1315                 1320
   Lys Val  Lys Tyr Arg Gln Ile  Ser Ser Val Tyr Asp  Ile Cys Asp
       1325                 1330                 1335
   Ser Glu  Glu Ala Val Tyr Leu  Phe Pro Ser Arg His  Lys Ser Gly
       1340                 1345                 1350
   Ser Arg  Pro Asp Phe Ile Leu  Pro Phe Leu Lys Ala  Val Leu Ile
       1355                 1360                 1365
   Ser Cys  Ile Ser Ser Gln Trp  Gln Val Val Tyr Ile  Ser Tyr Leu
       1370                 1375                 1380
   Ile Leu  Glu Ile Thr Tyr Tyr  Met His Arg Lys Ile  Ile Asp Glu
   1385 1390 1395
   Val Ser Gly Gly Ala Asn Phe Leu Ser Arg Leu Ile Ala Ala Ile
   1400 1405 1410
   Ile Glu Leu Asn Trp Ala Ile Asp Asp Glu Glu Cys Lys Gly Leu
   1415 1420 1425
   Lys Lys Leu Tyr Leu Leu Ser Gly Arg Ala Lys Asn Leu Ile Val
   1430 1435 1440
   Lys His Lys Val Arg Asn Glu Ala Val His Arg Trp Phe Gly Glu
   1445 1450 1455
   Glu Glu Ile Tyr Gly Ala Pro Lys Val Ile Thr Ile Ile Lys Ala
   1460 1465 1470
   Ser Thr Leu Ser Lys Asn Arg His Cys Ile Ile Cys Thr Ile Cys
   1475 1480 1485
   Glu Gly Lys Glu Trp Asn Gly Ala Asn Cys Pro Lys Cys Gly Arg
   1490 1495 1500
   Gln Gly Lys Pro Ile Thr Cys Gly Met Thr Leu Ala Asp Phe Glu
   1505 1510 1515
   Glu Lys His Tyr Lys Lys Ile Phe Ile Arg Glu Glu Ser Ser Cys
   1520 1525 1530
   Pro Val Pro Phe Asp Pro Ser Cys His Cys Asn Tyr Phe Arg His
   1535 1540 1545
   Asp Gly Pro Phe Arg Lys Glu Tyr Lys Gly Tyr Val Gln Tyr Thr
   1550 1555 1560
   Ala Arg Gly Gln Leu Phe Leu Arg Asn Leu Pro Ile Leu Ala Thr
   1565 1570 1575
   Lys Met Lys Leu Leu Met Val Gly Asn Leu Gly Ala Glu Ile Gly
   1580 1585 1590
   Asp Leu Glu His Leu Gly Trp Val Leu Arg Gly Pro Ala Val Cys
   1595 1600 1605
   Lys Lys Ile Thr Asn His Glu Lys Cys His Val Asn Ile Met Asp
   1610 1615 1620
   Lys Leu Thr Ala Phe Phe Gly Ile Met Pro Arg Gly Thr Thr Pro
   1625 1630 1635
   Arg Ala Pro Val Arg Phe Pro Thr Ala Leu Leu Lys Val Arg Arg
   1640 1645 1650
   Gly Leu Glu Thr Gly Trp Ala Tyr Thr His Gln Gly Gly Ile Ser
   1655 1660 1665
   Ser Val Asp His Val Thr Ala Gly Lys Asp Leu Leu Val Cys Asp
   1670 1675 1680
   Ser Met Gly Arg Thr Arg Val Val Cys His Ser Asn Asn Lys Met
   1685 1690 1695
   Thr Asp Glu Thr Glu Tyr Gly Ile Lys Thr Asp Ser Gly Cys Pro
   1700 1705 1710
   Glu Gly Ala Arg Cys Tyr Val Leu Asn Pro Glu Ala Val Asn Ile
   1715 1720 1725
   Ser Gly Thr Lys Gly Ala Met Val His Leu Gln Lys Thr Gly Gly
   1730 1735 1740
   Glu Phe Thr Cys Val Thr Ala Ser Gly Thr Pro Ala Phe Phe Asp
   1745 1750 1755
   Leu Lys Asn Leu Lys Gly Trp Ser Gly Leu Pro Ile Phe Glu Ala
   1760 1765 1770
   Ser Ser Gly Arg Val Val Gly Arg Val Lys Val Gly Lys Asn Glu
   1775 1780 1785
   Asp Ser Lys Pro Thr Lys Leu Met Ser Gly Ile Gln Thr Val Ser
   1790 1795 1800
   Lys Asn Gln Thr Asp Leu Ala Asp Ile Val Lys Lys Leu Thr Ser
   1805 1810 1815
   Met Asn Arg Gly Glu Phe Lys Gln Ile Thr Leu Ala Thr Gly Ala
   1820 1825 1830
   Gly Lys Thr Thr Glu Leu Pro Arg Ser Val Ile Glu Glu Ile Gly
   1835 1840 1845
   Arg His Lys Arg Val Leu Val Leu Ile Pro Leu Arg Ala Ala Ala
   1850 1855 1860
   Glu Ser Val Tyr Gln Tyr Met Arg Val Lys Tyr Pro Ser Ile Ser
   1865 1870 1875
   Phe Asn Leu Arg Ile Gly Asp Met Lys Glu Gly Asp Met Ala Thr
   1880 1885 1890
   Gly Ile Thr Tyr Ala Ser Tyr Gly Tyr Phe Cys Gln Leu Pro Gln
   1895 1900 1905
   Pro Lys Leu Arg Ala Ala Met Val Glu Tyr Ser Tyr Ile Phe Leu
   1910 1915 1920
   Asp Glu Tyr His Cys Ala Thr Pro Glu Gln Leu Ala Ile Ile Gly
   1925 1930 1935
   Lys Ile His Arg Phe Ala Glu Asn Leu Arg Val Val Ala Met Thr
   1940 1945 1950
   Ala Thr Pro Ala Gly Thr Val Thr Thr Thr Gly Gln Lys His Pro
   1955 1960 1965
   Ile Glu Glu Phe Ile Ala Pro Glu Val Met Lys Gly Glu Asp Leu
   1970 1975 1980
   Gly Ser Glu Tyr Leu Asp Ile Ala Gly Leu Lys Ile Pro Thr Glu
   1985 1990 1995
   Glu Met Lys Gly Asn Met Leu Val Phe Ala Pro Thr Arg Asn Met
   2000 2005 2010
   Ala Val Glu Thr Ala Lys Lys Leu Lys Ala Lys Gly Tyr Asn Ser
   2015 2020 2025
   Gly Tyr Tyr Tyr Ser Gly Glu Asn Pro Glu Asn Leu Arg Val Val
   2030 2035 2040
   Thr Ser Gln Ser Pro Tyr Val Val Val Ala Thr Asn Ala Ile Glu
   2045 2050 2055
   Ser Gly Val Thr Leu Pro Asp Leu Asp Thr Val Val Asp Thr Gly
   2060 2065 2070
   Leu Lys Cys Glu Lys Arg Val Arg Ile Ser Ser Lys Met Pro Phe
   2075 2080 2085
   Ile Val Thr Gly Leu Lys Arg Met Ala Val Thr Ile Gly Glu Gln
   2090 2095 2100
   Ala Gln Arg Arg Gly Arg Val Gly Arg Val Lys Pro Gly Arg Tyr
       2105                2110                2115
   Tyr Arg Ser Gln Glu Thr Ala Ser Gly Ser Lys Asp Tyr His Tyr
       2120                2125                2130
   Asp Leu Leu Gln Ala Gln Arg Tyr Gly Ile Glu Asp Gly Ile Asn
       2135                2140                2145
   Val Thr Lys Ser Phe Arg Glu Met Asn Tyr Asp Trp Ser Leu Tyr
       2150                2155                2160
   Glu Glu Asp Ser Leu Met Ile Thr Gln Leu Glu Val Leu Asn Asn
       2165                2170                2175
   Leu Leu Ile Ser Glu Asp Leu Pro Ala Ala Val Lys Asn Ile Met
       2180                2185                2190
   Ala Arg Thr Asp His Pro Glu Pro Ile Gln Leu Ala Tyr Asn Ser
       2195                2200                2205
   Tyr Glu Asn Gln Ile Pro Val Leu Phe Pro Lys Ile Lys Asn Gly
       2210                2215                2220
   Glu Val Thr Asp Ser Tyr Glu Asn Tyr Thr Tyr Leu Asn Ala Arg
       2225                2230                2235
   Lys Leu Gly Glu Asp Val Pro Ala Tyr Val Tyr Ala Thr Glu Asp
       2240                2245                2250
   Glu Asp Leu Ala Val Asp Leu Leu Gly Met Asp Trp Pro Asp Pro
       2255                2260                2265
   Gly Asn Gln Gln Val Val Glu Thr Gly Arg Ala Leu Lys Gln Val
       2270                2275                2280
   Thr Gly Leu Ser Thr Ala Glu Asn Ala Leu Leu Ile Ala Leu Phe
       2285                2290                2295
   Gly Tyr Val Gly Tyr Gln Thr Leu Ser Lys Arg His Ile Pro Met
       2300                2305                2310
   Ile Thr Asp Ile Tyr Thr Leu Glu Asp His Arg Leu Glu Asp Thr
       2315                2320                2325
   Thr His Leu Gln Phe Ala Pro Asn Ala Ile Arg Thr Asp Gly Lys
   2330 2335 2340
   Asp Ser Glu Leu Lys Glu Leu Ala Val Gly Asp Leu Asp Lys Tyr
   2345 2350 2355
   Val Asp Ala Leu Val Asp Tyr Ser Lys Gln Gly Met Lys Phe Ile
   2360 2365 2370
   Lys Val Gln Ala Glu Lys Val Arg Asp Ser Gln Ser Thr Lys Glu
   2375 2380 2385
   Gly Leu Gln Thr Ile Lys Glu Tyr Val Asp Lys Phe Ile Gln Ser
   2390 2395 2400
   Leu Thr Glu Asn Lys Glu Glu Ile Ile Arg Tyr Gly Leu Trp Gly
   2405 2410 2415
   Val His Thr Ala Leu Tyr Lys Ser Leu Ala Ala Arg Leu Gly His
   2420 2425 2430
   Glu Thr Ala Phe Ala Thr Leu Val Val Lys Trp Leu Ala Phe Gly
   2435 2440 2445
   Gly Glu Thr Val Ser Ala His Ile Lys Gln Val Ala Val Asp Leu
   2450 2455 2460
   Val Val Tyr Tyr Ile Ile Asn Lys Pro Ser Phe Pro Gly Asp Thr
   2465 2470 2475
   Glu Thr Gln Gln Glu Gly Arg Arg Phe Val Ala Ser Leu Phe Ile
   2480 2485 2490
   Ser Ala Leu Ala Thr Tyr Thr Tyr Lys Thr Trp Asn Tyr Asn Asn
   2495 2500 2505
   Leu Gln Arg Val Val Glu Pro Ala Leu Ala Tyr Leu Pro Tyr Ala
   2510 2515 2520
   Thr Ser Ala Leu Lys Leu Phe Thr Pro Thr Arg Leu Glu Ser Val
   2525 2530 2535
   Val Ile Leu Ser Ser Thr Ile Tyr Lys Thr Tyr Leu Ser Ile Arg
   2540 2545 2550
   Lys Gly Lys Ser Asp Gly Leu Leu Gly Thr Gly Ile Ser Ala Ala
   2555 2560 2565
   Met Glu  Ile Leu Asn Gln Asn  Pro Ile Ser Val Gly  Ile Ser Val
       2570                 2575                 2580
   Met Leu  Gly Val Gly Ala Ile  Ala Ala His Asn Ala  Ile Glu Ser
       2585                 2590                 2595
   Ser Glu  Gln Lys Arg Thr Leu  Leu Met Lys Val Phe  Val Lys Asn
       2600                 2605                 2610
   Phe Leu  Asp Gln Ala Ala Thr  Asp Glu Leu Val Lys  Glu Asn Pro
       2615                 2620                 2625
   Glu Lys  Ile Ile Met Ala Leu  Phe Glu Ala Val Gln  Thr Ile Gly
       2630                 2635                 2640
   Asn Pro  Leu Arg Leu Ile Tyr  His Leu Tyr Gly Val  Tyr Tyr Lys
       2645                 2650                 2655
   Gly Trp  Glu Ala Lys Glu Leu  Ala Glu Lys Thr Ala  Gly Arg Asn
       2660                 2665                 2670
   Leu Phe  Thr Leu Ile Met Phe  Glu Ala Phe Glu Leu  Leu Gly Met
       2675                 2680                 2685
   Asp Ser  Glu Gly Lys Ile Arg  Asn Leu Ser Gly Asn  Tyr Ile Leu
       2690                 2695                 2700
   Asp Leu  Ile Phe Asn Leu His  Asn Lys Leu Asn Lys  Gly Leu Lys
       2705                 2710                 2715
   Lys Leu  Val Leu Gly Trp Ala  Pro Ala Pro Leu Ser  Cys Asp Trp
       2720                 2725                 2730
   Thr Pro  Ser Asp Glu Arg Ile  Ser Leu Pro His Asn  Asn Tyr Leu
       2735                 2740                 2745
   Arg Val  Glu Thr Arg Cys Pro  Cys Gly Tyr Glu Met  Lys Ala Ile
       2750                 2755                 2760
   Lys Asn  Val Ala Gly Lys Leu  Thr Lys Val Glu Glu  Lys Gly Ser
       2765                 2770                 2775
   Phe Leu  Cys Arg Asn Arg Leu  Gly Arg Gly Pro Pro  Asn Phe Lys
       2780                 2785                 2790
   Val Thr  Lys Phe Tyr Asp Asp  Asn Leu Ile Glu Val  Lys Pro Val
       2795                 2800                 2805
   Ala Arg Leu Glu Gly Gln Val Asp Leu Tyr Tyr Lys Gly Val Thr
       2810                2815                2820
   Ala Lys Leu Asp Tyr Asn Asn Gly Lys Val Leu Leu Ala Thr Asn
       2825                2830                2835
   Lys Trp Glu Val Asp His Ala Phe Leu Thr Arg Leu Val Lys Lys
       2840                2845                2850
   His Thr Gly Ile Gly Phe Lys Gly Ala Tyr Leu Gly Asp Arg Pro
       2855                2860                2865
   Asp His Gln Asp Leu Val Asp Arg Asp Cys Ala Thr Ile Thr Lys
       2870                2875                2880
   Asn Ser Val Gln Phe Leu Lys Met Lys Lys Gly Cys Ala Phe Thr
       2885                2890                2895
   Tyr Asp Leu Thr Ile Ser Asn Leu Val Arg Leu Ile Glu Leu Val
       2900                2905                2910
   His Lys Asn Asn Leu Gln Glu Arg Glu Ile Pro Thr Val Thr Val
       2915                2920                2925
   Thr Thr Trp Leu Ala Tyr Ser Phe Val Asn Glu Asp Leu Gly Thr
       2930                2935                2940
   Ile Lys Pro Val Leu Gly Glu Lys Val Ile Pro Glu Pro Pro Glu
       2945                2950                2955
   Glu Leu Ser Leu Gln Pro Thr Val Arg Leu Val Thr Thr Glu Thr
       2960                2965                2970
   Ala Ile Thr Ile Thr Gly Glu Ala Glu Val Met Thr Thr Gly Ile
       2975                2980                2985
   Thr Pro Val Val Glu Met Lys Glu Glu Pro Gln Leu Asp His Gln
       2990                2995                3000
   Ser Thr Thr Leu Lys Val Gly Leu Lys Glu Gly Glu Tyr Pro Gly
       3005                3010                3015
   Pro Gly Val Asn Pro Asn His Leu Ala Glu Val Ile Asp Glu Lys
       3020                3025                3030
   Asp Asp Arg Pro Phe Val Leu Ile Ile Gly Asn Lys Gly Ser Thr
       3035                3040                3045
   Ser Asn Arg Ala Arg Thr Ala Lys Asn Ile Arg Leu Tyr Lys Gly
   3050 3055 3060
   Asn Asn Pro Arg Glu Ile Arg Asp Leu Met Ser Gln Gly Arg Ile
   3065 3070 3075
   Leu Thr Val Ala Leu Lys Glu Leu Asp Pro Glu Leu Lys Glu Leu
   3080 3085 3090
   Val Asp Tyr Lys Gly Thr Phe Leu Asn Arg Glu Ala Leu Glu Ala
   3095 3100 3105
   Leu Ser Leu Gly Lys Pro Ile Lys Arg Lys Thr Thr Thr Ala Met
   3110 3115 3120
   Ile Arg Arg Leu Ile Glu Pro Glu Val Glu Glu Glu Leu Pro Asp
   3125 3130 3135
   Trp Phe Gln Ala Glu Glu Pro Leu Phe Leu Glu Ala Lys Ile Gln
   3140 3145 3150
   Asn Asp Leu Tyr His Leu Ile Gly Ser Val Asp Ser Ile Lys Ser
   3155 3160 3165
   Lys Ala Lys Glu Leu Gly Ala Thr Asp Asn Thr Lys Ile Val Lys
   3170 3175 3180
   Glu Val Gly Ala Arg Thr Tyr Thr Met Lys Leu Ser Ser Trp Ser
   3185 3190 3195
   Thr Gln Val Thr Lys Lys Gln Met Ser Leu Ala Pro Leu Phe Glu
   3200 3205 3210
   Glu Leu Leu Leu Lys Cys Pro Pro Cys Ser Lys Ile Ser Lys Gly
   3215 3220 3225
   His Met Val Ser Ala Tyr Gln Leu Ala Gln Gly Asn Trp Glu Pro
   3230 3235 3240
   Leu Gly Cys Gly Val Tyr Met Gly Thr Ile Pro Ala Arg Arg Leu
   3245 3250 3255
   Lys Ile His Pro Tyr Glu Ala Tyr Leu Lys Leu Lys Glu Leu Val
   3260 3265 3270
   Glu Val Glu Ser Ser Arg Ala Thr Ala Lys Glu Ser Ile Ile Arg
   3275 3280 3285
   Glu His Asn Thr Trp Ile Leu Arg Lys Val Arg His Glu Gly Asn
   3290 3295 3300
   Leu Arg Thr Lys Ser Met Ile Asn Pro Gly Lys Ile Ser Asp Gln
   3305 3310 3315
   Leu Cys Arg Asp Gly His Lys Arg Asn Ile Tyr Asn Lys Ile Ile
   3320 3325 3330
   Gly Ser Thr Met Ala Ser Ala Gly Ile Arg Leu Glu Lys Leu Pro
   3335 3340 3345
   Val Val Arg Ala Gln Thr Asp Thr Thr Ser Phe His Gln Ala Ile
   3350 3355 3360
   Arg Glu Lys Ile Asp Lys Thr Glu Asn Lys Gln Thr Pro Glu Leu
   3365 3370 3375
   His Glu Glu Leu Met Lys Val Phe Asp Cys Leu Lys Ile Pro Glu
   3380 3385 3390
   Leu Lys Glu Ser Tyr Asp Glu Val Ser Trp Glu Gln Leu Glu Ala
   3395 3400 3405
   Gly Ile Asn Arg Lys Gly Ala Ala Gly Tyr Leu Glu Ser Lys Asn
   3410 3415 3420
   Ile Gly Glu Val Leu Asp Thr Glu Lys His Ile Val Glu Gln Leu
   3425 3430 3435
   Ile Lys Asp Leu Arg Lys Gly Lys Lys Ile Arg Tyr Tyr Glu Thr
   3440 3445 3450
   Ala Ile Pro Lys Asn Glu Lys Arg Asp Val Ser Asp Asp Trp Glu
   3455 3460 3465
   Ala Gly Glu Phe Val Asp Glu Lys Lys Pro Arg Val Ile Gln Tyr
   3470 3475 3480
   Pro Asp Ala Lys Val Arg Leu Ala Ile Thr Lys Val Met Tyr Lys
   3485 3490 3495
   Trp Val Lys Gln Lys Pro Val Val Ile Pro Gly Tyr Glu Gly Lys
   3500 3505 3510
   Thr Pro Leu Phe Asp Ile Phe Asn Lys Val Lys Lys Glu Trp Asp
   3515 3520 3525
   Ser Phe Gln Asp Pro Val Ala Val Ser Phe Asp Thr Lys Ala Trp
   3530 3535 3540
   Asp Thr Gln Val Thr Ser Arg Asp Leu Met Leu Ile Lys Asp Ile
   3545 3550 3555
   Gln Lys Tyr Tyr Phe Lys Arg Ser Ile His Lys Phe Leu Asp Thr
   3560 3565 3570
   Ile Thr Glu His Met Val Glu Val Pro Val Ile Thr Ala Asp Gly
   3575 3580 3585
   Glu Val Tyr Ile Arg Asn Gly Gln Arg Gly Ser Gly Gln Pro Asp
   3590 3595 3600
   Thr Ser Ala Gly Asn Ser Met Leu Asn Val Leu Thr Met Ile Tyr
   3605 3610 3615
   Ala Phe Cys Lys Ser Thr Gly Ile Pro Tyr Arg Gly Phe Ser Arg
   3620 3625 3630
   Val Ala Arg Ile His Val Cys Gly Asp Asp Gly Phe Leu Ile Thr
   3635 3640 3645
   Glu Arg Gly Leu Gly Leu Lys Phe Ser Glu Lys Gly Met Gln Ile
   3650 3655 3660
   Leu His Glu Ala Gly Lys Pro Gln Lys Ile Thr Glu Gly Asp Lys
   3665 3670 3675
   Met Lys Val Ala Tyr Arg Phe Glu Asp Ile Glu Phe Cys Ser His
   3680 3685 3690
   Thr Pro Val Pro Val Arg Trp Ala Asp Asn Thr Ser Ser Tyr Met
   3695 3700 3705
   Ala Gly Arg Ser Thr Ala Thr Ile Leu Ala Lys Met Ala Thr Arg
   3710 3715 3720
   Leu Asp Ser Ser Gly Glu Arg Gly Ser Thr Ala Tyr Glu Lys Ala
   3725 3730 3735
   Val Ala Phe Ser Phe Leu Leu Met Tyr Ser Trp Asn Pro Val Val
   3740 3745 3750
   Arg Arg Ile Cys Leu Leu Val Leu Ser Gln Phe Pro Glu Ile Ser
   3755 3760 3765
   Pro Ser Lys Asn Thr Ile Tyr Tyr Tyr Gln Gly Asp Pro Ile Ala
   3770 3775 3780
   Ala Tyr Arg Glu Val Ile Gly Lys Gln Leu Cys Glu Leu Lys Arg
   3785 3790 3795
   Thr Gly Phe Glu Lys Leu Ala Gly Leu Asn Leu Ser Met Thr Thr
   3800 3805 3810
   Leu Gly Ile Trp Thr Lys His Thr Ser Lys Arg Leu Ile Gln Ala
   3815 3820 3825
   Cys Val Glu Ile Gly Lys Arg Glu Gly Thr Trp Leu Val Asn Ala
   3830 3835 3840
   Asp Arg Leu Ile Ala Gly Lys Thr Gly Lys Phe Tyr Ile Pro Ser
   3845 3850 3855
   Thr Gly Val Thr Leu Leu Gly Lys His Tyr Glu Glu Ile Asn Leu
   3860 3865 3870
   Lys Gln Lys Ala Ala Gln Pro Pro Ile Glu Gly Val Asp Arg Tyr
   3875 3880 3885
   Lys Leu Gly Pro Ile Val Asn Val Ile Leu Arg Arg Leu Arg Val
   3890 3895 3900
   Met Leu Met Thr Val Ala Ser Gly Ser Trp
   3905 3910
<210> 6
   <211> 3749
   <212> PRT
   <213> Artificial Sequence: Mutated BVDV: XIKE-A-NdN
<400> 6
   Met Glu Leu Phe Ser Asp Glu Gly Ser Lys Gly Ala Thr Ser Lys Lys
   1 5 10 15
   Gln Pro Lys Pro Asp Arg Ile Glu Lys Gly Lys Met Lys Ile Ala Pro
   20 25 30
   Lys Glu Thr Glu Lys Asp Cys Lys Thr Arg Pro Pro Asp Ala Thr Ile
   35 40 45
   Val Val Glu Gly Val Lys Tyr Gln Val Lys Lys Lys Gly Lys Val Arg
       50                  55                  60
   Gly Lys Asn Thr Gln Asp Gly Leu Tyr His Asn Lys Asn Lys Pro Pro
   65                  70                  75                  80
   Glu Ser Arg Lys Lys Leu Glu Lys Ala Leu Leu Ala Trp Ala Ile Leu
                   85                  90                  95
   Ala Ala Val Leu Leu Gln Leu Val Thr Gly Glu Asn Ile Thr Gln Trp
               100                 105                 110
   Asn Leu Met Asp Asn Gly Thr Glu Gly Ile Gln Gln Ala Met Phe Leu
           115                 120                 125
   Arg Gly Val Asn Arg Ser Leu His Gly Ile Trp Pro Glu Lys Ile Cys
       130                 135                 140
   Thr Gly Val Pro Thr His Leu Ala Thr Asp Tyr Glu Leu Lys Glu Ile
   145                 150                 155                 160
   Val Gly Met Met Asp Ala Ser Glu Lys Thr Asn Tyr Thr Cys Cys Arg
                   165                 170                 175
   Leu Gln Arg His Glu Trp Asn Lys His Gly Trp Cys Asn Trp Phe His
               180                 185                 190
   Ile Glu Pro Trp Ile Trp Leu Met Asn Lys Thr Gln Asn Asn Leu Thr
           195                 200                 205
   Glu Gly Gln Pro Leu Arg Glu Cys Ala Val Thr Cys Arg Tyr Asp Lys
       210                 215                 220
   Glu Thr Glu Leu Asn Ile Val Thr Gln Ala Arg Asp Arg Pro Thr Thr
   225                 230                 235                 240
   Leu Thr Gly Cys Lys Lys Gly Lys Asn Phe Ser Phe Ala Gly Val Ile
                   245                 250                 255
   Leu Asp Gly Pro Cys Asn Phe Lys Val Ser Val Glu Asp Val Leu Phe
               260                 265                 270
   Lys Glu His Asp Cys Gly Asn Met Leu Gln Glu Thr Ala Ile Gln Leu
           275                 280                 285
   Leu Asp Gly Ala Thr Asn Thr Ile Glu Gly Ala Arg Val Gly Thr Ala
       290                 295                 300
   Lys Leu Thr Thr Trp Leu Gly Lys Gln Leu Gly Ile Leu Gly Lys Lys
   305                 310                 315                 320
   Leu Glu Asn Lys Ser Lys Ala Trp Phe Gly Ala His Ala Ala Ser Pro
                   325                 330                 335
   Tyr Cys Gly Val Glu Arg Lys Ile Gly Tyr Val Trp Tyr Thr Lys Asn
               340                 345                 350
   Cys Thr Pro Ala Cys Leu Pro Arg Asn Thr Arg Ile Ile Gly Pro Gly
           355                 360                 365
   Lys Phe Asp Thr Asn Ala Glu Asp Gly Lys Ile Leu His Glu Met Gly
       370                 375                 380
   Gly His Leu Ser Glu Phe Val Leu Leu Ser Leu Val Val Leu Ser Asp
   385                 390                 395                 400
   Phe Ala Pro Glu Thr Ala Ser Val Ile Tyr Leu Val Leu His Phe Ala
                   405                 410                 415
   Ile Pro Gln Ser His Val Asp Val Asp Thr Cys Asp Lys Asn Gln Leu
               420                 425                 430
   Asn Leu Thr Val Ala Thr Thr Val Ala Glu Val Ile Pro Gly Thr Val
           435                 440                 445
   Trp Asn Leu Gly Lys Tyr Val Cys Ile Arg Pro Asp Trp Trp Pro Tyr
       450                 455                 460
   Glu Thr Thr Thr Val Phe Val Ile Glu Glu Ala Gly Gln Val Ile Lys
   465                 470                 475                 480
   Leu Met Leu Arg Ala Ile Arg Asp Leu Thr Arg Ile Trp Asn Ala Ala
                   485                 490                 495
   Thr Thr Thr Ala Phe Leu Ile Phe Leu Val Lys Ala Leu Arg Gly Gln
               500                 505                 510
   Leu Ile Gln Gly Leu Leu Trp Leu Met Leu Ile Thr Gly Ala Gln Gly
           515                 520                 525
   Phe Pro Glu Cys Lys Glu Gly Phe Gln Tyr Ala Ile Ser Lys Asp Arg
       530                 535                 540
   Lys Met Gly Leu Leu Gly Pro Glu Ser Leu Thr Thr Thr Trp His Leu
   545                 550                 555                 560
   Pro Thr Lys Lys Ile Val Asp Ser Met Val His Val Trp Cys Glu Gly
                   565                 570                 575
   Lys Asp Leu Lys Ile Leu Lys Met Cys Thr Lys Glu Glu Arg Tyr Leu
               580                 585                 590
   Val Ala Val His Glu Arg Ala Leu Ser Thr Ser Ala Glu Phe Met Gln
           595                 600                 605
   Ile Ser Asp Gly Thr Ile Gly Pro Asp Val Ile Asp Met Pro Asp Asp
       610                 615                 620
   Phe Glu Phe Gly Leu Cys Pro Cys Asp Ser Lys Pro Val Ile Lys Gly
   625                 630                 635                 640
   Lys Phe Asn Ala Ser Leu Leu Asn Gly Pro Ala Phe Gln Met Val Cys
                   645                 650                 655
   Pro Gln Gly Trp Thr Gly Thr Ile Glu Cys Thr Leu Ala Asn Gln Asp
               660                 665                 670
   Thr Leu Asp Thr Thr Val Ile Arg Thr Tyr Arg Arg Thr Thr Pro Phe
           675                 680                 685
   Gln Arg Arg Lys Trp Cys Thr Tyr Glu Lys Ile Ile Gly Glu Asp Ile
       690                 695                 700
   Tyr Glu Cys Ile Leu Gly Gly Asn Trp Thr Cys Ile Thr Gly Asp His
   705                 710                 715                 720
   Ser Arg Leu Lys Asp Gly Pro Ile Lys Lys Cys Lys Trp Cys Gly His
                   725                 730                 735
   Asp Phe Val Asn Ser Glu Gly Leu Pro His Tyr Pro Ile Gly Lys Cys
               740                 745                 750
   Met Leu Ile Asn Glu Ser Gly Tyr Arg Tyr Val Asp Asp Thr Ser Cys
           755                 760                 765
   Asp Arg Gly Gly Val Ala Ile Val Pro Ser Gly Thr Val Lys Cys Arg
       770                 775                 780
   Ile Gly Asn Val Thr Val Gln Val Ile Ala Thr Asn Asn Asp Leu Gly
   785                 790                 795                 800
   Pro Met Pro Cys Ser Pro Ala Glu Val Ile Ala Ser Glu Gly Pro Val
                   805                 810                 815
   Glu Lys Thr Ala Cys Thr Phe Asn Tyr Ser Arg Thr Leu Pro Asn Lys
               820                 825                 830
   Tyr Tyr Glu Pro Arg Asp Arg Tyr Phe Gln Gln Tyr Met Leu Lys Gly
           835                 840                 845
   Glu Trp Gln Tyr Trp Phe Asp Leu Asp Ser Val Asp His His Lys Asp
       850                 855                 860
   Tyr Phe Ser Glu Phe Ile Ile Ile Ala Val Val Ala Leu Leu Gly Gly
   865                 870                 875                 880
   Lys Tyr Val Leu Trp Leu Leu Ile Thr Tyr Thr Ile Leu Ser Glu Gln
                   885                 890                 895
   Met Ala Met Gly Ala Gly Val Asn Thr Glu Glu Ile Val Met Ile Gly
               900                 905                 910
   Asn Leu Leu Thr Asp Ser Asp Ile Glu Val Val Val Tyr Phe Leu Leu
           915                 920                 925
   Leu Tyr Leu Ile Val Lys Glu Glu Leu Ala Arg Lys Trp Ile Ile Leu
       930                 935                 940
   Val Tyr His Ile Leu Val Ala Asn Pro Met Lys Thr Ile Gly Val Val
   945                 950                 955                 960
   Leu Leu Met Leu Gly Gly Val Val Lys Ala Ser Arg Ile Asn Ala Asp
                   965                 970                 975
   Asp Gln Ser Ala Met Asp Pro Cys Phe Leu Leu Val Thr Gly Val Val
               980                 985                 990
   Ala Val Leu Met Ile Ala Arg Arg  Glu Pro Ala Thr Leu  Pro Leu Ile
           995                 1000                1005
   Val Ala  Leu Leu Ala Ile Arg  Thr Ser Gly Phe Leu  Leu Pro Ala
       1010                1015                1020
   Ser Ile  Asp Val Thr Val Ala  Val Val Leu Ile Val  Leu Leu Leu
       1025                1030                1035
   Ala Ser  Tyr Ile Thr Asp Tyr  Phe Arg Tyr Lys Lys  Trp Leu Gln
       1040                1045                1050
   Leu Leu Phe Ser Leu Ile Ala Gly Ile Phe Ile Ile Arg Ser Leu
   1055 1060 1065
   Lys His Ile Asn Gln Met Glu Val Pro Glu Ile Ser Met Pro Ser
   1070 1075 1080
   Trp Arg Pro Leu Ala Leu Val Leu Phe Tyr Ile Thr Ser Thr Ala
   1085 1090 1095
   Ile Thr Thr Asn Trp Asp Ile Asp Leu Ala Gly Phe Leu Leu Gln
   1100 1105 1110
   Trp Ala Pro Ala Val Ile Met Met Ala Thr Met Trp Ala Asp Phe
   1115 1120 1125
   Leu Thr Leu Ile Ile Val Leu Pro Ser Tyr Glu Leu Ser Lys Leu
   1130 1135 1140
   Tyr Phe Leu Lys Asn Val Arg Thr Asp Val Glu Lys Asn Trp Leu
   1145 1150 1155
   Gly Lys Val Lys Tyr Arg Gln Ile Ser Ser Val Tyr Asp Ile Cys
   1160 1165 1170
   Asp Ser Glu Glu Ala Val Tyr Leu Phe Pro Ser Arg His Lys Ser
   1175 1180 1185
   Gly Ser Arg Pro Asp Phe Ile Leu Pro Phe Leu Lys Ala Val Leu
   1190 1195 1200
   Ile Ser Cys Ile Ser Ser Gln Trp Gln Val Val Tyr Ile Ser Tyr
   1205 1210 1215
   Leu Ile Leu Glu Ile Thr Tyr Tyr Met His Arg Lys Ile Ile Asp
   1220 1225 1230
   Glu Val Ser Gly Gly Ala Asn Phe Leu Ser Arg Leu Ile Ala Ala
   1235 1240 1245
   Ile Ile Glu Leu Asn Trp Ala Ile Asp Asp Glu Glu Cys Lys Gly
   1250 1255 1260
   Leu Lys Lys Leu Tyr Leu Leu Ser Gly Arg Ala Lys Asn Leu Ile
   1265 1270 1275
   Val Lys His Lys Val Arg Asn Glu Ala Val His Arg Trp Phe Gly
   1280 1285 1290
   Glu Glu  Glu Ile Tyr Gly Ala  Pro Lys Val Ile Thr  Ile Ile Lys
       1295                 1300                 1305
   Ala Ser  Thr Leu Ser Lys Asn  Arg His Cys Ile Ile  Cys Thr Ile
       1310                 1315                 1320
   Cys Glu  Gly Lys Glu Trp Asn  Gly Ala Asn Cys Pro  Lys Cys Gly
       1325                 1330                 1335
   Arg Gln  Gly Lys Pro Ile Thr  Cys Gly Met Thr Leu  Ala Asp Phe
       1340                 1345                 1350
   Glu Glu  Lys His Tyr Lys Lys  Ile Phe Ile Arg Glu  Glu Ser Ser
       1355                 1360                 1365
   Cys Pro  Val Pro Phe Asp Pro  Ser Cys His Cys Asn  Tyr Phe Arg
       1370                 1375                 1380
   His Asp  Gly Pro Phe Arg Lys  Glu Tyr Lys Gly Tyr  Val Gln Tyr
       1385                 1390                 1395
   Thr Ala  Arg Gly Gln Leu Phe  Leu Arg Asn Leu Pro  Ile Leu Ala
       1400                 1405                 1410
   Thr Lys  Met Lys Leu Leu Met  Val Gly Asn Leu Gly  Ala Glu Ile
       1415                 1420                 1425
   Gly Asp  Leu Glu His Leu Gly  Trp Val Leu Arg Gly  Pro Ala Val
       1430                 1435                 1440
   Cys Lys  Lys Ile Thr Asn His  Glu Lys Cys His Val  Asn Ile Met
       1445                 1450                 1455
   Asp Lys  Leu Thr Ala Phe Phe  Gly Ile Met Pro Arg  Gly Thr Thr
       1460                 1465                 1470
   Pro Arg  Ala Pro Val Arg Phe  Pro Thr Ala Leu Leu  Lys Val Arg
       1475                 1480                 1485
   Arg Gly  Leu Glu Thr Gly Trp  Ala Tyr Thr His Gln  Gly Gly Ile
       1490                 1495                 1500
   Ser Ser  Val Asp His Val Thr  Ala Gly Lys Asp Leu  Leu Val Cys
       1505                 1510                 1515
   Asp Ser  Met Gly Arg Thr Arg  Val Val Cys His Ser  Asn Asn Lys
   1520 1525 1530
   Met Thr Asp Glu Thr Glu Tyr Gly Ile Lys Thr Asp Ser Gly Cys
   1535 1540 1545
   Pro Glu Gly Ala Arg Cys Tyr Val Leu Asn Pro Glu Ala Val Asn
   1550 1555 1560
   Ile Ser Gly Thr Lys Gly Ala Met Val His Leu Gln Lys Thr Gly
   1565 1570 1575
   Gly Glu Phe Thr Cys Val Thr Ala Ser Gly Thr Pro Ala Phe Phe
   1580 1585 1590
   Asp Leu Lys Asn Leu Lys Gly Trp Ser Gly Leu Pro Ile Phe Glu
   1595 1600 1605
   Ala Ser Ser Gly Arg Val Val Gly Arg Val Lys Val Gly Lys Asn
   1610 1615 1620
   Glu Asp Ser Lys Pro Thr Lys Leu Met Ser Gly Ile Gln Thr Val
   1625 1630 1635
   Ser Lys Asn Gln Thr Asp Leu Ala Asp Ile Val Lys Lys Leu Thr
   1640 1645 1650
   Ser Met Asn Arg Gly Glu Phe Lys Gln Ile Thr Leu Ala Thr Gly
   1655 1660 1665
   Ala Gly Lys Thr Thr Glu Leu Pro Arg Ser Val Ile Glu Glu Ile
   1670 1675 1680
   Gly Arg His Lys Arg Val Leu Val Leu Ile Pro Leu Arg Ala Ala
   1685 1690 1695
   Ala Glu Ser Val Tyr Gln Tyr Met Arg Val Lys Tyr Pro Ser Ile
   1700 1705 1710
   Ser Phe Asn Leu Arg Ile Gly Asp Met Lys Glu Gly Asp Met Ala
   1715 1720 1725
   Thr Gly Ile Thr Tyr Ala Ser Tyr Gly Tyr Phe Cys Gln Leu Pro
   1730 1735 1740
   Gln Pro Lys Leu Arg Ala Ala Met Val Glu Tyr Ser Tyr Ile Phe
   1745 1750 1755
   Leu Asp Glu Tyr His Cys Ala Thr Pro Glu Gln Leu Ala Ile Ile
   1760 1765 1770
   Gly Lys Ile His Arg Phe Ala Glu Asn Leu Arg Val Val Ala Met
   1775 1780 1785
   Thr Ala Thr Pro Ala Gly Thr Val Thr Thr Thr Gly Gln Lys His
   1790 1795 1800
   Pro Ile Glu Glu Phe Ile Ala Pro Glu Val Met Lys Gly Glu Asp
   1805 1810 1815
   Leu Gly Ser Glu Tyr Leu Asp Ile Ala Gly Leu Lys Ile Pro Thr
   1820 1825 1830
   Glu Glu Met Lys Gly Asn Met Leu Val Phe Ala Pro Thr Arg Asn
   1835 1840 1845
   Met Ala Val Glu Thr Ala Lys Lys Leu Lys Ala Lys Gly Tyr Asn
   1850 1855 1860
   Ser Gly Tyr Tyr Tyr Ser Gly Glu Asn Pro Glu Asn Leu Arg Val
   1865 1870 1875
   Val Thr Ser Gln Ser Pro Tyr Val Val Val Ala Thr Asn Ala Ile
   1880 1885 1890
   Glu Ser Gly Val Thr Leu Pro Asp Leu Asp Thr Val Val Asp Thr
   1895 1900 1905
   Gly Leu Lys Cys Glu Lys Arg Val Arg Ile Ser Ser Lys Met Pro
   1910 1915 1920
   Phe Ile Val Thr Gly Leu Lys Arg Met Ala Val Thr Ile Gly Glu
   1925 1930 1935
   Gln Ala Gln Arg Arg Gly Arg Val Gly Arg Val Lys Pro Gly Arg
   1940 1945 1950
   Tyr Tyr Arg Ser Gln Glu Thr Ala Ser Gly Ser Lys Asp Tyr His
   1955 1960 1965
   Tyr Asp Leu Leu Gln Ala Gln Arg Tyr Gly Ile Glu Asp Gly Ile
   1970 1975 1980
   Asn Val Thr Lys Ser Phe Arg Glu Met Asn Tyr Asp Trp Ser Leu
   1985 1990 1995
   Tyr Glu Glu Asp Ser Leu Met Ile Thr Gln Leu Glu Val Leu Asn
   2000 2005 2010
   Asn Leu Leu Ile Ser Glu Asp Leu Pro Ala Ala Val Lys Asn Ile
   2015 2020 2025
   Met Ala Arg Thr Asp His Pro Glu Pro Ile Gln Leu Ala Tyr Asn
   2030 2035 2040
   Ser Tyr Glu Asn Gln Ile Pro Val Leu Phe Pro Lys Ile Lys Asn
   2045 2050 2055
   Gly Glu Val Thr Asp Ser Tyr Glu Asn Tyr Thr Tyr Leu Asn Ala
   2060 2065 2070
   Arg Lys Leu Gly Glu Asp Val Pro Ala Tyr Val Tyr Ala Thr Glu
   2075 2080 2085
   Asp Glu Asp Leu Ala Val Asp Leu Leu Gly Met Asp Trp Pro Asp
   2090 2095 2100
   Pro Gly Asn Gln Gln Val Val Glu Thr Gly Arg Ala Leu Lys Gln
   2105 2110 2115
   Val Thr Gly Leu Ser Thr Ala Glu Asn Ala Leu Leu Ile Ala Leu
   2120 2125 2130
   Phe Gly Tyr Val Gly Tyr Gln Thr Leu Ser Lys Arg His Ile Pro
   2135 2140 2145
   Met Ile Thr Asp Ile Tyr Thr Leu Glu Asp His Arg Leu Glu Asp
   2150 2155 2160
   Thr Thr His Leu Gln Phe Ala Pro Asn Ala Ile Arg Thr Asp Gly
   2165 2170 2175
   Lys Asp Ser Glu Leu Lys Glu Leu Ala Val Gly Asp Leu Asp Lys
   2180 2185 2190
   Tyr Val Asp Ala Leu Val Asp Tyr Ser Lys Gln Gly Met Lys Phe
   2195 2200 2205
   Ile Lys Val Gln Ala Glu Lys Val Arg Asp Ser Gln Ser Thr Lys
   2210 2215 2220
   Glu Gly Leu Gln Thr Ile Lys Glu Tyr Val Asp Lys Phe Ile Gln
   2225 2230 2235
   Ser Leu  Thr Glu Asn Lys Glu  Glu Ile Ile Arg Tyr  Gly Leu Trp
       2240                 2245                 2250
   Gly Val  His Thr Ala Leu Tyr  Lys Ser Leu Ala Ala  Arg Leu Gly
       2255                 2260                 2265
   His Glu  Thr Ala Phe Ala Thr  Leu Val Val Lys Trp  Leu Ala Phe
       2270                 2275                 2280
   Gly Gly  Glu Thr Val Ser Ala  His Ile Lys Gln Val  Ala Val Asp
       2285                 2290                 2295
   Leu Val  Val Tyr Tyr Ile Ile  Asn Lys Pro Ser Phe  Pro Gly Asp
       2300                 2305                 2310
   Thr Glu  Thr Gln Gln Glu Gly  Arg Arg Phe Val Ala  Ser Leu Phe
       2315                 2320                 2325
   Ile Ser  Ala Leu Ala Thr Tyr  Thr Tyr Lys Thr Trp  Asn Tyr Asn
       2330                 2335                 2340
   Asn Leu  Gln Arg Val Val Glu  Pro Ala Leu Ala Tyr  Leu Pro Tyr
       2345                 2350                 2355
   Ala Thr  Ser Ala Leu Lys Leu  Phe Thr Pro Thr Arg  Leu Glu Ser
       2360                 2365                 2370
   Val Val  Ile Leu Ser Ser Thr  Ile Tyr Lys Thr Tyr  Leu Ser Ile
       2375                 2380                 2385
   Arg Lys  Gly Lys Ser Asp Gly  Leu Leu Gly Thr Gly  Ile Ser Ala
       2390                 2395                 2400
   Ala Met  Glu Ile Leu Asn Gln  Asn Pro Ile Ser Val  Gly Ile Ser
       2405                 2410                 2415
   Val Met  Leu Gly Val Gly Ala  Ile Ala Ala His Asn  Ala Ile Glu
       2420                 2425                 2430
   Ser Ser  Glu Gln Lys Arg Thr  Leu Leu Met Lys Val  Phe Val Lys
       2435                 2440                 2445
   Asn Phe  Leu Asp Gln Ala Ala  Thr Asp Glu Leu Val  Lys Glu Asn
       2450                 2455                 2460
   Pro Glu  Lys Ile Ile Met Ala  Leu Phe Glu Ala Val  Gln Thr Ile
   2465 2470 2475
   Gly Asn Pro Leu Arg Leu Ile Tyr His Leu Tyr Gly Val Tyr Tyr
   2480 2485 2490
   Lys Gly Trp Glu Ala Lys Glu Leu Ala Glu Lys Thr Ala Gly Arg
   2495 2500 2505
   Asn Leu Phe Thr Leu Ile Met Phe Glu Ala Phe Glu Leu Leu Gly
   2510 2515 2520
   Met Asp Ser Glu Gly Lys Ile Arg Asn Leu Ser Gly Asn Tyr Ile
   2525 2530 2535
   Leu Asp Leu Ile Phe Asn Leu His Asn Lys Leu Asn Lys Gly Leu
   2540 2545 2550
   Lys Lys Leu Val Leu Gly Trp Ala Pro Ala Pro Leu Ser Cys Asp
   2555 2560 2565
   Trp Thr Pro Ser Asp Glu Arg Ile Ser Leu Pro His Asn Asn Tyr
   2570 2575 2580
   Leu Arg Val Glu Thr Arg Cys Pro Cys Gly Tyr Glu Met Lys Ala
   2585 2590 2595
   Ile Lys Asn Val Ala Gly Lys Leu Thr Lys Val Glu Glu Lys Gly
   2600 2605 2610
   Ser Phe Leu Cys Arg Asn Arg Leu Gly Arg Gly Pro Pro Asn Phe
   2615 2620 2625
   Lys Val Thr Lys Phe Tyr Asp Asp Asn Leu Ile Glu Val Lys Pro
   2630 2635 2640
   Val Ala Arg Leu Glu Gly Gln Val Asp Leu Tyr Tyr Lys Gly Val
   2645 2650 2655
   Thr Ala Lys Leu Asp Tyr Asn Asn Gly Lys Val Leu Leu Ala Thr
   2660 2665 2670
   Asn Lys Trp Glu Val Asp His Ala Phe Leu Thr Arg Leu Val Lys
   2675 2680 2685
   Lys His Thr Gly Ile Gly Phe Lys Gly Ala Tyr Leu Gly Asp Arg
   2690 2695 2700
   Pro Asp His Gln Asp Leu Val Asp Arg Asp Cys Ala Thr Ile Thr
   2705 2710 2715
   Lys Asn Ser Val Gln Phe Leu Lys Met Lys Lys Gly Cys Ala Phe
   2720 2725 2730
   Thr Tyr Asp Leu Thr Ile Ser Asn Leu Val Arg Leu Ile Glu Leu
   2735 2740 2745
   Val His Lys Asn Asn Leu Gln Glu Arg Glu Ile Pro Thr Val Thr
   2750 2755 2760
   Val Thr Thr Trp Leu Ala Tyr Ser Phe Val Asn Glu Asp Leu Gly
   2765 2770 2775
   Thr Ile Lys Pro Val Leu Gly Glu Lys Val Ile Pro Glu Pro Pro
   2780 2785 2790
   Glu Glu Leu Ser Leu Gln Pro Thr Val Arg Leu Val Thr Thr Glu
   2795 2800 2805
   Thr Ala Ile Thr Ile Thr Gly Glu Ala Glu Val Met Thr Thr Gly
   2810 2815 2820
   Ile Thr Pro Val Val Glu Met Lys Glu Glu Pro Gln Leu Asp His
   2825 2830 2835
   Gln Ser Thr Thr Leu Lys Val Gly Leu Lys Glu Gly Glu Tyr Pro
   2840 2845 2850
   Gly Pro Gly Val Asn Pro Asn His Leu Ala Glu Val Ile Asp Glu
   2855 2860 2865
   Lys Asp Asp Arg Pro Phe Val Leu Ile Ile Gly Asn Lys Gly Ser
   2870 2875 2880
   Thr Ser Asn Arg Ala Arg Thr Ala Lys Asn Ile Arg Leu Tyr Lys
   2885 2890 2895
   Gly Asn Asn Pro Arg Glu Ile Arg Asp Leu Met Ser Gln Gly Arg
   2900 2905 2910
   Ile Leu Thr Val Ala Leu Lys Glu Leu Asp Pro Glu Leu Lys Glu
   2915 2920 2925
   Leu Val Asp Tyr Lys Gly Thr Phe Leu Asn Arg Glu Ala Leu Glu
   2930 2935 2940
   Ala Leu Ser Leu Gly Lys Pro Ile Lys Arg Lys Thr Thr Thr Ala
   2945 2950 2955
   Met Ile Arg Arg Leu Ile Glu Pro Glu Val Glu Glu Glu Leu Pro
   2960 2965 2970
   Asp Trp Phe Gln Ala Glu Glu Pro Leu Phe Leu Glu Ala Lys Ile
   2975 2980 2985
   Gln Asn Asp Leu Tyr His Leu Ile Gly Ser Val Asp Ser Ile Lys
   2990 2995 3000
   Ser Lys Ala Lys Glu Leu Gly Ala Thr Asp Asn Thr Lys Ile Val
   3005 3010 3015
   Lys Glu Val Gly Ala Arg Thr Tyr Thr Met Lys Leu Ser Ser Trp
   3020 3025 3030
   Ser Thr Gln Val Thr Lys Lys Gln Met Ser Leu Ala Pro Leu Phe
   3035 3040 3045
   Glu Glu Leu Leu Leu Lys Cys Pro Pro Cys Ser Lys Ile Ser Lys
   3050 3055 3060
   Gly His Met Val Ser Ala Tyr Gln Leu Ala Gln Gly Asn Trp Glu
   3065 3070 3075
   Pro Leu Gly Cys Gly Val Tyr Met Gly Thr Ile Pro Ala Arg Arg
   3080 3085 3090
   Leu Lys Ile His Pro Tyr Glu Ala Tyr Leu Lys Leu Lys Glu Leu
   3095 3100 3105
   Val Glu Val Glu Ser Ser Arg Ala Thr Ala Lys Glu Ser Ile Ile
   3110 3115 3120
   Arg Glu His Asn Thr Trp Ile Leu Arg Lys Val Arg His Glu Gly
   3125 3130 3135
   Asn Leu Arg Thr Lys Ser Met Ile Asn Pro Gly Lys Ile Ser Asp
   3140 3145 3150
   Gln Leu Cys Arg Asp Gly His Lys Arg Asn Ile Tyr Asn Lys Ile
   3155 3160 3165
   Ile Gly Ser Thr Met Ala Ser Ala Gly Ile Arg Leu Glu Lys Leu
   3170 3175 3180
   Pro Val Val Arg Ala Gln Thr Asp Thr Thr Ser Phe His Gln Ala
       3185                3190                3195
   Ile Arg Glu Lys Ile Asp Lys Thr Glu Asn Lys Gln Thr Pro Glu
       3200                3205                3210
   Leu His Glu Glu Leu Met Lys Val Phe Asp Cys Leu Lys Ile Pro
       3215                3220                3225
   Glu Leu Lys Glu Ser Tyr Asp Glu Val Ser Trp Glu Gln Leu Glu
       3230                3235                3240
   Ala Gly Ile Asn Arg Lys Gly Ala Ala Gly Tyr Leu Glu Ser Lys
       3245                3250                3255
   Asn Ile Gly Glu Val Leu Asp Thr Glu Lys His Ile Val Glu Gln
       3260                3265                3270
   Leu Ile Lys Asp Leu Arg Lys Gly Lys Lys Ile Arg Tyr Tyr Glu
       3275                3280                3285
   Thr Ala Ile Pro Lys Asn Glu Lys Arg Asp Val Ser Asp Asp Trp
       3290                3295                3300
   Glu Ala Gly Glu Phe Val Asp Glu Lys Lys Pro Arg Val Ile Gln
       3305                3310                3315
   Tyr Pro Asp Ala Lys Val Arg Leu Ala Ile Thr Lys Val Met Tyr
       3320                3325                3330
   Lys Trp Val Lys Gln Lys Pro Val Val Ile Pro Gly Tyr Glu Gly
       3335                3340                3345
   Lys Thr Pro Leu Phe Asp Ile Phe Asn Lys Val Lys Lys Glu Trp
       3350                3355                3360
   Asp Ser Phe Gln Asp Pro Val Ala Val Ser Phe Asp Thr Lys Ala
       3365                3370                3375
   Trp Asp Thr Gln Val Thr Ser Arg Asp Leu Met Leu Ile Lys Asp
       3380                3385                3390
   Ile Gln Lys Tyr Tyr Phe Lys Arg Ser Ile His Lys Phe Leu Asp
       3395                3400                3405
   Thr Ile Thr Glu His Met Val Glu Val Pro Val Ile Thr Ala Asp
   3410 3415 3420
   Gly Glu Val Tyr Ile Arg Asn Gly Gln Arg Gly Ser Gly Gln Pro
   3425 3430 3435
   Asp Thr Ser Ala Gly Asn Ser Met Leu Asn Val Leu Thr Met Ile
   3440 3445 3450
   Tyr Ala Phe Cys Lys Ser Thr Gly Ile Pro Tyr Arg Gly Phe Ser
   3455 3460 3465
   Arg Val Ala Arg Ile His Val Cys Gly Asp Asp Gly Phe Leu Ile
   3470 3475 3480
   Thr Glu Arg Gly Leu Gly Leu Lys Phe Ser Glu Lys Gly Met Gln
   3485 3490 3495
   Ile Leu His Glu Ala Gly Lys Pro Gln Lys Ile Thr Glu Gly Asp
   3500 3505 3510
   Lys Met Lys Val Ala Tyr Arg Phe Glu Asp Ile Glu Phe Cys Ser
   3515 3520 3525
   His Thr Pro Val Pro Val Arg Trp Ala Asp Asn Thr Ser Ser Tyr
   3530 3535 3540
   Met Ala Gly Arg Ser Thr Ala Thr Ile Leu Ala Lys Met Ala Thr
   3545 3550 3555
   Arg Leu Asp Ser Ser Gly Glu Arg Gly Ser Thr Ala Tyr Glu Lys
   3560 3565 3570
   Ala Val Ala Phe Ser Phe Leu Leu Met Tyr Ser Trp Asn Pro Val
   3575 3580 3585
   Val Arg Arg Ile Cys Leu Leu Val Leu Ser Gln Phe Pro Glu Ile
   3590 3595 3600
   Ser Pro Ser Lys Asn Thr Ile Tyr Tyr Tyr Gln Gly Asp Pro Ile
   3605 3610 3615
   Ala Ala Tyr Arg Glu Val Ile Gly Lys Gln Leu Cys Glu Leu Lys
   3620 3625 3630
   Arg Thr Gly Phe Glu Lys Leu Ala Gly Leu Asn Leu Ser Met Thr
   3635 3640 3645
   Thr Leu Gly Ile Trp Thr Lys His Thr Ser Lys Arg Leu Ile Gln
   3650 3655 3660
   Ala Cys Val Glu Ile Gly Lys Arg Glu Gly Thr Trp Leu Val Asn
   3665 3670 3675
   Ala Asp Arg Leu Ile Ala Gly Lys Thr Gly Lys Phe Tyr Ile Pro
   3680 3685 3690
   Ser Thr Gly Val Thr Leu Leu Gly Lys His Tyr Glu Glu Ile Asn
   3695 3700 3705
   Leu Lys Gln Lys Ala Ala Gln Pro Pro Ile Glu Gly Val Asp Arg
   3710 3715 3720
   Tyr Lys Leu Gly Pro Ile Val Asn Val Ile Leu Arg Arg Leu Arg
   3725 3730 3735
   Val Met Leu Met Thr Val Ala Ser Gly Ser Trp
   3740 3745
<210> 7
   <211> 3912
   <212> PRT
   <213> Artificial Sequence: Mutated BVDV: XIKE-B
<400> 7
   Met Glu Leu Phe Ser Asn Glu Leu Leu Tyr Lys Thr Tyr Lys Gln Lys
   1 5 10 15
   Pro Ala Gly Val Val Glu Pro Val Tyr Asp Val Asn Gly Arg Pro Leu
   20 25 30
   Phe Gly Glu Ser Ser Asp Leu His Pro Gln Ser Thr Leu Lys Leu Pro
   35 40 45
   His Gln Arg Gly Ser Ala Asn Ile Leu Thr Asn Ala Arg Ser Leu Pro
   50 55 60
   Arg Lys Gly Asp Cys Arg Arg Gly Asn Val Tyr Gly Pro Val Ser Gly
   65 70 75 80
   Ile Tyr Ile Lys Pro Gly Pro Ile Tyr Tyr Gln Asp Tyr Val Gly Pro
   85 90 95
   Val Tyr His Arg Ala Pro Leu Glu Leu Cys Arg Glu Ala Ser Met Cys
   100 105 110
   Glu Thr Thr Arg Arg Val Gly Arg Val Thr Gly Ser Asp Gly Lys Leu
           115                 120                 125
   Tyr His Ile Tyr Ile Cys Ile Asp Gly Cys Ile Leu Leu Lys Arg Ala
       130                 135                 140
   Thr Arg Asn Gln Pro Glu Val Leu Lys Trp Val Tyr Asn Arg Leu Asn
   145                 150                 155                 160
   Cys Pro Leu Trp Val Thr Ser Cys Ser Asp Glu Gly Ser Lys Gly Ala
                   165                 170                 175
   Thr Ser Lys Lys Gln Pro Lys Pro Asp Arg Ile Glu Lys Gly Lys Met
               180                 185                 190
   Lys Ile Ala Pro Lys Glu Thr Glu Lys Asp Cys Lys Thr Arg Pro Pro
           195                 200                 205
   Asp Ala Thr Ile Val Val Glu Gly Val Lys Tyr Gln Val Lys Lys Lys
       210                 215                 220
   Gly Lys Val Arg Gly Lys Asn Thr Gln Asp Gly Leu Tyr His Asn Lys
   225                 230                 235                 240
   Asn Lys Pro Pro Glu Ser Arg Lys Lys Leu Glu Lys Ala Leu Leu Ala
                   245                 250                 255
   Trp Ala Ile Leu Ala Ala Val Leu Leu Gln Leu Val Thr Gly Glu Asn
               260                 265                 270
   Ile Thr Gln Trp Asn Leu Met Asp Asn Gly Thr Glu Gly Ile Gln Gln
           275                 280                 285
   Ala Met Phe Leu Arg Gly Val Asn Arg Ser Leu His Gly Ile Trp Pro
       290                 295                 300
   Glu Lys Ile Cys Thr Gly Val Pro Thr His Leu Ala Thr Asp Tyr Glu
   305                 310                 315                 320
   Leu Lys Glu Ile Val Gly Met Met Asp Ala Ser Glu Lys Thr Asn Tyr
                   325                 330                 335
   Thr Cys Cys Arg Leu Gln Arg His Glu Trp Asn Lys Gly Trp Cys Asn
               340                 345                 350
   Trp Phe His Ile Glu Pro Trp Ile Trp Leu Met Asn Lys Thr Gln Asn
           355                 360                 365
   Asn Leu Thr Glu Gly Gln Pro Leu Arg Glu Cys Ala Val Thr Cys Arg
       370                 375                 380
   Tyr Asp Lys Glu Thr Glu Leu Asn Ile Val Thr Gln Ala Arg Asp Arg
   385                 390                 395                 400
   Pro Thr Thr Leu Thr Gly Cys Lys Lys Gly Lys Asn Phe Ser Phe Ala
                   405                 410                 415
   Gly Val Ile Leu Asp Gly Pro Cys Asn Phe Lys Val Ser Val Glu Asp
               420                 425                 430
   Val Leu Phe Lys Glu His Asp Cys Gly Asn Met Leu Gln Glu Thr Ala
           435                 440                 445
   Ile Gln Leu Leu Asp Gly Ala Thr Asn Thr Ile Glu Gly Ala Arg Val
       450                 455                 460
   Gly Thr Ala Lys Leu Thr Thr Trp Leu Gly Lys Gln Leu Gly Ile Leu
   465                 470                 475                 480
   Gly Lys Lys Leu Glu Asn Lys Ser Lys Ala Trp Phe Gly Ala His Ala
                   485                 490                 495
   Ala Ser Pro Tyr Cys Gly Val Glu Arg Lys Ile Gly Tyr Val Trp Tyr
               500                 505                 510
   Thr Lys Asn Cys Thr Pro Ala Cys Leu Pro Arg Asn Thr Arg Ile Ile
           515                 520                 525
   Gly Pro Gly Lys Phe Asp Thr Asn Ala Glu Asp Gly Lys Ile Leu His
       530                 535                 540
   Glu Met Gly Gly His Leu Ser Glu Phe Val Leu Leu Ser Leu Val Val
   545                 550                 555                 560
   Leu Ser Asp Phe Ala Pro Glu Thr Ala Ser Val Ile Tyr Leu Val Leu
                   565                 570                 575
   His Phe Ala Ile Pro Gln Ser His Val Asp Val Asp Thr Cys Asp Lys
               580                 585                 590
   Asn Gln Leu Asn Leu Thr Val Ala Thr Thr Val Ala Glu Val Ile Pro
           595                 600                 605
   Gly Thr Val Trp Asn Leu Gly Lys Tyr Val Cys Ile Arg Pro Asp Trp
       610                 615                 620
   Trp Pro Tyr Glu Thr Thr Thr Val Phe Val Ile Glu Glu Ala Gly Gln
   625                 630                 635                 640
   Val Ile Lys Leu Met Leu Arg Ala Ile Arg Asp Leu Thr Arg Ile Trp
                   645                 650                 655
   Asn Ala Ala Thr Thr Thr Ala Phe Leu Ile Phe Leu Val Lys Ala Leu
               660                 665                 670
   Arg Gly Gln Leu Ile Gln Gly Leu Leu Trp Leu Met Leu Ile Thr Gly
           675                 680                 685
   Ala Gln Gly Phe Pro Glu Cys Lys Glu Gly Phe Gln Tyr Ala Ile Ser
       690                 695                 700
   Lys Asp Arg Lys Met Gly Leu Leu Gly Pro Glu Ser Leu Thr Thr Thr
   705                 710                 715                 720
   Trp His Leu Pro Thr Lys Lys Ile Val Asp Ser Met Val His Val Trp
                   725                 730                 735
   Cys Glu Gly Lys Asp Leu Lys Ile Leu Lys Met Cys Thr Lys Glu Glu
               740                 745                 750
   Arg Tyr Leu Val Ala Val His Glu Arg Ala Leu Ser Thr Ser Ala Glu
           755                 760                 765
   Phe Met Gln Ile Ser Asp Gly Thr Ile Gly Pro Asp Val Ile Asp Met
       770                 775                 780
   Pro Asp Asp Phe Glu Phe Gly Leu Cys Pro Cys Asp Ser Lys Pro Val
   785                 790                 795                 800
   Ile Lys Gly Lys Phe Asn Ala Ser Leu Leu Asn Gly Pro Ala Phe Gln
                   805                 810                 815
   Met Val Cys Pro Gln Gly Trp Thr Gly Thr Ile Glu Cys Thr Leu Ala
               820                 825                 830
   Asn Gln Asp Thr Leu Asp Thr Thr Val Ile Arg Thr Tyr Arg Arg Thr
           835                 840                 845
   Thr Pro Phe Gln Arg Arg Lys Trp Cys Thr Tyr Glu Lys Ile Ile Gly
       850                 855                 860
   Glu Asp Ile Tyr Glu Cys Ile Leu Gly Gly Asn Trp Thr Cys Ile Thr
   865                 870                 875                 880
   Gly Asp His Ser Arg Leu Lys Asp Gly Pro Ile Lys Lys Cys Lys Trp
                   885                 890                 895
   Cys Gly His Asp Phe Val Asn Ser Glu Gly Leu Pro His Tyr Pro Ile
               900                 905                 910
   Gly Lys Cys Met Leu Ile Asn Glu Ser Gly Tyr Arg Tyr Val Asp Asp
           915                 920                 925
   Thr Ser Cys Asp Arg Gly Gly Val Ala Ile Val Pro Ser Gly Thr Val
       930                 935                 940
   Lys Cys Arg Ile Gly Asn Val Thr Val Gln Val Ile Ala Thr Asn Asn
   945                 950                 955                 960
   Asp Leu Gly Pro Met Pro Cys Ser Pro Ala Glu Val Ile Ala Ser Glu
                   965                 970                 975
   Gly Pro Val Glu Lys Thr Ala Cys Thr Phe Asn Tyr Ser Arg Thr Leu
               980                 985                 990
   Pro Asn Lys Tyr Tyr Glu Pro Arg  Asp Arg Tyr Phe Gln  Gln Tyr Met
           995                 1000                 1005
   Leu Lys  Gly Glu Trp Gln Tyr  Trp Phe Asp Leu Asp  Ser Val Asp
       1010                 1015                 1020
   His His  Lys Asp Tyr Phe Ser  Glu Phe Ile Ile Ile  Ala Val Val
       1025                 1030                 1035
   Ala Leu  Leu Gly Gly Lys Tyr  Val Leu Trp Leu Leu  Ile Thr Tyr
       1040                 1045                 1050
   Thr Ile  Leu Ser Glu Gln Met  Ala Met Gly Ala Gly  Val Asn Thr
       1055                 1060                 1065
   Glu Glu  Ile Val Met Ile Gly  Asn Leu Leu Thr Asp  Ser Asp Ile
       1070                 1075                 1080
   Glu Val  Val Val Tyr Phe Leu  Leu Leu Tyr Leu Ile  Val Lys Glu
       1085                 1090                 1095
   Glu Leu  Ala Arg Lys Trp Ile  Ile Leu Val Tyr His  Ile Leu Val
       1100                 1105                 1110
   Ala Asn Pro Met Lys Thr Ile Gly Val Val Leu Leu Met Leu Gly
   1115 1120 1125
   Gly Val Val Lys Ala Ser Arg Ile Asn Ala Asp Asp Gln Ser Ala
   1130 1135 1140
   Met Asp Pro Cys Phe Leu Leu Val Thr Gly Val Val Ala Val Leu
   1145 1150 1155
   Met Ile Ala Arg Arg Glu Pro Ala Thr Leu Pro Leu Ile Val Ala
   1160 1165 1170
   Leu Leu Ala Ile Arg Thr Ser Gly Phe Leu Leu Pro Ala Ser Ile
   1175 1180 1185
   Asp Val Thr Val Ala Val Val Leu Ile Val Leu Leu Leu Ala Ser
   1190 1195 1200
   Tyr Ile Thr Asp Tyr Phe Arg Tyr Lys Lys Trp Leu Gln Leu Leu
   1205 1210 1215
   Phe Ser Leu Ile Ala Gly Ile Phe Ile Ile Arg Ser Leu Lys His
   1220 1225 1230
   Ile Asn Gln Met Glu Val Pro Glu Ile Ser Met Pro Ser Trp Arg
   1235 1240 1245
   Pro Leu Ala Leu Val Leu Phe Tyr Ile Thr Ser Thr Ala Ile Thr
   1250 1255 1260
   Thr Asn Trp Asp Ile Asp Leu Ala Gly Phe Leu Leu Gln Trp Ala
   1265 1270 1275
   Pro Ala Val Ile Met Met Ala Thr Met Trp Ala Asp Phe Leu Thr
   1280 1285 1290
   Leu Ile Ile Val Leu Pro Ser Tyr Glu Leu Ser Lys Leu Tyr Phe
   1295 1300 1305
   Leu Lys Asn Val Arg Thr Asp Val Glu Lys Asn Trp Leu Gly Lys
   1310 1315 1320
   Val Lys Tyr Arg Gln Ile Ser Ser Val Tyr Asp Ile Cys Asp Ser
   1325 1330 1335
   Glu Glu Ala Val Tyr Leu Phe Pro Ser Arg His Lys Ser Gly Ser
   1340 1345 1350
   Arg Pro Asp Phe Ile Leu Pro Phe Leu Lys Ala Val Leu Ile Ser
   1355 1360 1365
   Cys Ile Ser Ser Gln Trp Gln Val Val Tyr Ile Ser Tyr Leu Ile
   1370 1375 1380
   Leu Glu Ile Thr Tyr Tyr Met His Arg Lys Ile Ile Asp Glu Val
   1385 1390 1395
   Ser Gly Gly Ala Asn Phe Leu Ser Arg Leu Ile Ala Ala Ile Ile
   1400 1405 1410
   Glu Leu Asn Trp Ala Ile Asp Asp Glu Glu Cys Lys Gly Leu Lys
   1415 1420 1425
   Lys Leu Tyr Leu Leu Ser Gly Arg Ala Lys Asn Leu Ile Val Lys
   1430 1435 1440
   His Lys Val Arg Asn Glu Ala Val His Arg Trp Phe Gly Glu Glu
   1445 1450 1455
   Glu Ile Tyr Gly Ala Pro Lys Val Ile Thr Ile Ile Lys Ala Ser
   1460 1465 1470
   Thr Leu Ser Lys Asn Arg His Cys Ile Ile Cys Thr Ile Cys Glu
   1475 1480 1485
   Gly Lys Glu Trp Asn Gly Ala Asn Cys Pro Lys Cys Gly Arg Gln
   1490 1495 1500
   Gly Lys Pro Ile Thr Cys Gly Met Thr Leu Ala Asp Phe Glu Glu
   1505 1510 1515
   Lys His Tyr Lys Lys Ile Phe Ile Arg Glu Glu Ser Ser Cys Pro
   1520 1525 1530
   Val Pro Phe Asp Pro Ser Cys His Cys Asn Tyr Phe Arg His Asp
   1535 1540 1545
   Gly Pro Phe Arg Lys Glu Tyr Lys Gly Tyr Val Gln Tyr Thr Ala
   1550 1555 1560
   Arg Gly Gln Leu Phe Leu Arg Asn Leu Pro Ile Leu Ala Thr Lys
   1565 1570 1575
   Met Lys Leu Leu Met Val Gly Asn Leu Gly Ala Glu Ile Gly Asp
   1580 1585 1590
   Leu Glu His Leu Gly Trp Val Leu Arg Gly Pro Ala Val Cys Lys
   1595 1600 1605
   Lys Ile Thr Asn His Glu Lys Cys His Val Asn Ile Met Asp Lys
   1610 1615 1620
   Leu Thr Ala Phe Phe Gly Ile Met Pro Arg Gly Thr Thr Pro Arg
   1625 1630 1635
   Ala Pro Val Arg Phe Pro Thr Ala Leu Leu Lys Val Arg Arg Gly
   1640 1645 1650
   Leu Glu Thr Gly Trp Ala Tyr Thr His Gln Gly Gly Ile Ser Ser
   1655 1660 1665
   Val Asp His Val Thr Ala Gly Lys Asp Leu Leu Val Cys Asp Ser
   1670 1675 1680
   Met Gly Arg Thr Arg Val Val Cys His Ser Asn Asn Lys Met Thr
   1685 1690 1695
   Asp Glu Thr Glu Tyr Gly Ile Lys Thr Asp Ser Gly Cys Pro Glu
   1700 1705 1710
   Gly Ala Arg Cys Tyr Val Leu Asn Pro Glu Ala Val Asn Ile Ser
   1715 1720 1725
   Gly Thr Lys Gly Ala Met Val His Leu Gln Lys Thr Gly Gly Glu
   1730 1735 1740
   Phe Thr Cys Val Thr Ala Ser Gly Thr Pro Ala Phe Phe Asp Leu
   1745 1750 1755
   Lys Asn Leu Lys Gly Trp Ser Gly Leu Pro Ile Phe Glu Ala Ser
   1760 1765 1770
   Ser Gly Arg Val Val Gly Arg Val Lys Val Gly Lys Asn Glu Asp
   1775 1780 1785
   Ser Lys Pro Thr Lys Leu Met Ser Gly Ile Gln Thr Val Ser Lys
   1790 1795 1800
   Asn Gln Thr Asp Leu Ala Asp Ile Val Lys Lys Leu Thr Ser Met
   1805 1810 1815
   Asn Arg Gly Glu Phe Lys Gln Ile Thr Leu Ala Thr Gly Ala Gly
   1820 1825 1830
   Lys Thr Thr Glu Leu Pro Arg Ser Val Ile Glu Glu Ile Gly Arg
   1835 1840 1845
   His Lys Arg Val Leu Val Leu Ile Pro Leu Arg Ala Ala Ala Glu
   1850 1855 1860
   Ser Val Tyr Gln Tyr Met Arg Val Lys Tyr Pro Ser Ile Ser Phe
   1865 1870 1875
   Asn Leu Arg Ile Gly Asp Met Lys Glu Gly Asp Met Ala Thr Gly
   1880 1885 1890
   Ile Thr Tyr Ala Ser Tyr Gly Tyr Phe Cys Gln Leu Pro Gln Pro
   1895 1900 1905
   Lys Leu Arg Ala Ala Met Val Glu Tyr Ser Tyr Ile Phe Leu Asp
   1910 1915 1920
   Glu Tyr His Cys Ala Thr Pro Glu Gln Leu Ala Ile Ile Gly Lys
   1925 1930 1935
   Ile His Arg Phe Ala Glu Asn Leu Arg Val Val Ala Met Thr Ala
   1940 1945 1950
   Thr Pro Ala Gly Thr Val Thr Thr Thr Gly Gln Lys His Pro Ile
   1955 1960 1965
   Glu Glu Phe Ile Ala Pro Glu Val Met Lys Gly Glu Asp Leu Gly
   1970 1975 1980
   Ser Glu Tyr Leu Asp Ile Ala Gly Leu Lys Ile Pro Thr Glu Glu
   1985 1990 1995
   Met Lys Gly Asn Met Leu Val Phe Ala Pro Thr Arg Asn Met Ala
   2000 2005 2010
   Val Glu Thr Ala Lys Lys Leu Lys Ala Lys Gly Tyr Asn Ser Gly
   2015 2020 2025
   Tyr Tyr Tyr Ser Gly Glu Asn Pro Glu Asn Leu Arg Val Val Thr
   2030 2035 2040
   Ser Gln Ser Pro Tyr Val Val Val Ala Thr Asn Ala Ile Glu Ser
   2045 2050 2055
   Gly Val Thr Leu Pro Asp Leu Asp Thr Val Val Asp Thr Gly Leu
   2060 2065 2070
   Lys Cys Glu Lys Arg Val Arg Ile Ser Ser Lys Met Pro Phe Ile
   2075 2080 2085
   Val Thr Gly Leu Lys Arg Met Ala Val Thr Ile Gly Glu Gln Ala
   2090 2095 2100
   Gln Arg Arg Gly Arg Val Gly Arg Val Lys Pro Gly Arg Tyr Tyr
   2105 2110 2115
   Arg Ser Gln Glu Thr Ala Ser Gly Ser Lys Asp Tyr His Tyr Asp
   2120 2125 2130
   Leu Leu Gln Ala Gln Arg Tyr Gly Ile Glu Asp Gly Ile Asn Val
   2135 2140 2145
   Thr Lys Ser Phe Arg Glu Met Asn Tyr Asp Trp Ser Leu Tyr Glu
   2150 2155 2160
   Glu Asp Ser Leu Met Ile Thr Gln Leu Glu Val Leu Asn Asn Leu
   2165 2170 2175
   Leu Ile Ser Glu Asp Leu Pro Ala Ala Val Lys Asn Ile Met Ala
   2180 2185 2190
   Arg Thr Asp His Pro Glu Pro Ile Gln Leu Ala Tyr Asn Ser Tyr
   2195 2200 2205
   Glu Asn Gln Ile Pro Val Leu Phe Pro Lys Ile Lys Asn Gly Glu
   2210 2215 2220
   Val Thr Asp Ser Tyr Glu Asn Tyr Thr Tyr Leu Asn Ala Arg Lys
   2225 2230 2235
   Leu Gly Glu Asp Val Pro Ala Tyr Val Tyr Ala Thr Glu Asp Glu
   2240 2245 2250
   Asp Leu Ala Val Asp Leu Leu Gly Met Asp Trp Pro Asp Pro Gly
   2255 2260 2265
   Asn Gln Gln Val Val Glu Thr Gly Arg Ala Leu Lys Gln Val Thr
   2270 2275 2280
   Gly Leu Ser Thr Ala Glu Asn Ala Leu Leu Ile Ala Leu Phe Gly
   2285 2290 2295
   Tyr Val Gly Tyr Gln Thr Leu Ser Lys Arg His Ile Pro Met Ile
   2300 2305 2310
   Thr Asp Ile Tyr Thr Leu Glu Asp His Arg Leu Glu Asp Thr Thr
   2315 2320 2325
   His Leu Gln Phe Ala Pro Asn Ala Ile Arg Thr Asp Gly Lys Asp
   2330 2335 2340
   Ser Glu Leu Lys Glu Leu Ala Val Gly Asp Leu Asp Lys Tyr Val
   2345 2350 2355
   Asp Ala Leu Val Asp Tyr Ser Lys Gln Gly Met Lys Phe Ile Lys
   2360 2365 2370
   Val Gln Ala Glu Lys Val Arg Asp Ser Gln Ser Thr Lys Glu Gly
   2375 2380 2385
   Leu Gln Thr Ile Lys Glu Tyr Val Asp Lys Phe Ile Gln Ser Leu
   2390 2395 2400
   Thr Glu Asn Lys Glu Glu Ile Ile Arg Tyr Gly Leu Trp Gly Val
   2405 2410 2415
   His Thr Ala Leu Tyr Lys Ser Leu Ala Ala Arg Leu Gly His Glu
   2420 2425 2430
   Thr Ala Phe Ala Thr Leu Val Val Lys Trp Leu Ala Phe Gly Gly
   2435 2440 2445
   Glu Thr Val Ser Ala His Ile Lys Gln Val Ala Val Asp Leu Val
   2450 2455 2460
   Val Tyr Tyr Ile Ile Asn Lys Pro Ser Phe Pro Gly Asp Thr Glu
   2465 2470 2475
   Thr Gln Gln Glu Gly Arg Arg Phe Val Ala Ser Leu Phe Ile Ser
   2480 2485 2490
   Ala Leu Ala Thr Tyr Thr Tyr Lys Thr Trp Asn Tyr Asn Asn Leu
   2495 2500 2505
   Gln Arg Val Val Glu Pro Ala Leu Ala Tyr Leu Pro Tyr Ala Thr
   2510 2515 2520
   Ser Ala Leu Lys Leu Phe Thr Pro Thr Arg Leu Glu Ser Val Val
   2525 2530 2535
   Ile Leu Ser Ser Thr Ile Tyr Lys Thr Tyr Leu Ser Ile Arg Lys
   2540 2545 2550
   Gly Lys Ser Asp Gly Leu Leu Gly Thr Gly Ile Ser Ala Ala Met
   2555 2560 2565
   Glu Ile Leu Asn Gln Asn Pro Ile Ser Val Gly Ile Ser Val Met
   2570 2575 2580
   Leu Gly Val Gly Ala Ile Ala Ala His Asn Ala Ile Glu Ser Ser
   2585 2590 2595
   Glu Gln Lys Arg Thr Leu Leu Met Lys Val Phe Val Lys Asn Phe
   2600 2605 2610
   Leu Asp Gln Ala Ala Thr Asp Glu Leu Val Lys Glu Asn Pro Glu
   2615 2620 2625
   Lys Ile Ile Met Ala Leu Phe Glu Ala Val Gln Thr Ile Gly Asn
   2630 2635 2640
   Pro Leu Arg Leu Ile Tyr His Leu Tyr Gly Val Tyr Tyr Lys Gly
   2645 2650 2655
   Trp Glu Ala Lys Glu Leu Ala Glu Lys Thr Ala Gly Arg Asn Leu
   2660 2665 2670
   Phe Thr Leu Ile Met Phe Glu Ala Phe Glu Leu Leu Gly Met Asp
   2675 2680 2685
   Ser Glu Gly Lys Ile Arg Asn Leu Ser Gly Asn Tyr Ile Leu Asp
   2690 2695 2700
   Leu Ile Phe Asn Leu His Asn Lys Leu Asn Lys Gly Leu Lys Lys
   2705 2710 2715
   Leu Val Leu Gly Trp Ala Pro Ala Pro Leu Ser Cys Asp Trp Thr
   2720 2725 2730
   Pro Ser Asp Glu Arg Ile Ser Leu Pro His Asn Asn Tyr Leu Arg
   2735 2740 2745
   Val Glu Thr Arg Cys Pro Cys Gly Tyr Glu Met Lys Ala Ile Lys
   2750 2755 2760
   Asn Val Ala Gly Lys Leu Thr Lys Val Glu Glu Lys Gly Ser Phe
   2765 2770 2775
   Leu Cys Arg Asn Arg Leu Gly Arg Gly Pro Pro Asn Phe Lys Val
   2780 2785 2790
   Thr Lys Phe Tyr Asp Asp Asn Leu Ile Glu Val Lys Pro Val Ala
   2795 2800 2805
   Arg Leu Glu Gly Gln Val Asp Leu Tyr Tyr Lys Gly Val Thr Ala
   2810 2815 2820
   Lys Leu Asp Tyr Asn Asn Gly Lys Val Leu Leu Ala Thr Asn Lys
   2825 2830 2835
   Trp Glu Val Asp His Ala Phe Leu Thr Arg Leu Val Lys Lys His
   2840 2845 2850
   Thr Gly Ile Gly Phe Lys Gly Ala Tyr Leu Gly Asp Arg Pro Asp
   2855 2860 2865
   His Gln Asp Leu Val Asp Arg Asp Cys Ala Thr Ile Thr Lys Asn
   2870 2875 2880
   Ser Val Gln Phe Leu Lys Met Lys Lys Gly Cys Ala Phe Thr Tyr
   2885 2890 2895
   Asp Leu Thr Ile Ser Asn Leu Val Arg Leu Ile Glu Leu Val His
   2900 2905 2910
   Lys Asn Asn Leu Gln Glu Arg Glu Ile Pro Thr Val Thr Val Thr
   2915 2920 2925
   Thr Trp Leu Ala Tyr Ser Phe Val Asn Glu Asp Leu Gly Thr Ile
   2930 2935 2940
   Lys Pro Val Leu Gly Glu Lys Val Ile Pro Glu Pro Pro Glu Glu
   2945 2950 2955
   Leu Ser Leu Gln Pro Thr Val Arg Leu Val Thr Thr Glu Thr Ala
   2960 2965 2970
   Ile Thr Ile Thr Gly Glu Ala Glu Val Met Thr Thr Gly Ile Thr
   2975 2980 2985
   Pro Val Val Glu Met Lys Glu Glu Pro Gln Leu Asp His Gln Ser
   2990 2995 3000
   Thr Thr  Leu Lys Val Gly Leu  Lys Glu Gly Glu Tyr  Pro Gly Pro
       3005                 3010                 3015
   Gly Val  Asn Pro Asn His Leu  Ala Glu Val Ile Asp  Glu Lys Asp
       3020                 3025                 3030
   Asp Arg  Pro Phe Val Leu Ile  Ile Gly Asn Lys Gly  Ser Thr Ser
       3035                 3040                 3045
   Asn Arg  Ala Arg Thr Ala Lys  Asn Ile Arg Leu Tyr  Lys Gly Asn
       3050                 3055                 3060
   Asn Pro  Arg Glu Ile Arg Asp  Leu Met Ser Gln Gly  Arg Ile Leu
       3065                 3070                 3075
   Thr Val  Ala Leu Lys Glu Leu  Asp Pro Glu Leu Lys  Glu Leu Val
       3080                 3085                 3090
   Asp Tyr  Lys Gly Thr Phe Leu  Asn Arg Glu Ala Leu  Glu Ala Leu
       3095                 3100                 3105
   Ser Leu  Gly Lys Pro Ile Lys  Arg Lys Thr Thr Thr  Ala Met Ile
       3110                 3115                 3120
   Arg Arg  Leu Ile Glu Pro Glu  Val Glu Glu Glu Leu  Pro Asp Trp
       3125                 3130                 3135
   Phe Gln  Ala Glu Glu Pro Leu  Phe Leu Glu Ala Lys  Ile Gln Asn
       3140                 3145                 3150
   Asp Leu  Tyr His Leu Ile Gly  Ser Val Asp Ser Ile  Lys Ser Lys
       3155                 3160                 3165
   Ala Lys  Glu Leu Gly Ala Thr  Asp Asn Thr Lys Ile  Val Lys Glu
       3170                 3175                 3180
   Val Gly  Ala Arg Thr Tyr Thr  Met Lys Leu Ser Ser  Trp Ser Thr
       3185                 3190                 3195
   Gln Val  Thr Lys Lys Gln Met  Ser Leu Ala Pro Leu  Phe Glu Glu
       3200                 3205                 3210
   Leu Leu  Leu Lys Cys Pro Pro  Cys Ser Lys Ile Ser  Lys Gly His
       3215                 3220                 3225
   Met Val  Ser Ala Tyr Gln Leu  Ala Gln Gly Asn Trp  Glu Pro Leu
       3230                 3235                 3240
   Gly Cys Gly Val Tyr Met Gly Thr Ile Pro Ala Arg Arg Leu Lys
   3245 3250 3255
   Ile His Pro Tyr Glu Ala Tyr Leu Lys Leu Lys Glu Leu Val Glu
   3260 3265 3270
   Val Glu Ser Ser Arg Ala Thr Ala Lys Glu Ser Ile Ile Arg Glu
   3275 3280 3285
   His Asn Thr Trp Ile Leu Arg Lys Val Arg His Glu Gly Asn Leu
   3290 3295 3300
   Arg Thr Lys Ser Met Ile Asn Pro Gly Lys Ile Ser Asp Gln Leu
   3305 3310 3315
   Cys Arg Asp Gly His Lys Arg Asn Ile Tyr Asn Lys Ile Ile Gly
   3320 3325 3330
   Ser Thr Met Ala Ser Ala Gly Ile Arg Leu Glu Lys Leu Pro Val
   3335 3340 3345
   Val Arg Ala Gln Thr Asp Thr Thr Ser Phe His Gln Ala Ile Arg
   3350 3355 3360
   Glu Lys Ile Asp Lys Thr Glu Asn Lys Gln Thr Pro Glu Leu His
   3365 3370 3375
   Glu Glu Leu Met Lys Val Phe Asp Cys Leu Lys Ile Pro Glu Leu
   3380 3385 3390
   Lys Glu Ser Tyr Asp Glu Val Ser Trp Glu Gln Leu Glu Ala Gly
   3395 3400 3405
   Ile Asn Arg Lys Gly Ala Ala Gly Tyr Leu Glu Ser Lys Asn Ile
   3410 3415 3420
   Gly Glu Val Leu Asp Thr Glu Lys His Ile Val Glu Gln Leu Ile
   3425 3430 3435
   Lys Asp Leu Arg Lys Gly Lys Lys Ile Arg Tyr Tyr Glu Thr Ala
   3440 3445 3450
   Ile Pro Lys Asn Glu Lys Arg Asp Val Ser Asp Asp Trp Glu Ala
   3455 3460 3465
   Gly Glu Phe Val Asp Glu Lys Lys Pro Arg Val Ile Gln Tyr Pro
   3470 3475 3480
   Asp Ala  Lys Val Arg Leu Ala  Ile Thr Lys Val Met  Tyr Lys Trp
       3485                 3490                 3495
   Val Lys  Gln Lys Pro Val Val  Ile Pro Gly Tyr Glu  Gly Lys Thr
       3500                 3505                 3510
   Pro Leu  Phe Asp Ile Phe Asn  Lys Val Lys Lys Glu  Trp Asp Ser
       3515                 3520                 3525
   Phe Gln  Asp Pro Val Ala Val  Ser Phe Asp Thr Lys  Ala Trp Asp
       3530                 3535                 3540
   Thr Gln  Val Thr Ser Arg Asp  Leu Met Leu Ile Lys  Asp Ile Gln
       3545                 3550                 3555
   Lys Tyr  Tyr Phe Lys Arg Ser  Ile His Lys Phe Leu  Asp Thr Ile
       3560                 3565                 3570
   Thr Glu  His Met Val Glu Val  Pro Val Ile Thr Ala  Asp Gly Glu
       3575                 3580                 3585
   Val Tyr  Ile Arg Asn Gly Gln  Arg Gly Ser Gly Gln  Pro Asp Thr
       3590                 3595                 3600
   Ser Ala  Gly Asn Ser Met Leu  Asn Val Leu Thr Met  Ile Tyr Ala
       3605                 3610                 3615
   Phe Cys  Lys Ser Thr Gly Ile  Pro Tyr Arg Gly Phe  Ser Arg Val
       3620                 3625                 3630
   Ala Arg  Ile His Val Cys Gly  Asp Asp Gly Phe Leu  Ile Thr Glu
       3635                 3640                 3645
   Arg Gly  Leu Gly Leu Lys Phe  Ser Glu Lys Gly Met  Gln Ile Leu
       3650                 3655                 3660
   His Glu  Ala Gly Lys Pro Gln  Lys Ile Thr Glu Gly  Asp Lys Met
       3665                 3670                 3675
   Lys Val  Ala Tyr Arg Phe Glu  Asp Ile Glu Phe Cys  Ser His Thr
       3680                 3685                 3690
   Pro Val  Pro Val Arg Trp Ala  Asp Asn Thr Ser Ser  Tyr Met Ala
       3695                 3700                 3705
   Gly Arg  Ser Thr Ala Thr Ile  Leu Ala Lys Met Ala  Thr Arg Leu
               3710                3715                3720
   Asp Ser Ser Gly Glu Arg Gly Ser Thr Ala Tyr Glu Lys Ala Val
       3725                3730                3735
   Ala Phe Ser Phe Leu Leu Met Tyr Ser Trp Asn Pro Val Val Arg
       3740                3745                3750
   Arg Ile Cys Leu Leu Val Leu Ser Gln Phe Pro Glu Ile Ser Pro
       3755                3760                3765
   Ser Lys Asn Thr Ile Tyr Tyr Tyr Gln Gly Asp Pro Ile Ala Ala
       3770                3775                3780
   Tyr Arg Glu Val Ile Gly Lys Gln Leu Cys Glu Leu Lys Arg Thr
       3785                3790                3795
   Gly Phe Glu Lys Leu Ala Gly Leu Asn Leu Ser Met Thr Thr Leu
       3800                3805                3810
   Gly Ile Trp Thr Lys His Thr Ser Lys Arg Leu Ile Gln Ala Cys
       3815                3820                3825
   Val Glu Ile Gly Lys Arg Glu Gly Thr Trp Leu Val Asn Ala Asp
       3830                3835                3840
   Arg Leu Ile Ala Gly Lys Thr Gly Lys Phe Tyr Ile Pro Ser Thr
       3845                3850                3855
   Gly Val Thr Leu Leu Gly Lys His Tyr Glu Glu Ile Asn Leu Lys
       3860                3865                3870
   Gln Lys Ala Ala Gln Pro Pro Ile Glu Gly Val Asp Arg Tyr Lys
       3875                3880                3885
   Leu Gly Pro Ile Val Asn Val Ile Leu Arg Arg Leu Arg Val Met
       3890                3895                3900
   Leu Met Thr Val Ala Ser Gly Ser Trp
       3905                3910
<210> 8
   <211> 3748
   <212> PRT
   <213> Artificial Sequence: Mutated BVDV: XIKE-B-NdN
<400> 8
   Met Glu Leu Phe Ser Asp Glu Gly Ser Lys Gly Ala Thr Ser Lys Lys
   1 5 10 15
   Gln Pro Lys Pro Asp Arg Ile Glu Lys Gly Lys Met Lys Ile Ala Pro
   20 25 30
   Lys Glu Thr Glu Lys Asp Cys Lys Thr Arg Pro Pro Asp Ala Thr Ile
   35 40 45
   Val Val Glu Gly Val Lys Tyr Gln Val Lys Lys Lys Gly Lys Val Arg
   50 55 60
   Gly Lys Asn Thr Gln Asp Gly Leu Tyr His Asn Lys Asn Lys Pro Pro
   65 70 75 80
   Glu Ser Arg Lys Lys Leu Glu Lys Ala Leu Leu Ala Trp Ala Ile Leu
   85 90 95
   Ala Ala Val Leu Leu Gln Leu Val Thr Gly Glu Asn Ile Thr Gln Trp
   100 105 110
   Asn Leu Met Asp Asn Gly Thr Glu Gly Ile Gln Gln Ala Met Phe Leu
   115 120 125
   Arg Gly Val Asn Arg Ser Leu His Gly Ile Trp Pro Glu Lys Ile Cys
   130 135 140
   Thr Gly Val Pro Thr His Leu Ala Thr Asp Tyr Glu Leu Lys Glu Ile
   145 150 155 160
   Val Gly Met Met Asp Ala Ser Glu Lys Thr Asn Tyr Thr Cys Cys Arg
   165 170 175
   Leu Gln Arg His Glu Trp Asn Lys Gly Trp Cys Asn Trp Phe His Ile
   180 185 190
   Glu Pro Trp Ile Trp Leu Met Asn Lys Thr Gln Asn Asn Leu Thr Glu
   195 200 205
   Gly Gln Pro Leu Arg Glu Cys Ala Val Thr Cys Arg Tyr Asp Lys Glu
   210 215 220
   Thr Glu Leu Asn Ile Val Thr Gln Ala Arg Asp Arg Pro Thr Thr Leu
   225 230 235 240
   Thr Gly Cys Lys Lys Gly Lys Asn Phe Ser Phe Ala Gly Val Ile Leu
   245 250 255
   Asp Gly Pro Cys Asn Phe Lys Val Ser Val Glu Asp Val Leu Phe Lys
               260                 265                 270
   Glu His Asp Cys Gly Asn Met Leu Gln Glu Thr Ala Ile Gln Leu Leu
           275                 280                 285
   Asp Gly Ala Thr Asn Thr Ile Glu Gly Ala Arg Val Gly Thr Ala Lys
       290                 295                 300
   Leu Thr Thr Trp Leu Gly Lys Gln Leu Gly Ile Leu Gly Lys Lys Leu
   305                 310                 315                 320
   Glu Asn Lys Ser Lys Ala Trp Phe Gly Ala His Ala Ala Ser Pro Tyr
                   325                 330                 335
   Cys Gly Val Glu Arg Lys Ile Gly Tyr Val Trp Tyr Thr Lys Asn Cys
               340                 345                 350
   Thr Pro Ala Cys Leu Pro Arg Asn Thr Arg Ile Ile Gly Pro Gly Lys
           355                 360                 365
   Phe Asp Thr Asn Ala Glu Asp Gly Lys Ile Leu His Glu Met Gly Gly
       370                 375                 380
   His Leu Ser Glu Phe Val Leu Leu Ser Leu Val Val Leu Ser Asp Phe
   385                 390                 395                 400
   Ala Pro Glu Thr Ala Ser Val Ile Tyr Leu Val Leu His Phe Ala Ile
                   405                 410                 415
   Pro Gln Ser His Val Asp Val Asp Thr Cys Asp Lys Asn Gln Leu Asn
               420                 425                 430
   Leu Thr Val Ala Thr Thr Val Ala Glu Val Ile Pro Gly Thr Val Trp
           435                 440                 445
   Asn Leu Gly Lys Tyr Val Cys Ile Arg Pro Asp Trp Trp Pro Tyr Glu
       450                 455                 460
   Thr Thr Thr Val Phe Val Ile Glu Glu Ala Gly Gln Val Ile Lys Leu
   465                 470                 475                 480
   Met Leu Arg Ala Ile Arg Asp Leu Thr Arg Ile Trp Asn Ala Ala Thr
                   485                 490                 495
   Thr Thr Ala Phe Leu Ile Phe Leu Val Lys Ala Leu Arg Gly Gln Leu
               500                 505                 510
   Ile Gln Gly Leu Leu Trp Leu Met Leu Ile Thr Gly Ala Gln Gly Phe
           515                 520                 525
   Pro Glu Cys Lys Glu Gly Phe Gln Tyr Ala Ile Ser Lys Asp Arg Lys
       530                 535                 540
   Met Gly Leu Leu Gly Pro Glu Ser Leu Thr Thr Thr Trp His Leu Pro
   545                 550                 555                 560
   Thr Lys Lys Ile Val Asp Ser Met Val His Val Trp Cys Glu Gly Lys
                   565                 570                 575
   Asp Leu Lys Ile Leu Lys Met Cys Thr Lys Glu Glu Arg Tyr Leu Val
               580                 585                 590
   Ala Val His Glu Arg Ala Leu Ser Thr Ser Ala Glu Phe Met Gln Ile
           595                 600                 605
   Ser Asp Gly Thr Ile Gly Pro Asp Val Ile Asp Met Pro Asp Asp Phe
       610                 615                 620
   Glu Phe Gly Leu Cys Pro Cys Asp Ser Lys Pro Val Ile Lys Gly Lys
   625                 630                 635                 640
   Phe Asn Ala Ser Leu Leu Asn Gly Pro Ala Phe Gln Met Val Cys Pro
                   645                 650                 655
   Gln Gly Trp Thr Gly Thr Ile Glu Cys Thr Leu Ala Asn Gln Asp Thr
               660                 665                 670
   Leu Asp Thr Thr Val Ile Arg Thr Tyr Arg Arg Thr Thr Pro Phe Gln
           675                 680                 685
   Arg Arg Lys Trp Cys Thr Tyr Glu Lys Ile Ile Gly Glu Asp Ile Tyr
       690                 695                 700
   Glu Cys Ile Leu Gly Gly Asn Trp Thr Cys Ile Thr Gly Asp His Ser
   705                 710                 715                 720
   Arg Leu Lys Asp Gly Pro Ile Lys Lys Cys Lys Trp Cys Gly His Asp
                   725                 730                 735
   Phe Val Asn Ser Glu Gly Leu Pro His Tyr Pro Ile Gly Lys Cys Met
               740                 745                 750
   Leu Ile Asn Glu Ser Gly Tyr Arg Tyr Val Asp Asp Thr Ser Cys Asp
           755                 760                 765
   Arg Gly Gly Val Ala Ile Val Pro Ser Gly Thr Val Lys Cys Arg Ile
       770                 775                 780
   Gly Asn Val Thr Val Gln Val Ile Ala Thr Asn Asn Asp Leu Gly Pro
   785                 790                 795                 800
   Met Pro Cys Ser Pro Ala Glu Val Ile Ala Ser Glu Gly Pro Val Glu
                   805                 810                 815
   Lys Thr Ala Cys Thr Phe Asn Tyr Ser Arg Thr Leu Pro Asn Lys Tyr
               820                 825                 830
   Tyr Glu Pro Arg Asp Arg Tyr Phe Gln Gln Tyr Met Leu Lys Gly Glu
           835                 840                 845
   Trp Gln Tyr Trp Phe Asp Leu Asp Ser Val Asp His His Lys Asp Tyr
       850                 855                 860
   Phe Ser Glu Phe Ile Ile Ile Ala Val Val Ala Leu Leu Gly Gly Lys
   865                 870                 875                 880
   Tyr Val Leu Trp Leu Leu Ile Thr Tyr Thr Ile Leu Ser Glu Gln Met
                   885                 890                 895
   Ala Met Gly Ala Gly Val Asn Thr Glu Glu Ile Val Met Ile Gly Asn
               900                 905                 910
   Leu Leu Thr Asp Ser Asp Ile Glu Val Val Val Tyr Phe Leu Leu Leu
           915                 920                 925
   Tyr Leu Ile Val Lys Glu Glu Leu Ala Arg Lys Trp Ile Ile Leu Val
       930                 935                 940
   Tyr His Ile Leu Val Ala Asn Pro Met Lys Thr Ile Gly Val Val Leu
   945                 950                 955                 960
   Leu Met Leu Gly Gly Val Val Lys Ala Ser Arg Ile Asn Ala Asp Asp
                   965                 970                 975
   Gln Ser Ala Met Asp Pro Cys Phe Leu Leu Val Thr Gly Val Val Ala
               980                 985                 990
   Val Leu Met Ile Ala Arg Arg Glu  Pro Ala Thr Leu Pro  Leu Ile Val
           995                 1000                1005
   Ala Leu  Leu Ala Ile Arg Thr  Ser Gly Phe Leu Leu  Pro Ala Ser
   1010 1015 1020
   Ile Asp Val Thr Val Ala Val Val Leu Ile Val Leu Leu Leu Ala
   1025 1030 1035
   Ser Tyr Ile Thr Asp Tyr Phe Arg Tyr Lys Lys Trp Leu Gln Leu
   1040 1045 1050
   Leu Phe Ser Leu Ile Ala Gly Ile Phe Ile Ile Arg Ser Leu Lys
   1055 1060 1065
   His Ile Asn Gln Met Glu Val Pro Glu Ile Ser Met Pro Ser Trp
   1070 1075 1080
   Arg Pro Leu Ala Leu Val Leu Phe Tyr Ile Thr Ser Thr Ala Ile
   1085 1090 1095
   Thr Thr Asn Trp Asp Ile Asp Leu Ala Gly Phe Leu Leu Gln Trp
   1100 1105 1110
   Ala Pro Ala Val Ile Met Met Ala Thr Met Trp Ala Asp Phe Leu
   1115 1120 1125
   Thr Leu Ile Ile Val Leu Pro Ser Tyr Glu Leu Ser Lys Leu Tyr
   1130 1135 1140
   Phe Leu Lys Asn Val Arg Thr Asp Val Glu Lys Asn Trp Leu Gly
   1145 1150 1155
   Lys Val Lys Tyr Arg Gln Ile Ser Ser Val Tyr Asp Ile Cys Asp
   1160 1165 1170
   Ser Glu Glu Ala Val Tyr Leu Phe Pro Ser Arg His Lys Ser Gly
   1175 1180 1185
   Ser Arg Pro Asp Phe Ile Leu Pro Phe Leu Lys Ala Val Leu Ile
   1190 1195 1200
   Ser Cys Ile Ser Ser Gln Trp Gln Val Val Tyr Ile Ser Tyr Leu
   1205 1210 1215
   Ile Leu Glu Ile Thr Tyr Tyr Met His Arg Lys Ile Ile Asp Glu
   1220 1225 1230
   Val Ser Gly Gly Ala Asn Phe Leu Ser Arg Leu Ile Ala Ala Ile
   1235 1240 1245
   Ile Glu Leu Asn Trp Ala Ile Asp Asp Glu Glu Cys Lys Gly Leu
       1250                1255                1260
   Lys Lys Leu Tyr Leu Leu Ser Gly Arg Ala Lys Asn Leu Ile Val
       1265                1270                1275
   Lys His Lys Val Arg Asn Glu Ala Val His Arg Trp Phe Gly Glu
       1280                1285                1290
   Glu Glu Ile Tyr Gly Ala Pro Lys Val Ile Thr Ile Ile Lys Ala
       1295                1300                1305
   Ser Thr Leu Ser Lys Asn Arg His Cys Ile Ile Cys Thr Ile Cys
       1310                1315                1320
   Glu Gly Lys Glu Trp Asn Gly Ala Asn Cys Pro Lys Cys Gly Arg
       1325                1330                1335
   Gln Gly Lys Pro Ile Thr Cys Gly Met Thr Leu Ala Asp Phe Glu
       1340                1345                1350
   Glu Lys His Tyr Lys Lys Ile Phe Ile Arg Glu Glu Ser Ser Cys
       1355                1360                1365
   Pro Val Pro Phe Asp Pro Ser Cys His Cys Asn Tyr Phe Arg His
       1370                1375                1380
   Asp Gly Pro Phe Arg Lys Glu Tyr Lys Gly Tyr Val Gln Tyr Thr
       1385                1390                1395
   Ala Arg Gly Gln Leu Phe Leu Arg Asn Leu Pro Ile Leu Ala Thr
       1400                1405                1410
   Lys Met Lys Leu Leu Met Val Gly Asn Leu Gly Ala Glu Ile Gly
       1415                1420                1425
   Asp Leu Glu His Leu Gly Trp Val Leu Arg Gly Pro Ala Val Cys
       1430                1435                1440
   Lys Lys Ile Thr Asn His Glu Lys Cys His Val Asn Ile Met Asp
       1445                1450                1455
   Lys Leu Thr Ala Phe Phe Gly Ile Met Pro Arg Gly Thr Thr Pro
       1460                1465                1470
   Arg Ala Pro Val Arg Phe Pro Thr Ala Leu Leu Lys Val Arg Arg
       1475                1480                1485
   Gly Leu  Glu Thr Gly Trp Ala  Tyr Thr His Gln Gly  Gly Ile Ser
       1490                 1495                 1500
   Ser Val  Asp His Val Thr Ala  Gly Lys Asp Leu Leu  Val Cys Asp
       1505                 1510                 1515
   Ser Met  Gly Arg Thr Arg Val  Val Cys His Ser Asn  Asn Lys Met
       1520                 1525                 1530
   Thr Asp  Glu Thr Glu Tyr Gly  Ile Lys Thr Asp Ser  Gly Cys Pro
       1535                 1540                 1545
   Glu Gly  Ala Arg Cys Tyr Val  Leu Asn Pro Glu Ala  Val Asn Ile
       1550                 1555                 1560
   Ser Gly  Thr Lys Gly Ala Met  Val His Leu Gln Lys  Thr Gly Gly
       1565                 1570                 1575
   Glu Phe  Thr Cys Val Thr Ala  Ser Gly Thr Pro Ala  Phe Phe Asp
       1580                 1585                 1590
   Leu Lys  Asn Leu Lys Gly Trp  Ser Gly Leu Pro Ile  Phe Glu Ala
       1595                 1600                 1605
   Ser Ser  Gly Arg Val Val Gly  Arg Val Lys Val Gly  Lys Asn Glu
       1610                 1615                 1620
   Asp Ser  Lys Pro Thr Lys Leu  Met Ser Gly Ile Gln  Thr Val Ser
       1625                 1630                 1635
   Lys Asn  Gln Thr Asp Leu Ala  Asp Ile Val Lys Lys  Leu Thr Ser
       1640                 1645                 1650
   Met Asn  Arg Gly Glu Phe Lys  Gln Ile Thr Leu Ala  Thr Gly Ala
       1655                 1660                 1665
   Gly Lys  Thr Thr Glu Leu Pro  Arg Ser Val Ile Glu  Glu Ile Gly
       1670                 1675                 1680
   Arg His  Lys Arg Val Leu Val  Leu Ile Pro Leu Arg  Ala Ala Ala
       1685                 1690                 1695
   Glu Ser  Val Tyr Gln Tyr Met  Arg Val Lys Tyr Pro  Ser Ile Ser
       1700                 1705                 1710
   Phe Asn  Leu Arg Ile Gly Asp  Met Lys Glu Gly Asp  Met Ala Thr
       1715                 1720                 1725
   Gly Ile Thr Tyr Ala Ser Tyr Gly Tyr Phe Cys Gln Leu Pro Gln
   1730 1735 1740
   Pro Lys Leu Arg Ala Ala Met Val Glu Tyr Ser Tyr Ile Phe Leu
   1745 1750 1755
   Asp Glu Tyr His Cys Ala Thr Pro Glu Gln Leu Ala Ile Ile Gly
   1760 1765 1770
   Lys Ile His Arg Phe Ala Glu Asn Leu Arg Val Val Ala Met Thr
   1775 1780 1785
   Ala Thr Pro Ala Gly Thr Val Thr Thr Thr Gly Gln Lys His Pro
   1790 1795 1800
   Ile Glu Glu Phe Ile Ala Pro Glu Val Met Lys Gly Glu Asp Leu
   1805 1810 1815
   Gly Ser Glu Tyr Leu Asp Ile Ala Gly Leu Lys Ile Pro Thr Glu
   1820 1825 1830
   Glu Met Lys Gly Asn Met Leu Val Phe Ala Pro Thr Arg Asn Met
   1835 1840 1845
   Ala Val Glu Thr Ala Lys Lys Leu Lys Ala Lys Gly Tyr Asn Ser
   1850 1855 1860
   Gly Tyr Tyr Tyr Ser Gly Glu Asn Pro Glu Asn Leu Arg Val Val
   1865 1870 1875
   Thr Ser Gln Ser Pro Tyr Val Val Val Ala Thr Asn Ala Ile Glu
   1880 1885 1890
   Ser Gly Val Thr Leu Pro Asp Leu Asp Thr Val Val Asp Thr Gly
   1895 1900 1905
   Leu Lys Cys Glu Lys Arg Val Arg Ile Ser Ser Lys Met Pro Phe
   1910 1915 1920
   Ile Val Thr Gly Leu Lys Arg Met Ala Val Thr Ile Gly Glu Gln
   1925 1930 1935
   Ala Gln Arg Arg Gly Arg Val Gly Arg Val Lys Pro Gly Arg Tyr
   1940 1945 1950
   Tyr Arg Ser Gln Glu Thr Ala Ser Gly Ser Lys Asp Tyr His Tyr
   1955 1960 1965
   Asp Leu Leu Gln Ala Gln Arg Tyr Gly Ile Glu Asp Gly Ile Asn
   1970 1975 1980
   Val Thr Lys Ser Phe Arg Glu Met Asn Tyr Asp Trp Ser Leu Tyr
   1985 1990 1995
   Glu Glu Asp Ser Leu Met Ile Thr Gln Leu Glu Val Leu Asn Asn
   2000 2005 2010
   Leu Leu Ile Ser Glu Asp Leu Pro Ala Ala Val Lys Asn Ile Met
   2015 2020 2025
   Ala Arg Thr Asp His Pro Glu Pro Ile Gln Leu Ala Tyr Asn Ser
   2030 2035 2040
   Tyr Glu Asn Gln Ile Pro Val Leu Phe Pro Lys Ile Lys Asn Gly
   2045 2050 2055
   Glu Val Thr Asp Ser Tyr Glu Asn Tyr Thr Tyr Leu Asn Ala Arg
   2060 2065 2070
   Lys Leu Gly Glu Asp Val Pro Ala Tyr Val Tyr Ala Thr Glu Asp
   2075 2080 2085
   Glu Asp Leu Ala Val Asp Leu Leu Gly Met Asp Trp Pro Asp Pro
   2090 2095 2100
   Gly Asn Gln Gln Val Val Glu Thr Gly Arg Ala Leu Lys Gln Val
   2105 2110 2115
   Thr Gly Leu Ser Thr Ala Glu Asn Ala Leu Leu Ile Ala Leu Phe
   2120 2125 2130
   Gly Tyr Val Gly Tyr Gln Thr Leu Ser Lys Arg His Ile Pro Met
   2135 2140 2145
   Ile Thr Asp Ile Tyr Thr Leu Glu Asp His Arg Leu Glu Asp Thr
   2150 2155 2160
   Thr His Leu Gln Phe Ala Pro Asn Ala Ile Arg Thr Asp Gly Lys
   2165 2170 2175
   Asp Ser Glu Leu Lys Glu Leu Ala Val Gly Asp Leu Asp Lys Tyr
   2180 2185 2190
   Val Asp Ala Leu Val Asp Tyr Ser Lys Gln Gly Met Lys Phe Ile
   2195 2200 2205
   Lys Val Gln Ala Glu Lys Val Arg Asp Ser Gln Ser Thr Lys Glu
   2210 2215 2220
   Gly Leu Gln Thr Ile Lys Glu Tyr Val Asp Lys Phe Ile Gln Ser
   2225 2230 2235
   Leu Thr Glu Asn Lys Glu Glu Ile Ile Arg Tyr Gly Leu Trp Gly
   2240 2245 2250
   Val His Thr Ala Leu Tyr Lys Ser Leu Ala Ala Arg Leu Gly His
   2255 2260 2265
   Glu Thr Ala Phe Ala Thr Leu Val Val Lys Trp Leu Ala Phe Gly
   2270 2275 2280
   Gly Glu Thr Val Ser Ala His Ile Lys Gln Val Ala Val Asp Leu
   2285 2290 2295
   Val Val Tyr Tyr Ile Ile Asn Lys Pro Ser Phe Pro Gly Asp Thr
   2300 2305 2310
   Glu Thr Gln Gln Glu Gly Arg Arg Phe Val Ala Ser Leu Phe Ile
   2315 2320 2325
   Ser Ala Leu Ala Thr Tyr Thr Tyr Lys Thr Trp Asn Tyr Asn Asn
   2330 2335 2340
   Leu Gln Arg Val Val Glu Pro Ala Leu Ala Tyr Leu Pro Tyr Ala
   2345 2350 2355
   Thr Ser Ala Leu Lys Leu Phe Thr Pro Thr Arg Leu Glu Ser Val
   2360 2365 2370
   Val Ile Leu Ser Ser Thr Ile Tyr Lys Thr Tyr Leu Ser Ile Arg
   2375 2380 2385
   Lys Gly Lys Ser Asp Gly Leu Leu Gly Thr Gly Ile Ser Ala Ala
   2390 2395 2400
   Met Glu Ile Leu Asn Gln Asn Pro Ile Ser Val Gly Ile Ser Val
   2405 2410 2415
   Met Leu Gly Val Gly Ala Ile Ala Ala His Asn Ala Ile Glu Ser
   2420 2425 2430
   Ser Glu Gln Lys Arg Thr Leu Leu Met Lys Val Phe Val Lys Asn
   2435 2440 2445
   Phe Leu Asp Gln Ala Ala Thr Asp Glu Leu Val Lys Glu Asn Pro
   2450 2455 2460
   Glu Lys Ile Ile Met Ala Leu Phe Glu Ala Val Gln Thr Ile Gly
   2465 2470 2475
   Asn Pro Leu Arg Leu Ile Tyr His Leu Tyr Gly Val Tyr Tyr Lys
   2480 2485 2490
   Gly Trp Glu Ala Lys Glu Leu Ala Glu Lys Thr Ala Gly Arg Asn
   2495 2500 2505
   Leu Phe Thr Leu Ile Met Phe Glu Ala Phe Glu Leu Leu Gly Met
   2510 2515 2520
   Asp Ser Glu Gly Lys Ile Arg Asn Leu Ser Gly Asn Tyr Ile Leu
   2525 2530 2535
   Asp Leu Ile Phe Asn Leu His Asn Lys Leu Asn Lys Gly Leu Lys
   2540 2545 2550
   Lys Leu Val Leu Gly Trp Ala Pro Ala Pro Leu Ser Cys Asp Trp
   2555 2560 2565
   Thr Pro Ser Asp Glu Arg Ile Ser Leu Pro His Asn Asn Tyr Leu
   2570 2575 2580
   Arg Val Glu Thr Arg Cys Pro Cys Gly Tyr Glu Met Lys Ala Ile
   2585 2590 2595
   Lys Asn Val Ala Gly Lys Leu Thr Lys Val Glu Glu Lys Gly Ser
   2600 2605 2610
   Phe Leu Cys Arg Asn Arg Leu Gly Arg Gly Pro Pro Asn Phe Lys
   2615 2620 2625
   Val Thr Lys Phe Tyr Asp Asp Asn Leu Ile Glu Val Lys Pro Val
   2630 2635 2640
   Ala Arg Leu Glu Gly Gln Val Asp Leu Tyr Tyr Lys Gly Val Thr
   2645 2650 2655
   Ala Lys Leu Asp Tyr Asn Asn Gly Lys Val Leu Leu Ala Thr Asn
   2660 2665 2670
   Lys Trp Glu Val Asp His Ala Phe Leu Thr Arg Leu Val Lys Lys
   2675 2680 2685
   His Thr Gly Ile Gly Phe Lys Gly Ala Tyr Leu Gly Asp Arg Pro
   2690 2695 2700
   Asp His Gln Asp Leu Val Asp Arg Asp Cys Ala Thr Ile Thr Lys
   2705 2710 2715
   Asn Ser Val Gln Phe Leu Lys Met Lys Lys Gly Cys Ala Phe Thr
   2720 2725 2730
   Tyr Asp Leu Thr Ile Ser Asn Leu Val Arg Leu Ile Glu Leu Val
   2735 2740 2745
   His Lys Asn Asn Leu Gln Glu Arg Glu Ile Pro Thr Val Thr Val
   2750 2755 2760
   Thr Thr Trp Leu Ala Tyr Ser Phe Val Asn Glu Asp Leu Gly Thr
   2765 2770 2775
   Ile Lys Pro Val Leu Gly Glu Lys Val Ile Pro Glu Pro Pro Glu
   2780 2785 2790
   Glu Leu Ser Leu Gln Pro Thr Val Arg Leu Val Thr Thr Glu Thr
   2795 2800 2805
   Ala Ile Thr Ile Thr Gly Glu Ala Glu Val Met Thr Thr Gly Ile
   2810 2815 2820
   Thr Pro Val Val Glu Met Lys Glu Glu Pro Gln Leu Asp His Gln
   2825 2830 2835
   Ser Thr Thr Leu Lys Val Gly Leu Lys Glu Gly Glu Tyr Pro Gly
   2840 2845 2850
   Pro Gly Val Asn Pro Asn His Leu Ala Glu Val Ile Asp Glu Lys
   2855 2860 2865
   Asp Asp Arg Pro Phe Val Leu Ile Ile Gly Asn Lys Gly Ser Thr
   2870 2875 2880
   Ser Asn Arg Ala Arg Thr Ala Lys Asn Ile Arg Leu Tyr Lys Gly
   2885 2890 2895
   Asn Asn Pro Arg Glu Ile Arg Asp Leu Met Ser Gln Gly Arg Ile
           2900                2905                2910
   Leu Thr  Val Ala Leu Lys Glu  Leu Asp Pro Glu Leu  Lys Glu Leu
       2915                2920                2925
   Val Asp  Tyr Lys Gly Thr Phe  Leu Asn Arg Glu Ala  Leu Glu Ala
       2930                2935                2940
   Leu Ser  Leu Gly Lys Pro Ile  Lys Arg Lys Thr Thr  Thr Ala Met
       2945                2950                2955
   Ile Arg  Arg Leu Ile Glu Pro  Glu Val Glu Glu Glu  Leu Pro Asp
       2960                2965                2970
   Trp Phe  Gln Ala Glu Glu Pro  Leu Phe Leu Glu Ala  Lys Ile Gln
       2975                2980                2985
   Asn Asp  Leu Tyr His Leu Ile  Gly Ser Val Asp Ser  Ile Lys Ser
       2990                2995                3000
   Lys Ala  Lys Glu Leu Gly Ala  Thr Asp Asn Thr Lys  Ile Val Lys
       3005                3010                3015
   Glu Val  Gly Ala Arg Thr Tyr  Thr Met Lys Leu Ser  Ser Trp Ser
       3020                3025                3030
   Thr Gln  Val Thr Lys Lys Gln  Met Ser Leu Ala Pro  Leu Phe Glu
       3035                3040                3045
   Glu Leu  Leu Leu Lys Cys Pro  Pro Cys Ser Lys Ile  Ser Lys Gly
       3050                3055                3060
   His Met  Val Ser Ala Tyr Gln  Leu Ala Gln Gly Asn  Trp Glu Pro
       3065                3070                3075
   Leu Gly  Cys Gly Val Tyr Met  Gly Thr Ile Pro Ala  Arg Arg Leu
       3080                3085                3090
   Lys Ile  His Pro Tyr Glu Ala  Tyr Leu Lys Leu Lys  Glu Leu Val
       3095                3100                3105
   Glu Val  Glu Ser Ser Arg Ala  Thr Ala Lys Glu Ser  Ile Ile Arg
       3110                3115                3120
   Glu His  Asn Thr Trp Ile Leu  Arg Lys Val Arg His  Glu Gly Asn
       3125                3130                3135
   Leu Arg  Thr Lys Ser Met Ile  Asn Pro Gly Lys Ile  Ser Asp Gln
       3140                 3145                 3150
   Leu Cys  Arg Asp Gly His Lys  Arg Asn Ile Tyr Asn  Lys Ile Ile
       3155                 3160                 3165
   Gly Ser  Thr Met Ala Ser Ala  Gly Ile Arg Leu Glu  Lys Leu Pro
       3170                 3175                 3180
   Val Val  Arg Ala Gln Thr Asp  Thr Thr Ser Phe His  Gln Ala Ile
       3185                 3190                 3195
   Arg Glu  Lys Ile Asp Lys Thr  Glu Asn Lys Gln Thr  Pro Glu Leu
       3200                 3205                 3210
   His Glu  Glu Leu Met Lys Val  Phe Asp Cys Leu Lys  Ile Pro Glu
       3215                 3220                 3225
   Leu Lys  Glu Ser Tyr Asp Glu  Val Ser Trp Glu Gln  Leu Glu Ala
       3230                 3235                 3240
   Gly Ile  Asn Arg Lys Gly Ala  Ala Gly Tyr Leu Glu  Ser Lys Asn
       3245                 3250                 3255
   Ile Gly  Glu Val Leu Asp Thr  Glu Lys His Ile Val  Glu Gln Leu
       3260                 3265                 3270
   Ile Lys  Asp Leu Arg Lys Gly  Lys Lys Ile Arg Tyr  Tyr Glu Thr
       3275                 3280                 3285
   Ala Ile  Pro Lys Asn Glu Lys  Arg Asp Val Ser Asp  Asp Trp Glu
       3290                 3295                 3300
   Ala Gly  Glu Phe Val Asp Glu  Lys Lys Pro Arg Val  Ile Gln Tyr
       3305                 3310                 3315
   Pro Asp  Ala Lys Val Arg Leu  Ala Ile Thr Lys Val  Met Tyr Lys
       3320                 3325                 3330
   Trp Val  Lys Gln Lys Pro Val  Val Ile Pro Gly Tyr  Glu Gly Lys
       3335                 3340                 3345
   Thr Pro  Leu Phe Asp Ile Phe  Asn Lys Val Lys Lys  Glu Trp Asp
       3350                 3355                 3360
   Ser Phe  Gln Asp Pro Val Ala  Val Ser Phe Asp Thr  Lys Ala Trp
       3365                 3370                 3375
   Asp Thr Gln Val Thr Ser Arg Asp Leu Met Leu Ile Lys Asp Ile
   3380 3385 3390
   Gln Lys Tyr Tyr Phe Lys Arg Ser Ile His Lys Phe Leu Asp Thr
   3395 3400 3405
   Ile Thr Glu His Met Val Glu Val Pro Val Ile Thr Ala Asp Gly
   3410 3415 3420
   Glu Val Tyr Ile Arg Asn Gly Gln Arg Gly Ser Gly Gln Pro Asp
   3425 3430 3435
   Thr Ser Ala Gly Asn Ser Met Leu Asn Val Leu Thr Met Ile Tyr
   3440 3445 3450
   Ala Phe Cys Lys Ser Thr Gly Ile Pro Tyr Arg Gly Phe Ser Arg
   3455 3460 3465
   Val Ala Arg Ile His Val Cys Gly Asp Asp Gly Phe Leu Ile Thr
   3470 3475 3480
   Glu Arg Gly Leu Gly Leu Lys Phe Ser Glu Lys Gly Met Gln Ile
   3485 3490 3495
   Leu His Glu Ala Gly Lys Pro Gln Lys Ile Thr Glu Gly Asp Lys
   3500 3505 3510
   Met Lys Val Ala Tyr Arg Phe Glu Asp Ile Glu Phe Cys Ser His
   3515 3520 3525
   Thr Pro Val Pro Val Arg Trp Ala Asp Asn Thr Ser Ser Tyr Met
   3530 3535 3540
   Ala Gly Arg Ser Thr Ala Thr Ile Leu Ala Lys Met Ala Thr Arg
   3545 3550 3555
   Leu Asp Ser Ser Gly Glu Arg Gly Ser Thr Ala Tyr Glu Lys Ala
   3560 3565 3570
   Val Ala Phe Ser Phe Leu Leu Met Tyr Ser Trp Asn Pro Val Val
   3575 3580 3585
   Arg Arg Ile Cys Leu Leu Val Leu Ser Gln Phe Pro Glu Ile Ser
   3590 3595 3600
   Pro Ser Lys Asn Thr Ile Tyr Tyr Tyr Gln Gly Asp Pro Ile Ala
   3605 3610 3615
   Ala Tyr Arg Glu Val Ile Gly Lys Gln Leu Cys Glu Leu Lys Arg
   3620 3625 3630
   Thr Gly Phe Glu Lys Leu Ala Gly Leu Asn Leu Ser Met Thr Thr
   3635 3640 3645
   Leu Gly Ile Trp Thr Lys His Thr Ser Lys Arg Leu Ile Gln Ala
   3650 3655 3660
   Cys Val Glu Ile Gly Lys Arg Glu Gly Thr Trp Leu Val Asn Ala
   3665 3670 3675
   Asp Arg Leu Ile Ala Gly Lys Thr Gly Lys Phe Tyr Ile Pro Ser
   3680 3685 3690
   Thr Gly Val Thr Leu Leu Gly Lys His Tyr Glu Glu Ile Asn Leu
   3695 3700 3705
   Lys Gln Lys Ala Ala Gln Pro Pro Ile Glu Gly Val Asp Arg Tyr
   3710 3715 3720
   Lys Leu Gly Pro Ile Val Asn Val Ile Leu Arg Arg Leu Arg Val
   3725 3730 3735
   Met Leu Met Thr Val Ala Ser Gly Ser Trp
   3740 3745
<210> 9
   <211> 3913
   <212> PRT
   <213> Artificial sequence:XIKE-C BVDV-Sequence
<400> 9
   Met Glu Leu Phe Ser Asn Glu Leu Leu Tyr Lys Thr Tyr Lys Gln Lys
   1 5 10 15
   Pro Ala Gly Val Val Glu Pro Val Tyr Asp Val Asn Gly Arg Pro Leu
   20 25 30
   Phe Gly Glu Ser Ser Asp Leu His Pro Gln Ser Thr Leu Lys Leu Pro
   35 40 45
   His Gln Arg Gly Ser Ala Asn Ile Leu Thr Asn Ala Arg Ser Leu Pro
   50 55 60
   Arg Lys Gly Asp Cys Arg Arg Gly Asn Val Tyr Gly Pro Val Ser Gly
   65 70 75 80
   Ile Tyr Ile Lys Pro Gly Pro Ile Tyr Tyr Gln Asp Tyr Val Gly Pro
                   85                  90                  95
   Val Tyr His Arg Ala Pro Leu Glu Leu Cys Arg Glu Ala Ser Met Cys
               100                 105                 110
   Glu Thr Thr Arg Arg Val Gly Arg Val Thr Gly Ser Asp Gly Lys Leu
           115                 120                 125
   Tyr His Ile Tyr Ile Cys Ile Asp Gly Cys Ile Leu Leu Lys Arg Ala
       130                 135                 140
   Thr Arg Asn Gln Pro Glu Val Leu Lys Trp Val Tyr Asn Arg Leu Asn
   145                 150                 155                 160
   Cys Pro Leu Trp Val Thr Ser Cys Ser Asp Glu Gly Ser Lys Gly Ala
                   165                 170                 175
   Thr Ser Lys Lys Gln Pro Lys Pro Asp Arg Ile Glu Lys Gly Lys Met
               180                 185                 190
   Lys Ile Ala Pro Lys Glu Thr Glu Lys Asp Cys Lys Thr Arg Pro Pro
           195                 200                 205
   Asp Ala Thr Ile Val Val Glu Gly Val Lys Tyr Gln Val Lys Lys Lys
       210                 215                 220
   Gly Lys Val Arg Gly Lys Asn Thr Gln Asp Gly Leu Tyr His Asn Lys
   225                 230                 235                 240
   Asn Lys Pro Pro Glu Ser Arg Lys Lys Leu Glu Lys Ala Leu Leu Ala
                   245                 250                 255
   Trp Ala Ile Leu Ala Ala Val Leu Leu Gln Leu Val Thr Gly Glu Asn
               260                 265                 270
   Ile Thr Gln Trp Asn Leu Met Asp Asn Gly Thr Glu Gly Ile Gln Gln
           275                 280                 285
   Ala Met Phe Leu Arg Gly Val Asn Arg Ser Leu Leu Gly Ile Trp Pro
       290                 295                 300
   Glu Lys Ile Cys Thr Gly Val Pro Thr His Leu Ala Thr Asp Tyr Glu
   305                 310                 315                 320
   Leu Lys Glu Ile Val Gly Met Met Asp Ala Ser Glu Lys Thr Asn Tyr
                   325                 330                 335
   Thr Cys Cys Arg Leu Gln Arg His Glu Trp Asn Lys His Gly Trp Cys
               340                 345                 350
   Asn Trp Phe His Ile Glu Pro Trp Ile Trp Leu Met Asn Lys Thr Gln
           355                 360                 365
   Asn Asn Leu Thr Glu Gly Gln Pro Leu Arg Glu Cys Ala Val Thr Cys
       370                 375                 380
   Arg Tyr Asp Lys Glu Thr Glu Leu Asn Ile Val Thr Gln Ala Arg Asp
   385                 390                 395                 400
   Arg Pro Thr Thr Leu Thr Gly Cys Lys Lys Gly Lys Asn Phe Ser Phe
                   405                 410                 415
   Ala Gly Val Ile Leu Asp Gly Pro Cys Asn Phe Lys Val Ser Val Glu
               420                 425                 430
   Asp Val Leu Phe Lys Glu His Asp Cys Gly Asn Met Leu Gln Glu Thr
           435                 440                 445
   Ala Ile Gln Leu Leu Asp Gly Ala Thr Asn Thr Ile Glu Gly Ala Arg
       450                 455                 460
   Val Gly Thr Ala Lys Leu Thr Thr Trp Leu Gly Lys Gln Leu Gly Ile
   465                 470                 475                 480
   Leu Gly Lys Lys Leu Glu Asn Lys Ser Lys Ala Trp Phe Gly Ala His
                   485                 490                 495
   Ala Ala Ser Pro Tyr Cys Gly Val Glu Arg Lys Ile Gly Tyr Val Trp
               500                 505                 510
   Tyr Thr Lys Asn Cys Thr Pro Ala Cys Leu Pro Arg Asn Thr Arg Ile
           515                 520                 525
   Ile Gly Pro Gly Lys Phe Asp Thr Asn Ala Glu Asp Gly Lys Ile Leu
       530                 535                 540
   His Glu Met Gly Gly His Leu Ser Glu Phe Val Leu Leu Ser Leu Val
   545                 550                 555                 560
   Val Leu Ser Asp Phe Ala Pro Glu Thr Ala Ser Val Ile Tyr Leu Val
                   565                 570                 575
   Leu His Phe Ala Ile Pro Gln Ser His Val Asp Val Asp Thr Cys Asp
               580                 585                 590
   Lys Asn Gln Leu Asn Leu Thr Val Ala Thr Thr Val Ala Glu Val Ile
           595                 600                 605
   Pro Gly Thr Val Trp Asn Leu Gly Lys Tyr Val Cys Ile Arg Pro Asp
       610                 615                 620
   Trp Trp Pro Tyr Glu Thr Thr Thr Val Phe Val Ile Glu Glu Ala Gly
   625                 630                 635                 640
   Gln Val Ile Lys Leu Met Leu Arg Ala Ile Arg Asp Leu Thr Arg Ile
                   645                 650                 655
   Trp Asn Ala Ala Thr Thr Thr Ala Phe Leu Ile Phe Leu Val Lys Ala
               660                 665                 670
   Leu Arg Gly Gln Leu Ile Gln Gly Leu Leu Trp Leu Met Leu Ile Thr
           675                 680                 685
   Gly Ala Gln Gly Phe Pro Glu Cys Lys Glu Gly Phe Gln Tyr Ala Ile
       690                 695                 700
   Ser Lys Asp Arg Lys Met Gly Leu Leu Gly Pro Glu Ser Leu Thr Thr
   705                 710                 715                 720
   Thr Trp His Leu Pro Thr Lys Lys Ile Val Asp Ser Met Val His Val
                   725                 730                 735
   Trp Cys Glu Gly Lys Asp Leu Lys Ile Leu Lys Met Cys Thr Lys Glu
               740                 745                 750
   Glu Arg Tyr Leu Val Ala Val His Glu Arg Ala Leu Ser Thr Ser Ala
           755                 760                 765
   Glu Phe Met Gln Ile Ser Asp Gly Thr Ile Gly Pro Asp Val Ile Asp
       770                 775                 780
   Met Pro Asp Asp Phe Glu Phe Gly Leu Cys Pro Cys Asp Ser Lys Pro
   785                 790                 795                 800
   Val Ile Lys Gly Lys Phe Asn Ala Ser Leu Leu Asn Gly Pro Ala Phe
                   805                 810                 815
   Gln Met Val Cys Pro Gln Gly Trp Thr Gly Thr Ile Glu Cys Thr Leu
               820                 825                 830
   Ala Asn Gln Asp Thr Leu Asp Thr Thr Val Ile Arg Thr Tyr Arg Arg
           835                 840                 845
   Thr Thr Pro Phe Gln Arg Arg Lys Trp Cys Thr Tyr Glu Lys Ile Ile
       850                 855                 860
   Gly Glu Asp Ile Tyr Glu Cys Ile Leu Gly Gly Asn Trp Thr Cys Ile
   865                 870                 875                 880
   Thr Gly Asp His Ser Arg Leu Lys Asp Gly Pro Ile Lys Lys Cys Lys
                   885                 890                 895
   Trp Cys Gly His Asp Phe Val Asn Ser Glu Gly Leu Pro His Tyr Pro
               900                 905                 910
   Ile Gly Lys Cys Met Leu Ile Asn Glu Ser Gly Tyr Arg Tyr Val Asp
           915                 920                 925
   Asp Thr Ser Cys Asp Arg Gly Gly Val Ala Ile Val Pro Ser Gly Thr
       930                 935                 940
   Val Lys Cys Arg Ile Gly Asn Val Thr Val Gln Val Ile Ala Thr Asn
   945                 950                 955                 960
   Asn Asp Leu Gly Pro Met Pro Cys Ser Pro Ala Glu Val Ile Ala Ser
                   965                 970                 975
   Glu Gly Pro Val Glu Lys Thr Ala Cys Thr Phe Asn Tyr Ser Arg Thr
               980                 985                 990
   Leu Pro Asn Lys Tyr Tyr Glu Pro  Arg Asp Arg Tyr Phe  Gln Gln Tyr
           995                 1000                1005
   Met Leu  Lys Gly Glu Trp Gln  Tyr Trp Phe Asp Leu  Asp Ser Val
       1010                1015                1020
   Asp His  His Lys Asp Tyr Phe  Ser Glu Phe Ile Ile  Ile Ala Val
       1025                1030                1035
   Val Ala  Leu Leu Gly Gly Lys  Tyr Val Leu Trp Leu  Leu Ile Thr
       1040                1045                1050
   Tyr Thr  Ile Leu Ser Glu Gln  Met Ala Met Gly Ala  Gly Val Asn
       1055                1060                1065
   Thr Glu  Glu Ile Val Met Ile  Gly Asn Leu Leu Thr  Asp Ser Asp
       1070                1075                1080
   Ile Glu  Val Val Val Tyr Phe  Leu Leu Leu Tyr Leu  Ile Val Lys
       1085                 1090                 1095
   Glu Glu  Leu Ala Arg Lys Trp  Ile Ile Leu Val Tyr  His Ile Leu
       1100                 1105                 1110
   Val Ala  Asn Pro Met Lys Thr  Ile Gly Val Val Leu  Leu Met Leu
       1115                 1120                 1125
   Gly Gly  Val Val Lys Ala Ser  Arg Ile Asn Ala Asp  Asp Gln Ser
       1130                 1135                 1140
   Ala Met  Asp Pro Cys Phe Leu  Leu Val Thr Gly Val  Val Ala Val
       1145                 1150                 1155
   Leu Met  Ile Ala Arg Arg Glu  Pro Ala Thr Leu Pro  Leu Ile Val
       1160                 1165                 1170
   Ala Leu  Leu Ala Ile Arg Thr  Ser Gly Phe Leu Leu  Pro Ala Ser
       1175                 1180                 1185
   Ile Asp  Val Thr Val Ala Val  Val Leu Ile Val Leu  Leu Leu Ala
       1190                 1195                 1200
   Ser Tyr  Ile Thr Asp Tyr Phe  Arg Tyr Lys Lys Trp  Leu Gln Leu
       1205                 1210                 1215
   Leu Phe  Ser Leu Ile Ala Gly  Ile Phe Ile Ile Arg  Ser Leu Lys
       1220                 1225                 1230
   His Ile  Asn Gln Met Glu Val  Pro Glu Ile Ser Met  Pro Ser Trp
       1235                 1240                 1245
   Arg Pro  Leu Ala Leu Val Leu  Phe Tyr Ile Thr Ser  Thr Ala Ile
       1250                 1255                 1260
   Thr Thr  Asn Trp Asp Ile Asp  Leu Ala Gly Phe Leu  Leu Gln Trp
       1265                 1270                 1275
   Ala Pro  Ala Val Ile Met Met  Ala Thr Met Trp Ala  Asp Phe Leu
       1280                 1285                 1290
   Thr Leu  Ile Ile Val Leu Pro  Ser Tyr Glu Leu Ser  Lys Leu Tyr
       1295                 1300                 1305
   Phe Leu  Lys Asn Val Arg Thr  Asp Val Glu Lys Asn  Trp Leu Gly
   1310 1315 1320
   Lys Val Lys Tyr Arg Gln Ile Ser Ser Val Tyr Asp Ile Cys Asp
   1325 1330 1335
   Ser Glu Glu Ala Val Tyr Leu Phe Pro Ser Arg His Lys Ser Gly
   1340 1345 1350
   Ser Arg Pro Asp Phe Ile Leu Pro Phe Leu Lys Ala Val Leu Ile
   1355 1360 1365
   Ser Cys Ile Ser Ser Gln Trp Gln Val Val Tyr Ile Ser Tyr Leu
   1370 1375 1380
   Ile Leu Glu Ile Thr Tyr Tyr Met His Arg Lys Ile Ile Asp Glu
   1385 1390 1395
   Val Ser Gly Gly Ala Asn Phe Leu Ser Arg Leu Ile Ala Ala Ile
   1400 1405 1410
   Ile Glu Leu Asn Trp Ala Ile Asp Asp Glu Glu Cys Lys Gly Leu
   1415 1420 1425
   Lys Lys Leu Tyr Leu Leu Ser Gly Arg Ala Lys Asn Leu Ile Val
   1430 1435 1440
   Lys His Lys Val Arg Asn Glu Ala Val His Arg Trp Phe Gly Glu
   1445 1450 1455
   Glu Glu Ile Tyr Gly Ala Pro Lys Val Ile Thr Ile Ile Lys Ala
   1460 1465 1470
   Ser Thr Leu Ser Lys Asn Arg His Cys Ile Ile Cys Thr Ile Cys
   1475 1480 1485
   Glu Gly Lys Glu Trp Asn Gly Ala Asn Cys Pro Lys Cys Gly Arg
   1490 1495 1500
   Gln Gly Lys Pro Ile Thr Cys Gly Met Thr Leu Ala Asp Phe Glu
   1505 1510 1515
   Glu Lys His Tyr Lys Lys Ile Phe Ile Arg Glu Glu Ser Ser Cys
   1520 1525 1530
   Pro Val Pro Phe Asp Pro Ser Cys His Cys Asn Tyr Phe Arg His
   1535 1540 1545
   Asp Gly Pro Phe Arg Lys Glu Tyr Lys Gly Tyr Val Gln Tyr Thr
   1550 1555 1560
   Ala Arg Gly Gln Leu Phe Leu Arg Asn Leu Pro Ile Leu Ala Thr
   1565 1570 1575
   Lys Met Lys Leu Leu Met Val Gly Asn Leu Gly Ala Glu Ile Gly
   1580 1585 1590
   Asp Leu Glu His Leu Gly Trp Val Leu Arg Gly Pro Ala Val Cys
   1595 1600 1605
   Lys Lys Ile Thr Asn His Glu Lys Cys His Val Asn Ile Met Asp
   1610 1615 1620
   Lys Leu Thr Ala Phe Phe Gly Ile Met Pro Arg Gly Thr Thr Pro
   1625 1630 1635
   Arg Ala Pro Val Arg Phe Pro Thr Ala Leu Leu Lys Val Arg Arg
   1640 1645 1650
   Gly Leu Glu Thr Gly Trp Ala Tyr Thr His Gln Gly Gly Ile Ser
   1655 1660 1665
   Ser Val Asp His Val Thr Ala Gly Lys Asp Leu Leu Val Cys Asp
   1670 1675 1680
   Ser Met Gly Arg Thr Arg Val Val Cys His Ser Asn Asn Lys Met
   1685 1690 1695
   Thr Asp Glu Thr Glu Tyr Gly Ile Lys Thr Asp Ser Gly Cys Pro
   1700 1705 1710
   Glu Gly Ala Arg Cys Tyr Val Leu Asn Pro Glu Ala Val Asn Ile
   1715 1720 1725
   Ser Gly Thr Lys Gly Ala Met Val His Leu Gln Lys Thr Gly Gly
   1730 1735 1740
   Glu Phe Thr Cys Val Thr Ala Ser Gly Thr Pro Ala Phe Phe Asp
   1745 1750 1755
   Leu Lys Asn Leu Lys Gly Trp Ser Gly Leu Pro Ile Phe Glu Ala
   1760 1765 1770
   Ser Ser Gly Arg Val Val Gly Arg Val Lys Val Gly Lys Asn Glu
   1775 1780 1785
   Asp Ser  Lys Pro Thr Lys Leu  Met Ser Gly Ile Gln  Thr Val Ser
       1790                 1795                 1800
   Lys Asn  Gln Thr Asp Leu Ala  Asp Ile Val Lys Lys  Leu Thr Ser
       1805                 1810                 1815
   Met Asn  Arg Gly Glu Phe Lys  Gln Ile Thr Leu Ala  Thr Gly Ala
       1820                 1825                 1830
   Gly Lys  Thr Thr Glu Leu Pro  Arg Ser Val Ile Glu  Glu Ile Gly
       1835                 1840                 1845
   Arg His  Lys Arg Val Leu Val  Leu Ile Pro Leu Arg  Ala Ala Ala
       1850                 1855                 1860
   Glu Ser  Val Tyr Gln Tyr Met  Arg Val Lys Tyr Pro  Ser Ile Ser
       1865                 1870                 1875
   Phe Asn  Leu Arg Ile Gly Asp  Met Lys Glu Gly Asp  Met Ala Thr
       1880                 1885                 1890
   Gly Ile  Thr Tyr Ala Ser Tyr  Gly Tyr Phe Cys Gln  Leu Pro Gln
       1895                 1900                 1905
   Pro Lys  Leu Arg Ala Ala Met  Val Glu Tyr Ser Tyr  Ile Phe Leu
       1910                 1915                 1920
   Asp Glu  Tyr His Cys Ala Thr  Pro Glu Gln Leu Ala  Ile Ile Gly
       1925                 1930                 1935
   Lys Ile  His Arg Phe Ala Glu  Asn Leu Arg Val Val  Ala Met Thr
       1940                 1945                 1950
   Ala Thr  Pro Ala Gly Thr Val  Thr Thr Thr Gly Gln  Lys His Pro
       1955                 1960                 1965
   Ile Glu  Glu Phe Ile Ala Pro  Glu Val Met Lys Gly  Glu Asp Leu
       1970                 1975                 1980
   Gly Ser  Glu Tyr Leu Asp Ile  Ala Gly Leu Lys Ile  Pro Thr Glu
       1985                 1990                 1995
   Glu Met  Lys Gly Asn Met Leu  Val Phe Ala Pro Thr  Arg Asn Met
       2000                 2005                 2010
   Ala Val  Glu Thr Ala Lys Lys  Leu Lys Ala Lys Gly  Tyr Asn Ser
       2015                 2020                 2025
   Gly Tyr  Tyr Tyr Ser Gly Glu  Asn Pro Glu Asn Leu  Arg Val Val
       2030                 2035                 2040
   Thr Ser  Gln Ser Pro Tyr Val  Val Val Ala Thr Asn  Ala Ile Glu
       2045                 2050                 2055
   Ser Gly  Val Thr Leu Pro Asp  Leu Asp Thr Val Val  Asp Thr Gly
       2060                 2065                 2070
   Leu Lys  Cys Glu Lys Arg Val  Arg Ile Ser Ser Lys  Met Pro Phe
       2075                 2080                 2085
   Ile Val  Thr Gly Leu Lys Arg  Met Ala Val Thr Ile  Gly Glu Gln
       2090                 2095                 2100
   Ala Gln  Arg Arg Gly Arg Val  Gly Arg Val Lys Pro  Gly Arg Tyr
       2105                 2110                 2115
   Tyr Arg  Ser Gln Glu Thr Ala  Ser Gly Ser Lys Asp  Tyr His Tyr
       2120                 2125                 2130
   Asp Leu  Leu Gln Ala Gln Arg  Tyr Gly Ile Glu Asp  Gly Ile Asn
       2135                 2140                 2145
   Val Thr  Lys Ser Phe Arg Glu  Met Asn Tyr Asp Trp  Ser Leu Tyr
       2150                 2155                 2160
   Glu Glu  Asp Ser Leu Met Ile  Thr Gln Leu Glu Val  Leu Asn Asn
       2165                 2170                 2175
   Leu Leu  Ile Ser Glu Asp Leu  Pro Ala Ala Val Lys  Asn Ile Met
       2180                 2185                 2190
   Ala Arg  Thr Asp His Pro Glu  Pro Ile Gln Leu Ala  Tyr Asn Ser
       2195                 2200                 2205
   Tyr Glu  Asn Gln Ile Pro Val  Leu Phe Pro Lys Ile  Lys Asn Gly
       2210                 2215                 2220
   Glu Val  Thr Asp Ser Tyr Glu  Asn Tyr Thr Tyr Leu  Asn Ala Arg
       2225                 2230                 2235
   Lys Leu  Gly Glu Asp Val Pro  Ala Tyr Val Tyr Ala  Thr Glu Asp
       2240                 2245                 2250
   Glu Asp  Leu Ala Val Asp Leu  Leu Gly Met Asp Trp  Pro Asp Pro
   2255 2260 2265
   Gly Asn Gln Gln Val Val Glu Thr Gly Arg Ala Leu Lys Gln Val
   2270 2275 2280
   Thr Gly Leu Ser Thr Ala Glu Asn Ala Leu Leu Ile Ala Leu Phe
   2285 2290 2295
   Gly Tyr Val Gly Tyr Gln Thr Leu Ser Lys Arg His Ile Pro Met
   2300 2305 2310
   Ile Thr Asp Ile Tyr Thr Leu Glu Asp His Arg Leu Glu Asp Thr
   2315 2320 2325
   Thr His Leu Gln Phe Ala Pro Asn Ala Ile Arg Thr Asp Gly Lys
   2330 2335 2340
   Asp Ser Glu Leu Lys Glu Leu Ala Val Gly Asp Leu Asp Lys Tyr
   2345 2350 2355
   Val Asp Ala Leu Val Asp Tyr Ser Lys Gln Gly Met Lys Phe Ile
   2360 2365 2370
   Lys Val Gln Ala Glu Lys Val Arg Asp Ser Gln Ser Thr Lys Glu
   2375 2380 2385
   Gly Leu Gln Thr Ile Lys Glu Tyr Val Asp Lys Phe Ile Gln Ser
   2390 2395 2400
   Leu Thr Glu Asn Lys Glu Glu Ile Ile Arg Tyr Gly Leu Trp Gly
   2405 2410 2415
   Val His Thr Ala Leu Tyr Lys Ser Leu Ala Ala Arg Leu Gly His
   2420 2425 2430
   Glu Thr Ala Phe Ala Thr Leu Val Val Lys Trp Leu Ala Phe Gly
   2435 2440 2445
   Gly Glu Thr Val Ser Ala His Ile Lys Gln Val Ala Val Asp Leu
   2450 2455 2460
   Val Val Tyr Tyr Ile Ile Asn Lys Pro Ser Phe Pro Gly Asp Thr
   2465 2470 2475
   Glu Thr Gln Gln Glu Gly Arg Arg Phe Val Ala Ser Leu Phe Ile
   2480 2485 2490
   Ser Ala Leu Ala Thr Tyr Thr Tyr Lys Thr Trp Asn Tyr Asn Asn
   2495 2500 2505
   Leu Gln Arg Val Val Glu Pro Ala Leu Ala Tyr Leu Pro Tyr Ala
   2510 2515 2520
   Thr Ser Ala Leu Lys Leu Phe Thr Pro Thr Arg Leu Glu Ser Val
   2525 2530 2535
   Val Ile Leu Ser Ser Thr Ile Tyr Lys Thr Tyr Leu Ser Ile Arg
   2540 2545 2550
   Lys Gly Lys Ser Asp Gly Leu Leu Gly Thr Gly Ile Ser Ala Ala
   2555 2560 2565
   Met Glu Ile Leu Asn Gln Asn Pro Ile Ser Val Gly Ile Ser Val
   2570 2575 2580
   Met Leu Gly Val Gly Ala Ile Ala Ala His Asn Ala Ile Glu Ser
   2585 2590 2595
   Ser Glu Gln Lys Arg Thr Leu Leu Met Lys Val Phe Val Lys Asn
   2600 2605 2610
   Phe Leu Asp Gln Ala Ala Thr Asp Glu Leu Val Lys Glu Asn Pro
   2615 2620 2625
   Glu Lys Ile Ile Met Ala Leu Phe Glu Ala Val Gln Thr Ile Gly
   2630 2635 2640
   Asn Pro Leu Arg Leu Ile Tyr His Leu Tyr Gly Val Tyr Tyr Lys
   2645 2650 2655
   Gly Trp Glu Ala Lys Glu Leu Ala Glu Lys Thr Ala Gly Arg Asn
   2660 2665 2670
   Leu Phe Thr Leu Ile Met Phe Glu Ala Phe Glu Leu Leu Gly Met
   2675 2680 2685
   Asp Ser Glu Gly Lys Ile Arg Asn Leu Ser Gly Asn Tyr Ile Leu
   2690 2695 2700
   Asp Leu Ile Phe Asn Leu His Asn Lys Leu Asn Lys Gly Leu Lys
   2705 2710 2715
   Lys Leu Val Leu Gly Trp Ala Pro Ala Pro Leu Ser Cys Asp Trp
   2720 2725 2730
   Thr Pro  Ser Asp Glu Arg Ile  Ser Leu Pro His Asn  Asn Tyr Leu
       2735                 2740                 2745
   Arg Val  Glu Thr Arg Cys Pro  Cys Gly Tyr Glu Met  Lys Ala Ile
       2750                 2755                 2760
   Lys Asn  Val Ala Gly Lys Leu  Thr Lys Val Glu Glu  Lys Gly Ser
       2765                 2770                 2775
   Phe Leu  Cys Arg Asn Arg Leu  Gly Arg Gly Pro Pro  Asn Phe Lys
       2780                 2785                 2790
   Val Thr  Lys Phe Tyr Asp Asp  Asn Leu Ile Glu Val  Lys Pro Val
       2795                 2800                 2805
   Ala Arg  Leu Glu Gly Gln Val  Asp Leu Tyr Tyr Lys  Gly Val Thr
       2810                 2815                 2820
   Ala Lys  Leu Asp Tyr Asn Asn  Gly Lys Val Leu Leu  Ala Thr Asn
       2825                 2830                 2835
   Lys Trp  Glu Val Asp His Ala  Phe Leu Thr Arg Leu  Val Lys Lys
       2840                 2845                 2850
   His Thr  Gly Ile Gly Phe Lys  Gly Ala Tyr Leu Gly  Asp Arg Pro
       2855                 2860                 2865
   Asp His  Gln Asp Leu Val Asp  Arg Asp Cys Ala Thr  Ile Thr Lys
       2870                 2875                 2880
   Asn Ser  Val Gln Phe Leu Lys  Met Lys Lys Gly Cys  Ala Phe Thr
       2885                 2890                 2895
   Tyr Asp  Leu Thr Ile Ser Asn  Leu Val Arg Leu Ile  Glu Leu Val
       2900                 2905                 2910
   His Lys  Asn Asn Leu Gln Glu  Arg Glu Ile Pro Thr  Val Thr Val
       2915                 2920                 2925
   Thr Thr  Trp Leu Ala Tyr Ser  Phe Val Asn Glu Asp  Leu Gly Thr
       2930                 2935                 2940
   Ile Lys  Pro Val Leu Gly Glu  Lys Val Ile Pro Glu  Pro Pro Glu
       2945                 2950                 2955
   Glu Leu  Ser Leu Gln Pro Thr  Val Arg Leu Val Thr  Thr Glu Thr
       2960                 2965                 2970
   Ala Ile Thr Ile Thr Gly Glu Ala Glu Val Met Thr Thr Gly Ile
   2975 2980 2985
   Thr Pro Val Val Glu Met Lys Glu Glu Pro Gln Leu Asp His Gln
   2990 2995 3000
   Ser Thr Thr Leu Lys Val Gly Leu Lys Glu Gly Glu Tyr Pro Gly
   3005 3010 3015
   Pro Gly Val Asn Pro Asn His Leu Ala Glu Val Ile Asp Glu Lys
   3020 3025 3030
   Asp Asp Arg Pro Phe Val Leu Ile Ile Gly Asn Lys Gly Ser Thr
   3035 3040 3045
   Ser Asn Arg Ala Arg Thr Ala Lys Asn Ile Arg Leu Tyr Lys Gly
   3050 3055 3060
   Asn Asn Pro Arg Glu Ile Arg Asp Leu Met Ser Gln Gly Arg Ile
   3065 3070 3075
   Leu Thr Val Ala Leu Lys Glu Leu Asp Pro Glu Leu Lys Glu Leu
   3080 3085 3090
   Val Asp Tyr Lys Gly Thr Phe Leu Asn Arg Glu Ala Leu Glu Ala
   3095 3100 3105
   Leu Ser Leu Gly Lys Pro Ile Lys Arg Lys Thr Thr Thr Ala Met
   3110 3115 3120
   Ile Arg Arg Leu Ile Glu Pro Glu Val Glu Glu Glu Leu Pro Asp
   3125 3130 3135
   Trp Phe Gln Ala Glu Glu Pro Leu Phe Leu Glu Ala Lys Ile Gln
   3140 3145 3150
   Asn Asp Leu Tyr His Leu Ile Gly Ser Val Asp Ser Ile Lys Ser
   3155 3160 3165
   Lys Ala Lys Glu Leu Gly Ala Thr Asp Asn Thr Lys Ile Val Lys
   3170 3175 3180
   Glu Val Gly Ala Arg Thr Tyr Thr Met Lys Leu Ser Ser Trp Ser
   3185 3190 3195
   Thr Gln Val Thr Lys Lys Gln Met Ser Leu Ala Pro Leu Phe Glu
   3200 3205 3210
   Glu Leu Leu Leu Lys Cys Pro Pro Cys Ser Lys Ile Ser Lys Gly
   3215 3220 3225
   His Met Val Ser Ala Tyr Gln Leu Ala Gln Gly Asn Trp Glu Pro
   3230 3235 3240
   Leu Gly Cys Gly Val Tyr Met Gly Thr Ile Pro Ala Arg Arg Leu
   3245 3250 3255
   Lys Ile His Pro Tyr Glu Ala Tyr Leu Lys Leu Lys Glu Leu Val
   3260 3265 3270
   Glu Val Glu Ser Ser Arg Ala Thr Ala Lys Glu Ser Ile Ile Arg
   3275 3280 3285
   Glu His Asn Thr Trp Ile Leu Arg Lys Val Arg His Glu Gly Asn
   3290 3295 3300
   Leu Arg Thr Lys Ser Met Ile Asn Pro Gly Lys Ile Ser Asp Gln
   3305 3310 3315
   Leu Cys Arg Asp Gly His Lys Arg Asn Ile Tyr Asn Lys Ile Ile
   3320 3325 3330
   Gly Ser Thr Met Ala Ser Ala Gly Ile Arg Leu Glu Lys Leu Pro
   3335 3340 3345
   Val Val Arg Ala Gln Thr Asp Thr Thr Ser Phe His Gln Ala Ile
   3350 3355 3360
   Arg Glu Lys Ile Asp Lys Thr Glu Asn Lys Gln Thr Pro Glu Leu
   3365 3370 3375
   His Glu Glu Leu Met Lys Val Phe Asp Cys Leu Lys Ile Pro Glu
   3380 3385 3390
   Leu Lys Glu Ser Tyr Asp Glu Val Ser Trp Glu Gln Leu Glu Ala
   3395 3400 3405
   Gly Ile Asn Arg Lys Gly Ala Ala Gly Tyr Leu Glu Ser Lys Asn
   3410 3415 3420
   Ile Gly Glu Val Leu Asp Thr Glu Lys His Ile Val Glu Gln Leu
   3425 3430 3435
   Ile Lys  Asp Leu Arg Lys Gly  Lys Lys Ile Arg Tyr  Tyr Glu Thr
       3440                 3445                 3450
   Ala Ile  Pro Lys Asn Glu Lys  Arg Asp Val Ser Asp  Asp Trp Glu
       3455                 3460                 3465
   Ala Gly  Glu Phe Val Asp Glu  Lys Lys Pro Arg Val  Ile Gln Tyr
       3470                 3475                 3480
   Pro Asp  Ala Lys Val Arg Leu  Ala Ile Thr Lys Val  Met Tyr Lys
       3485                 3490                 3495
   Trp Val  Lys Gln Lys Pro Val  Val Ile Pro Gly Tyr  Glu Gly Lys
       3500                 3505                 3510
   Thr Pro  Leu Phe Asp Ile Phe  Asn Lys Val Lys Lys  Glu Trp Asp
       3515                 3520                 3525
   Ser Phe  Gln Asp Pro Val Ala  Val Ser Phe Asp Thr  Lys Ala Trp
       3530                 3535                 3540
   Asp Thr  Gln Val Thr Ser Arg  Asp Leu Met Leu Ile  Lys Asp Ile
       3545                 3550                 3555
   Gln Lys  Tyr Tyr Phe Lys Arg  Ser Ile His Lys Phe  Leu Asp Thr
       3560                 3565                 3570
   Ile Thr  Glu His Met Val Glu  Val Pro Val Ile Thr  Ala Asp Gly
       3575                 3580                 3585
   Glu Val  Tyr Ile Arg Asn Gly  Gln Arg Gly Ser Gly  Gln Pro Asp
       3590                 3595                 3600
   Thr Ser  Ala Gly Asn Ser Met  Leu Asn Val Leu Thr  Met Ile Tyr
       3605                 3610                 3615
   Ala Phe  Cys Lys Ser Thr Gly  Ile Pro Tyr Arg Gly  Phe Ser Arg
       3620                 3625                 3630
   Val Ala  Arg Ile His Val Cys  Gly Asp Asp Gly Phe  Leu Ile Thr
       3635                 3640                 3645
   Glu Arg  Gly Leu Gly Leu Lys  Phe Ser Glu Lys Gly  Met Gln Ile
       3650                 3655                 3660
   Leu His  Glu Ala Gly Lys Pro  Gln Lys Ile Thr Glu  Gly Asp Lys
       3665                 3670                 3675
   Met Lys Val Ala Tyr Arg Phe Glu Asp Ile Glu Phe Cys Ser His
       3680                3685                3690
   Thr Pro Val Pro Val Arg Trp Ala Asp Asn Thr Ser Ser Tyr Met
       3695                3700                3705
   Ala Gly Arg Ser Thr Ala Thr Ile Leu Ala Lys Met Ala Thr Arg
       3710                3715                3720
   Leu Asp Ser Ser Gly Glu Arg Gly Ser Thr Ala Tyr Glu Lys Ala
       3725                3730                3735
   Val Ala Phe Ser Phe Leu Leu Met Tyr Ser Trp Asn Pro Val Val
       3740                3745                3750
   Arg Arg Ile Cys Leu Leu Val Leu Ser Gln Phe Pro Glu Ile Ser
       3755                3760                3765
   Pro Ser Lys Asn Thr Ile Tyr Tyr Tyr Gln Gly Asp Pro Ile Ala
       3770                3775                3780
   Ala Tyr Arg Glu Val Ile Gly Lys Gln Leu Cys Glu Leu Lys Arg
       3785                3790                3795
   Thr Gly Phe Glu Lys Leu Ala Gly Leu Asn Leu Ser Met Thr Thr
       3800                3805                3810
   Leu Gly Ile Trp Thr Lys His Thr Ser Lys Arg Leu Ile Gln Ala
       3815                3820                3825
   Cys Val Glu Ile Gly Lys Arg Glu Gly Thr Trp Leu Val Asn Ala
       3830                3835                3840
   Asp Arg Leu Ile Ala Gly Lys Thr Gly Lys Phe Tyr Ile Pro Ser
       3845                3850                3855
   Thr Gly Val Thr Leu Leu Gly Lys His Tyr Glu Glu Ile Asn Leu
       3860                3865                3870
   Lys Gln Lys Ala Ala Gln Pro Pro Ile Glu Gly Val Asp Arg Tyr
       3875                3880                3885
   Lys Leu Gly Pro Ile Val Asn Val Ile Leu Arg Arg Leu Arg Val
       3890                3895                3900
   Met Leu Met Thr Val Ala Ser Gly Ser Trp
       3905                3910
<210> 10
   <211> 12332
   <212> DNA
   <213> Artificial-sequence:XIKE-C BVDV-Sequence
<400> 10
   gtatacgaga ttagctaaag aactcgtata tggattggac gtcaacaaat ttttaattgg 60
   caacgtaggg aaccttcccc tcagcgaagg ccgaaaagag gctagccatg cccttagtag 120
   gactagcaaa agtaggggac tagcggtagc agtgagttcg ttggatggcc gaacccctga 180
   gtacagggga gtcgtcaatg gttcgacact ccattagtcg aggagtctcg agatgccatg 240
   tggacgaggg catgcccacg gcacatctta acccatgcgg gggttgcatg ggtgaaagcg 300
   ctattcgtgg cgttatggac acagcctgat agggtgtagc agagacctgc tattccgcta 360
   gtaaaaactc tgctgtacat ggcacatgga gttgttttca aatgaacttt tatacaaaac 420
   atataaacaa aaaccagcag gcgtcgtgga acctgtttac gacgtcaacg ggcgcccact 480
   gtttggagag agcagtgact tgcacccgca gtcaacacta aaactaccac accaacgagg 540
   cagcgccaac atcctgacca atgctaggtc cctaccgcgg aaaggtgact gccggagagg 600
   taatgtgtat ggaccggtga gtggcatcta tatcaaacca ggaccgatct actaccagga 660
   ttatgtgggc cccgtctatc atagagcccc actggaacta tgtagggagg caagtatgtg 720
   cgaaacaact aggagagttg gcagagtgac cggtagtgat gggaaattat atcatatcta 780
   catctgcata gatgggtgta tcctcctgaa gagggcgact aggaaccaac cagaagtcct 840
   gaaatgggta tacaacagat taaattgtcc tttatgggtc accagctgct ccgatgaagg 900
   gagcaagggt gctacaagta agaagcagcc taagccagat aggatagaaa aaggtaagat 960
   gaaaatagcc ccaaaagaga cagaaaaaga ttgcaaaacc agaccccccg acgcgactat 1020
   agtagtagaa ggggttaagt accaggtgaa gaaaaaagga aaggtaaggg gaaaaaatac 1080
   tcaagatggg ttatatcaca acaagaataa gccccctgaa tcaagaaaaa aattggaaaa 1140
   ggcactgctg gcttgggcca tcttagcagc ggtcctgctt cagctggtaa caggagagaa 1200
   tatcacccag tggaacttga tggacaacgg caccgaggga atacagcaag cgatgttcct 1260
   aagaggggtg aacaggagtc tattaggaat ttggccagag aaaatttgca ccggagtacc 1320
   aactcactta gcaacagact atgagcttaa agagatagtg gggatgatgg acgcgagtga 1380
   gaagaccaac tacacgtgtt gcaggttgca aagacatgag tggaataaac atggttggtg 1440
   taactggttt catatagaac cgtggatatg gttgatgaac aaaacccaaa acaacctgac 1500
   agaagggcaa ccgcttaggg agtgtgctgt gacttgtagg tatgacaagg aaacagaatt 1560
   gaacatcgtg acacaggcta gggacagacc tacaactctg acaggttgca agaaaggcaa 1620
   gaatttctct ttcgcaggtg ttatactgga tgggccctgt aactttaaag tatcggttga 1680
   agatgtgctg ttcaaggagc acgattgcgg caacatgctg caagagaccg cgatacagct 1740
   actcgatggg gcaaccaaca ccattgaggg agcaagggta gggacggcca agttgacaac 1800
   ctggttaggg aagcaattag ggatccttgg taagaagttg gagaacaaaa gcaaagcatg 1860
   gtttggtgca catgcagcaa gtccatactg cggagtggag aggaagatcg gttacgtatg 1920
   gtatacaaaa aactgcactc cagcttgcct tccaagaaac actagaataa taggccccgg 1980
   gaaatttgat accaacgccg aagatggaaa aatactccat gagatggggg ggcacctctc 2040
   agaatttgtc ctattgtcct tggtggttct gtctgacttt gccccggaaa ccgcgagcgt 2100
   catctacttg gttctacatt ttgcgatccc gcaaagccac gttgatgtag acacatgcga 2160
   caagaaccag ctgaatttaa cggtagcaac cacagtagca gaggtcatac cagggacagt 2220
   gtggaaccta gggaagtatg tctgcataag accagactgg tggccatatg agacgacgac 2280
   agtcttcgtc atagaggaag cagggcaagt aatcaaattg atgctaaggg ccatcagaga 2340
   cttaactagg atatggaatg ctgccactac cacagctttc ttaatctttt tagtaaaagc 2400
   actgagggga caactaatcc aagggctatt gtggctgatg ctaataacag gagcacaggg 2460
   cttccctgaa tgcaaagagg gcttccaata tgccatatct aaagacagga aaatggggtt 2520
   attggggcca gagagcttaa ctacaacatg gcacctcccc accaaaaaaa tagtggattc 2580
   catggtgcat gtatggtgtg aaggaaaaga cttgaaaata ttaaaaatgt gcacaaagga 2640
   agagaggtat ctagtggctg tgcacgagag agccttatca accagtgccg agtttatgca 2700
   gatcagtgat gggacaatag gcccagacgt gatagatatg cctgatgact ttgagtttgg 2760
   actctgccct tgtgactcaa aaccagtgat aaagggcaaa tttaatgcca gcttactgaa 2820
   tggaccagct ttccagatgg tatgcccaca ggggtggact ggtacaatag aatgcaccct 2880
   agcgaaccaa gacaccttgg acacaactgt cattaggaca tatagaagaa ctaccccatt 2940
   tcagcggaga aaatggtgta cctatgaaaa aataataggg gaagatatct atgaatgcat 3000
   tctaggtgga aactggacat gcataaccgg tgaccatagc aggttgaaag acggacctat 3060
   caagaagtgt aagtggtgtg gccatgactt cgtcaactca gaggggctac cacactaccc 3120
   aataggcaag tgcatgctca tcaacgagag tgggtacagg tatgtagatg acacctcttg 3180
   cgataggggt ggtgtagcca tagttccatc tggcaccgta aagtgtagaa taggtaacgt 3240
   cacggtgcaa gttatcgcta ctaacaatga tctgggaccc atgccttgca gcccagctga 3300
   agtgatagca agtgaaggac cagtggaaaa gactgcatgc acattcaact attcaaggac 3360
   tctacctaat aagtattatg agccaaggga ccggtacttc caacaataca tgttaaaagg 3420
   ggagtggcaa tattggttcg acctggattc tgtagaccac cacaaagact acttctcaga 3480
   gttcataatc atagcagtgg tcgccttgtt gggtggtaag tacgtactgt ggctcttgat 3540
   aacatacaca atactgtctg agcagatggc tatgggtgct ggagtgaata ctgaagagat 3600
   agtcatgata ggcaatttgc tgacagacag tgatattgag gttgtggttt atttccttct 3660
   tctgtactta atagttaaag aggaactggc gaggaaatgg attatactgg tataccacat 3720
   ccttgtagcc aaccctatga aaacaattgg ggtcgtctta ctaatgctag ggggagtggt 3780
   gaaggccagc agaatcaatg ctgatgacca aagtgctatg gacccatgct ttcttctcgt 3840
   gacaggcgta gtggctgttt tgatgatcgc tagaagagaa cctgccacat taccactgat 3900
   tgtagcattg ctagcaataa gaacatcagg attcctactg cccgctagca ttgatgtaac 3960
   tgtagcagta gtattaattg tacttttgtt ggctagctac ataacagact actttagata 4020
   taaaaagtgg cttcaactct tatttagtct gatagctggt atctttatta taaggagctt 4080
   aaaacatatc aaccagatgg aggtaccaga aatatctatg ccaagttgga gacctctagc 4140
   tctggtcctt ttctatataa catctacagc aataaccact aattgggaca ttgacttagc 4200
   aggcttcctg ctgcaatggg cgccagcagt gatcatgatg gctaccatgt gggcagactt 4260
   tttgactctg atcatagtcc tgcccagtta cgagttatct aagctttact tcctaaagaa 4320
   cgtcaggaca gacgtggaaa agaactggct cggcaaagtg aaatacagac agatcagttc 4380
   agtttatgac atctgtgaca gtgaggaagc agtgtaccta tttccatcaa ggcataagag 4440
   tggaagcagg ccagatttca tattaccttt tttgaaagcc gtgttaataa gctgcatcag 4500
   cagccaatgg caagtggttt acatttctta cctaatactg gaaattacat actatatgca 4560
   caggaaaatc atagatgagg tgtcaggagg agcaaatttt ctatcaagac tcatagcagc 4620
   catcatagaa ttaaattggg ccatagatga tgaggaatgt aaaggactga agaaactgta 4680
   tctcttgtca gggagagcga agaatttgat agttaaacat aaggtaagaa atgaagccgt 4740
   ccacagatgg tttggtgagg aggaaatata cggggcaccc aaggtgatca ctatcataaa 4800
   agctagtacc ctaagtaaaa acaggcactg cataatctgc acgatctgtg aagggaaaga 4860
   atggaatgga gccaactgcc caaagtgtgg aagacaagga aagcccataa catgtggaat 4920
   gacactcgca gactttgagg agaaacatta caaaaagata tttataagag aagaatcttc 4980
   ttgtcctgtg ccttttgatc cttcttgcca ttgtaattat tttcgccacg atgggccttt 5040
   caggaaagag tataagggtt acgtccaata cacagccaga ggacaactct ttctgaggaa 5100
   cctaccaatt ctagcgacga agatgaagct attaatggtg ggaaacctcg gcgcagaaat 5160
   tggcgacctg gaacatctag gatgggtact gagagggcca gccgtgtgca aaaaaattac 5220
   caaccatgag aagtgccacg taaacatcat ggataagcta actgcatttt ttggaatcat 5280
   gcctagaggc acgaccccta gggcacctgt gaggttcccc acagcactac taaaagtgag 5340
   aagggggcta gagacgggat gggcttacac gcaccaagga gggatcagct cggtagacca 5400
   tgtcacagcc ggaaaggatt tactagtgtg tgacagtatg ggcaggacca gggttgtctg 5460
   tcatagtaac aataagatga ctgatgagac tgagtatggc atcaagaccg actcagggtg 5520
   tcccgaaggt gcgaggtgtt acgtgctaaa cccagaagct gttaacattt ctggcacaaa 5580
   aggagctatg gtacacctcc agaaaacggg gggggagttc acatgtgtca ctgcctcagg 5640
   gaccccggct ttcttcgatc tgaaaaatct aaaaggctgg tccgggctac caatttttga 5700
   agcatccagt ggcagggtgg ttggtagggt gaaagtcggc aagaatgagg attccaagcc 5760
   caccaaacta atgagcggaa tccagacagt gtctaagaac cagacagacc tagcggacat 5820
   cgtaaaaaaa ttgactagta tgaacagagg agagttcaaa cagataacat tagccactgg 5880
   ggcaggaaaa actacggaac tgccaaggtc cgtcatagag gagataggga ggcacaaaag 5940
   ggtcttagtc ctgataccat tgagagcagc agcagagtca gtgtatcagt atatgagagt 6000
   gaagtaccca agtatatctt tcaatttgag aataggagat atgaaggaag gtgacatggc 6060
   cactggtatc acctacgcct catatgggta cttttgtcag cttcctcagc ccaaactgag 6120
   agctgccatg gtagagtact catatatatt cttagatgag taccactgtg ctacacccga 6180
   gcaattagca ataattggaa agatacacag gtttgctgaa aatcttagag tggtagcaat 6240
   gacagcaacc ccagctggaa cggtcacaac gactggtcag aaacacccta tagaggagtt 6300
   catagcccca gaggtgatga aaggtgaaga tctaggtagt gaatacttgg atattgcagg 6360
   gttgaagata ccgactgaag agatgaaagg caacatgctc gtgttcgcgc caactaggaa 6420
   catggcagta gaaacagcta agaaattgaa ggctaaggga tacaactctg gatactatta 6480
   cagtggggaa aacccagaga acttgagggt ggtaacctcg caatccccgt atgtggtagt 6540
   agccaccaat gccatagagt caggtgtgac attaccagac ttagacacag ttgtagacac 6600
   tggactaaag tgtgagaaga gggtgaggat ttcttcaaaa atgcccttca ttgtaacagg 6660
   acttaagaga atggcagtca caatcggaga gcaagcccag cgcaggggta gagtaggaag 6720
   agtcaagcca ggtaggtact ataggagtca agaaacagct tcagggtcaa aagattacca 6780
   ttacgaccta ctgcaagccc agaggtacgg aatagaagat ggaattaatg taacaaagtc 6840
   attcagggag atgaactatg attggagcct ttacgaagag gacagcttga tgataactca 6900
   actcgaggtc cttaacaacc tccttatatc agaagacctg cctgccgcag tgaagaacat 6960
   catggcccgg accgatcacc cagaacccat acaactggcc tataacagtt atgaaaacca 7020
   aattccagtg ctgttcccaa agatcaaaaa tggtgaggtg acagacagtt atgagaatta 7080
   cacatatctc aatgcaagaa aattaggaga ggacgtgccg gcatatgtgt acgccacaga 7140
   ggatgaggat ctagcagtgg atcttctggg tatggattgg ccggacccag gcaaccaaca 7200
   ggtggtagag acagggaggg cattaaaaca agtaactggc ttatccacag cagaaaacgc 7260
   cctcttgata gccctattcg gctacgtcgg gtaccagaca ctttcaaaaa ggcacatacc 7320
   catgattact gacatctata cacttgaaga ccacaggctt gaggacacaa cccacctcca 7380
   gtttgcccca aacgctataa ggaccgacgg caaggactca gagttgaagg aattagctgt 7440
   gggagacctt gataaatatg tggacgcact ggtagactac tccaaacaag ggatgaaatt 7500
   catcaaagtc caagctgaaa aggtcagaga ctcccagtct acgaaggaag gcttgcaaac 7560
   cattaaggag tatgtggata agtttataca atcactaaca gagaataagg aggagatcat 7620
   caggtatgga ctatggggag ttcacacggc actctacaaa agcttggcag cgagactggg 7680
   gcatgaaaca gcttttgcaa ctttagtggt aaaatggttg gcttttgggg gcgaaacggt 7740
   atctgctcac atcaagcaag tagcagttga tctagtagta tattatatca tcaacaaacc 7800
   atcttttcct ggagatacag agacccaaca agaggggagg aagtttgtgg ctagtctttt 7860
   tatatctgca ctagcaacat acacatataa aacctggaat tacaacaatc tgcaacgggt 7920
   tgtcgaacct gccttagctt acctcccata tgctacaagt gccttgaagt tgttcacacc 7980
   cacaagatta gagagtgtgg tcatactcag ttctacaatt tacaagacat acctctctat 8040
   aaggaagggt aagagtgacg gcttgttagg tacaggcata agtgcagcca tggagatctt 8100
   aaaccaaaac ccaatctcag taggtatatc tgtgatgctg ggggtaggtg ccatcgccgc 8160
   ccataatgca atagaatcta gtgaacagaa aagaactttg ctgatgaagg tctttgtaaa 8220
   aaacttctta gaccaagcag caacagatga gctagtcaaa gagaaccctg aaaaaataat 8280
   catggctcta tttgaagcag tccagaccat aggaaacccc ctaagactca tctaccatct 8340
   gtacggggtg tactataagg ggtgggaagc aaaagaactc gcagagaaaa ctgctggccg 8400
   caacttattc acattgatca tgtttgaggc ctttgagctt ttaggtatgg actcagaagg 8460
   aaagataaga aacttgtcag gcaactacat actggactta atcttcaact tgcataataa 8520
   attaaacaag gggctcaaaa aactagtcct tgggtgggct cctgcacctt tgagctgtga 8580
   ttggacacca agtgatgaga gaataagcct acctcataac aactacttaa gggtagaaac 8640
   caggtgtcct tgtggctatg agatgaaggc aataaaaaat gttgctggta aattgacaaa 8700
   agttgaagaa aaggggtcct tcctatgcag gaatagatta gggagaggac ctccaaactt 8760
   caaagtaaca aagttctatg atgataactt gatagaagtc aagccagtag ctaggctaga 8820
   aggccaggtg gacctctatt acaagggagt aacagctaag ttagactaca acaatgggaa 8880
   agtactgtta gctaccaaca agtgggaggt ggaccacgct ttcctgacca gactagtaaa 8940
   gaagcacaca gggataggtt ttaaaggtgc atatttgggt gaccgaccag accatcaaga 9000
   tcttgtcgat agagattgtg caactataac gaagaactca gtacagttcc taaaaatgaa 9060
   gaagggttgc gctttcacat atgacctaac aatctctaac cttgtcaggc ttattgaact 9120
   agtccataag aataatttac aagaaagaga gatccctacc gtgacagtaa ctacttggct 9180
   tgcatattct tttgtcaatg aagacctggg gactatcaag cctgtattgg gggagaaagt 9240
   catcccagaa ccccccgagg agttgagtct ccaacccacc gtgagactag tcaccactga 9300
   aacagcaata accataacag gggaggctga agtgatgacg acagggatca caccagtggt 9360
   agagatgaaa gaagaacctc agctggacca ccagtcaact accctaaagg tagggttgaa 9420
   ggaaggggaa tatccagggc caggagttaa ccctaaccat ttagcagagg tgatagatga 9480
   gaaagatgac aggccttttg tcctaatcat cggtaacaaa ggttctacct cgaacagagc 9540
   aagaacggcc aagaatatac ggctgtacaa aggaaacaac ccaagagaga tcagggatct 9600
   gatgagccaa ggaagaatat tgacggttgc tctaaaagag ttggacccgg aattaaaaga 9660
   attagtagat tacaagggga cctttctcaa tagggaagct ttagaagccc taagcttagg 9720
   taagccaatc aagaggaaaa ccacaacagc aatgatcagg aggttaatag agccagaggt 9780
   tgaggaggaa ctaccagatt ggttccaagc ggaagaaccc ctatttttgg aagcaaaaat 9840
   acagaatgac ttataccacc taattggcag tgtagatagt ataaaaagca aagcaaagga 9900
   attaggggcc acagataaca caaagatagt gaaggaagtt ggggctagga cctatacgat 9960
   gaaattgagc agctggagca cacaagttac aaaaaaacag atgagtctag cccctctctt 10020
   tgaagagctg ttattaaagt gccctccatg tagtaaaatt tcaaagggac atatggtgtc 10080
   agcataccaa ctggctcaag gaaactggga acccctcggg tgtggggtct atatgggaac 10140
   cataccagct aggcgtctca agatccaccc ttatgaggct taccttaaac tcaaagagct 10200
   ggtggaagtt gaatcttcga gggccactgc aaaagaatcc atcataagag aacataacac 10260
   ctggatcctg cggaaggtga gacatgaagg gaacctaaga accaaatcaa tgatcaaccc 10320
   tgggaaaata tcagatcagc tatgcagaga tggacacaaa agaaacatat ataataagat 10380
   cataggctca acaatggcct ctgctggtat taggctggag aaactgccag tagtccgagc 10440
   ccaaactgac acaaccagtt tccaccaagc cataagagaa aaaattgata aaacagaaaa 10500
   caagcagacc cctgaattgc atgaagaact aatgaaggtc ttcgactgct taaagatccc 10560
   agagctgaag gaatcgtatg atgaagtttc atgggaacaa ttagaagccg ggataaaccg 10620
   taagggtgca gcaggctatc tagagagcaa gaacataggg gaagtcctag acacagagaa 10680
   acacatagta gagcagctga tcaaggatct gaggaagggg aagaagatta ggtactatga 10740
   aacagccatc cccaagaatg agaagagaga cgtcagcgac gactgggaag ccggagagtt 10800
   cgttgatgaa aagaaaccaa gagtaatcca gtacccggac gccaaggtga gactggccat 10860
   tacaaaagtg atgtacaaat gggtaaagca aaaaccagtg gtgatacccg gctatgaagg 10920
   taaaacacct ctatttgaca tattcaacaa agtgaagaag gaatgggatt cattccagga 10980
   ccccgtagca gtgagctttg acaccaaagc gtgggataca caagtcacca gtagagacct 11040
   aatgttgata aaggatatcc agaaatatta tttcaagaga agtatacaca aatttttaga 11100
   tacaataaca gaacacatgg tggaggtacc tgtcattaca gcagacggtg aagtttacat 11160
   aaggaatggt cagaggggta gtggccaacc cgacacaagt gctggtaata gtatgttgaa 11220
   tgtcctaacc atgatatatg ctttctgtaa aagtacaggc ataccttaca ggggattcag 11280
   cagagtggca agaatccatg tgtgtggtga tgatggcttt ttgataacag agagaggact 11340
   gggactgaaa ttctctgaga agggtatgca gatattacat gaggccggga agccccagaa 11400
   aataactgaa ggggacaaaa tgaaagtggc atacagattc gaggacatag agttttgttc 11460
   ccatactccc gtgccagtca gatgggcaga taacaccagt agttacatgg cagggaggag 11520
   cacagccact atactagcta agatggcaac caggctggat tccagcggag agaggggtag 11580
   cacagcttat gagaaggccg tagccttcag cttccttttg atgtactcat ggaatcccgt 11640
   agttagaagg atctgcttac tggtgttgtc acagtttcca gaaatatccc catccaaaaa 11700
   cacaatatac tactaccaag gggatcccat agctgcgtac agagaagtga tagggaaaca 11760
   gctgtgtgaa ctgaaaagaa caggatttga gaagctggct ggtctgaatt tgagtatgac 11820
   cactctaggc atctggacaa aacatactag taaaagacta atccaagcct gtgtagaaat 11880
   aggtaagaga gaaggtacct ggttagttaa tgctgacaga ctgattgcag gaaagactgg 11940
   gaagttttac atcccaagca ctggtgtcac tctgttggga aaacactatg aggaaattaa 12000
   cttaaagcaa aaggcggcac aaccgccgat agagggggtt gacagatata agttgggccc 12060
   catagttaat gttatcttga gaaggctgag ggtgatgctg atgacagttg ccagcggaag 12120
   ctggtgaatc cgtccggagc gtcgtgccct cactcaaggt ttttaattgt aaatattgta 12180
   aatagacagc taagatattt attgtagttg gatagtaatg cagtgatagt aaatacccca 12240
   atttaacact acctccaatg cactaagcac tttagctgtg tgaggttaac tcgacgtcca 12300
   cggttggact agggaagacc tctaacagcc cc 12332
<210> 11
   <211> 11840
   <212> DNA
   <213> Artificial sequence:XIKE-C-NdN BVDV-Sequence
<400> 11
   gtatacgaga ttagctaaag aactcgtata tggattggac gtcaacaaat ttttaattgg     60
   caacgtaggg aaccttcccc tcagcgaagg ccgaaaagag gctagccatg cccttagtag    120
   gactagcaaa agtaggggac tagcggtagc agtgagttcg ttggatggcc gaacccctga    180
   gtacagggga gtcgtcaatg gttcgacact ccattagtcg aggagtctcg agatgccatg    240
   tggacgaggg catgcccacg gcacatctta acccatgcgg gggttgcatg ggtgaaagcg    300
   ctattcgtgg cgttatggac acagcctgat agggtgtagc agagacctgc tattccgcta    360
   gtaaaaactc tgctgtacat ggcacatgga gttgttttcc gatgaaggga gcaagggtgc    420
   tacaagtaag aagcagccta agccagatag gatagaaaaa ggtaagatga aaatagcccc 480
   aaaagagaca gaaaaagatt gcaaaaccag accccccgac gcgactatag tagtagaagg 540
   ggttaagtac caggtgaaga aaaaaggaaa ggtaagggga aaaaatactc aagatgggtt 600
   atatcacaac aagaataagc cccctgaatc aagaaaaaaa ttggaaaagg cactgctggc 660
   ttgggccatc ttagcagcgg tcctgcttca gctggtaaca ggagagaata tcacccagtg 720
   gaacttgatg gacaacggca ccgagggaat acagcaagcg atgttcctaa gaggggtgaa 780
   caggagtcta ttaggaattt ggccagagaa aatttgcacc ggagtaccaa ctcacttagc 840
   aacagactat gagcttaaag agatagtggg gatgatggac gcgagtgaga agaccaacta 900
   cacgtgttgc aggttgcaaa gacatgagtg gaataaacat ggttggtgta actggtttca 960
   tatagaaccg tggatatggt tgatgaacaa aacccaaaac aacctgacag aagggcaacc 1020
   gcttagggag tgtgctgtga cttgtaggta tgacaaggaa acagaattga acatcgtgac 1080
   acaggctagg gacagaccta caactctgac aggttgcaag aaaggcaaga atttctcttt 1140
   cgcaggtgtt atactggatg ggccctgtaa ctttaaagta tcggttgaag atgtgctgtt 1200
   caaggagcac gattgcggca acatgctgca agagaccgcg atacagctac tcgatggggc 1260
   aaccaacacc attgagggag caagggtagg gacggccaag ttgacaacct ggttagggaa 1320
   gcaattaggg atccttggta agaagttgga gaacaaaagc aaagcatggt ttggtgcaca 1380
   tgcagcaagt ccatactgcg gagtggagag gaagatcggt tacgtatggt atacaaaaaa 1440
   ctgcactcca gcttgccttc caagaaacac tagaataata ggccccggga aatttgatac 1500
   caacgccgaa gatggaaaaa tactccatga gatggggggg cacctctcag aatttgtcct 1560
   attgtccttg gtggttctgt ctgactttgc cccggaaacc gcgagcgtca tctacttggt 1620
   tctacatttt gcgatcccgc aaagccacgt tgatgtagac acatgcgaca agaaccagct 1680
   gaatttaacg gtagcaacca cagtagcaga ggtcatacca gggacagtgt ggaacctagg 1740
   gaagtatgtc tgcataagac cagactggtg gccatatgag acgacgacag tcttcgtcat 1800
   agaggaagca gggcaagtaa tcaaattgat gctaagggcc atcagagact taactaggat 1860
   atggaatgct gccactacca cagctttctt aatcttttta gtaaaagcac tgaggggaca 1920
   actaatccaa gggctattgt ggctgatgct aataacagga gcacagggct tccctgaatg 1980
   caaagagggc ttccaatatg ccatatctaa agacaggaaa atggggttat tggggccaga 2040
   gagcttaact acaacatggc acctccccac caaaaaaata gtggattcca tggtgcatgt 2100
   atggtgtgaa ggaaaagact tgaaaatatt aaaaatgtgc acaaaggaag agaggtatct 2160
   agtggctgtg cacgagagag ccttatcaac cagtgccgag tttatgcaga tcagtgatgg 2220
   gacaataggc ccagacgtga tagatatgcc tgatgacttt gagtttggac tctgcccttg 2280
   tgactcaaaa ccagtgataa agggcaaatt taatgccagc ttactgaatg gaccagcttt 2340
   ccagatggta tgcccacagg ggtggactgg tacaatagaa tgcaccctag cgaaccaaga 2400
   caccttggac acaactgtca ttaggacata tagaagaact accccatttc agcggagaaa 2460
   atggtgtacc tatgaaaaaa taatagggga agatatctat gaatgcattc taggtggaaa 2520
   ctggacatgc ataaccggtg accatagcag gttgaaagac ggacctatca agaagtgtaa 2580
   gtggtgtggc catgacttcg tcaactcaga ggggctacca cactacccaa taggcaagtg 2640
   catgctcatc aacgagagtg ggtacaggta tgtagatgac acctcttgcg ataggggtgg 2700
   tgtagccata gttccatctg gcaccgtaaa gtgtagaata ggtaacgtca cggtgcaagt 2760
   tatcgctact aacaatgatc tgggacccat gccttgcagc ccagctgaag tgatagcaag 2820
   tgaaggacca gtggaaaaga ctgcatgcac attcaactat tcaaggactc tacctaataa 2880
   gtattatgag ccaagggacc ggtacttcca acaatacatg ttaaaagggg agtggcaata 2940
   ttggttcgac ctggattctg tagaccacca caaagactac ttctcagagt tcataatcat 3000
   agcagtggtc gccttgttgg gtggtaagta cgtactgtgg ctcttgataa catacacaat 3060
   actgtctgag cagatggcta tgggtgctgg agtgaatact gaagagatag tcatgatagg 3120
   caatttgctg acagacagtg atattgaggt tgtggtttat ttccttcttc tgtacttaat 3180
   agttaaagag gaactggcga ggaaatggat tatactggta taccacatcc ttgtagccaa 3240
   ccctatgaaa acaattgggg tcgtcttact aatgctaggg ggagtggtga aggccagcag 3300
   aatcaatgct gatgaccaaa gtgctatgga cccatgcttt cttctcgtga caggcgtagt 3360
   ggctgttttg atgatcgcta gaagagaacc tgccacatta ccactgattg tagcattgct 3420
   agcaataaga acatcaggat tcctactgcc cgctagcatt gatgtaactg tagcagtagt 3480
   attaattgta cttttgttgg ctagctacat aacagactac tttagatata aaaagtggct 3540
   tcaactctta tttagtctga tagctggtat ctttattata aggagcttaa aacatatcaa 3600
   ccagatggag gtaccagaaa tatctatgcc aagttggaga cctctagctc tggtcctttt 3660
   ctatataaca tctacagcaa taaccactaa ttgggacatt gacttagcag gcttcctgct 3720
   gcaatgggcg ccagcagtga tcatgatggc taccatgtgg gcagactttt tgactctgat 3780
   catagtcctg cccagttacg agttatctaa gctttacttc ctaaagaacg tcaggacaga 3840
   cgtggaaaag aactggctcg gcaaagtgaa atacagacag atcagttcag tttatgacat 3900
   ctgtgacagt gaggaagcag tgtacctatt tccatcaagg cataagagtg gaagcaggcc 3960
   agatttcata ttaccttttt tgaaagccgt gttaataagc tgcatcagca gccaatggca 4020
   agtggtttac atttcttacc taatactgga aattacatac tatatgcaca ggaaaatcat 4080
   agatgaggtg tcaggaggag caaattttct atcaagactc atagcagcca tcatagaatt 4140
   aaattgggcc atagatgatg aggaatgtaa aggactgaag aaactgtatc tcttgtcagg 4200
   gagagcgaag aatttgatag ttaaacataa ggtaagaaat gaagccgtcc acagatggtt 4260
   tggtgaggag gaaatatacg gggcacccaa ggtgatcact atcataaaag ctagtaccct 4320
   aagtaaaaac aggcactgca taatctgcac gatctgtgaa gggaaagaat ggaatggagc 4380
   caactgccca aagtgtggaa gacaaggaaa gcccataaca tgtggaatga cactcgcaga 4440
   ctttgaggag aaacattaca aaaagatatt tataagagaa gaatcttctt gtcctgtgcc 4500
   ttttgatcct tcttgccatt gtaattattt tcgccacgat gggcctttca ggaaagagta 4560
   taagggttac gtccaataca cagccagagg acaactcttt ctgaggaacc taccaattct 4620
   agcgacgaag atgaagctat taatggtggg aaacctcggc gcagaaattg gcgacctgga 4680
   acatctagga tgggtactga gagggccagc cgtgtgcaaa aaaattacca accatgagaa 4740
   gtgccacgta aacatcatgg ataagctaac tgcatttttt ggaatcatgc ctagaggcac 4800
   gacccctagg gcacctgtga ggttccccac agcactacta aaagtgagaa gggggctaga 4860
   gacgggatgg gcttacacgc accaaggagg gatcagctcg gtagaccatg tcacagccgg 4920
   aaaggattta ctagtgtgtg acagtatggg caggaccagg gttgtctgtc atagtaacaa 4980
   taagatgact gatgagactg agtatggcat caagaccgac tcagggtgtc ccgaaggtgc 5040
   gaggtgttac gtgctaaacc cagaagctgt taacatttct ggcacaaaag gagctatggt 5100
   acacctccag aaaacggggg gggagttcac atgtgtcact gcctcaggga ccccggcttt 5160
   cttcgatctg aaaaatctaa aaggctggtc cgggctacca atttttgaag catccagtgg 5220
   cagggtggtt ggtagggtga aagtcggcaa gaatgaggat tccaagccca ccaaactaat 5280
   gagcggaatc cagacagtgt ctaagaacca gacagaccta gcggacatcg taaaaaaatt 5340
   gactagtatg aacagaggag agttcaaaca gataacatta gccactgggg caggaaaaac 5400
   tacggaactg ccaaggtccg tcatagagga gatagggagg cacaaaaggg tcttagtcct 5460
   gataccattg agagcagcag cagagtcagt gtatcagtat atgagagtga agtacccaag 5520
   tatatctttc aatttgagaa taggagatat gaaggaaggt gacatggcca ctggtatcac 5580
   ctacgcctca tatgggtact tttgtcagct tcctcagccc aaactgagag ctgccatggt 5640
   agagtactca tatatattct tagatgagta ccactgtgct acacccgagc aattagcaat 5700
   aattggaaag atacacaggt ttgctgaaaa tcttagagtg gtagcaatga cagcaacccc 5760
   agctggaacg gtcacaacga ctggtcagaa acaccctata gaggagttca tagccccaga 5820
   ggtgatgaaa ggtgaagatc taggtagtga atacttggat attgcagggt tgaagatacc 5880
   gactgaagag atgaaaggca acatgctcgt gttcgcgcca actaggaaca tggcagtaga 5940
   aacagctaag aaattgaagg ctaagggata caactctgga tactattaca gtggggaaaa 6000
   cccagagaac ttgagggtgg taacctcgca atccccgtat gtggtagtag ccaccaatgc 6060
   catagagtca ggtgtgacat taccagactt agacacagtt gtagacactg gactaaagtg 6120
   tgagaagagg gtgaggattt cttcaaaaat gcccttcatt gtaacaggac ttaagagaat 6180
   ggcagtcaca atcggagagc aagcccagcg caggggtaga gtaggaagag tcaagccagg 6240
   taggtactat aggagtcaag aaacagcttc agggtcaaaa gattaccatt acgacctact 6300
   gcaagcccag aggtacggaa tagaagatgg aattaatgta acaaagtcat tcagggagat 6360
   gaactatgat tggagccttt acgaagagga cagcttgatg ataactcaac tcgaggtcct 6420
   taacaacctc cttatatcag aagacctgcc tgccgcagtg aagaacatca tggcccggac 6480
   cgatcaccca gaacccatac aactggccta taacagttat gaaaaccaaa ttccagtgct 6540
   gttcccaaag atcaaaaatg gtgaggtgac agacagttat gagaattaca catatctcaa 6600
   tgcaagaaaa ttaggagagg acgtgccggc atatgtgtac gccacagagg atgaggatct 6660
   agcagtggat cttctgggta tggattggcc ggacccaggc aaccaacagg tggtagagac 6720
   agggagggca ttaaaacaag taactggctt atccacagca gaaaacgccc tcttgatagc 6780
   cctattcggc tacgtcgggt accagacact ttcaaaaagg cacataccca tgattactga 6840
   catctataca cttgaagacc acaggcttga ggacacaacc cacctccagt ttgccccaaa 6900
   cgctataagg accgacggca aggactcaga gttgaaggaa ttagctgtgg gagaccttga 6960
   taaatatgtg gacgcactgg tagactactc caaacaaggg atgaaattca tcaaagtcca 7020
   agctgaaaag gtcagagact cccagtctac gaaggaaggc ttgcaaacca ttaaggagta 7080
   tgtggataag tttatacaat cactaacaga gaataaggag gagatcatca ggtatggact 7140
   atggggagtt cacacggcac tctacaaaag cttggcagcg agactggggc atgaaacagc 7200
   ttttgcaact ttagtggtaa aatggttggc ttttgggggc gaaacggtat ctgctcacat 7260
   caagcaagta gcagttgatc tagtagtata ttatatcatc aacaaaccat cttttcctgg 7320
   agatacagag acccaacaag aggggaggaa gtttgtggct agtcttttta tatctgcact 7380
   agcaacatac acatataaaa cctggaatta caacaatctg caacgggttg tcgaacctgc 7440
   cttagcttac ctcccatatg ctacaagtgc cttgaagttg ttcacaccca caagattaga 7500
   gagtgtggtc atactcagtt ctacaattta caagacatac ctctctataa ggaagggtaa 7560
   gagtgacggc ttgttaggta caggcataag tgcagccatg gagatcttaa accaaaaccc 7620
   aatctcagta ggtatatctg tgatgctggg ggtaggtgcc atcgccgccc ataatgcaat 7680
   agaatctagt gaacagaaaa gaactttgct gatgaaggtc tttgtaaaaa acttcttaga 7740
   ccaagcagca acagatgagc tagtcaaaga gaaccctgaa aaaataatca tggctctatt 7800
   tgaagcagtc cagaccatag gaaaccccct aagactcatc taccatctgt acggggtgta 7860
   ctataagggg tgggaagcaa aagaactcgc agagaaaact gctggccgca acttattcac 7920
   attgatcatg tttgaggcct ttgagctttt aggtatggac tcagaaggaa agataagaaa 7980
   cttgtcaggc aactacatac tggacttaat cttcaacttg cataataaat taaacaaggg 8040
   gctcaaaaaa ctagtccttg ggtgggctcc tgcacctttg agctgtgatt ggacaccaag 8100
   tgatgagaga ataagcctac ctcataacaa ctacttaagg gtagaaacca ggtgtccttg 8160
   tggctatgag atgaaggcaa taaaaaatgt tgctggtaaa ttgacaaaag ttgaagaaaa 8220
   ggggtccttc ctatgcagga atagattagg gagaggacct ccaaacttca aagtaacaaa 8280
   gttctatgat gataacttga tagaagtcaa gccagtagct aggctagaag gccaggtgga 8340
   cctctattac aagggagtaa cagctaagtt agactacaac aatgggaaag tactgttagc 8400
   taccaacaag tgggaggtgg accacgcttt cctgaccaga ctagtaaaga agcacacagg 8460
   gataggtttt aaaggtgcat atttgggtga ccgaccagac catcaagatc ttgtcgatag 8520
   agattgtgca actataacga agaactcagt acagttccta aaaatgaaga agggttgcgc 8580
   tttcacatat gacctaacaa tctctaacct tgtcaggctt attgaactag tccataagaa 8640
   taatttacaa gaaagagaga tccctaccgt gacagtaact acttggcttg catattcttt 8700
   tgtcaatgaa gacctgggga ctatcaagcc tgtattgggg gagaaagtca tcccagaacc 8760
   ccccgaggag ttgagtctcc aacccaccgt gagactagtc accactgaaa cagcaataac 8820
   cataacaggg gaggctgaag tgatgacgac agggatcaca ccagtggtag agatgaaaga 8880
   agaacctcag ctggaccacc agtcaactac cctaaaggta gggttgaagg aaggggaata 8940
   tccagggcca ggagttaacc ctaaccattt agcagaggtg atagatgaga aagatgacag 9000
   gccttttgtc ctaatcatcg gtaacaaagg ttctacctcg aacagagcaa gaacggccaa 9060
   gaatatacgg ctgtacaaag gaaacaaccc aagagagatc agggatctga tgagccaagg 9120
   aagaatattg acggttgctc taaaagagtt ggacccggaa ttaaaagaat tagtagatta 9180
   caaggggacc tttctcaata gggaagcttt agaagcccta agcttaggta agccaatcaa 9240
   gaggaaaacc acaacagcaa tgatcaggag gttaatagag ccagaggttg aggaggaact 9300
   accagattgg ttccaagcgg aagaacccct atttttggaa gcaaaaatac agaatgactt 9360
   ataccaccta attggcagtg tagatagtat aaaaagcaaa gcaaaggaat taggggccac 9420
   agataacaca aagatagtga aggaagttgg ggctaggacc tatacgatga aattgagcag 9480
   ctggagcaca caagttacaa aaaaacagat gagtctagcc cctctctttg aagagctgtt 9540
   attaaagtgc cctccatgta gtaaaatttc aaagggacat atggtgtcag cataccaact 9600
   ggctcaagga aactgggaac ccctcgggtg tggggtctat atgggaacca taccagctag 9660
   gcgtctcaag atccaccctt atgaggctta ccttaaactc aaagagctgg tggaagttga 9720
   atcttcgagg gccactgcaa aagaatccat cataagagaa cataacacct ggatcctgcg 9780
   gaaggtgaga catgaaggga acctaagaac caaatcaatg atcaaccctg ggaaaatatc 9840
   agatcagcta tgcagagatg gacacaaaag aaacatatat aataagatca taggctcaac 9900
   aatggcctct gctggtatta ggctggagaa actgccagta gtccgagccc aaactgacac 9960
   aaccagtttc caccaagcca taagagaaaa aattgataaa acagaaaaca agcagacccc 10020
   tgaattgcat gaagaactaa tgaaggtctt cgactgctta aagatcccag agctgaagga 10080
   atcgtatgat gaagtttcat gggaacaatt agaagccggg ataaaccgta agggtgcagc 10140
   aggctatcta gagagcaaga acatagggga agtcctagac acagagaaac acatagtaga 10200
   gcagctgatc aaggatctga ggaaggggaa gaagattagg tactatgaaa cagccatccc 10260
   caagaatgag aagagagacg tcagcgacga ctgggaagcc ggagagttcg ttgatgaaaa 10320
   gaaaccaaga gtaatccagt acccggacgc caaggtgaga ctggccatta caaaagtgat 10380
   gtacaaatgg gtaaagcaaa aaccagtggt gatacccggc tatgaaggta aaacacctct 10440
   atttgacata ttcaacaaag tgaagaagga atgggattca ttccaggacc ccgtagcagt 10500
   gagctttgac accaaagcgt gggatacaca agtcaccagt agagacctaa tgttgataaa 10560
   ggatatccag aaatattatt tcaagagaag tatacacaaa tttttagata caataacaga 10620
   acacatggtg gaggtacctg tcattacagc agacggtgaa gtttacataa ggaatggtca 10680
   gaggggtagt ggccaacccg acacaagtgc tggtaatagt atgttgaatg tcctaaccat 10740
   gatatatgct ttctgtaaaa gtacaggcat accttacagg ggattcagca gagtggcaag 10800
   aatccatgtg tgtggtgatg atggcttttt gataacagag agaggactgg gactgaaatt 10860
   ctctgagaag ggtatgcaga tattacatga ggccgggaag ccccagaaaa taactgaagg 10920
   ggacaaaatg aaagtggcat acagattcga ggacatagag ttttgttccc atactcccgt 10980
   gccagtcaga tgggcagata acaccagtag ttacatggca gggaggagca cagccactat 11040
   actagctaag atggcaacca ggctggattc cagcggagag aggggtagca cagcttatga 11100
   gaaggccgta gccttcagct tccttttgat gtactcatgg aatcccgtag ttagaaggat 11160
   ctgcttactg gtgttgtcac agtttccaga aatatcccca tccaaaaaca caatatacta 11220
   ctaccaaggg gatcccatag ctgcgtacag agaagtgata gggaaacagc tgtgtgaact 11280
   gaaaagaaca ggatttgaga agctggctgg tctgaatttg agtatgacca ctctaggcat 11340
   ctggacaaaa catactagta aaagactaat ccaagcctgt gtagaaatag gtaagagaga 11400
   aggtacctgg ttagttaatg ctgacagact gattgcagga aagactggga agttttacat 11460
   cccaagcact ggtgtcactc tgttgggaaa acactatgag gaaattaact taaagcaaaa 11520
   ggcggcacaa ccgccgatag agggggttga cagatataag ttgggcccca tagttaatgt 11580
   tatcttgaga aggctgaggg tgatgctgat gacagttgcc agcggaagct ggtgaatccg 11640
   tccggagcgt cgtgccctca ctcaaggttt ttaattgtaa atattgtaaa tagacagcta 11700
   agatatttat tgtagttgga tagtaatgca gtgatagtaa ataccccaat ttaacactac 11760
   ctccaatgca ctaagcactt tagctgtgtg aggttaactc gacgtccacg gttggactag  11820
   ggaagacctc taacagcccc                                              11840
<210> 12
   <211> 3749
   <212> PRT
   <213> Artificial sequence:XIKE-C-NdN
<400> 12
   Met Glu Leu Phe Ser Asp Glu Gly Ser Lys Gly Ala Thr Ser Lys Lys
   1               5                   10                  15
   Gln Pro Lys Pro Asp Arg Ile Glu Lys Gly Lys Met Lys Ile Ala Pro
               20                  25                  30
   Lys Glu Thr Glu Lys Asp Cys Lys Thr Arg Pro Pro Asp Ala Thr Ile
           35                  40                  45
   Val Val Glu Gly Val Lys Tyr Gln Val Lys Lys Lys Gly Lys Val Arg
       50                  55                  60
   Gly Lys Asn Thr Gln Asp Gly Leu Tyr His Asn Lys Asn Lys Pro Pro
   65                  70                  75                  80
   Glu Ser Arg Lys Lys Leu Glu Lys Ala Leu Leu Ala Trp Ala Ile Leu
                   85                  90                  95
   Ala Ala Val Leu Leu Gln Leu Val Thr Gly Glu Asn Ile Thr Gln Trp
               100                 105                 110
   Asn Leu Met Asp Asn Gly Thr Glu Gly Ile Gln Gln Ala Met Phe Leu
           115                 120                 125
   Arg Gly Val Asn Arg Ser Leu Leu Gly Ile Trp Pro Glu Lys Ile Cys
       130                 135                 140
   Thr Gly Val Pro Thr His Leu Ala Thr Asp Tyr Glu Leu Lys Glu Ile
   145                 150                 155                 160
   Val Gly Met Met Asp Ala Ser Glu Lys Thr Asn Tyr Thr Cys Cys Arg
                   165                 170                 175
   Leu Gln Arg His Glu Trp Asn Lys His Gly Trp Cys Asn Trp Phe His
               180                 185                 190
   Ile Glu Pro Trp Ile Trp Leu Met Asn Lys Thr Gln Asn Asn Leu Thr
           195                 200                 205
   Glu Gly Gln Pro Leu Arg Glu Cys Ala Val Thr Cys Arg Tyr Asp Lys
       210                 215                 220
   Glu Thr Glu Leu Asn Ile Val Thr Gln Ala Arg Asp Arg Pro Thr Thr
   225                 230                 235                 240
   Leu Thr Gly Cys Lys Lys Gly Lys Asn Phe Ser Phe Ala Gly Val Ile
                   245                 250                 255
   Leu Asp Gly Pro Cys Asn Phe Lys Val Ser Val Glu Asp Val Leu Phe
               260                 265                 270
   Lys Glu His Asp Cys Gly Asn Met Leu Gln Glu Thr Ala Ile Gln Leu
           275                 280                 285
   Leu Asp Gly Ala Thr Asn Thr Ile Glu Gly Ala Arg Val Gly Thr Ala
       290                 295                 300
   Lys Leu Thr Thr Trp Leu Gly Lys Gln Leu Gly Ile Leu Gly Lys Lys
   305                 310                 315                 320
   Leu Glu Asn Lys Ser Lys Ala Trp Phe Gly Ala His Ala Ala Ser Pro
                   325                 330                 335
   Tyr Cys Gly Val Glu Arg Lys Ile Gly Tyr Val Trp Tyr Thr Lys Asn
               340                 345                 350
   Cys Thr Pro Ala Cys Leu Pro Arg Asn Thr Arg Ile Ile Gly Pro Gly
           355                 360                 365
   Lys Phe Asp Thr Asn Ala Glu Asp Gly Lys Ile Leu His Glu Met Gly
       370                 375                 380
   Gly His Leu Ser Glu Phe Val Leu Leu Ser Leu Val Val Leu Ser Asp
   385                 390                 395                 400
   Phe Ala Pro Glu Thr Ala Ser Val Ile Tyr Leu Val Leu His Phe Ala
                   405                 410                 415
   Ile Pro Gln Ser His Val Asp Val Asp Thr Cys Asp Lys Asn Gln Leu
               420                 425                 430
   Asn Leu Thr Val Ala Thr Thr Val Ala Glu Val Ile Pro Gly Thr Val
           435                 440                 445
   Trp Asn Leu Gly Lys Tyr Val Cys Ile Arg Pro Asp Trp Trp Pro Tyr
       450                 455                 460
   Glu Thr Thr Thr Val Phe Val Ile Glu Glu Ala Gly Gln Val Ile Lys
   465                 470                 475                 480
   Leu Met Leu Arg Ala Ile Arg Asp Leu Thr Arg Ile Trp Asn Ala Ala
                   485                 490                 495
   Thr Thr Thr Ala Phe Leu Ile Phe Leu Val Lys Ala Leu Arg Gly Gln
               500                 505                 510
   Leu Ile Gln Gly Leu Leu Trp Leu Met Leu Ile Thr Gly Ala Gln Gly
           515                 520                 525
   Phe Pro Glu Cys Lys Glu Gly Phe Gln Tyr Ala Ile Ser Lys Asp Arg
       530                 535                 540
   Lys Met Gly Leu Leu Gly Pro Glu Ser Leu Thr Thr Thr Trp His Leu
   545                 550                 555                 560
   Pro Thr Lys Lys Ile Val Asp Ser Met Val His Val Trp Cys Glu Gly
                   565                 570                 575
   Lys Asp Leu Lys Ile Leu Lys Met Cys Thr Lys Glu Glu Arg Tyr Leu
               580                 585                 590
   Val Ala Val His Glu Arg Ala Leu Ser Thr Ser Ala Glu Phe Met Gln
           595                 600                 605
   Ile Ser Asp Gly Thr Ile Gly Pro Asp Val Ile Asp Met Pro Asp Asp
       610                 615                 620
   Phe Glu Phe Gly Leu Cys Pro Cys Asp Ser Lys Pro Val Ile Lys Gly
   625                 630                 635                 640
   Lys Phe Asn Ala Ser Leu Leu Asn Gly Pro Ala Phe Gln Met Val Cys
                   645                 650                 655
   Pro Gln Gly Trp Thr Gly Thr Ile Glu Cys Thr Leu Ala Asn Gln Asp
               660                 665                 670
   Thr Leu Asp Thr Thr Val Ile Arg Thr Tyr Arg Arg Thr Thr Pro Phe
           675                 680                 685
   Gln Arg Arg Lys Trp Cys Thr Tyr Glu Lys Ile Ile Gly Glu Asp Ile
       690                 695                 700
   Tyr Glu Cys Ile Leu Gly Gly Asn Trp Thr Cys Ile Thr Gly Asp His
   705 710 715 720
   Ser Arg Leu Lys Asp Gly Pro Ile Lys Lys Cys Lys Trp Cys Gly His
   725 730 735
   Asp Phe Val Asn Ser Glu Gly Leu Pro His Tyr Pro Ile Gly Lys Cys
   740 745 750
   Met Leu Ile Asn Glu Ser Gly Tyr Arg Tyr Val Asp Asp Thr Ser Cys
   755 760 765
   Asp Arg Gly Gly Val Ala Ile Val Pro Ser Gly Thr Val Lys Cys Arg
   770 775 780
   Ile Gly Asn Val Thr Val Gln Val Ile Ala Thr Asn Asn Asp Leu Gly
   785 790 795 800
   Pro Met Pro Cys Ser Pro Ala Glu Val Ile Ala Ser Glu Gly Pro Val
   805 810 815
   Glu Lys Thr Ala Cys Thr Phe Asn Tyr Ser Arg Thr Leu Pro Asn Lys
   820 825 830
   Tyr Tyr Glu Pro Arg Asp Arg Tyr Phe Gln Gln Tyr Met Leu Lys Gly
   835 840 845
   Glu Trp Gln Tyr Trp Phe Asp Leu Asp Ser Val Asp His His Lys Asp
   850 855 860
   Tyr Phe Ser Glu Phe Ile Ile Ile Ala Val Val Ala Leu Leu Gly Gly
   865 870 875 880
   Lys Tyr Val Leu Trp Leu Leu Ile Thr Tyr Thr Ile Leu Ser Glu Gln
   885 890 895
   Met Ala Met Gly Ala Gly Val Asn Thr Glu Glu Ile Val Met Ile Gly
   900 905 910
   Asn Leu Leu Thr Asp Ser Asp Ile Glu Val Val Val Tyr Phe Leu Leu
   915 920 925
   Leu Tyr Leu Ile Val Lys Glu Glu Leu Ala Arg Lys Trp Ile Ile Leu
   930 935 940
   Val Tyr His Ile Leu Val Ala Asn Pro Met Lys Thr Ile Gly Val Val
   945 950 955 960
   Leu Leu Met Leu Gly Gly Val Val Lys Ala Ser Arg Ile Asn Ala Asp
                   965                 970                 975
   Asp Gln Ser Ala Met Asp Pro Cys Phe Leu Leu Val Thr Gly Val Val
               980                 985                 990
   Ala Val Leu Met Ile Ala Arg Arg  Glu Pro Ala Thr Leu  Pro Leu Ile
           995                 1000                 1005
   Val Ala  Leu Leu Ala Ile Arg  Thr Ser Gly Phe Leu  Leu Pro Ala
       1010                 1015                 1020
   Ser Ile  Asp Val Thr Val Ala  Val Val Leu Ile Val  Leu Leu Leu
       1025                 1030                 1035
   Ala Ser  Tyr Ile Thr Asp Tyr  Phe Arg Tyr Lys Lys  Trp Leu Gln
       1040                 1045                 1050
   Leu Leu  Phe Ser Leu Ile Ala  Gly Ile Phe Ile Ile  Arg Ser Leu
       1055                 1060                 1065
   Lys His  Ile Asn Gln Met Glu  Val Pro Glu Ile Ser  Met Pro Ser
       1070                 1075                 1080
   Trp Arg  Pro Leu Ala Leu Val  Leu Phe Tyr Ile Thr  Ser Thr Ala
       1085                 1090                 1095
   Ile Thr  Thr Asn Trp Asp Ile  Asp Leu Ala Gly Phe  Leu Leu Gln
       1100                 1105                 1110
   Trp Ala  Pro Ala Val Ile Met  Met Ala Thr Met Trp  Ala Asp Phe
       1115                 1120                 1125
   Leu Thr  Leu Ile Ile Val Leu  Pro Ser Tyr Glu Leu  Ser Lys Leu
       1130                 1135                 1140
   Tyr Phe  Leu Lys Asn Val Arg  Thr Asp Val Glu Lys  Asn Trp Leu
       1145                 1150                 1155
   Gly Lys  Val Lys Tyr Arg Gln  Ile Ser Ser Val Tyr  Asp Ile Cys
       1160                 1165                 1170
   Asp Ser  Glu Glu Ala Val Tyr  Leu Phe Pro Ser Arg  His Lys Ser
       1175                 1180                 1185
   Gly Ser  Arg Pro Asp Phe Ile  Leu Pro Phe Leu Lys  Ala Val Leu
       1190                 1195                 1200
   Ile Ser Cys Ile Ser Ser Gln Trp Gln Val Val Tyr Ile Ser Tyr
   1205 1210 1215
   Leu Ile Leu Glu Ile Thr Tyr Tyr Met His Arg Lys Ile Ile Asp
   1220 1225 1230
   Glu Val Ser Gly Gly Ala Asn Phe Leu Ser Arg Leu Ile Ala Ala
   1235 1240 1245
   Ile Ile Glu Leu Asn Trp Ala Ile Asp Asp Glu Glu Cys Lys Gly
   1250 1255 1260
   Leu Lys Lys Leu Tyr Leu Leu Ser Gly Arg Ala Lys Asn Leu Ile
   1265 1270 1275
   Val Lys His Lys Val Arg Asn Glu Ala Val His Arg Trp Phe Gly
   1280 1285 1290
   Glu Glu Glu Ile Tyr Gly Ala Pro Lys Val Ile Thr Ile Ile Lys
   1295 1300 1305
   Ala Ser Thr Leu Ser Lys Asn Arg His Cys Ile Ile Cys Thr Ile
   1310 1315 1320
   Cys Glu Gly Lys Glu Trp Asn Gly Ala Asn Cys Pro Lys Cys Gly
   1325 1330 1335
   Arg Gln Gly Lys Pro Ile Thr Cys Gly Met Thr Leu Ala Asp Phe
   1340 1345 1350
   Glu Glu Lys His Tyr Lys Lys Ile Phe Ile Arg Glu Glu Ser Ser
   1355 1360 1365
   Cys Pro Val Pro Phe Asp Pro Ser Cys His Cys Asn Tyr Phe Arg
   1370 1375 1380
   His Asp Gly Pro Phe Arg Lys Glu Tyr Lys Gly Tyr Val Gln Tyr
   1385 1390 1395
   Thr Ala Arg Gly Gln Leu Phe Leu Arg Asn Leu Pro Ile Leu Ala
   1400 1405 1410
   Thr Lys Met Lys Leu Leu Met Val Gly Asn Leu Gly Ala Glu Ile
   1415 1420 1425
   Gly Asp Leu Glu His Leu Gly Trp Val Leu Arg Gly Pro Ala Val
   1430 1435 1440
   Cys Lys Lys Ile Thr Asn His Glu Lys Cys His Val Asn Ile Met
       1445                1450                1455
   Asp Lys Leu Thr Ala Phe Phe Gly Ile Met Pro Arg Gly Thr Thr
       1460                1465                1470
   Pro Arg Ala Pro Val Arg Phe Pro Thr Ala Leu Leu Lys Val Arg
       1475                1480                1485
   Arg Gly Leu Glu Thr Gly Trp Ala Tyr Thr His Gln Gly Gly Ile
       1490                1495                1500
   Ser Ser Val Asp His Val Thr Ala Gly Lys Asp Leu Leu Val Cys
       1505                1510                1515
   Asp Ser Met Gly Arg Thr Arg Val Val Cys His Ser Asn Asn Lys
       1520                1525                1530
   Met Thr Asp Glu Thr Glu Tyr Gly Ile Lys Thr Asp Ser Gly Cys
       1535                1540                1545
   Pro Glu Gly Ala Arg Cys Tyr Val Leu Asn Pro Glu Ala Val Asn
       1550                1555                1560
   Ile Ser Gly Thr Lys Gly Ala Met Val His Leu Gln Lys Thr Gly
       1565                1570                1575
   Gly Glu Phe Thr Cys Val Thr Ala Ser Gly Thr Pro Ala Phe Phe
       1580                1585                1590
   Asp Leu Lys Asn Leu Lys Gly Trp Ser Gly Leu Pro Ile Phe Glu
       1595                1600                1605
   Ala Ser Ser Gly Arg Val Val Gly Arg Val Lys Val Gly Lys Asn
       1610                1615                1620
   Glu Asp Ser Lys Pro Thr Lys Leu Met Ser Gly Ile Gln Thr Val
       1625                1630                1635
   Ser Lys Asn Gln Thr Asp Leu Ala Asp Ile Val Lys Lys Leu Thr
       1640                1645                1650
   Ser Met Asn Arg Gly Glu Phe Lys Gln Ile Thr Leu Ala Thr Gly
       1655                1660                1665
   Ala Gly Lys Thr Thr Glu Leu Pro Arg Ser Val Ile Glu Glu Ile
   1670 1675 1680
   Gly Arg His Lys Arg Val Leu Val Leu Ile Pro Leu Arg Ala Ala
   1685 1690 1695
   Ala Glu Ser Val Tyr Gln Tyr Met Arg Val Lys Tyr Pro Ser Ile
   1700 1705 1710
   Ser Phe Asn Leu Arg Ile Gly Asp Met Lys Glu Gly Asp Met Ala
   1715 1720 1725
   Thr Gly Ile Thr Tyr Ala Ser Tyr Gly Tyr Phe Cys Gln Leu Pro
   1730 1735 1740
   Gln Pro Lys Leu Arg Ala Ala Met Val Glu Tyr Ser Tyr Ile Phe
   1745 1750 1755
   Leu Asp Glu Tyr His Cys Ala Thr Pro Glu Gln Leu Ala Ile Ile
   1760 1765 1770
   Gly Lys Ile His Arg Phe Ala Glu Asn Leu Arg Val Val Ala Met
   1775 1780 1785
   Thr Ala Thr Pro Ala Gly Thr Val Thr Thr Thr Gly Gln Lys His
   1790 1795 1800
   Pro Ile Glu Glu Phe Ile Ala Pro Glu Val Met Lys Gly Glu Asp
   1805 1810 1815
   Leu Gly Ser Glu Tyr Leu Asp Ile Ala Gly Leu Lys Ile Pro Thr
   1820 1825 1830
   Glu Glu Met Lys Gly Asn Met Leu Val Phe Ala Pro Thr Arg Asn
   1835 1840 1845
   Met Ala Val Glu Thr Ala Lys Lys Leu Lys Ala Lys Gly Tyr Asn
   1850 1855 1860
   Ser Gly Tyr Tyr Tyr Ser Gly Glu Asn Pro Glu Asn Leu Arg Val
   1865 1870 1875
   Val Thr Ser Gln Ser Pro Tyr Val Val Val Ala Thr Asn Ala Ile
   1880 1885 1890
   Glu Ser Gly Val Thr Leu Pro Asp Leu Asp Thr Val Val Asp Thr
   1895 1900 1905
   Gly Leu Lys Cys Glu Lys Arg Val Arg Ile Ser Ser Lys Met Pro
   1910 1915 1920
   Phe Ile Val Thr Gly Leu Lys Arg Met Ala Val Thr Ile Gly Glu
   1925 1930 1935
   Gln Ala Gln Arg Arg Gly Arg Val Gly Arg Val Lys Pro Gly Arg
   1940 1945 1950
   Tyr Tyr Arg Ser Gln Glu Thr Ala Ser Gly Ser Lys Asp Tyr His
   1955 1960 1965
   Tyr Asp Leu Leu Gln Ala Gln Arg Tyr Gly Ile Glu Asp Gly Ile
   1970 1975 1980
   Asn Val Thr Lys Ser Phe Arg Glu Met Asn Tyr Asp Trp Ser Leu
   1985 1990 1995
   Tyr Glu Glu Asp Ser Leu Met Ile Thr Gln Leu Glu Val Leu Asn
   2000 2005 2010
   Asn Leu Leu Ile Ser Glu Asp Leu Pro Ala Ala Val Lys Asn Ile
   2015 2020 2025
   Met Ala Arg Thr Asp His Pro Glu Pro Ile Gln Leu Ala Tyr Asn
   2030 2035 2040
   Ser Tyr Glu Asn Gln Ile Pro Val Leu Phe Pro Lys Ile Lys Asn
   2045 2050 2055
   Gly Glu Val Thr Asp Ser Tyr Glu Asn Tyr Thr Tyr Leu Asn Ala
   2060 2065 2070
   Arg Lys Leu Gly Glu Asp Val Pro Ala Tyr Val Tyr Ala Thr Glu
   2075 2080 2085
   Asp Glu Asp Leu Ala Val Asp Leu Leu Gly Met Asp Trp Pro Asp
   2090 2095 2100
   Pro Gly Asn Gln Gln Val Val Glu Thr Gly Arg Ala Leu Lys Gln
   2105 2110 2115
   Val Thr Gly Leu Ser Thr Ala Glu Asn Ala Leu Leu Ile Ala Leu
   2120 2125 2130
   Phe Gly Tyr Val Gly Tyr Gln Thr Leu Ser Lys Arg His Ile Pro
   2135 2140 2145
   Met Ile  Thr Asp Ile Tyr Thr  Leu Glu Asp His Arg  Leu Glu Asp
       2150                 2155                 2160
   Thr Thr  His Leu Gln Phe Ala  Pro Asn Ala Ile Arg  Thr Asp Gly
       2165                 2170                 2175
   Lys Asp  Ser Glu Leu Lys Glu  Leu Ala Val Gly Asp  Leu Asp Lys
       2180                 2185                 2190
   Tyr Val  Asp Ala Leu Val Asp  Tyr Ser Lys Gln Gly  Met Lys Phe
       2195                 2200                 2205
   Ile Lys  Val Gln Ala Glu Lys  Val Arg Asp Ser Gln  Ser Thr Lys
       2210                 2215                 2220
   Glu Gly  Leu Gln Thr Ile Lys  Glu Tyr Val Asp Lys  Phe Ile Gln
       2225                 2230                 2235
   Ser Leu  Thr Glu Asn Lys Glu  Glu Ile Ile Arg Tyr  Gly Leu Trp
       2240                 2245                 2250
   Gly Val  His Thr Ala Leu Tyr  Lys Ser Leu Ala Ala  Arg Leu Gly
       2255                 2260                 2265
   His Glu  Thr Ala Phe Ala Thr  Leu Val Val Lys Trp  Leu Ala Phe
       2270                 2275                 2280
   Gly Gly  Glu Thr Val Ser Ala  His Ile Lys Gln Val  Ala Val Asp
       2285                 2290                 2295
   Leu Val  Val Tyr Tyr Ile Ile  Asn Lys Pro Ser Phe  Pro Gly Asp
       2300                 2305                 2310
   Thr Glu  Thr Gln Gln Glu Gly  Arg Arg Phe Val Ala  Ser Leu Phe
       2315                 2320                 2325
   Ile Ser  Ala Leu Ala Thr Tyr  Thr Tyr Lys Thr Trp  Asn Tyr Asn
       2330                 2335                 2340
   Asn Leu  Gln Arg Val Val Glu  Pro Ala Leu Ala Tyr  Leu Pro Tyr
       2345                 2350                 2355
   Ala Thr  Ser Ala Leu Lys Leu  Phe Thr Pro Thr Arg  Leu Glu Ser
       2360                 2365                 2370
   Val Val  Ile Leu Ser Ser Thr  Ile Tyr Lys Thr Tyr  Leu Ser Ile
       2375                 2380                 2385
   Arg Lys Gly Lys Ser Asp Gly Leu Leu Gly Thr Gly Ile Ser Ala
       2390                2395                2400
   Ala Met Glu Ile Leu Asn Gln Asn Pro Ile Ser Val Gly Ile Ser
       2405                2410                2415
   Val Met Leu Gly Val Gly Ala Ile Ala Ala His Asn Ala Ile Glu
       2420                2425                2430
   Ser Ser Glu Gln Lys Arg Thr Leu Leu Met Lys Val Phe Val Lys
       2435                2440                2445
   Asn Phe Leu Asp Gln Ala Ala Thr Asp Glu Leu Val Lys Glu Asn
       2450                2455                2460
   Pro Glu Lys Ile Ile Met Ala Leu Phe Glu Ala Val Gln Thr Ile
       2465                2470                2475
   Gly Asn Pro Leu Arg Leu Ile Tyr His Leu Tyr Gly Val Tyr Tyr
       2480                2485                2490
   Lys Gly Trp Glu Ala Lys Glu Leu Ala Glu Lys Thr Ala Gly Arg
       2495                2500                2505
   Asn Leu Phe Thr Leu Ile Met Phe Glu Ala Phe Glu Leu Leu Gly
       2510                2515                2520
   Met Asp Ser Glu Gly Lys Ile Arg Asn Leu Ser Gly Asn Tyr Ile
       2525                2530                2535
   Leu Asp Leu Ile Phe Asn Leu His Asn Lys Leu Asn Lys Gly Leu
       2540                2545                2550
   Lys Lys Leu Val Leu Gly Trp Ala Pro Ala Pro Leu Ser Cys Asp
       2555                2560                2565
   Trp Thr Pro Ser Asp Glu Arg Ile Ser Leu Pro His Asn Asn Tyr
       2570                2575                2580
   Leu Arg Val Glu Thr Arg Cys Pro Cys Gly Tyr Glu Met Lys Ala
       2585                2590                2595
   Ile Lys Asn Val Ala Gly Lys Leu Thr Lys Val Glu Glu Lys Gly
       2600                2605                2610
   Ser Phe Leu Cys Arg Asn Arg Leu Gly Arg Gly Pro Pro Asn Phe
   2615 2620 2625
   Lys Val Thr Lys Phe Tyr Asp Asp Asn Leu Ile Glu Val Lys Pro
   2630 2635 2640
   Val Ala Arg Leu Glu Gly Gln Val Asp Leu Tyr Tyr Lys Gly Val
   2645 2650 2655
   Thr Ala Lys Leu Asp Tyr Asn Asn Gly Lys Val Leu Leu Ala Thr
   2660 2665 2670
   Asn Lys Trp Glu Val Asp His Ala Phe Leu Thr Arg Leu Val Lys
   2675 2680 2685
   Lys His Thr Gly Ile Gly Phe Lys Gly Ala Tyr Leu Gly Asp Arg
   2690 2695 2700
   Pro Asp His Gln Asp Leu Val Asp Arg Asp Cys Ala Thr Ile Thr
   2705 2710 2715
   Lys Asn Ser Val Gln Phe Leu Lys Met Lys Lys Gly Cys Ala Phe
   2720 2725 2730
   Thr Tyr Asp Leu Thr Ile Ser Asn Leu Val Arg Leu Ile Glu Leu
   2735 2740 2745
   Val His Lys Asn Asn Leu Gln Glu Arg Glu Ile Pro Thr Val Thr
   2750 2755 2760
   Val Thr Thr Trp Leu Ala Tyr Ser Phe Val Asn Glu Asp Leu Gly
   2765 2770 2775
   Thr Ile Lys Pro Val Leu Gly Glu Lys Val Ile Pro Glu Pro Pro
   2780 2785 2790
   Glu Glu Leu Ser Leu Gln Pro Thr Val Arg Leu Val Thr Thr Glu
   2795 2800 2805
   Thr Ala Ile Thr Ile Thr Gly Glu Ala Glu Val Met Thr Thr Gly
   2810 2815 2820
   Ile Thr Pro Val Val Glu Met Lys Glu Glu Pro Gln Leu Asp His
   2825 2830 2835
   Gln Ser Thr Thr Leu Lys Val Gly Leu Lys Glu Gly Glu Tyr Pro
   2840 2845 2850
   Gly Pro  Gly Val Asn Pro Asn  His Leu Ala Glu Val  Ile Asp Glu
       2855                 2860                 2865
   Lys Asp  Asp Arg Pro Phe Val  Leu Ile Ile Gly Asn  Lys Gly Ser
       2870                 2875                 2880
   Thr Ser  Asn Arg Ala Arg Thr  Ala Lys Asn Ile Arg  Leu Tyr Lys
       2885                 2890                 2895
   Gly Asn  Asn Pro Arg Glu Ile  Arg Asp Leu Met Ser  Gln Gly Arg
       2900                 2905                 2910
   Ile Leu  Thr Val Ala Leu Lys  Glu Leu Asp Pro Glu  Leu Lys Glu
       2915                 2920                 2925
   Leu Val  Asp Tyr Lys Gly Thr  Phe Leu Asn Arg Glu  Ala Leu Glu
       2930                 2935                 2940
   Ala Leu  Ser Leu Gly Lys Pro  Ile Lys Arg Lys Thr  Thr Thr Ala
       2945                 2950                 2955
   Met Ile  Arg Arg Leu Ile Glu  Pro Glu Val Glu Glu  Glu Leu Pro
       2960                 2965                 2970
   Asp Trp  Phe Gln Ala Glu Glu  Pro Leu Phe Leu Glu  Ala Lys Ile
       2975                 2980                 2985
   Gln Asn  Asp Leu Tyr His Leu  Ile Gly Ser Val Asp  Ser Ile Lys
       2990                 2995                 3000
   Ser Lys  Ala Lys Glu Leu Gly  Ala Thr Asp Asn Thr  Lys Ile Val
       3005                 3010                 3015
   Lys Glu  Val Gly Ala Arg Thr  Tyr Thr Met Lys Leu  Ser Ser Trp
       3020                 3025                 3030
   Ser Thr  Gln Val Thr Lys Lys  Gln Met Ser Leu Ala  Pro Leu Phe
       3035                 3040                 3045
   Glu Glu  Leu Leu Leu Lys Cys  Pro Pro Cys Ser Lys  Ile Ser Lys
       3050                 3055                 3060
   Gly His  Met Val Ser Ala Tyr  Gln Leu Ala Gln Gly  Asn Trp Glu
       3065                 3070                 3075
   Pro Leu  Gly Cys Gly Val Tyr  Met Gly Thr Ile Pro  Ala Arg Arg
       3080                 3085                 3090
   Leu Lys Ile His Pro Tyr Glu Ala Tyr Leu Lys Leu Lys Glu Leu
   3095 3100 3105
   Val Glu Val Glu Ser Ser Arg Ala Thr Ala Lys Glu Ser Ile Ile
   3110 3115 3120
   Arg Glu His Asn Thr Trp Ile Leu Arg Lys Val Arg His Glu Gly
   3125 3130 3135
   Asn Leu Arg Thr Lys Ser Met Ile Asn Pro Gly Lys Ile Ser Asp
   3140 3145 3150
   Gln Leu Cys Arg Asp Gly His Lys Arg Asn Ile Tyr Asn Lys Ile
   3155 3160 3165
   Ile Gly Ser Thr Met Ala Ser Ala Gly Ile Arg Leu Glu Lys Leu
   3170 3175 3180
   Pro Val Val Arg Ala Gln Thr Asp Thr Thr Ser Phe His Gln Ala
   3185 3190 3195
   Ile Arg Glu Lys Ile Asp Lys Thr Glu Asn Lys Gln Thr Pro Glu
   3200 3205 3210
   Leu His Glu Glu Leu Met Lys Val Phe Asp Cys Leu Lys Ile Pro
   3215 3220 3225
   Glu Leu Lys Glu Ser Tyr Asp Glu Val Ser Trp Glu Gln Leu Glu
   3230 3235 3240
   Ala Gly Ile Asn Arg Lys Gly Ala Ala Gly Tyr Leu Glu Ser Lys
   3245 3250 3255
   Asn Ile Gly Glu Val Leu Asp Thr Glu Lys His Ile Val Glu Gln
   3260 3265 3270
   Leu Ile Lys Asp Leu Arg Lys Gly Lys Lys Ile Arg Tyr Tyr Glu
   3275 3280 3285
   Thr Ala Ile Pro Lys Asn Glu Lys Arg Asp Val Ser Asp Asp Trp
   3290 3295 3300
   Glu Ala Gly Glu Phe Val Asp Glu Lys Lys Pro Arg Val Ile Gln
   3305 3310 3315
   Tyr Pro Asp Ala Lys Val Arg Leu Ala Ile Thr Lys Val Met Tyr
   3320 3325 3330
   Lys Trp Val Lys Gln Lys Pro Val Val Ile Pro Gly Tyr Glu Gly
   3335 3340 3345
   Lys Thr Pro Leu Phe Asp Ile Phe Asn Lys Val Lys Lys Glu Trp
   3350 3355 3360
   Asp Ser Phe Gln Asp Pro Val Ala Val Ser Phe Asp Thr Lys Ala
   3365 3370 3375
   Trp Asp Thr Gln Val Thr Ser Arg Asp Leu Met Leu Ile Lys Asp
   3380 3385 3390
   Ile Gln Lys Tyr Tyr Phe Lys Arg Ser Ile His Lys Phe Leu Asp
   3395 3400 3405
   Thr Ile Thr Glu His Met Val Glu Val Pro Val Ile Thr Ala Asp
   3410 3415 3420
   Gly Glu Val Tyr Ile Arg Asn Gly Gln Arg Gly Ser Gly Gln Pro
   3425 3430 3435
   Asp Thr Ser Ala Gly Asn Ser Met Leu Asn Val Leu Thr Met Ile
   3440 3445 3450
   Tyr Ala Phe Cys Lys Ser Thr Gly Ile Pro Tyr Arg Gly Phe Ser
   3455 3460 3465
   Arg Val Ala Arg Ile His Val Cys Gly Asp Asp Gly Phe Leu Ile
   3470 3475 3480
   Thr Glu Arg Gly Leu Gly Leu Lys Phe Ser Glu Lys Gly Met Gln
   3485 3490 3495
   Ile Leu His Glu Ala Gly Lys Pro Gln Lys Ile Thr Glu Gly Asp
   3500 3505 3510
   Lys Met Lys Val Ala Tyr Arg Phe Glu Asp Ile Glu Phe Cys Ser
   3515 3520 3525
   His Thr Pro Val Pro Val Arg Trp Ala Asp Asn Thr Ser Ser Tyr
   3530 3535 3540
   Met Ala Gly Arg Ser Thr Ala Thr Ile Leu Ala Lys Met Ala Thr
   3545 3550 3555
   Arg Leu Asp Ser Ser Gly Glu Arg Gly Ser Thr Ala Tyr Glu Lys
   3560 3565 3570
   Ala Val Ala Phe Ser Phe Leu Leu Met Tyr Ser Trp Asn Pro Val
   3575 3580 3585
   Val Arg Arg Ile Cys Leu Leu Val Leu Ser Gln Phe Pro Glu Ile
   3590 3595 3600
   Ser Pro Ser Lys Asn Thr Ile Tyr Tyr Tyr Gln Gly Asp Pro Ile
   3605 3610 3615
   Ala Ala Tyr Arg Glu Val Ile Gly Lys Gln Leu Cys Glu Leu Lys
   3620 3625 3630
   Arg Thr Gly Phe Glu Lys Leu Ala Gly Leu Asn Leu Ser Met Thr
   3635 3640 3645
   Thr Leu Gly Ile Trp Thr Lys His Thr Ser Lys Arg Leu Ile Gln
   3650 3655 3660
   Ala Cys Val Glu Ile Gly Lys Arg Glu Gly Thr Trp Leu Val Asn
   3665 3670 3675
   Ala Asp Arg Leu Ile Ala Gly Lys Thr Gly Lys Phe Tyr Ile Pro
   3680 3685 3690
   Ser Thr Gly Val Thr Leu Leu Gly Lys His Tyr Glu Glu Ile Asn
   3695 3700 3705
   Leu Lys Gln Lys Ala Ala Gln Pro Pro Ile Glu Gly Val Asp Arg
   3710 3715 3720
   Tyr Lys Leu Gly Pro Ile Val Asn Val Ile Leu Arg Arg Leu Arg
   3725 3730 3735
   Val Met Leu Met Thr Val Ala Ser Gly Ser Trp
   3740 3745
<210> 13
   <211> 13
   <212> PRT
   <213> Artificial sequence BVDV
<400> 13
   Ser Leu His Gly Ile Trp Phe Glu Lys Ile Cys Thr Gly
   1 5 10
<210> 14
   <211> 20
   <212> PRT
   <213> Artificial sequence BVDV
<400> 14
   Leu Gln Arg His Glu Trp Asn Lys His Gly Trp Cys Asn Trp Phe His
   1               5                   10                  15
   Ile Glu Pro Trp
               20
<210> 15
   <211> 20
   <212> PRT
   <213> Artificial sequence BVDV
<400> 15
   Leu Gln Arg His Glu Trp Asn Lys His Gly Trp Cys Asn Trp Tyr Asn
   1               5                   10                  15
   Ile Glu Pro Trp
               20
<210> 16
   <211> 11
   <212> PRT
   <213> Artificial sequence BVDV
<400> 16
   Ser Leu His Gly Ile Trp Pro Glu Lys Ile Cys
   1               5                   10
<210> 17
   <211> 12
   <212> PRT
   <213> Artificial sequence BVDV
<400> 17
   Arg His Glu Trp Asn Lys His Gly Trp Cys Asn Trp
   1               5                   10
<210> 18
   <211> 4
   <212> PRT
   <213> Artificial sequence BVDV
<400> 18
   Met Glu Leu Phe
   1
<210> 19
   <211> 6
   <212> PRT
   <213> Artificial sequence BVDV
<400> 19
   Met Glu Leu Phe Ser Asn
   1 5
<210> 20
   <211> 7
   <212> PRT
   <213> Artificial sequence BVDV
<400> 20
   Met Glu Leu Phe Ser Asn Glu
   1 5
<210> 21
   <211> 8
   <212> PRT
   <213> Artificial sequence BVDV
<400> 21
   Met Glu Leu Phe Ser Asn Glu Leu
   1 5
<210> 22
   <211> 9
   <212> PRT
   <213> Artificial sequence BVDV
<400> 22
   Met Glu Leu Phe Ser Asn Glu Leu Leu
   1 5
<210> 23
   <211> 10
   <212> PRT
   <213> Artificial sequence BVDV
<400> 23
   Met Glu Leu Phe Ser Asn Glu Leu Leu Tyr
   1 5 10
<210> 24
   <211> 11
   <212> PRT
   <213> Artificial sequence BVDV
<400> 24
   Met Glu Leu Phe Ser Asn Glu Leu Leu Tyr Lys
   1 5 10
<210> 25
   <211> 12
   <212> PRT
   <213> Artificial sequence BVDV
<400> 25
   Met Glu Leu Phe Ser Asn Glu Leu Leu Tyr Lys Thr
   1 5 10
<210> 26
   <211> 6
   <212> PRT
   <213> Artificial sequence BVDV
<400> 26
   Ser Asn Glu Gly Ser Lys
   1 5
<210> 27
   <211> 6
   <212> PRT
   <213> Artificial sequence BVDV
<400> 27
   Ser Asp Glu Gly Ser Lys
   1 5
<210> 28
   <211> 8
   <212> PRT
   <213> Artificial sequence BVDV
<400> 28
   Met Glu Ser Asp Gln Gly Ser Lys
   1 5
<210> 29
   <211> 11
   <212> PRT
   <213> Artificial sequence BVDV
<400> 29
   Met Glu Leu Phe Ser Ser Asp Gln Gly Ser Lys
   1 5 10
<210> 30
   <211> 13
   <212> PRT
   <213> Artificial sequence BVDV
<400> 30
   Met Glu Leu Phe Ser Asn Glu Ser Asp Gln Gly Ser Lys
   1 5 10
<210> 31
   <211> 14
   <212> PRT
   <213> Artificial sequence BVDV
<400> 31
<210> 32
   <211> 15
   <212> PRT
   <213> Artificial sequence BVDV
<400> 32
   Met Glu Leu Phe Ser Asn Glu Leu Leu Ser Asp Gln Gly Ser Lys
   1 5 10 15
<210> 33
   <211> 16
   <212> PRT
   <213> Artificial sequence BVDV
<400> 33
   Met Glu Leu Phe Ser Asn Glu Leu Leu Tyr Ser Asp Gln Gly Ser Lys
   1 5 10 15
<210> 34
   <211> 17
   <212> PRT
   <213> Artificial sequence BVDV
<400> 34
   Met Glu Leu Phe Ser Asn Glu Leu Leu Tyr Lys Ser Asp Gln Gly Ser
   1 5 10 15
   Lys
<210> 35
   <211> 18
   <212> PRT
   <213> Artificial sequence BVDV
<400> 35
   Met Glu Leu Phe Ser Asn Glu Leu Leu Tyr Lys Thr Ser Asp Gln Gly
   1 5 10 15
   Ser Lys
<210> 36
   <211> 12
   <212> PRT
   <213> Artificial sequence BVDV
<400> 36
   Met Glu Leu Ile Ser Asn Glu Leu Leu Tyr Lys Thr
   1               5                   10
<210> 37
   <211> 12
   <212> PRT
   <213> Artificial sequence BVDV
<400> 37
   Met Glu Leu Ile Thr Asn Glu Leu Leu Tyr Lys Thr
   1               5                   10
<210> 38
   <211> 12
   <212> PRT
   <213> Artificial sequence BVDV
<400> 38
   Met Glu Leu Asn His Phe Glu Leu Leu Tyr Lys Thr
   1               5                   10
<210> 39
   <211> 12
   <212> PRT
   <213> Artificial sequence BVDV
<400> 39
   Met Glu Leu Asn Lys Phe Glu Leu Leu Tyr Lys Thr
   1               5                   10

## Description

### BACKGROUND OF THE INVENTION

### TECHNICAL FIELD

The present invention relates to the field of animal health and in particular to combinantion vaccines which comprise an attenuated bovine viral diarrhea virus (BVDV) and at least one further immunological active component for treating or preventing diseases or disorders in cattle caused by infectious agents.

### BACKGROUND INFORMATION

***Bovine viral diarrhea virus*** (BVDV) type 1 (BVDV-1) and type 2 (BVDV-2) cause bovine viral diarrhea (BVD) and mucosal disease (MD) in cattle (Baker, 1987; Moennig and Plagemann, 1992; Thiel et al., 1996). The division of BVDV into 2 species is based on significant differences at the level of genomic sequences (summarized in Heinz et al., 2000) which are also obvious from limited cross neutralizing antibody reactions (Ridpath et al. 1994). The viral proteins of BVDV, and any other virus of the pestivirus family, are arranged in the polyprotein in the order NH₂-N^{pro}-C-E^{rns}-E1-E2-p7-NS2-NS3-NS4A-NS4B-NS5A-NS5B-COOH (Lindenbach and Rice, 2001). Protein C (= core- or capsidprotein) and the glycoproteins E^{rns}, E1 and E2 represent structural components of the BVDV. E^{rns} and E2 were found to be targets for antibody neutralization (Donis et al., 1988; Paton et al., 1992; van Rijn et al., 1993; Weiland et al., 1990,1992). E^{rns} lacks a typical membrane anchor and is secreted in considerable amounts from the infected cells; this protein has been reported to exhibit RNase activity (Hulst et al., 1994; Schneider et al., 1993; Windisch et al., 1996). The function of this enzymatic activity for the viral life cycle is presently unknown. The enzymatic activity depends on the presence of two stretches of amino acids conserved between the pestivirus E^{rns} and different known RNases of plant and fungal origin. Both of these conserved sequences contain a histidine residue (Schneider et al., 1993). Exchange of each of these residues against lysine in the E^{rns} protein of a Classical Swine Fever Virus (CSFV) vaccine strain resulted in the destruction of RNase activity (Hulst et al., 1998). Introduction of these mutations into the genome of the CSFV vaccine strain did not influence viral viability or growth properties but led to a virus exhibiting a cytopathogenic phenotype (Hulst et al., 1998). Similarly, Meyers et al. showed that an RNase negative variant of the virulent CSFV strain Alfort/Tübingen was fully viable. However, the respective virus mutant showed no cytopathogenic phenotype (Meyers et al., 1999).

N^{pro} represents the first protein encoded by the long open reading frame in the pestivirus RNA. N^{pro} represents a nonstructural protein that has protease activity and cleaves itself of the nascent polyprotein (Stark et al., 1993; Wiskerchen et al., 1991) presumably already during translation. N^{pro} is a cysteine protease (Rümenapf et al., 1998) that is not essential for virus replication (Tratschin et al., 1998). Recently, it was shown that N^{pro} somehow interferes with the cellular antiviral defense so that it can be hypothesized to modulate the immune system within an infected host (Rüggli et al., 2003). Mayer and coworkers presented indications for an attenuation of CSFV in consequence of a deletion of the N^{pro} gene (Mayer et al., 2004).

Present BVDV vaccines for the prevention and treatment of BVDV infections still have drawbacks (Oirschot et al. 1999). Vaccines against the classical BVDV-1 provide only partial protection from BVDV-2 infection, and vaccinated dams may produce calves that are persistently infected with virulent BVDV-2 (Bolin et al., 1991, Ridpath et al., 1994). This problem is probably due to the great antigenic diversity between type 1 and type 2 strains which is most pronounced in the glycoprotein E2, the major antigen for virus neutralization (Tijssen et al., 1996). Most monoclonal antibodies against type 1 strains fail to bind to type 2 viruses (Ridpath et al., 1994).

Currently, licensed BVDV MLV vaccines are produced using attenuated viruses obtained via repeated passage in bovine or porcine cells (Coggins et al.,Cornell Vet. 51: 539- , 1961; Philips et al., Am. J. Vet. Res. 36: 135-, 1975), or using chemically modified viruses which exhibit a temperature-sensitive phenotype (Lobmann et al., Am. J. Vet. Res. 45: 2498-, 1984; 47: 557-561,1986). A single dose of MLV vaccine is sufficient for immunization, and duration of the immunity can last for years in vaccinated cattle. However, as these vaccines have been developed using type I BVDV virus strains, the protection is against type I virus only. Moreover, these vaccines, although attenuated, are most often associated with safety problems. The vaccine viruses may cross the placenta of pregnant animals, e.g. cows and lead to clinical manifestations in the fetus and/or the induction of persistently infected calves. Therefore, they cannot be applied to breeding herds that contain pregnant cows. Pregnant cows have to be kept separate from vaccinated cattle to protect fetuses and must not be vaccinated themselves.

***Parainfluenza-3 virus*** (PI-3) is an RNA virus classified in the paramyxovirus family. Infections caused by PI-3 are common in cattle. Although PI-3 is capable of causing disease, it is usually associated with mild to subclinical infections. The most important role of PI-3 is to serve as an initiator that can lead to the development of secondary bacterial pneumonia. Clinical signs include pyrexia, cough, serous nasal and lacrimal discharge, increased respiratory rate, and increased breath sounds. The severity of signs worsen with the onset of bacterial pneumonia. Fatalities from uncomplicated PI-3 pneumonia are rare. Lesions include cranioventral lung consolidation, bronchiolitis, and alveolitis with marked congestion and hemorrhage. Inclusion bodies may be identified. Most fatal cases will also have a concurrent bacterial bronchopneumonia.

***Bovine Respiratory Syncytial Virus*** (BRSV) is an RNA virus classified as a pneumovirus in the paramyxovirus family. In addition to cattle, sheep and goats can also be infected by respiratory syncytial viruses. This virus was named for its characteristic cytopathic effect-the formation of syncytial cells. Antigenic subtypes are known to exist for BRSV, and preliminary evidence suggests that there may be antigenic subtypes of BRSV. BRSV is distributed worldwide, and the virus is indigenous in the cattle population. BRSV infections associated with respiratory disease occur predominantly in young beef and dairy cattle. Passively derived immunity does not appear to prevent BRSV infections but will reduce the severity of disease. Initial exposures to the virus are associated with severe respiratory disease; subsequent exposures result in mild to subclinical disease. BRSV appears to be an important virus in the bovine respiratory disease complex because of its frequency of occurrence, predilection for the lower respiratory tract, and its ability to predispose the respiratory tract to secondary bacterial infection. In outbreaks, morbidity tends to be high, and case fatality can be 0-20%. Signs include increased rectal temperature 40-42°C, depression, decreased feed intake, increased respiratory rate, cough, and nasal and lacrimal discharge. Generally, respiratory signs predominate. Dyspnea may become pronounced in the later stages of the disease. Subcutaneous emphysema is sometimes reported. Secondary bacterial pneumonia is a frequent occurrence. A biphasic disease pattern has been described but is not consistent. Gross lesions include a diffuse interstitial pneumonia with subpleural and interstitial emphysema along with interstitial edema. These lesions are similar to and must be differentiated from other causes of interstitial pneumonia. *See also* atypical interstitial pneumonia. Histologic examination reveals syncytial cells in bronchiolar epithelium and lung parenchyma, intracytoplasmic inclusion bodies, proliferation and/or degeneration of bronchiolar epithelium, alveolar epithelialization, edema, and hyaline membrane formation.

***Bovine Herpesvirus (BHV-1)*** is associated with several diseases and symptoms in cattle: Infectious bovine rhinotracheitis (IBR), infectious pustular vulvovaginitis (IPV), balanoposthitis, conjunctivitis, abortion, encephalomyelitis, and mastitis. Only a single serotype of BHV-1 is recognized; however, three subtypes of BHV-1 have been described on the basis of endonuclease cleavage patterns of viral DNA. These types are referred to as BHV-1.1 (respiratory subtype), BHV-1.2 (genital subtype), and BHV-1.3 (encephalitic subtype). Recently, BHV-1.3 has been reclassified as a distinct herpesvirus designated BHV-5. BHV-1 infections are widespread in the cattle population. In feedlot cattle, the respiratory form is most common. The viral infection alone is not life-threatening but predisposes cattle to secondary bacterial pneumonia, which may result in death. In breeding cattle, abortion or genital infections are more common. Genital infections can occur in bulls (infectious pustular balanoposthitis) and cows (IPV) within 1-3 days of mating or close contact with an infected animal. Transmission can occur in the absence of visible lesions and through artificial insemination with semen from subclinically infected bulls. Cattle with latent BHV-1 infections generally show no clinical signs when the virus is reactivated, but they do serve as a source of infection for other susceptible animals and thus perpetuate the disease. The incubation period for the respiratory and genital forms is 2-6 days. In the respiratory form, clinical signs range from mild to severe, depending on the presence of secondary bacterial pneumonia. Clinical signs include pyrexia, anorexia, coughing, excessive salivation, nasal discharge that progresses from serous to mucopurulent, conjunctivitis with lacrimal discharge, inflamed nares (hence the common name "red nose"), and dyspnea if the larynx becomes occluded with purulent material. Pustules may develop on the nasal mucosa and later form diphtheritic plaques. Conjunctivitis with corneal opacity may develop as the only manifestation of BHV-1 infection. In the absence of bacterial pneumonia, recovery generally occurs 4-5 days after the onset of clinical signs. Abortions may occur concurrently with respiratory disease but can also occur up to 100 days after infection. Abortions can occur regardless of the severity of disease in the dam. Abortions generally occur during the second half of pregnancy, but early embryonic death may also occur. The first signs of genital infections in cows are frequent urination, elevation of the tailhead, and a mild vaginal discharge. The vulva is swollen, and small papules, then erosions and ulcers, are present on the mucosal surface. If secondary bacterial infections do not occur, animals recover in 10-14 days. If bacterial infection occurs, there may be inflammation of the uterus and transient infertility, with purulent vaginal discharge for several weeks. In bulls, similar lesions occur on the penis and prepuce. BHV-1 infection can be severe in young calves and cause a generalized disease. Pyrexia, ocular and nasal discharges, respiratory distress, diarrhea, incoordination, and eventually convulsions and death may occur in a short period after generalized viral infection. IBR is rarely fatal in cattle unless complicated by bacterial pneumonia. In uncomplicated IBR infections, most lesions are restricted to the upper respiratory tract and trachea. Petechial to ecchymotic hemorrhages may be found in the mucous membranes of the nasal cavity and the paranasal sinuses. Focal areas of necrosis develop in the nose, pharynx, larynx, and trachea. The lesions may coalesce to form plaques. The sinuses are often filled with a serous or serofibrinous exudate. As the disease progresses, the pharynx becomes covered with a serofibrinous exudate, and blood-tinged fluid may be found in the trachea. The pharyngeal and pulmonary lymph nodes may be acutely swollen and hemorrhagic. The tracheitis may extend into the bronchi and bronchioles; when this occurs, epithelium is sloughed in the airways. The viral lesions are often masked by secondary bacterial infections. In young animals with generalized BHV-1 infection, erosions and ulcers overlaid with debris may be found in the nose, esophagus, and forestomachs. In addition, white foci may be found in the liver, kidney, spleen, and lymph nodes. Aborted fetuses may have pale, focal, necrotic lesions in all tissues, but which are especially visible in the liver.

A number of other Bovine Respiratory Viruses have been identified as being involved in BRD. ***Bovine herpesvirus-4*** has been implicated in several diseases, including BRD. Bovine adenovirus has been associated with a wide spectrum of diseases, with bovine adenovirus type 3 being the serotype most often associated with BRD. Two serotypes of bovine rhinovirus have been recognized to cause respiratory tract infections in cattle. Other viruses reported to be associated with BRD include ***bovine reovirus, enterovirus,*** and ***coronavirus.*** These viruses have a role similar to the other viruses previously discussed in that, in combination with other stressors, they can serve as initiators of bacterial pneumonia. ***Bovine coronavirus*** is also commonly associated with diarrhea in calves. It replicates in the epithelium of the upper respiratory tract and in the enterocytes of the intestine, where it produces similar lesions to rotavirus but also infects the epithelial cells of the large intestine to produce atrophy of the colonic ridges. Vaccines are not available for prevention of these viral respiratory diseases.

***Bovine rotavirus*** is the most common viral cause of diarrhea in calves. Group A and B rotavirus are involved, but group A is the most prevalent and clinically important and contains several serotypes of differing virulence. Rotavirus replicates in the mature absorptive and enzyme-producing enterocytes on the villi of the small intestine, leading to rupture and sloughing of the enterocytes with release of virus to infect adjacent cells. Rotavirus does not infect the immature cells of the crypts. With virulent strains of rotavirus, the loss of enterocytes exceeds the ability of the intestinal crypts to replace them; hence, villous height is reduced, with a consequent decrease in intestinal absorptive surface area and intestinal digestive enzyme activity.

Other viruses, including Breda virus, a calici-like virus, ***Adenovirus, Astrovirus*** and ***Parvovirus,*** have been demonstrated in the feces of calves with diarrhea and can produce diarrhea in calves experimentally. However, these agents can also be demonstrated in the feces of healthy calves. The importance of these agents in the syndrome of neonatal diarrhea has yet to be determined. ***Manheimia haemolytica*** (formerly *Pasteurella haemolytica*) biotype A, serotype 1 is the bacterium most frequently isolated from the lungs of cattle with BRD. Although less frequently cultured than *M. haemolytica, **Pasteurella multocida*** is also an important cause of bacterial pneumonia. When pulmonary abscessation occurs, generally in association with chronic pneumonia, ***Actinomyces pyogenes*** is frequently isolated. Under normal conditions, *M. haemolytica* remains confined to the upper respiratory tract, in particular the tonsillar crypts, and is difficult to culture from healthy cattle. After stress or viral infection, the replication rate of *M. haemolytica* in the upper respiratory tract increases rapidly, as does the likelihood of culturing the bacterium. The increased bacterial growth rate and colonization of the lungs may be due to suppression of the host's defense mechanism related to environmental stressors or viral infections. It is during this log phase of growth that virulence factors are elaborated by *M. haemolytica* , such as an exotoxin that has been referred to as leukotoxin. The interaction between the virulence factors of the bacteria and host defenses results in tissue damage and development of pneumonia. Clinical signs of bacterial pneumonia are often preceded by signs of viral infection of the respiratory tract. With the onset of bacterial pneumonia, the severity of clinical signs increases and are characterized by depression and toxemia. There will be pyrexia (40-41°C); serous to mucopurulent nasal discharge; moist cough; and a rapid, shallow respiratory rate. Auscultation of the cranioventral lung field reveals increased bronchial sounds, crackles, and wheezes. In severe cases, pleurisy may develop, which is characterized by an irregular breathing pattern and grunting on expiration. The animal will become unthrifty in appearance if the pneumonia becomes chronic, which is usually associated with the formation of pulmonary abscesses. *M. haemolytica* causes a severe, acute fibrinous pneumonia or fibrinonecrotic pneumonia. The pneumonia has a bronchopneumonic pattern. Grossly, there is extensive reddish black to grayish brown cranioventral regions of consolidation with gelatinous thickening of interlobular septa and fibrinous pleuritis. There are extensive thromboses, foci of lung necrosis, and limited evidence of bronchitis and bronchiolitis. *P. multocida* is associated with a less fulminating fibrinous to fibrinopurulent bronchopneumonia. In contrast to *M. haemolytica, P. multocida* is associated with only small amounts of fibrin exudation, some thromboses, limited lung necrosis, and suppurative bronchitis and bronchiolitis.

***Haemophilus somnus*** is being increasingly recognized as an important pathogen in BRD; these bacteria are normal inhabitants of the nasopharynx of cattle. *H. somnus* infection of the lungs results in purulent bronchopneumonia that may be followed by septicemia and infection of multiple organs. Occasionally, *H. somnus* is associated with extensive pleuritis. *H. somnus* can cause an acute, usually fatal, septicemic disease that can involve the nervous, musculoskeletal, circulatory, and respiratory systems, either singly or together. The reproductive system is often affected but usually without the other systems being clinically involved. The disease may be characterized by fever, severe depression, ataxia, weakness, blindness, coma, and death within several hours to several days. It occurs sporadically in individual beef and dairy cattle and is found nearly worldwide. *H. somnus* is a gram-negative, nonmotile, nonsporeforming, pleomorphic coccobacillus that requires an enriched medium and a microaerophilic atmosphere for culture. It appears to be identical to ***Histophilus ovis*** and ***Haemophilus agni**,* etiologic agents of ovine septicemia, mastitis, and epididymitis; however, transmission of *H. somnus* between sheep and cattle has not been demonstrated. Pathogenic and nonpathogenic strains have been differentiated by intracisternal inoculation of young calves with organisms from various sources. Pathogenic and nonpathogenic strains of *H. somnus* are carried in the sheath and prepuce of males, the vagina of female cattle, and in the nasal passages of both sexes. The organism may colonize the respiratory tract, presumably after inhalation, and is frequently found in urine. Prevalence of the organism in cattle is probably high because high titers of specific antibodies are found in a large proportion of tested cattle. Several disease syndromes caused by *H. somnus* have been recognized, including thrombomeningoencephalitis, fibrinopurulent bronchopneumonia, fibrinous pleuritis, and polyarthritis. Myocardial and skeletal muscle necrosis occur. Suppurative vaginitis, cervicitis, and endometritis have been documented in cows infected experimentally and naturally after breeding, and the organism is a cause of sporadic abortion. Strains of *H. somnus* that cause disease adhere to the endothelium of vessels, resulting in contraction, exposure of collagen, platelet adhesion, and thrombosis. TME results when this occurs in the brain and associated membranes, after invasion of the organism into the bloodstream of susceptible cattle. Strains may adhere to endothelium in vessels of the pleura, myocardium, synovium, or a variety of other tissues and produce inflammation in those sites (e.g., infections of the larynx and middle ear have been recorded). The susceptibility of individual animals and variations in the preference of strains of the organism for vessels in different tissues may be important in the development of the form of disease, but the mechanisms involved are incompletely understood. Reproductive problems may not necessarily be preceded by bacteremia, but the pathogenesis is poorly defined. A fever as high as 42°C is often the first sign of disease; however, this usually falls to normal or subnormal within hours. Other findings are determined by the system(s) involved and may include rapid respiration, stiffness, knuckling at the fetlocks, severe depression, ataxia, paralysis, and opisthotonos, followed by coma and death within several hours. Affected animals may be blind, and retinal hemorrhages with gray foci of retinal necrosis are sometimes seen. Signs such as hypersensitivity, convulsions, excitement, nystagmus, and circling occur inconsistently and may be related to the regions of the CNS affected in the course of disease development. Occasionally, animals are found dead, indicating a rapidly fatal course. A marked change in the total and differential WBC count is common; leukopenia and neutropenia occur in severe, usually acute, fatal disease, while neutrophilia may be present in less severe disease. In TME, the total cell count of the CSF is markedly increased, and neutrophils predominate. During septicemia, the organism can be recovered from blood, synovial fluid, CSF, brain, kidneys, urine, and a variety of other organs. The lesions are characterized by vascular thrombosis and infarction of the surrounding tissue. Randomly distributed red to brown foci of necrosis with hemorrhage on the surface and cut sections of the brain and spinal cord, retina, skeletal muscle, myocardium, kidney, intestine, and spleen are characteristic. A fibrinopurulent meningitis with cloudy CSF may sometimes be seen on the surface of the brain and spinal cord, and a polyserositis, especially of joints and pleura, may occur. An acute fibrinous bronchopneumonia with tissue necrosis may develop after airborne infections.

The exact role of mycoplasmas and ureaplasmas in BRD requires better definition. Mycoplasmas can be recovered from the respiratory tract of nonpneumonic calves, but the frequency of isolation is greater in those with respiratory tract disease. The mycoplasmas commonly recovered from the lungs of pneumonic calves include ***Mycoplasma dispar, Mycoplasma bovis,*** and ***Ureaplasma* spp.** Experimental infections usually result in inapparent to mild signs of respiratory disease. This does not preclude a synergistic role for mycoplasmas in conjunction with viruses and bacteria in BRD. Lesions described include peribronchial and peribronchiolar lymphoid cuffing and alveolitis. Culture of these organisms requires special media and conditions and may take up to a week for growth of the organisms.

Chlamydiae have been identified in various parts of the world as a cause of enzootic pneumonia in calves. The causative agent is ***Chlamydia psittaci***. Some respiratory isolates from calves have properties of immunotypes 1 and 6 and are similar to strains recovered from intestinal infections and abortions of cattle and sheep. Immunotype 6 has been recovered from pneumonic lungs of calves and pigs. Thus, the GI tract must be considered as an important site in the pathogenesis of chlamydial infections and as a natural reservoir and source of the organisms. Chlamydial pneumonia has affected calves under a whole range of conditions as well as on dairy farms. A synergism between *Chlamydia* and *P. haemolytica* has been demonstrated experimentally. Calves with chlamydial pneumonia are usually febrile, lethargic, and dyspneic, and have a serous and later mucopurulent nasal discharge and a dry hacking cough. Calves of weanling age are affected most frequently, but older cattle may also show signs. The acute pulmonary lesion is a bronchointerstitial pneumonia. The anteroventral parts of the lungs are affected but, in severe cases, entire lobes can be involved. The dry cough is attributed to tracheitis. Microscopic changes in the lungs include suppurative bronchitis and alveolitis progressing to type II pneumocyte hyperplasia and interstitial thickening.

Bovine genital campylobacteriosis is a venereal disease of cattle characterized primarily by early embryonic death, infertility, a protracted calving season, and occasionally, abortion. Distribution is probably worldwide. The cause is the motile, gram-negative, curved or spiral, polar flagellated bacterium ***Campylobacter fetus venerealis*** or ***Campylobacter fetus fetus***. For many years, it was thought that *C. fetus fetus* (formerly *C. fetus intestinalis*) was generally an intestinal organism, only occasionally caused abortion in cattle, and was not a cause of infertility. However, it has been shown that *C. fetus fetus* can also be a significant cause of the classic infertility syndrome usually attributed to *Campylobacter fetus venerealis.* There are several strains of *C. fetus fetus,* and the only way to determine if a strain is a cause of infertility is to test that possibility in a group of heifers. *Campylobacter* spp are very labile and are destroyed quickly by heating, drying, and exposure to the atmosphere. Unless cultured quickly after collection from the animal and grown under microaerophilic or anaerobic conditions, campylobacters will not grow. *Campylobacter fetus* is transmitted venereally and also by contaminated instruments, bedding, or by artificial insemination using contaminated semen. Individual bulls vary in their susceptibility to infection because some become permanent carriers, while others appear to be resistant to infection. Bulls can also transmit the infection mechanically for several hours after copulating with an infected cow. In cows, the duration of the carrier state is also variable; some clear the infection rapidly, while others can carry *C. fetus* for ≥ 2 yr. IgA antibodies are shed in cervical mucus in significant amounts in -50% of cows for several months after infection and are useful diagnostically. Although most of the genital tract may be free of infection when a cow eventually conceives, the vagina may remain chronically infected, even through pregnancy. Cows are systemically normal, but there is a variable degree of mucopurulent endometritis that causes early embryonic death, prolonged luteal phases, irregular estrous cycles, repeat breeding and, as a result, protracted calving periods. Observed abortions are not common. In herds not managed intensively, disease may be noticed only when pregnancy examinations reveal low or marginally low pregnancy rates but, more importantly, great variations in gestation lengths, especially when the disease has recently been introduced to the herd. In subsequent years, infertility is usually confined to replacement heifers and a few susceptible cows. Bulls are asymptomatic and produce normal semen.

Leptospirosis is a contagious disease of animals, including man, caused by various immunologically distinct leptospiral serovars, most of which are regarded as subgroups of ***Leptospira interrogans.*** Infections may be asymptomatic or cause various signs, including fever, icterus, hemoglobinuria, renal failure, infertility, abortion, and death. After acute infection, leptospires frequently localize in the kidneys or reproductive organs and are shed in the urine, sometimes in large numbers for months or years. Because the organisms survive in surface waters for extended periods, the disease is often waterborne. In the USA, the disease is primarily due to the serovars ***Leptospira hardjo, Leptospira pomona,*** and ***Leptospira grippotyphosa**.* However, ***Leptospira canicola*** and ***Leptospira icterohaemorrhagiae*** serovars also have been isolated. Calves may have fever, anorexia, and dyspnea, and in *Leptospira pomona* infections, icterus, hemoglobinuria, and anemia. Body temperature may rise suddenly to 40.5-41°C. Hemoglobinuria rarely lasts longer than 48-72 hrs. Icterus clears rapidly and is followed by anemia. The RBC's begin to increase in number by 4-5 days and return to normal 7-10 days later. However, *Leptospira hardjo* infections usually do not cause hemolytic anemia, which makes diagnosis more difficult. Morbidity and mortality are higher in calves than in adult cattle. In older cattle, signs vary greatly and diagnosis is more difficult. Enzootic *Leptospira hardjo* infections, which usually result in abnormal milk, are more obvious in dairy than in beef cattle. Signs usually are restricted to lowered milk and calf production; a hemolytic crisis does not occur. The milk is thick, yellow, and blood-tinged; it may contain clots, although there is little evidence of mammary inflammation. Milk production returns to normal in 10-14 days, even in the absence of treatment. Abortion and stillbirths, which are common in *Leptospira pomona* infections and sporadic in *Leptospira hardjo* infections, generally occur 3-10 weeks after initial infection. The abortions are more common during the third trimester. An abortion storm in a breeding herd is often the first indication that leptospirosis exists, because the mild initial signs often pass unnoticed. In endemically infected herds, abortions occur mostly in younger animals and are sporadic, rather than being manifested as abortion storms. Calves reared by previously infected cows are protected by colostral antibodies for up to 6 mos. The calves generally have an antibody titer similar to that of their dams. In the acute form, anemia, icterus, hemoglobinuria, and submucosal hemorrhages are prominent. The kidneys are swollen, with multifocal petechial and ecchymotic hemorrhages that become pale with time. The liver may be swollen, with minute areas of focal necrosis. Petechiae in other organs are seen in fulminating cases; however, in the more prevalent *Leptospira hardjo* infections, the lesions are primarily restricted to the kidneys.

Brucellosis is caused by bacteria of the genus *Brucella* and is characterized by abortion, retained placenta, and to a lesser extent, orchitis and infection of the accessory sex glands in males. The disease in cattle, water buffalo, and bison is caused almost exclusively by ***Brucella abortus***; however, ***Brucella suis*** or ***Brucella melitensis*** is occasionally implicated in some cattle herds. *Brucella suis* does not appear to be contagious from cow to cow. Infection spreads rapidly and causes many abortions in unvaccinated herds. Typically, in a herd in which disease is endemic, an infected cow aborts only once after exposure; subsequent gestations and lactations appear normal. After exposure, many cattle become bacteremic for a short period and develop agglutinins and other antibodies; others resist infection, and a small percentage of infected cows recover. A positive serum agglutination test usually precedes abortion or a normal parturition but may be delayed in -15% of animals. The incubation period may be variable and is related to the stage of gestation at time of exposure. Organisms are shed in milk and uterine discharges, and the cow may become temporarily sterile. Bacteria may be found in the uterus during pregnancy, uterine involution, and infrequently, for a prolonged time in the nongravid uterus. Shedding from the vagina largely disappears with reduction of the fluids after parturition. Some infected cows that aborted previously shed brucellae from the uterus at subsequent normal parturitions. Organisms are shed in milk for a variable length of time-in most cattle for life. Natural transmission occurs by ingestion of organisms, which are present in large numbers in aborted fetuses, fetal membranes, and uterine discharges. Cattle may ingest contaminated feed and water, or lick contaminated genitals of other animals. Venereal transmission by infected bulls to susceptible cows appears to be rare. Transmission may occur by artificial insemination when *Brucella* -contaminated semen is deposited in the uterus but, reportedly, not when deposited in the midcervix. Brucellae may enter the body through mucous membranes, conjunctivae, wounds, or even intact skin. Mechanical vectors (eg, other animals, including man) may spread infection. Brucellae have been recovered from fetuses and from manure that has remained in a cool environment for > 2 mo. Exposure to direct sunlight kills the organisms within a few hours. Abortion is the most obvious manifestation. Infections may also cause stillborn or weak calves, retained placentas, and reduced milk yield. Usually, general health is not impaired in uncomplicated abortions. Seminal vesicles, ampullae, testicles, and epididymides may be infected in bulls; therefore, organisms are in the semen. Agglutinins may be demonstrated in seminal plasma from infected bulls. Testicular abscesses may occur. Long-standing infections may result in arthritic joints in some cattle.

***Actinomyces (Corynebacterium) pyogenes*** causes sporadic abortion in the last trimester. Rarely, the incidence in a herd may reach enzootic (64%) levels. The bacteria are present on mucous membranes of many normal cows, as well as in uterine and abscess discharges. They gain entry to the bloodstream and cause an endometritis and placentitis, which is diffuse with a reddish brown to brown color. The fetus is usually autolyzed, with fibrinous pericarditis, pleuritis, or peritonitis possible.

Clostridia are relatively large, anaerobic, spore-forming, rod-shaped organisms. The spores are oval, sometimes spherical, and are central, subterminal, or terminal in position. The vegetative forms of clostridia in tissue fluids of infected animals occur singly, in pairs, or rarely in chains. Differentiation of the various pathogenic and related species is based on cultural characteristics, spore shape and position, biochemical reactions, and the antigenic specificity of toxins or surface antigens. The natural habitats of the organisms are the soil and intestinal tract of animals, including man. Pathogenic strains may be acquired by susceptible animals either by wound contamination or by ingestion. Diseases thus produced are a constant threat to successful livestock production in many parts of the world.

***Clostridium haemolyticum*** is a soil-borne organism that may be found naturally in the GI tract of cattle. It can survive for long periods in contaminated soil or in bones from carcasses of animals that had been infected. After ingestion, latent spores ultimately become lodged in the liver. The incubation period is extremely variable, and the onset depends on the presence of a locus of anaerobiosis in the liver. Such a nidus for germination is most often caused by fluke infection, much less often by high nitrate content of the diet, accidental liver puncture, liver biopsy, or any other cause of localized necrosis. When conditions for anaerobiosis are favorable, the spores germinate, and the resulting vegetative cells multiply and produce β toxin (phospholipase C), which causes intravascular hemolysis and its sequelae, including hemolytic anemia and hemoglobinuria. Cattle may be found dead without premonitory signs. Usually, there is a sudden onset of severe depression, fever, abdominal pain, dyspnea, dysentery, and hemoglobinuria. Anemia and jaundice are present in varying degrees. Edema of the brisket may occur. Hgb and RBC levels are quite low. The duration of clinical signs varies from -12 hr in pregnant cows to -3-4 days in other cattle. The mortality in untreated animals is -95%. Some cattle suffer from subclinical attacks of the disease and thereafter act as immune carriers. Dehydration, anemia, and sometimes subcutaneous edema are present. There is bloody fluid in the abdominal and thoracic cavities. The lungs are not grossly affected, and the trachea contains bloody froth with hemorrhages in the mucosa. The small intestine and occasionally the large intestine are hemorrhagic; their contents often contain free or clotted blood. An anemic infarct in the liver is virtually pathognomonic; it is slightly elevated, lighter in color than the surrounding tissue, and outlined by a bluish red zone of congestion. The kidneys are dark, friable, and usually studded with petechiae. The bladder contains purplish red urine. After death, *rigor mortis* sets in more rapidly than usual.

***Clostridium chauvoei*** occurs naturally in the intestinal tract of animals. It probably can remain viable in the soil for many years, although it does not actively grow there. Contaminated pasture appears to be a source of organisms. Outbreaks of blackleg have occurred in cattle on farms in which recent excavations have occurred, which suggests that disturbance of soil may activate latent spores. The organisms probably are ingested, pass through the wall of the GI tract, and after gaining access to the bloodstream, are deposited in muscle and other tissues. In cattle, blackleg infection is endogenous, in contrast to malignant edema. Lesions develop without any history of wounds, although bruising or excessive exercise may precipitate some cases. Commonly, the animals that contract blackleg are of the beef breeds, in excellent health, gaining weight, and usually the best animals of their group. Outbreaks occur in which a few new cases are found each day for several days. Most cases occur in cattle from 6 months to 2 years old, but thrifty calves as young as 6 weeks and cattle as old as 10-12 years may be affected. The disease usually occurs in summer and fall and is uncommon during the winter. In sheep, the disease is not restricted to the young, and most cases follow some form of injury such as shearing cuts, docking, crutching, or castration. Usually, onset is sudden and a few cattle may be found dead without premonitory signs. Acute lameness and marked depression are common. Initially, there is a fever but, by the time clinical signs are obvious, the temperature may be normal or subnormal. Characteristic edematous and crepitant swellings develop in the hip, shoulder, chest, back, neck, or elsewhere. At first, the swelling is small, hot, and painful. As the disease rapidly progresses, the swelling enlarges, there is crepitation on palpation, and the skin becomes cold and insensitive as the blood supply to the area diminishes. General signs include prostration and tremors. Death occurs in 12-48 hrs. In some cattle, the lesions are restricted to the myocardium and the diaphragm, with no reliable ante mortem evidence of the localized lesion.

***Clostridium novyi*** has been suspected but not yet confirmed as a cause of sudden death in cattle and pigs fed high-level grain diets, and in which pre-existing lesions of the liver were not detectable. The lethal and necrotizing toxins (primarily α toxin) damage hepatic parenchyma, thereby permitting the bacteria to multiply and produce a lethal amount of toxin. Usually, death is sudden with no well-defined signs. Affected animals tend to lag behind the flock, assume sternal recumbency, and die within a few hours. Most cases occur in the summer and early fall when liver fluke infection is at its height. The disease is most prevalent in 1- to 4-year-old sheep and is limited to animals infected with liver flukes. Differentiation from acute fascioliasis may be difficult, but peracute deaths of animals that show typical lesions on necropsy should arouse suspicion of infectious necrotic hepatitis. The most characteristic lesions are the grayish yellow necrotic foci in the liver that often follow the migratory tracks of the young flukes. Other common findings are an enlarged pericardial sac filled with straw-colored fluid, and excess fluid in the peritoneal and thoracic cavities. Usually, there is extensive rupture of the capillaries in the subcutaneous tissue, which causes the adjacent skin to turn black (hence the common name, black disease).

***Clostridium septicum*** is found in soil and intestinal contents of animals (including man) throughout the world. Infection ordinarily occurs through contamination of wounds containing devitalized tissue, soil, or some other tissue-debilitant. Wounds caused by accident, castration, docking, insanitary vaccination, and parturition may become infected. General signs, such as anorexia, intoxication, and high fever, as well as local lesions, develop within a few hours to a few days after predisposing injury. The local lesions are soft swellings that pit on pressure and extend rapidly because of the formation of large quantities of exudate that infiltrates the subcutaneous and intramuscular connective tissue of the affected areas. The muscle in such areas is dark brown to black. Accumulations of gas are uncommon. Severe edema of the head of rams occurs after infection of wounds inflicted by fighting. Malignant edema associated with lacerations of the vulva at parturition is characterized by marked edema of the vulva, severe toxemia, and death in 24-48 hours. Similarity to blackleg is marked, and differentiation made on necropsy is unreliable; laboratory confirmation is the only certain procedure. Horses and pigs are susceptible to malignant edema but not to blackleg.

Infectious disease caused by ***Clostridium sordellii*** are also characterized by a nongaseous, nonhemorrhagic, edematous swelling of the head, face, and neck of young rams. This infection is initiated in young rams by their continual butting of one another. The bruised and battered subcutaneous tissues provide conditions suitable for growth of pathogenic clostridia, and the breaks in the skin offer an opportunity for their entrance

Infection with ***C. perfringens* types A, B and C** causes severe enteritis, dysentery, toxemia, and high mortality in young calves. Types B and C both produce the highly necrotizing and lethal β toxin that is responsible for the severe intestinal damage. This toxin is sensitive to proteolytic enzymes, and disease is associated with inhibition of proteolysis in the intestine. Sow colostrum, which contains a trypsin inhibitor, has been suggested as a factor in the susceptibility of young piglets. Type C also causes enterotoxemia in adult cattle. In calves, there is acute diarrhea, dysentery, abdominal pain, convulsions, and opisthotonos. Death may occur in a few hours, but less severe cases survive for a few days, and recovery over a period of several days is possible. Hemorrhagic enteritis with ulceration of the mucosa is the major lesion in all species. Grossly, the affected portion of the intestine is deep blue-purple and appears at first glance to be an infarction associated with mesenteric torsion. Smears of intestinal contents can be examined for large numbers of gram-positive, rod-shaped bacteria, and filtrates made for detection of toxin and subsequent identification by neutralization with specific antiserum.

This classic enterotoxemia caused by ***C. perfringens* type D** rarely occurs in cattle. It is worldwide in distribution and may occur in animals of any age. The disease has been suspected in well-nourished beef calves nursing high-producing cows grazing lush pasture and in sudden death syndrome in feedlot cattle; however, supportive laboratory evidence in the latter is lacking. Acutely affected calves not found dead show mania, convulsions, blindness, and death in a few hours. Subacutely affected calves are stuporous for a few days and may recover.

Tetanus toxemia is caused by a specific neurotoxin produced by ***Clostridium tetani*** in necrotic tissue. Almost all mammals are susceptible to this disease. Although tetanus is worldwide in distribution, there are some areas, such as the northern Rocky Mountain section of the USA, where the organism is rarely found in the soil and where tetanus is almost unknown. In general, the occurrence of C tetani in the soil and the incidence of tetanus in man and horses is higher in the warmer parts of the various continents. Clostridium tetani , an anaerobe with terminal, spherical spores, is found in soil and intestinal tracts. In most cases, it is introduced into the tissues through wounds, particularly deep puncture wounds, that provide a suitable anaerobic environment.

Infection with ***Salmonella spp*** can produce diarrhea in animals of all ages, especially those that are stressed, closely stocked, or exposed to a heavily contaminated feed or water supply. Salmonellosis is caused by many species of salmonellae and characterized clinically by one or more of three major syndromes-septicemia, acute enteritis, and chronic enteritis. The incidence has increased with the intensification of livestock production. Young calves usually develop the septicemic form. Adult cattle, develop acute enteritis. Conic enteritis may develop occasionally in cattle. Pregnant animals may abort. In older animals, the disease is manifested by dysentery and toxemia, and mortality can be significant. While many other Salmonella spp may cause disease, the more relevant in cattle are ***S. typhimurium, S. dublin,*** and ***S. newport.*** Although their resulting clinical patterns are not distinct, different species of salmonellae tend to differ in their epidemiology. Plasmid profile and drug-resistance patterns are sometimes useful markers for epidemiologic studies. Feces of infected animals can contaminate feed and water, milk, fresh and processed meats from abattoirs, plant and animal products used as fertilizers or feedstuffs, pasture and rangeland, and many inert materials. The organisms may survive for months in wet, warm areas such as in feeder pig barns or in water dugouts but survive less than 1 week in composted cattle manure. Rodents and wild birds also are sources of infection. The prevalence of infection varies among species and countries and is much higher than the incidence of clinical disease, which is commonly precipitated by stressful situations such as sudden deprivation of feed, transportation, drought, crowding, parturition, and the administration of some drugs.

Further relevant gastro-intestinal pathogens are ***Cryptosporidium parvum*** and ***Mycobacterium avium paratuberculosis.*** Paratuberculosis is a chronic, contagious enteritis characterized by persistent and progressive diarrhea, weight loss, debilitation, and eventually death. It affects cattle, sheep, goats, llamas, camels, farmed deer, and other domestic, exotic, and wild ruminants. It has also been recognized in wild rabbits; horses and pigs can be infected experimentally. Distribution is worldwide. There are conflicting data on the involvement of the organism in Crohn's disease, a chronic enteritis in people. Animals with paratuberculosis should be considered as potential zoonotic risks until the situation is clarified. The causative organism is ***Mycobacterium avium paratuberculosis,*** formerly known as ***M. paratuberculosis*** or ***M. johnei.*** Occasionally, other *M. avium* subspecies are isolated from cases. The organism is quite resistant and can survive on pasture for more than 1 year, but sunlight, alkaline soils, and drying reduce its survival rate. It is shed in large numbers in feces of infected animals, and infection is acquired by ingestion of contaminated feed and water. Introduction of the disease into a clean herd is usually by subclinically infected carriers. Infection is acquired early in life, but clinical signs rarely develop in cattle < 2 yrs old. Resistance increases with age, and cattle first exposed as adults are unlikely to become infected. Most calves are infected soon after birth either by nursing udders contaminated with feces from infected animals or by being housed in contaminated pens. The organism can also be present in colostrum and milk of infected cows, and intrauterine infections have also been described. After ingestion, the bacteria infect macrophages in the mucosa of the lower small intestine and in associated lymph nodes. Most animals will eliminate infection by an early cell-mediated immune response that encourages microbicidal activity in macrophages. In susceptible animals, the organisms multiply and provoke a chronic enteritis that leads to clinical disease. This may take months to years to develop and is usually paralleled by a decline in cell-mediated immunity and a rise in ineffective serum antibody. However, fecal shedding begins before clinical signs are apparent. *Mycobacterium avium paratuberculosis* can be isolated from feces, mesenteric and ileocecal lymph nodes, thickened intestinal walls, and less frequently the udder and the reproductive tracts of both sexes.

Cryptosporidiosis is an enterocolitis of cosmopolitan distribution caused by the coccidian parasite ***Cryptosporidium parvum.*** It is not host-specific and is common in young ruminants, particularly calves; it is also found in man and pigs and is rare in dogs, cats, and horses. Other cryptosporidia cause disease in reptiles and birds. The disease in calves, characterized by weight loss and watery diarrhea, is clinically indistinguishable from many other causes of calf diarrhea. *Cryptosporidium parvum* is a minute protozoan that is transmitted by the fecal-oral route. Oocysts are sporulated (four sporozoites) when shed in the feces and, therefore, are immediately infective. The mean incubation period is ∼4 days. Calves 1-3 weeks old seem to be most susceptible. Signs such as anorexia, weight loss, diarrhea, and tenesmus, resemble those caused by several other intestinal pathogens; however, infections without signs do occur. Uncomplicated cryptosporidiosis is seldom fatal. Disease can be severe in immunocompromised individuals. If severe disease in calves is seen, other disease agents or concurrent infections should be ruled out. Although *C. parvum* can infect virtually the entire intestinal tract, the distal small intestine usually is affected most severely. Infection in horses is limited to the small intestine. Gross lesions may consist of hyperemic intestinal mucosa and yellowish intestinal contents. Microscopically, mild to severe villous atrophy with spherical organisms in the brush border is evident. Unlike *Eimeria* and *Isospora* spp , which are intracellular parasites, *C. parvum* is intramembranous and resides within the brush border of the intestinal epithelial cells.

***Chlamydia psittaci*** causes sporadic abortion after the fourth month of gestation but usually in the last trimester. The chlamydia cause placentitis, fetal pneumonia, and hepatitis. Stained smears of cotyledons may reveal the organisms; if not, tissues may be cultured in embryonating chicken eggs. Abortion is usually sporadic in cows, but an ovine chlamydial vaccine has been used in cattle.

Inflammation of the mammary gland (mastitis) is almost always due to the effects of infection by bacterial or mycotic pathogens. Mastitis may be associated with infection by many other organisms, including ***Streptococcus uberis, Streptococcus dysgalactiae, Klebsiella spp. Pseudomonas aeruginosa, Actinomyces pyogenes, Mycoplasma spp, Nocardia asteroides, Serratia, Mycobacterium spp, Clostridium perfringens, Pasteurella spp, yeasts, and Prototheca spp.***

Dermatomycoses (Dermatophytosis) in animals are anthropozoonotic diseases of the skin and related tissue. Clinical symptoms are characterized by loss of hair in the affected area, hyperemia, scaling and asbestos-like scabs. Inflammation is often accompanied by suppuration. Dermatomycoses are often also characterized by localized infection of the skin. Dermatomycoses in animals carry a substantial socioeconomic impact. Diseased animals required prolonged treatment and can spread infection to both animals and humans. Dermatophytosis are caused by mycosis infections of ***Trichophyton spp.*** or ***Microsporum spp.*** Most relevant causes for cattle are ***Trichophyton verrucosum, Trichophyton mentagrophytes*** or ***Trichophyton sarkisovii.***

An infection of the lower respiratory tract, usually resulting in bronchitis or pneumonia, can be caused by any of several parasitic nematodes, including ***Dictyocaulus viviparus*** in cattle. This lungworm belongs to the superfamily *Trichostrongyloidea* and has direct life cycles. The cattle lungworm is common in northwest Europe and is the cause of severe outbreaks of "husk" or "hoose" in young grazing cattle. Because *D. viviparus* infection in cattle is the most economically important, it has been most investigated and many of the observations from it are applicable to the other species. Clinical disease usually develops on first exposure to sufficient infective larvae. In cattle, this usually occurs during their first season at pasture; however, an increase in the number of older cattle affected has been reported. Signs of lungworm infection range from moderate coughing with slightly increased respiratory rates to severe persistent coughing and respiratory distress and even failure. Reduced weight-gains, reduced milk yields, and weight loss accompany many infections in cattle. Patent subclinical infections can occur in all species. The most consistent signs in cattle are tachypnea and coughing.

Trichomoniasis is a venereal protozoal disease of cattle characterized primarily by early fetal death and infertility, resulting in extended calving intervals. Distribution is probably worldwide. The causative protozoan, ***Trichomonas** (**Tritrichomonas**) **foetus,*** is pyriform and ordinarily 10-15 × 5-10 µm, but there is considerable pleomorphism. It may become spherical when cultured in artificial media. At its anterior end, there are three flagella about the same length as the body of the parasite. An undulating membrane extends the length of the body and is bordered by a marginal filament that continues beyond the membrane as a posterior flagellum. Although *T foetus* can survive the process used for freezing semen, it is killed by drying or high temperatures. Trichomonas foetus is found in the genital tracts of cattle. When cows are bred naturally by an infected bull, 30-90% become infected, suggesting that strain differences exist. Variation in breed susceptibility to trichomoniasis may also exist. Bulls of all ages can remain infected indefinitely but this is less likely in younger males. By contrast, most cows are free of infection within 3 months after breeding. However, immunity is not long lasting and reinfection does occur. Transmission can also occur when the semen from infected bulls is used for artificial insemination. The most common sign is infertility caused by embryonic death. This results in repeat breeding and a prolonged calving season. Fetal death and abortions can also occur but are not as common as losses earlier in gestation. *Trichomonas foetus* has been found in vaginal cultures taken as late as 8 months of gestation and, apparently, live calves can be born to infected dams. Pyometra occasionally develops after breeding.

***Neospora caninum*** is an obligate intracellular protozoan parasite that has been confused previously with ***Toxoplasma gondii***. Only asexual stages are known, and they resemble *T gondii.* The complete life cycle of *N caninum* is unknown, but it can be transmitted transplacentally in dogs, cattle, goats, sheep, and cats, and subsequent offspring may be affected. Tachyzoites are 5-7 × 1-5 µm, depending on the stage of division. They divide by endodyogeny. Tachyzoites are found in myocytes, neural cells, dermal cells, macrophages, and other cells. Tissue cysts up to 100 µm in diameter are found in neural cells; the cyst wall is amorphous and up to 4 µm thick. Cysts have no septa and enclose slender 7 × 1.5 µm bradyzoites. In dairy cattle, *N caninum* is a major cause of abortion in many countries, particularly in the USA. Calves may be aborted, stillborn, born underweight, weak, or paralyzed, or they may become paralyzed within 4 weeks of birth. Non-suppurative encephalitis is the main lesion in aborted fetal tissues. Abortion can occur throughout gestation, and some cows may abort again; dams of these calves are clinically normal.

Babesiosis is caused by intraerythrocytic protozoan parasites of the genus *Babesia* . A wide range of domestic and wild animals and occasionally man is affected by the disease, which is transmitted by ticks and has a worldwide distribution. Two important species in cattle- ***Babesia bigemina*** and ***Babesia bovis*** - are widespread in tropical and subtropical areas and are the focus of this discussion. In endemic areas, two features are important in determining the risk of clinical disease: 1) calves have a degree of immunity (related both to colostral-derived antibodies and to age) that persists for ~6 months, and 2) animals that recover from *Babesia* infections are immune for life. Thus, at high levels of tick transmission, all newborn calves will become infected with *Babesia* by 6 mos. of age, show few if any clinical signs, and subsequently be immune. This situation of endemic stability can be upset by either a natural (eg, climatic) or artificial (eg, acaricide treatment) reduction in tick numbers to levels where tick transmission of *Babesia* to calves is insufficient to ensure all are infected during this critical early period. Other circumstances that can lead to clinical outbreaks include the introduction of susceptible cattle to endemic areas and the incursion of *Babesia* -infected ticks into previously tick-free areas. Strain variation in immunity has been demonstrated but is probably not of significance in the field. The acute disease generally runs a course of ∼1 week. The first sign is fever (frequently 41°C or higher), which persists throughout, and is accompanied later by inappetence, increased respiratory rate, muscle tremors, anemia, jaundice, and loss of weight with hemoglobinemia and hemoglobinuria in the final stages. CNS involvement due to sludging of parasitized erythrocytes in brain capillaries occurs frequently with *B. bovis* infection. Either constipation or diarrhea may be present. Pregnant cows often abort. With virulent strains of *B. bovis,* a hypotensive shock syndrome, combined with generalized nonspecific inflammation, coagulation disturbances, and erythrocytic stasis in capillaries, contribute to the pathogenesis. With most strains of *B. bigemina,* the pathogenic effects relate more directly to erythrocyte destruction. Animals that recover from the acute disease remain infected for a number of years with *B. bovis* and for a few months in the case of *B. bigemina*. No signs are apparent during this carrier state. Lesions include an enlarged and friable spleen; a swollen liver with an enlarged gallbladder containing thick granular bile; congested, dark-colored kidneys; and generalized anemia and jaundice. The urine is often, but not invariably, red. Other organs, including the brain and heart, may show congestion or petechial hemorrhages. The susceptibility of cattle breeds to *Babesia* infections varies; for example, Brahman cattle are more resistant to *B. bovis* infection than are British breeds.
D1 - EP1104676 A1 relates to "safe attenuated bovine viral diarrhea viruses for use in pregnant cows"
D2 - WO03023041 A2 relates to "Infectious bovine viral diarrhea virus clone"
D3 - US2005002966 A1 relates to "Attenuated pestiviruses"
D4 - US2004146854 A1 relates to "Attenuated forms of bovine viral diarrhea virus"
D5 - EP1013757 A2 relates to "Attenuated forms of bovine viral diarrhea virus"

### BRIEF DESCRIPTION OF THE DRAWINGS

Fig. 1 Serum neutralisation against NY93/C (BVDV type II)
Fig. 2 Serum neutralisation assay against KE9 (BVDV type I)
Fig. 3 Serum neutralisation assay against NY93/C (BVDV type II)

All subsequent sequences show the deleted regions indicated with dashes (-), which are also numbered, whereas the sequences in the sequence listing attached hereto are continuously numbered without the deleted regions or amino acid codons.

| | |
|---|---|
| SEQ ID NO:1 | XIKE-A-cDNA sequence |
| SEQ ID NO:2 | XIKE-A-NdN-cDNA sequence |
| SEQ ID NO:3 | XIKE-B-cDNA sequence |
| SEQ ID NO:4 | XIKE-B-NdN-cDNA |
| SEQ ID NO:5 | XIKE-A amino acid sequence |
| SEQ ID NO:6 | XIKE-A-NdN amino acid sequence |
| SEQ ID NO:7 | XIKE-B amino acid sequence |
| SEQ ID NO:8 | XIKE-B-NdN amino acid sequence |
| SEQ ID NO:9 | XIKE-C-NdN amino acid sequence |
| SEQ ID NO:10 | XIKE-C-NdN-cDNA sequence |
| SEQ ID NO:11 | XIKE-C-cDNA sequence |
| SEQ ID NO:12 | XIKE-C amino acid sequence |

### BRIEF SUMMARY OF THE INVENTION

The present invention relates to combination vaccines for the treatment and/or prophylaxis of cattle against microbiological infections, wherein one of the infections is caused by BVDV. The combination vaccine as described herein comprises at least one attenuated BVDV, wherein said attenuated BVDV comprises at least one mutation in the coding sequence for glycoprotein E^{rns} and at least another mutation in the coding sequence for N^{pro} which preferably leads to combined inactivation of the RNase activity residing in glycoprotein E^{rns} in addition to the inactivation of the (hypothesized) immunomodulating activity residing in N^{pro}. The dislcosure also relates to methods for producing such combination vaccines.

According to a preferred embodiment, the present invention relates to a combination vaccine for the treatment and/or prophylaxis of cattle against infections of the respiratory and reproductive system in cattle, wherein the combination vaccine comprises an attenuated BVDV as described herein and at least one further immunological active component effective for the treatment and/or prophylaxis of infections caused by Bovine Herpes virus (BHV), Bovine Respiratory Syncytial Virus (BRSV), Parainfluenza Virus (PI-3), Campylobacter fetus, Leptospira canicola, Leptospira grippotyphosa, Leptospira borgpetersenii, Leptospira prajitno, Leptospira icterohaemmorrhagiae, Leptospira hardjo, Leptospira bovis, Leptospira interrogans and/or Leptospira ponoma. According to a more preferred embodiment, the combination vaccine comprises an attenuated BVDV as described herein and at least one antigen of BHV, BRSV, PI-3, Campylobacter fetus, Leptospira canicola, Leptospira grippotyphosa, Leptospira borgpetersenii Leptospira prajitno, Leptospira hardjo (Leptospira hardjo prajitno and Leptospira hardjo-bovis), Leptospira icterohaemmorrhagiae, Leptospira bovis, Leptospira interrogans and/or Leptospira ponoma.

According to a more preferred embodiment, the present invention relates to a combination vaccine for the treatment and /or prophylaxis of cattle against viral infections of the respiratory and reproductive system in cattle caused by BVDV, PI-3, BRSV, IBR and/or BHV, wherein said vaccine comprises at least an attenuated BVDV as described herein and at least one further further immunological active component effective for the treatment and/or prophylaxis of infections caused by PI-3, BRSV IBR, and BHV. According to a further more preferred embodiment, the present invention relates to a combination vaccine for the treatment and /or prophylaxis of cattle against viral infections of the respiratory and reproductive system in cattle caused by BVDV, PI-3, IBR, BRSV and/or BHV, wherein said vaccine comprises at least an attenuated BVDV as described herein and at least one antigen of PI-3, IBR, BRSV and/or BHV.

### DETAILED DESCRIPTION OF THE INVENTION

### DEFINITIONS OF TERMS USED IN THE DESCRIPTION:

Before the embodiments of the present invention it must be noted that as used herein and in the appended claims, the singular forms "a", "an", and "the" include plural reference unless the context clearly dictates otherwise. Thus, for example, reference to "a BVDV" includes a plurality of such BVDV, reference to the "cell" is a reference to one or more cells and equivalents thereof known to those skilled in the art, and so forth. Unless defined otherwise, all technical and scientific terms used herein have the same meanings as commonly understood by one of ordinary skill in the art to which this invention belongs.

The term "BVDV" as used herein refers to all viruses belonging to species bovine viral diarrhea virus (BVDV) type 1 (BVDV-1) and BVDV type 2 (BVDV-2), including any sub-species such as 1a, 1b, 2a, 2,b, and the like in the genus *Pestivirus* within the family *Flaviviridae* (Heinz et al., 2000). The more classical BVDV type 1 strains and the more recently recognized BVDV type 2 strains display some limited but distinctive differences in nucleotide and amino acid sequences.

"Protein C" or "C protein" or "C-protein" as used herein relates to a structural component of the pestivirus virion (Thiel et al., 1991). "Protein C" is the capsid or core protein of pestiviruses. Said term, depending on the context, may also relate to the "Protein C" with one or several amino acids exchanges resulting from mutation of the encoding nucleotide sequence.

The term "N^{pro}" as understood herein relates to the first protein encoded by the viral open reading frame that cleaves itself from the rest of the synthesized polyprotein (Stark, et al., J. Virol. 67:7088-7093 (1993); Wiskerchen, et al., Virol. 65:4508-4514 (1991)). Said term, depending on the context, may also relate to the remaining "N^{pro}" amino acids after mutation of the encoding nucleotide sequence or to the coding nucleotide sequence for said protein itself. "Protease activity residing in N^{pro}" relates to the polypeptide cleavage activity of said "N^{pro}".

Inactivation of N^{pro} as used herein means the prevention or considerable reduction of the probable immunemodulating activity of N^{pro} by mutation. In a preferred embodiment this mutation prevents or considerably reduces the interference of N^{pro} with the induction of an interferon response by the infected cells as described by Rüggli et al., (2003). In this case, the inactivation of N^{pro} would allow the cell to mount a normal interferon response.

"Processing signal" as used herein relates to a substance that ensures the generation of a functional N-terminal of the C protein of the pestivirus, preferably of BVDV, in particular a substance selected from the group of ubiquitin, LC3, SUMO-1, NEDD8, GATE-16 and GABA(A)RAP. Also proteases selected from the group of Intein, picornavirus 3C, caridovirus 2A and p15 of rabbit hemorrhagic disease virus are understood as "processing signals" as used herein. Any other similar processing signal known to the skilled person that ensures the generation of a functional N-terminal of the C protein shall also be comprised in the term "processing signal".

"E^{rns}" as used herein relates to the glycoprotein E^{rns} which represents a structural component of the pestivirus virion (Thiel et al., 1991). E^{rns} lacks a typical membrane anchor and is secreted in considerable amounts from the infected cells; this protein has been reported to exhibit RNase activity (Hulst et al., 1994; Schneider et al., 1993; Windisch et al., 1996). It should be noted that the term glycoprotein E0 is often used synonymously with glycoprotein E^{rns} in publications. Said term, depending on the context, may also relate to the mutated "E^{rns}" protein after mutation of the encoding nucleotide sequence or to the coding nucleotide sequence for said protein itself. "RNase activity residing in glycoprotein E^{rns}" relates to the RNA cleavage activity of said glycoprotein, i.e. the ability of the glycoprotein E^{rns} to hydrolyze RNA. The term "inactivation of the RNase activity residing in said glycoprotein" refers to the inability or reduced capability of a modified glycoprotein E^{rns} to hydrolyze RNA as compared to the unmodified wild type of said glycoprotein E^{rns}.

Inactivation of E^{rns} as used herein means RNase activity not significantly above the level measured for noninfected control cells in an RNase assay as described in Meyers et al., 1999. "Not significantly above the level measured for noninfected control cells in an RNase assay as described in Meyers et al., 1999", means for example, that the RNase activity is less than 150% compared to the noninfected control cells.

Attenuation: "An attenuated pestivirus or BVDV particle" as used herein means that there is a statistically significant difference between the virulence of attenuated pestivirus or BVDV particles of the present invention, wherein said attenuated viral particles being attenuated by a method described herein, and wild-type pestivirus or BVDV isolates from which said attenuated pestivirus or BVDV particles have been derived, for the predominant clinical parameters, in case of BVDV for diarrhea, pyrexia and lethality in animals infected with the same dose, preferably 6x10⁶TCID₅₀. Thus, said attenuated BVDV particles do not cause diarrhea, pyrexia and lethality and thus may be used in a vaccine.

"Bovine pathogen" as used herein means a microorganism that has an impact on the healthiness of cattle.

"Immunological active component" or "immunologically active component" as used herein means a component that induces or stimulates the immune response in an animal to which said component is administered. According to a preferred embodiment, said immune response is directed to said component or to an microorganism comprising said component. According to a further preferred embodiment, the immunological active component is an attenuated microorganism, including modified live virus (MLV), a killed-microorganism or at least an immunological active part of a microorganism.

"Immunological active part of a microorganism" as used herein means a protein-, sugar-, and or glycoprotein containing fraction of a microorganism that comprises at least one antigen that induces or stimulates the immune response in an animal to which said component is administered. According to a preferred embodiment, said immune response is directed to said immunological active part of a microorganism or to a microorganism comprising said immunological active part.

The term "vaccine" as used herein refers to a pharmaceutical composition comprising at least one immunologically active component that induces an immunological response in an animal and possibly but not necessarily one or more additional components that enhance the immunological activity of said active component. A vaccine may additionally comprise further components typical to pharmaceutical compostions. The immunologically active component of a vaccine may comprise complete virus particles in either their original form or as attenuated particles in a so-called modified live vaccine (MLV) or particles inactivated by appropriate methods in a so-called killed vaccine (KV). In another form, the immunologically active component of a vaccine may comprise appropriate elements of said organisms (subunit vaccines) whereby these elements are generated either by destroying the whole particle or the growth cultures containing such particles and optionally, subsequent purification steps yielding the desired structure(s), or by synthetic processes including an appropriate manipulation by use of a suitable system based on, for example, bacteria, insects, mammalian or other species, plus optionally subsequent isolation and purification procedures, or by induction of said synthetic processes in the animal needing a vaccine by direct incorporation of genetic material using suitable pharmaceutical compositions (polynucleotide vaccination). A vaccine may comprise one or simultaneously more than one of the elements described above. The term "vaccine" as understood herein is a vaccine for veterinary use comprising antigenic substances and is administered for the purpose of inducing a specific and active immunity against a disease provoked by a microbiological infection, preferably by a BVDV infection. The BVDV as described herein, confer active immunity that may be transferred passively via maternal antibodies against the immunogens it contains and sometimes also against antigenically related organisms. A vaccine of the invention refers to a vaccine as defined above, wherein one immunologically active component is a BVDV or derived from a nucleotide sequence that is more than 70% homologous to any known BVDV sequence (sense or antisense).

The term "live vaccine" refers to a vaccine comprising a replication competent, in particular, a replication compentent viral active component.

"Combination vaccine" as used herein means a vaccine that comprises attenuated BVDV as desribed herein together with a monovalent, bivalent or multivalent combination of immunological active component(s).

"Microbiological infection" as used herein means an infection as caused by a microorganism that is pathogenic for cattle. Such microorganisms inculde but are not limited to bacteria, viruses, yeasts or fungi, mycoplasms, and parasites.

A fragment" according to the invention is any subunit of a polynucleotide molecule according to the invention, i.e. any subset. For DNA, said fragment is characterized in that it is shorter than the DNA covering the full-length viral genome.

A functional variant" of the nucleotide molecule as used herein is a nucleotide molecule which possesses a biological activity (either functional or structural) that is substantially similar to the nucleotide molecule according to the invention. The term "functional variant" also includes "a fragment", "a functional variant", "a variant based on the degenerative nucleic acid code" or "a chemical derivative". Such "a functional variant" e.g. may carry one or several nucleotide exchanges, deletions or insertions. Said functional variant at least partially retains its biological activity, e.g. functions as an infectious clone or a vaccine strain, or even exhibits improved biological activity.

"Possess a biological activity that is substantially similar" means with respect to the pestiviruses provided herewith, for example, that said pestivirus is attenuated in a manner described herein and results in an non-pathogenic virus suitable for the production of live attenuated virus, which loses ability to pass the placenta but mediates an immune response after vaccination.

A "variant based on the degenerative nature of the genetic code" is a variant resulting from the fact that a certain amino acid may be encoded by several different nucleotide triplets. Said variant at least partially retains its biological activity, or even exhibits improved biological activity.

A molecule is "substantially similar" to another molecule if both molecules have substantially similar nucleotide sequences or biological activity. Thus, provided that two molecules possess a similar activity, they are considered variants as that term is used herein if the nucleotide sequence is not identical, and two molecules which have a similar nucleotide sequence are considered variants as that term is used herein even if their biological activity is not identical.

A "mutation" as used herein relates to modifications in the nucleic acid molecules encoding the proteins / amino acids according to the invention. Said mutations relate to, but are not limited to, substitutions (replacement of one or several nucleotides/base pairs), deletions (removal of one or several nucleotides/base pairs), and/or insertions (addition of one or several nucleotides/base pairs). As used herein, mutation may refer to a single mutation or several mutations, therefore, often the term "mutation(s)" is used and relates to both a single mutation and several mutations. Said mutations include, but are not limited to point mutations (single nucleotide mutations) or larger mutations wherein e.g. parts of the encoding nucleic acid molecules are deleted, substituted and/or additional coding nucleic acids are inserted. Said mutations may result in a modified expressed polypeptide due to the change in the coding sequence. Such modified polypeptides are desired, as set out in the disclosure of the invention as set out below.

Additional components to enhance the immune response are constituents commonly referred to as "adjuvants", like e.g. aluminiumhydroxide, mineral or other oils or ancillary molecules added to the vaccine or generated by the body after the respective induction by such additional components, like but not restricted to interferons, interleukins or growth factors.

A "pharmaceutical composition" essentially consists of one or more ingredients capable of modifying physiological e.g. immunological functions of the organism it is administered to, or of organisms living in or on the organism. The term includes, but is not restricted to, antibiotics or antiparasitics, as well as other constituents commonly used to achieve certain other objectives like, but not limited to, processing traits, sterility, stability, feasibility to administer the composition via enteral or parenteral routes such as oral, intranasal, intravenous, intramuscular, subcutaneous, intradermal or other suitable route, tolerance after administration, and controlled release properties. One non-limiting example of such a pharmaceutical composition, solely given for demonstration purposes, could be prepared as follows: Cell culture supernatant of an infected cell culture is mixed with a stabilizer (e.g. spermidine and/or BSA (bovine serum albumin)) and the mixture is subsequently lyophilized or dehydrated by other methods. Prior to vaccination, said mixture is then rehydrated in aqueous (e.g. saline, PBS (phosphate buffered saline)) or non-aqueous solutions (e.g. oil emulsion, aluminum-based adjuvant).

### DISCLOSURE OF THE INVENTION

The solution to the above technical problem is achieved by the description and the embodiments characterized in the claims.

The present invention relates to a combinantion vaccine for the treatment and/or prophylaxis of microbiological infection in cattle, that comprises a live attenuated BVDV as described herein and at least one further immunological active component for treating or preventing diseases or disorders in cattle caused by an infectious agents other than BVDV.

### Attenuated BVDV

It was decribed in WO 99/64604 that BVDV can be attenuated by introducing at least one mutation in the coding sequence of glycoporiteion E^{rns}, wherein said mutation(s) result in an incatvation of the RNAse activity residing in the E^{rns} gene region. Moreover, it has also surprisingly been found that BVDV can be more effectively attenuated by introducing at least one mutation in the coding sequence for glycoprotein E^{rns} and at least another mutation in the coding sequence for N^{pro} which preferably leads to the combined inactivation of the RNase activity residing in glycoprotein E^{rns} in addition to the inactivation of the immunomodulating activity residing in N^{pro} (WO2005/111201). An immunomodulating effect in one aspect is indicated but not limited to the indicated function for one pestivirus in an exemplary manner by Rüggli et al. (2003).Thus according to one aspect, the present invention provides a combination vaccine that comprises at least an attenuated BVDV having at least one mutation in the coding sequence for glycoprotein E^{rns} and at least another mutation in the coding sequence for N^{pro}. Preferably, in such attenuated BVDV, said mutation in the coding sequence for glycoprotein E^{rns} leads to inactivation of the RNase activity residing in E^{rns} and/or said mutation in the coding sequence for N^{pro} leads to inactivation of said N^{pro}.

The attenuated BVDV as described herein can be advantageously used in combination vaccines for the treatment and/or prophylaxis of microbiological infections in cattle. Surprisingly, the BVDV as described herein, comprising any of the modifications in the N^{pro} and E^{rns} gene region are safe for use in pregnant animals as they do not cross the placenta. This is exemplified in a non-limiting manner for BVDV in example 3. Furthermore, the BVDV with defined mutations within the N^{pro} and E^{rns} as a basis for attenuation will allow to avoid the risk of reversion to a more pathogenic strain. A further advantage of said attenuating mutations lies in their molecular uniqueness, which allows to use them as distinctive labels for an attenuated BVDV and to distinguish them from BVDV from the field. Therefore, in a further aspect the present invention provides a combination vaccine that comprises at least an attenuated BVDV having at least one mutation in the coding sequence for glycoprotein E^{rns} and at least another mutation in the coding sequence for N^{pro}. Preferably, in such attenuated BVDV, said mutation in the coding sequence for glycoprotein E^{rns} leads to inactivation of the RNase activity residing in E^{rns} and/or said mutation in the coding sequence for N^{pro} leads to inactivation of said N^{pro}. Said inactivation may take place by any mutation known to the person skilled in the art of the E^{rns}- and the N^{pro}-coding sequence, wherein the mutations are any mutation as defined in the "definitions" section, such as deletions, insertion mutations and/or substitution mutations. Most preferably, the mutation(s) are deletions, as the likelihood for reversion to the wild type is the lowest for deletions.

Altogether, the term attenuated BVDV or attenuated pestivirus in general as used herein, means but is not limited to any attenuated BVDV or pestivirus, having at least one modification in the coding sequence for glycoprotein E^{rns} and/or at least one modification in the coding sequence for N^{pro}. In the following, specific embodiments of any of such modification in the E^{rns} and N^{pro} are described more in detail. According to a preferred aspect, the term attenuated BVDV or attenuated pestivirus in general as used herein, means, but is not limited to, any attenuated BVDV or pestivirus, having at least one modification in the coding sequence for glycoprotein E^{rns} and at least one modification in the coding sequence for N^{pro} However, it is hereby understood that the present invention shall not be limited to the specific modification described herein. A person skilled in the art with the knowledge of the teaching provided herewith, is able to generate and introduce further modifications within the glycoprotein E^{rns} and/or N^{pro} having the effect of attenuation as described herein.

### Modifications of the E^{rns} of BVDV

It has been shown that the glycoprotein E^{rns} forms a disulfide-bonded homodimer of about 97 kD, wherein each monomer consists of 227 amino acids corresponding to the amino acids 268 to 494 of the CSFV polyprotein as described by Rümenapf et al. (1993). The genome sequence of the Alfort/Tiibingen strain of CSFV is available in the GenBank/EMBL data library under accession number J04358; alternatively, the amino acid sequence for the BVDV strain CP7 can be accessed in the GenBank/EMBL data library (accession number U63479); in the BVDV CP7 polyprotein, the E^{rns} protein corresponds to residues 271 to 497. Two regions of amino acids are highly conserved in glycoprotein E^{rns} as well as in some plant and fungal RNase-active proteins (Schneider et al., 1993). These two regions are of particular importance to the RNase enzymatic activity. The first region consists of the region at the amino acids at position 295 to 307 (298 to 310 for BVDV strain cp7) and the second region consists of the amino acids at position 338 to 357 (341 to 360 for BVDV strain cp7) of said viral polyprotein as exemplified for the Alfort strain of CSFV in Meyers et al., 1999 (numbering according to the published deduced amino acid sequence of CSFV strain Alfort/Tübingen (Meyers et al., 1989). The amino acids of particular importance to the RNase activity as mentioned above are by no means limited to the exact position as defined for the Alfort/Tübingen strain of CSFV but are simply used in an exemplary manner to point out the preferred amino acids being at that position or corresponding to that position in other strains such as found in BVDV, BDV and pestiviruses in general since they are highly conserved. For pestiviruses other than the CSFV Alfort/Tübingen strain the numbering of the positions of the preferred amino acids can be different but an expert in the field of the molecular biology of pestiviruses will easily identify these preferred amino acids by the high degree of conservation of this amino acid sequence and the position of these motifs in the sequence context. In one particular non-limiting example, the position of CSFV Alfort/Tübingen 346 is identical to position 349 of BVDV strain cp7.

As a consequence, the present invention preferably relates to a BVDV according to the invention, wherein said mutation(s) in the coding sequence for glycoprotein E^{rns} are located in the encoding nucleotide sequence corresponding to amino acids at position 298 to 310 and/or position 341 to 360. Preferably, such a mutation is (amino acids are given in the one letter symbols; the amino acid before the position number indicates the amino acid to be substituted, the amino acid after the position number the substituting amino acid (del indicates deletion) for example, H300L, which means that histidine at position 300 was substituted by leucine:
Suitable modification of the glycoprotein E^{rns} are for example, the single substitutions/deletions: S298G, H300K, H300L, H300R, H300del, W303G, P304del, E305A, C308G, R343G, E345del, W346G, K348A, H349K, H349L, H349del, H349Q, H349SV (mutation H349S and insertion of V), K348R, W351P, W351G, W351L, W351K, W351H; the double substitutions/deletions: H300L/H349L, K348del/H349del, H349del/G350del, E345del/H349del, W303G/E305A, H300K/H349K, H300K/H349L and the triple deletions: L299del/H300del/G301del, K348del/H349del/G350del. Numbering is according to the published amino acid sequence of BVDV CP7 for all the mutants listed above. All the above-listed mutants were at least tested as BVDV mutants without mutations in the N^{pro}region. Suitable mutants of the pestiviral such BVDV glycoprotein E^{rns} are provided, for example, by WO 99/64604. It should be noted, however, that according to a further preferred embodiment of the present invention, at least one additional mutation in the N^{pro} region, as disclosed in further detail below, must be present.

It was particularly found that deletion or substitution of the histidine residue at position 349 (BVDV) leads to effective inactivation of E^{rns} and therefore leads to particularly useful BVDV live vaccines. The present invention demonstrates that BVDV are viable and code for an E^{rns} protein without RNase activity when the histidine residue at position at position 349 (numbering according to the published sequence of BVDV CP7 (Meyers et al., 1996b)) is deleted. Thus, preferably, the BVDV as used in the combination vaccine bears a mutation in the coding sequence for glycoprotein E^{rns} is a deletion or substitution of the histidine residue at position 349. Even more specifically, the putative active site of the RNase is represented by the conserved E^{rns} sequences SLHGIWPEKICTG (SEQ ID NO 13) and/or LQRHEWNKHGWCNWFHIEPW (SEQ ID NO 14) (sequence of the BVDV-2 New York'93 protein is given here in an exemplary manner; minor changes can possibly be found in other BVDV sequences but the identity of the motif will always be obvious for an expert in the field). As an example, the corresponding amino acid sequences of BVDV-1 CP7 would be SLHGIWPEKICTG (SEQ ID NO 13) and/or LQRHEWNKHGWCNWYNIEPW (SEQ ID NO 15). Thus, preferably, the BVDV of the combination vaccine bears mutation(s) in the coding sequence for glycoprotein E^{rns} are located in the nucleotide sequence coding for the conserved E^{rns} sequence SLHGIWPEKICTG (SEQ ID NO 13) and/or LQRHEWNKHGWCNWFHIEPW (SEQ ID NO 14). These sequences are representing the putative active site of the RNase. The sequences SLHGIWPEKIC (SEQ ID NO 16) and RHEWNKHGWCNW (SEQ ID NO 17) of the putative E^{rns} active site are even more conserved across pestiviruses. Thus, preferably, the BVDV used for the preparation of a combination vaccine as described herein has at least one mutation in the coding sequence of the N^{pro} protein and/or the glycoprotein E^{rns}, wherein said mutation(s) in the coding sequence for glycoprotein E^{rns} are located in the nucleotide sequence coding for the conserved E^{rns} sequence SLHGIWPEKIC (SEQ ID NO 16) and/or RHEWNKHGWCNW (SEQ ID NO 15). Preferably, the mutation is located in only one of said sequences. Thus the BVDV of the combination vaccine described herein having at least one mutation in the coding sequence of the N^{pro} protein and/or the glycoprotein E^{rns}, wherein said mutation(s) in the coding sequence for glycoprotein E^{rns} are located in the nucleotide sequence coding for the conserved E^{rns} sequence SLHGIWPEKIC (SEQ ID NO 16) or RHEWNKHGWCNW (SEQ ID NO 17). Preferably, such mutations concern two different amino acids, i.e. are double mutations. Thus, said mutations may be 1 to 3 nucleotide mutations in two different triplets encoding two amino acids. Thus, the invention also relates to a combination vaccine comprising a live attenuated BVDV having at least one mutation in the coding sequence of the N^{pro} protein and/or the glycoprotein E^{rns}, wherein said mutation(s) in the coding sequence for glycoprotein E^{rns} are two mutations located in the nucleotide sequence coding for the conserved E^{rns} sequence SLHGIWPEKIC (SEQ ID NO 16) and/or RHEWNKHGWCNW (SEQ ID NO 17). Preferably, such mutations concern a single amino acid. Thus, said mutation may be 1 to 3 nucleotide mutations in one triplett encoding one amino acid. Thus, the invention also relates to combination vaccine for the treatment and/or prophylaxis of cattle against microbiological infections comprising a live attenuated BVDV having at least one mutation in the coding sequence of the N^{pro} protein and/or the glycoprotein E^{rns}, wherein a single mutation is located in the conserved E^{rns} sequence SLHGIWPEKIC (SEQ ID NO 16) and/or RHEWNKHGWCNW (SEQ ID NO 17).

### Modification of the N^{pro}_gene of BVDV

As mentioned above, the attenuated BVDV provided herein, having at least one mutation in the coding sequence of the glycoprotein E^{rns} and/or in the coding sequence of the the N^{Pro} protein, wherein said mutation preferably results in inactivation of the RNase activity residing in the glycoprotein E^{RNS} and/or of the immunomodulating activity residing in N^{pro}. Inactivation of N^{pro} is achieved in BVDV of the specified formula described more in detail below, wherein between 0 and all amino acids of N^{pro} are present; ubiquitin or LC3 or another sequence serves as processing signal (e.g. SUMO-1, NEDD8, GATE-16,GABA(A)RAP, or proteases like e.g. Intein, picornavirus 3C, cardovirus 2A, or p15 of rabbit hemorrhagic disease virus) are present or absent. In case a processing signal is present, the coding sequence of the processing signal is inserted at or close to the C-terminal end of the (remaining part of the) N^{pro}-protein. Only in the case that a processing signal is present, any number of amino acids coding for N^{pro} (=N^{pro} amino acids) may be present. In case no processing signal sequence is inserted, a maximum of 12 amino acids, preferably aminoterminal amino acids, of N^{pro} may be present, the remaining amino acids have to be deleted. Furthermore, other than the E^{rns} mutations as disclosed above (at least one of which has to be present in the attenuated BVDV as described herein), the remaining sequences of the attenuated BVDV may remain unchanged, i.e. are not mutated, or may also have mutations close to the N-terminal end of the C-protein. A number of more specific embodiments as disclosed below exemplify this.

Thus, the attenuated BVDV of the combination vaccine is modified in the coding regions of the E^{rns} as described above and/or in the N^{pro}, wherein said mutation(s) in the coding sequence for N^{pro} leads to an encoded polyprotein as characterized by the following formula:

[N^{pro}]ₓ-[PS]_{y}-[C-term]

and wherein:
[**N^{pro}**] relates to the N^{pro} portion of said polyprotein, wherein "x" represents the number of amino acids of the N^{pro} present in the polyprotein;
**[PS]** relates to a processing signal selected from: ubiquitin, LC3, SUMO-1, NEDD8, GATE-16 or GABA(A)RAP or proteases like e.g. Intein, picornavirus 3C, cardovirus 2A, or p15 of rabbit hemorrhagic disease virus, or any processing signal known to the skilled person that ensures the generation of a functional N-terminal of the C-protein. "Y" may be = 0, which means that no processing signal is present (= PS is absent), or "Y" may be = 1, which means that a processing signal is present (= PS present).
[**C-term**] relates to the complete pestivirus, in particular the complete BVDV polyprotein except for N^{pro}, but including the capsid (C)-protein and any other protein present in the pestivirus polyprotein, in particular in the BVDV polyprotein including the carboxyterminal NS5B. Preferably, the glycoprotein E^{rns} in said [C-term] is mutated, in such that the RNase activity residing in the glycoprotein E^{rns} is inactivated. The term "any other protein present in the pestivirus polyprotein /BVDV polyprotein" relates to E^{rns}, E1, E2, p7, NS2, NS3, NS4A, NS4B and NS5A, wherein glycoprotein E^{rns} is mutated, preferably as disclosed herein (see above), in such that the RNase activity residing in the glycoprotein E^{rns} is inactivated. Preferably, the pestivirus, in particular the BVDV according to the invention has a C-protein which is not mutated except for the amino acid at position 2 which is changed from D to N. Therefore, [C-term*] is the same as [C-term] but with a mutation at position 2 of the C-protein (N instead of D);
if "y" is = 0 (means no [PS] present) then"x" is 0 to 12, (means no N^{pro} specific amino acid or 1 to 12 amino acids of N^{pro}, preferably of the N-terminus of N^{pro}, are present);
if "y" is = 1 (means [PS] is present) then "x" is 0 to 168; (means no N^{pro} specific amino acid or 1 to all 168 amino acids of N^{pro}, preferably of the N-terminus of N^{pro}, are present).

Also more preferably, the mutation(s) in the coding sequence for N^{pro} lead to an encoded polyprotein as characterized by the following formula:

[N^{pro}]₁-[PS]₀-[C-term]

and wherein the definitions are as defined above.

A specific example thereof is disclosed below, wherein the N-terminal methionine is followed by the C-protein and any other protein present in the polyprotein including the carboxyterminal NS5B Hence, most preferably, the attenunated BVDV as described herein is encoded by a polyprotein as characterized by the following formula:

M[C-term].

and wherein the definitions are as defined above.

Also more preferably, the invention relates to a combination vaccine for the treatment and/or prophylaxis of cattle against microbiological infections, wherein said vaccine comprises an attenuated BVDV that is modified in the coding regions of the E^{rns} as described above and/or in the N^{pro}, wherein said mutation(s) in the coding sequence for N^{pro} leads to an encoded polyprotein as characterized by the following formula:

[N^{pro}]₃-[PS]₀-[C-term]

and wherein the definitions are as defined above.

A further specific example of an attenuated BVDV is disclosed below, wherein the N-terminal methionine is followed by the N^{pro} sequence EL and the C-protein and any other protein present in the polyprotein including the carboxyterminal NS5B. Hence, most preferably, the attenuated BVDV as described herein is encoded by a polyprotein as characterized by the following formula:

MEL-[C-term]

and wherein the definitions are as defined above.

Also more preferably, the invention relates to a combination vaccine for the treatment and/or prophylaxis of cattle against microbiological infections, wherein said vaccine comprises an attenuated BVDV that is modified in the coding regions of the E^{rns} as described above and/or in the N^{pro}, wherein said mutation(s) in the coding sequence for N^{pro} leads to an encoded polyprotein as characterized by the following formula:

[N^{pro}]₄-[PS]₀-[C-term]

and wherein the definitions are as defined above.

A further specific example of BVDV is disclosed below, wherein the N-terminal methionine is followed by the N^{pro} sequence ELF (SEQ ID NO 18) and the C-protein and any other protein present in the polyprotein including the carboxyterminal NS5B. Hence, most preferably, the invention refers to a BVDV, wherein said mutation(s) in the coding sequence for N^{pro} leads to an encoded polyprotein as characterized by the following formula:

MELF-[C-term].

and wherein the definitions are as defined above.

Also more preferably, the invention relates to a combination vaccine for the treatment and/or prophylaxis of cattle against microbiological infections, wherein said vaccine comprises an attenuated BVDV that is modified in the coding regions of the E^{rns} as described above and/or in the N^{pro}, wherein said mutation(s) in the coding sequence for N^{pro} leads to an encoded polyprotein as characterized by the following formula:

[N^{pro}]₆-[PS]₀-[C-term]

and wherein the definitions are as defined above.

A further specific example of an attenuated BVDV as described herein is given below, wherein the N-terminal methionine is followed by the N^{pro} sequence ELFSN (SEQ ID NO 19) and the C-protein and any other protein present in the polyprotein including the carboxyterminal NS5B. Hence, most preferably, the invention relates to a combination vaccine for the treatment and/or prophylaxis of cattle against microbiological infections, wherein said vaccine comprises an attenuated BVDV that is modified in the coding regions of the E^{rns} as described above and/or in the N^{pro}, wherein said mutation(s) in the coding sequence for N^{pro} leads to an encoded polyprotein as characterized by the following formula:

MELFSN-[C-term].

and wherein the definitions are as defined above.

A further specific example of an attenuated BVDV as described herein is given below, wherein the N-terminal methionine is followed by the N^{pro} sequence ELFSNE (SEQ ID NO 20); ELFSNEL (SEQ ID NO 21); ELFSNELL (SEQ ID NO 22); ELFSNELLY (SEQ ID NO 23); ELFSNELLYK (SEQ ID NO 24); or ELFSNELLYKT (SEQ ID NO 25) and the C-protein and any other protein present in the polyprotein including the carboxyterminal NS5B. Hence, most preferably, the invention relates to a combination vaccine for the treatment and/or prophylaxis of cattle against microbiological infections, wherein said vaccine comprises an attenuated BVDV that is modified in the coding regions of the E^{rns} as described above and/or in the N^{pro}, wherein said mutation(s) in the coding sequence for N^{pro} leads to an encoded polyprotein as characterized by the following formula:

MELFSN-[C-term].

MELFSNE-[C-term];

MELFSNEL--[C-term];

MELFSNELL-[C-term];

MELFSNELLY-[C-term];

MELFSNELLYK-[C- TERM]

MELFSNELLYKT-[C- TERM]

and wherein the definitions are as defined above.

Also more preferably, the invention relates to a combination vaccine for the treatment and/or prophylaxis of cattle against microbiological infections, wherein said vaccine comprises an attenuated BVDV that is modified in the coding regions of the E^{rns} as described above and/or in the N^{pro}, wherein said mutation(s) in the coding sequence for N^{pro} leads to an encoded polyprotein as characterized by the following formula:

[N^{pro}]₄-[PS]₀-[C-term*]

and wherein the definitions are as defined above except for the fact that the aminoterminal part of the C-protein is changed, also as described above.

A further specific example of BVDV is disclosed below, wherein the N-terminal methionine is followed by the N^{pro} sequence ELF and in the C-protein sequence, the amino acid at position 2 is changed from D to N. Therefore, the aminoterminal C-protein sequence is SNEGSK (SEQ ID NO 26), instead of SDEGSK (SEQ ID NO 27). Hence, most preferably, the invention relates to a combination vaccine for the treatment and/or prophylaxis of cattle against microbiological infections, wherein said vaccine comprises an attenuated BVDV that is modified in the coding regions of the E^{rns} as described above and/or in the N^{pro}, wherein said mutation(s) in the coding sequence for N^{pro} leads to an encoded polyprotein as characterized by the following formula:

MELF-[C-term*],

wherein in the C-protein the amino acid at position 2 is changed from D to N, and wherein the definitions are as defined above.

Also more preferably, the invention relates to a combination vaccine for the treatment and/or prophylaxis of cattle against microbiological infections, wherein said vaccine comprises an attenuated BVDV that is modified in the coding regions of the E^{rns} as described above and/or in the N^{pro}, wherein said mutation(s) in the coding sequence for N^{pro} lead to an encoded polyprotein as characterized by the following formula:

[N^{pro}]ₓ-[PS]₁-[C-term],

wherein the definitions are as defined as above,
and wherein PS is any of the PS disclosed above, and more preferably selected from the group of ubiquitin or LC3.

A further specific example of BVDV is disclosed below, wherein the N-terminal methionine is followed by any 21 or 28 N^{pro} amino acids, ubiquitin or LC3, and the C-protein. Hence most preferably, the invention relates to a combination vaccine for the treatment and/or prophylaxis of cattle against microbiological infections, wherein said vaccine comprises an attenuated BVDV that is modified in the coding regions of the E^{rns} as described above and/or of the N^{pro}, wherein said mutation(s) in the coding sequence for N^{pro} lead to an encoded polyprotein as characterized by the following formula:

[N^{pro}]₂₂-[PS]₁-[C-term],

wherein preferably, the PS is ubiquitin or LC3 or

[N^{pro}]₂₉-[PS]₁-[C-term],

wherein preferably, the PS is ubiquitin or LC3.

Ubiquitin is a well known highly conserved cellular protein of 76 amino acids. Among other functions, ubiquitin is a key player in protein catabolism since conjugation with ubiquitin can mark a protein for degradation via the proteasome. Ubiquitin conjugated with or fused to other proteins via the carboxyterminal glycine can be cleaved off by cellular ubiquitin-specific proteases. Thus, fusion of a protein to the carboxyterminus of ubiquitin will usually result in defined proteolytic cleavage of the fusion protein into its components when expressed within a cell.

LC3 (light chain 3 of microtubule associated proteins) represents a cellular protein of 125 amino acids that serves a variety of functions (length given for bovine LC3). Recently, a fundamental role of the protein in autophagy has been defined. During this process, LC3 is activated by carboxyterminal cleavage. Thereby, a new carboxyterminus is generated that consists of glycine. LC3 is then conjugated via the carboxyterminal glycine to phosphatidylethanolamine present in the membranes of autophagic vesicles. Because of this process, a protein fused to the carboxyterminus of LC3 will be cleaved off by a cellular protease at a defined position.

Also more preferably the invention relates to a combination vaccine for the treatment and/or prophylaxis of cattle against microbiological infections, wherein said vaccine comprises an attenuated BVDV that is modified in the coding regions of the E^{rns} as described above and/or in the N^{pro}, wherein said mutation(s) in the coding sequence for N^{pro} lead to an encoded polyprotein as characterized by the following formula selected from the group of :
[N^{pro}]₂-[PS]_{y}-[C-term] and preferably ME-[PS]_{y}-[C-term];
[N^{pro}]₅-[PS]_{y}-[C-term] and preferably MELFS-[PS]_{y}-[C-term];
[N^{pro}]₇-[PS]_{y}-[C-term] and preferably MELFSNE-[PS]_{y}-[C-term];
[N^{pro}]₈-[PS]_{y}-[C-term] and preferably MELFSNEL-[PS]_{y}-[C-term];
[N^{Pro}]₉-[PS]_{y}-[C-term] and preferably MELFSNELL-[PS]_{y}-[C-term];
[N^{pro}]₁₀-[PS]_{y}-[C-term] and preferably MELFSNELLY-[PS]_{y}-[C-term];
[N^{pro}]₁₁-[PS]_{y}-[C-term] and preferably MELFSNELLYK-[PS]_{y}-[C-term]; and
[N^{pro}]₁₂-[PS]_{y}-[C-term] and preferably MELFSNELLYKT-[PS]_{y}-[C-term]
and wherein the definitions are as defined as above. The preferably disclosed embodiments refer to BVDV.
Most preferably, y is 0 (no PS present).

Suitable E^{RNS} N^{pro} double mutants of BVDV include those listed in the table below:

**Table: BVDV E^{Rns} N^{pro} double mutants:**

| ***Modification* of *E^{RNS}*** | ***Modification of N^{Pro}*** |
|---|---|
| S298G | |
| H300K | |
| H300L | |
| | |
| H300del | |
| W303G | |
| P304del | |
| E305A | |
| C308G | |
| R343G | |
| E345del | |
| W346G | |
| K348A | |
| | |
| K348R | |
| H349K | |
| H349L | |
| H349Q | |
| H349del | |
| H349SV (mutation H349S and insertion of V) | |
| W351P | |
| W351K | |
| H300L/H349L | |
| | |
| H300K/H349K | |
| H300K/H349L | |
| W303G/E305A | |
| K348del/H349del | |
| E345del/H349del | |
| H349del/G350del | |
| L299del/H300del/G301 del | |
| L299del/H300del/G301 del | |

The N^{pro} -mutations are defined by their remaining amino terminal sequences. For example; MELF-mutant means that the coding region of N^{pro} is completely deleted except of the amino terminal amino acids MELF.

According to one embodiment of the present invention, the attenuated BVDV of the combination vaccine as provided herein is a BVDV type 1. Preferably, the attenuated BVDV is based on one of the following BVDV type 1 strains: NADL, Osloss, SD-1, CP7 or KE9, wherein each of the strains comprises at least one of the E^{RNS} and/or N^{pro} mutations as described above, and preferably at least one of the double-mutants within the E^{RNS} and N^{pro} region as listed in the table above. According to a further embodiment, said attenuated BVDV of the combination vaccine as provided herein is a BVDV type 2. Preferably, the attenuated BVDV is based on one of the following BVDV type 2 strains: 890, C413, or New York'93C, wherein each of the strains comprises at least one of the E^{RNS} and/or N^{pro} mutations as described above, and preferably at least one of the double-mutants within the E^{RNS} and N^{pro} region as listed in the table above.

BVDV-1 and BVDV-2 are differentiated according to features of their genomic sequences (Heinz et al., 2000 and references therein). BVDV-1 as disclosed herein may be used in the manufacture of a composition for use in the prevention and/or treatment of BVDV type 1 infections in breeding stocks of cattle, in pregnant cows and in the induction of fetal protection against BVDV type 1 infection is pregnant cows. Surprisingly, a BVDV-2 as disclosed herein may be used in the manufacture of a combination vaccine for use in the prevention and/or treatment of BVDV type 1 infections in breeding stocks of cattle. In particular, the invention relates to the use of a BVDV type 2 as described herein in the manufacture of a combination vaccine for use in the prevention and/or treatment of BVDV type 1 infections in pregnant cows. Preferably, the BVDV type 2 as provided herein may be used in the manufacture of a combination vaccine for use in the induction of fetal protection against BVDV type 1 infections in pregnant cows. Surprisingly also, a BVDV-1 as disclosed herein may be used in the manufacture of a combination vaccine for use in the prevention and/or treatment of BVDV type 2 infections in breeding stocks of cattle. In particular, the invention relates to the use of a BVDV type 1 as described herein in the manufacture of a combination vaccine for use in the prevention and/or treatment of BVDV type 2 infections in pregnant cows. Preferably, the BVDV type 1 according to the invention may be used in the manufacture of a combination vaccine for use in the induction of fetal protection against BVDV type 2 infections in pregnant cows.

Most preferred is the use of BVDV type 1 and type 2 in combination for the manufacture of the combination vaccines described herein for use in the prevention and/or treatment of BVDV type 1 and or type 2 infections in breeding stocks of cattle, in pregnant cows and in the induction of fetal protection against BVDV type 1 and/or type 2 infections in pregnant cows. Thus, preferably the combination vaccines provided herewith comprise one or more attenuated BVDV type 1 and type 2 as desribed above. For example, the combination vaccines provided herewith comprise an attenuated BVDV of type 1 and type 2, based on the strains: NADL/890; NADL/C413; NADL/NewYork'93/C; CP7/890; CP7/C413; CP7/NewYork'93/C; KE9/890; KE9/C413; KE9/NewYork'93/C, wherein each of the strains comprises at least one of the E^{RNS} and/or N^{pro} mutations as described above, and more preferably at least one of the double-mutants within the E^{RNS} and N^{pro} region as listed in the table above. More preferably, any of the combination vaccines provided herewith may include one of more sub-types of attenuated BVDV type 1 and one or more sub-types of attenuated BVDV-2, e.g one or more attenuated BVDV of sub-types 1a, 1b, 1c, Id, 1e, If, 1g, 1h, and the like and one or more attenuated BVDV of sub-types 2a, 2b and the like. Most preferred is a combination vaccine comprising attenuated BVDV of sub-types 1a, 1b, and 2a. Thus, according to a preferred embodiment of the present invention, the phrase "attenuated BVDV (types 1 and/or 2)" includes but is not limited to combinations of BVD viruses comprising one or more attenuated BVDV of type 1, preferably of sub-type 1b and one or more attenuated BVDV of type 2, preferably of subtype 2a. According to further embodiment of the present invention the phrase "attenuated BVDV (types 1 and/or 2)" includes but is not limited to combinations of BVD viruses comprising one or more "attenuated BVDV of sub-type 1a, one or more attenuated BVDV of sub-type 1b, and one or more attenuated BVDV of type 2, preferably of sub-type 2a.

If more than one attenuated BVDV is used in the combination vaccines as described herein, each of the attenuated BVDV should mutated in same genomic site of the E^{RNS} and/or the N^{pro} such that the none of the attenuated BVDV can recombine with any of the others to eliminate the mutations which are essential and responsible for the attenuation of the viruses. For example if BVDV type 1a with one of the following E^{RNS} mution is used: H349K, H349L, H349del, H349Q, H349SV (mutation H349S and insertion of V), a BVDV type 1b and/or type 2 should be used, which are/is mutated in the same site of the E^{RNS}, i.e. at position 349 or at amino acid position which corresponds to position 349 of BVDV type 1 in order to avoid revertation of attenuated BVDV type 1 or type 2. This principle also applies to any mutation within the N^{pro} region. Thus, according to a prefered embodiment of the present invention, the phrase "attenuated BVDV (types 1 and/or 2)" includes but is not limited to combinations of BVD viruses comprising one or more attenuated BVDV of type 1 and one or more attenuated BVDV of type 2, wherein each of the attenuated BVDV is mutated in same genomic site of the E^{RNS} and/or the N^{pro} such that none of the attenuated BVDV can recombine with any of the others to eliminate the mutations within the E^{RNS} and N^{pro} which are essential and responsible for the attenuation of the viruses. According to another embodiment of the present invention, the phrase "attenuated BVDV (types 1 and/or 2)" includes, but is not limited to, a combination of BVD viruses comprising one or more "attenuated BVDV of sub-type 1a, one or more attenuated BVDV of sub-type 1b, and one or more attenuated BVDV of type 2, preferably of sub-type 2a, wherein each of the attenuated BVDV is mutated in same genomic site of the E^{RNS} and/or the N^{pro} such that none of attenuated BVDV can recombine with any of the others to eliminate the mutations within the E^{RNS} and/or N^{pro} which are essential and responsible for the attenuation of the viruses.

### Preparation of the attenuated BVDV

Another important aspect of the disclosure is a method for attenuating a pestivirus, characterized in that at least one mutation in the coding sequence for glycoprotein E^{rns} and/or at least another mutation in the coding sequence for N^{pro} is generated in the BVDV genome.

According to a more preferred aspect, said method comprises the steps:
a) reverse transcription of a wild-type pestivirus nucleotide sequence into a cDNA;
b) cloning said cDNA;
c) introducing mutations selected from the group of deletions, insertion mutations and/or substitution mutations into said cDNA, wherein said mutations are located in the coding sequence encoding glycoprotein E^{rns} and/or the protease N^{pro},
d) incorporating the cDNA into a plasmid or into a DNA virus capable of directing the transcription of BVDV cDNA into RNA in vitro or upon infection of suitable cells.

Regarding the method for attenuating a BVDV according to the disclosure, said preferred method comprises the steps:
a) reverse transcription of a wild-type BVDV nucleotide sequence into a cDNA;
b) cloning said cDNA;
c) introducing mutations selected from the group of deletions, insertion mutations and/or substitution mutations into said cDNA, wherein said mutations are located in the coding sequence encoding glycoprotein E^{rns} and/or the protease N^{pro},
d) incorporating the cDNA into a plasmid or into a DNA virus capable of directing the transcription of BVDV cDNA into RNA in vitro or upon infection of suitable cells.

There are several nucleotide sequences known in the art, which represents the basis for the production of a polynucleotide molecule coding for a BVDV attenuated as decribed herein, having at least one mutation in the coding sequence of N^{pro} and/or at least one in the coding sequence of glycoprotein E^{rns}, wherein said mutations result in a combined inactivation of the RNase activity residing in glycoprotein E^{rns} and in the inactivation of the immunomodulating activity residing in N^{pro}. Examples of nuclecic acid sequences of wild-type sequences of several BVDVstrains are listed below:

**Bovine viral diarrhea virus 1**

| | |
|---|---|
| Strain NADL | NCBI GenBank Accession No. [M31182] |
| Strain Osloss | NCBI GenBank Accession No. [M96687] |
| Strain SD-1 | NCBI GenBank Accession No. [M96751] |
| Strain CP7 | NCBI GenBank Accession No. [U63479] |
| Strain KE9 | (SEQ ID NO:1) |

**Bovine viral diarrhea virus 2**

| | |
|---|---|
| Strain 890 | NCBI GenBank Accession No. [U18059] |
| Strain C413 | NCBI GenBank Accession No. [AF002227] |
| Strain NewYork'93/C | NCBI GenBank Accession No. [AF502399] |

The mutations/modifications relating to the coding sequences of N^{pro} and E^{rns} are described above more in detail. Having this information, a person skilled in the art is able to realize the manufacture of any polynucleotide/polynucleic acid coding for an attenuated BVDV as provided herewith. Molecular methods for introducing a mutation into a polynucleotide sequence, as well as the cloning and amplification of said mutated polynucleotide are for example provided by Sambrook et al. 1989 or Ausubel et al. 1994.

Most preferably, the wild type BVDV which is to be mutated as disclosed herein corresponds to amino acid sequence SEQ ID No. 5 (termed XIKE A) or is a functional variant thereof. Most preferably also, the BVDV has a N^{pro} mutation as described herein corresponding to amino acid sequence SEQ ID No. 6 (termed XIKE-A-NdN) or is a functional variant thereof. Preferably, such a functional variant is at least 65% homologous to the amino acid sequence disclosed herein. On the amino acid level, homologies are very roughly: BVDV-1/-BVDV-1: 93%; BVDV-1/-BVDV-2: 84%; BVDV-2/-BVDV-2: 98%. Therefore, more preferably, such a functional variant is at least 70%, 71%, 72%, 73%, 74%, 75%, 76%, 77%, 78%, 79%, 80%, 81%, 82%, 83%, 84%, 85%, 86%, 87%, 88%, 89% or 90% homologous to the amino acid sequence disclosed herein. More preferably also, such functional variant is at least 91%, 92%, 93%, 94%, 95%, 96%, 97% or 98% homologous to the amino acid sequence disclosed herein. Most preferably, such functional variant is at least 99% or 99.9% homologous to the amino acid sequence disclosed herein.

Most preferably also, the attenuated BVDV as described herein has a E^{rns} mutation which has a deletion of the codon coding for histidine 349 (termed XIKE-B), or is a functional variant thereof. Most preferably also, the attenuated BVDV has both a E^{rns} mutation and/or a N^{pro} mutation as described herein, wherein the codon coding for histidine 349 of E^{rns} is deleted and also the complete N^{pro} coding region is deleted, except for codons 1 to 4, thus amino acids MELF of N^{pro} remain. Said double mutant corresponds to amino acid sequence SEQ ID No. 8 (termed XIKE-B-NdN) or is a functional variant thereof. Preferably, such a functional variant is at least 65% homologous to the amino acid sequence disclosed herein. More preferably, such a functional variant is at least 70%, 71%, 72%, 73%, 74%, 75%, 76%, 77%, 78%, 79%, 80%, 81%, 82%, 83%, 84%, 85%, 86%, 87%, 88%, 89% or 90% homologous to the amino acid sequence disclosed herein. More preferably also, such functional variant is at least 91%, 92%, 93%, 94%, 95%, 96%, 97% or 98% homologous to the amino acid sequence disclosed herein. Most preferably, such functional variant is at least 99% or 99.9% homologous to the amino acid sequence disclosed herein.

Most preferably also, the BVDV according to the invention has an E^{rns} mutation according to the invention which has a substitution of the codon coding for histidine 300 by the codon coding for leucine (termed XIKE-C), or is a functional variant thereof. Most preferably also, the BVDV according to the invention has both a E^{rns} mutation and a N^{pro} mutation according to the invention, wherein the codon coding for histidine 300 is substituted by the codon coding for leucine and also the complete N^{pro} coding region is deleted, except for codons 1 to 4, thus amino acids MELF of N^{pro} remain. Said mutant corresponds to amino acid sequence SEQ ID No. 10 (termed XIKE-C NdN) or is a functional variant thereof. Preferably, such a functional variant is at least 65% homologous to the amino acid sequence disclosed herein. More preferably, such a functional variant is at least 70%, 71%, 72%, 73%, 74%, 75%, 76%, 77%, 78%, 79%, 80%, 81%, 82%, 83%, 84%, 85%, 86%, 87%, 88%, 89% or 90% homologous to the amino acid sequence disclosed herein. More preferably also, such functional variant is at least 91%, 92%, 93%, 94%, 95%, 96%, 97% or 98% homologous to the amino acid sequence disclosed herein. Most preferably, such functional variant is at least 99% or 99.9% homologous to the amino acid sequence disclosed herein.

### Combination partners:

As described above, the present invention relates to a combination vaccine for the treatment and/or prophylaxis of cattle against microbiological infections, wherein said combination vaccine comprises attenuated BVDV (types 1 and/or 2) as decribed above, and at least one further immunological active component effective for the treatment and/or prophylaxis of infections caused by a bovine pathogen other than BVDV. As mentioned above, the combination vaccine preferably comprises attenuated BVDV type 1 and BVDV type 2, both having at least one mutation in the coding sequence for glycoprotein E^{rns} and/or at least another mutation in the coding sequence for N^{pro}, wherein said mutation in the coding sequence for glycoprotein E^{rns} leads to inactivation of RNase activity residing in E^{rns} and/or said mutation in the coding sequence for N^{pro} leads to inactivation of said N^{pro}. Even more preferably, each of the attenuated BVDV is mutated in same genomic site of the E^{RNS} and/or the N^{pro} such that none of the attenuated BVDV can recombine with any of the others to eliminate the mutations within the E^{RNS} and/or N^{pro}, which are essential and responsible for the attenuation of the viruses. Even more preferably, the combination vaccine comprises attenuated BVDV type 1 and BVDV type 2, both having at least one mutation in the coding sequence for glycoprotein E^{rns} and at least another mutation in the coding sequence for N^{pro}, wherein said mutation in the coding sequence for glycoprotein E^{rns} leads to inactivation of RNase activity residing in E^{rns} and/or said mutation in the coding sequence for N^{pro} leads to inactivation of said N^{pro}. Most preferably, each of the attenuated BVDV is mutated in same genomic site of the E^{RNS} and the N^{pro} such that none of the attenuated BVDV can recombine with any of the others to eliminate the mutations within the E^{RNS} and/or N^{pro}, which are essential and responsible for the attenuation of the viruses.

Relevant bovine pathogens other than BVDV include but are not limited to: i) **pathogens of viral origin** such as Parainfluenza-3 Virus (PI-3), Infectious Bovine Rhinotracheitis virus (IBR), Bovine Respiratory Syncytial Virus (BRSV), Bovine Herpesvirus (BHV), Bovine Rotavirus (BRV), Bovine Enterovirus (BEV), Bovine Coronovirus (BCV), Bovine Rabies (BR), Bovine Parvovirus (PPV), and Adenovirus and Astrovirus; **ii) pathogens of bacterial origin,** such as Mannheimia haemolytica (formerly Pasteurella haemolytica), Pasteurella multocida, Haemophilus somnus (Histophilus ovis and Haemophilus agni), Actinomyces (Corynebacterium), Actinomyces pyogenes, Chlamydia psittaci, Campylobacter fetus venerealis and Campylobacter fetus fetus (formerly C fetus intestinalis), Leptospira interrogans, Leptospira pomona, and Leptospira grippotyphosa, Leptospira canicola, Leptospira grippotyphosa, Leptospira hardjo (Leptospira hardjoprajitno and Leptospira hardjo-bovis), Brucella abortus, Brucella suis and Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium; Salmonella newport, Mycobacterium avium paratuberculosis, Staphylococcus aureus, Streptococcus dysgalactiae, and Streptococcus uberus **iii) pathogens of other origin,** such as Mycoplasma dispar, Mycoplasma bovis, and Ureaplasma spp., Tritrichomonas foetus, Trichophyton verrucosum, Trichophyton mentagrophytes, Trichophyton sarkisovii, Neospora caninum (formerly Toxoplasma gondii), Cryptsporidium parvum, Cryptsporidium hominis, Babesia bigemina and Babesia bovis, and Dictyocaulus viviparous (Lungworm disease).

Thus, the present invention relates to a combination vaccine for the treatment and /or prophylaxis of cattle against microbiological infections in cattle, wherein said vaccine comprises attenuated BVDV (types 1 and/or 2) as described herein and a further immunological active component effective for the treatment and/or prophylaxis of infections caused by Parainfluenza-3 Virus (PI-3), Infectious Bovine Rhinotracheitis virus (IBR), Bovine Respiratory Syncytial Virus (BRSV), Bovine Herpesvirus (BHV), Bovine Rotavirus (BRV), Bovine Enterovirus (BEV), Bovine Coronovirus (BCV), Bovine Rabies (BR), Bovie Parvovirus (PPV), Adenovirus Astrovirus, Mannheimia haemolytica (formerly Pasteurella haemolytica), Pasteurella multocida, Haemophilus somnus (Histophilus ovis and Haemophilus agni), Actinomyces (Corynebacterium), Actinomyces pyogenes, Chlamydia psittaci, Campylobacter fetus venerealis and Campylobacter fetus fetus (formerly C fetus intestinalis), Leptospira interrogans, Leptospira pomona, and Leptospira grippotyphosa, Leptospira canicola, Leptospira grippotyphosa, Leptospira hardjo (Leptospira hardjoprajitno and Leptospira hardjo-bovis), Brucella abortus, Brucella suis and Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium; Salmonella newport, Mycobacterium avium paratuberculosis, Cryptsporidium parvum, Cryptsporidium hominis, Staphylococcus aureus, Streptococcus dysgalactiae, Streptococcus uberus, Mycoplasma spp, Mycoplasma dispar, Mycoplasma bovis, and Ureaplasma spp., Tritrichomonas foetus, Trichophyton verrucosum, Trichophyton mentagrophytes, Trichophyton sarkisovii, Neospora caninum (formerly Toxoplasma gondii), Babesia bigemina and Babesia bovis, and Dictyocaulus viviparous (Lungworm disease).

According to a further embodiment, the present invention relates to a combination vaccine for the treatment and /or prophylaxis of cattle against microbiological infections in cattle, wherein said vaccine comprises attenuated BVDV (types 1 and/or 2) as described herein and at least one antigen of Parainfluenza-3 Virus (PI-3), Infectious Bovine Rhinotracheitis virus (IBR), Bovine Respiratory Syncytial Virus (BRSV), Bovine Herpesvirus (BHV), Bovine Rotavirus (BRV), Bovine Enterovirus (BEV), Bovine Coronovirus (BCV), Bovine Rabies (BR), Bovine Parvovirus (PPV), Adenovirus Astrovirus, Mannheimia haemolytica (formerly Pasteurella haemolytica), Pasteurella multocida, Haemophilus somnus (Histophilus ovis and Haemophilus agni), Actinomyces (Corynebacterium), Actinomyces pyogenes, Chlamydia psittaci, Campylobacter fetus venerealis and Campylobacter fetus fetus (formerly C fetus intestinalis), Leptospira interrogans, Leptospira hardjo, Leptospira pomona, and Leptospira grippotyphosa, Leptospira canicola, Leptospira grippotyphosa, Leptospira hardjo (Leptospira hardjoprajitno and Leptospira hardjo-bovis), Brucella abortus, Brucella suis and Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium; Salmonella newport, Mycobacterium avium paratuberculosis, Cryptsporidium parvum, Cryptsporidium hominis, Staphylococcus aureus, Streptococcus dysgalactiae, Streptococcus uberus, Mycoplasma spp, Mycoplasma dispar, Mycoplasma bovis, and Ureaplasma spp., Tritrichomonas foetus, Trichophyton verrucosum, Trichophyton mentagrophytes, Trichophyton sarkisovii, Neospora caninum (formerly Toxoplasma gondii), Babesia bigemina and Babesia bovis, and Dictyocaulus viviparous (Lungworm disease)

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR [combo 001].** According to a preferred embodiment, the combination vaccine comprises attenuated BVDV (types 1 and/or 2) as described herein and at least one antigen of IBR **[combo 002].** According to a preferred embodiment, the IBR antigen is a **live modified virus [combo 003].** According to a further embodiment, the combination vaccine of attenuated BVDV and IBR **contains an antibiotic, e.g. neomycin, for preservation [combo 004].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **PI-3 [combo 005].** According to a preferred embodiment, the combination vaccine comprises attenuated BVDV (types 1 and/or 2) as described herein and at least one antigen of PI-3 **[combo 006]**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **BRSV [combo 007].** According to a more preferred embodiment, the combination vaccine comprises attenuated BVDV (types 1 and/or 2) as described herein and at least one antigen of BRSV **[combo 008].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **BHV [combo 009].** According to a preferred embodiment, the combination vaccine comprises attenuated BVDV (types 1 and/or 2) as described herein and at least one antigen of BHV **[combo 010].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR and PI-3 [combo 011].** According to a preferred embodiment, the combination vaccine comprises attenuated BVDV (types 1 and/or 2) as described herein and at least one antigen of IBR and PI-3 **[combo 012].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR and BRSV [combo 013].** According to a preferred embodiment, the combination vaccine comprises attenuated BVDV (types 1 and/or 2) as described herein and at least one antigen of IBR and BRSV **[combo 014].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR and BHV [combo 015].** According to a preferred embodiment, the combination vaccine comprises attenuated BVDV (types 1 and/or 2) as described herein and at least one antigen of IBR and BHV **[combo 016].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **PI-3 and BRSV [combo 017].** According to a preferred embodiment, the combination vaccine comprises attenuated BVDV (types 1 and/or 2) as described herein and at least one antigen of PI-3 and BRSV **[combo 018].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **PI-3 and BHV [combo 019].** According to a preferred embodiment, the combination vaccine comprises attenuated BVDV (types 1 and/or 2) as described herein and at least one antigen of PI-3 and BHV**[combo 020].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3 and BRSV [combo 021].** According to a preferred embodiment, the combination vaccine comprises attenuated BVDV (types 1 and/or 2) as described herein and at least one antigen of IBR, PI-3 and BRSV **[combo 022].** Preferably, **all viral antigens are modified live viruses [combo 023].** According to a further embodiment of said combination vaccine, the **IBR and PI-3 antigens are modified live viruses and BRSV antigen is a killed virus [combo 024].** According to a further embodiment of said combination vaccine, the **IBR, PI-3 and BRSV antigens are killed viruses [combo 025].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, BRSV and BHV [combo 026].** According to a preferred embodiment, the combination vaccine comprises attenuated BVDV (types 1 and/or 2) as described herein and at least one antigen of IBR, BRSV and BHV **[combo 027].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **PI-3, BRSV and BHV [combo 028].** According to a preferred embodiment, the combination vaccine comprises attenuated BVDV (types 1 and/or 2) as described herein and at least one antigen of PI-3, BRSV and BHV **[combo 029].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3 and BHV [combo 030].** According to a preferred embodiment, the combination vaccine comprises attenuated BVDV (types 1 and/or 2) as described herein and at least one antigen of IBR, PI-3 and BHV **[combo 031].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3, BRSV** and **BHV [combo 032].** According to a more preferred embodiment, the combination vaccine comprises attenuated BVDV (types 1 and/or 2) as described herein and at least one antigen of IBR, PI-3, BRSV and BHV **[combo 033].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **H. somnus [combo 034].** According to a more preferred embodiment, the combination vaccine comprises attenuated BVDV (types 1 and/or 2) as described herein and at least one antigen of H. somnus **[combo 035].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR** and **H. somnus [combo 036].** According to a more preferred embodiment, the combination vaccine comprises attenuated BVDV (types 1 and/or 2) as described herein and at least one antigen of H. somnus **[combo 037].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3,** and **H. somnus [combo 038].** According to a more preferred embodiment, the combination vaccine comprises attenuated BVDV (types 1 and/or 2) as described herein and at least one antigen of IBR, PI-3, and H. somnus **[combo 039].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3, BRSV** and **H. somnus [combo 040].** According to a more preferred embodiment, the combination vaccine comprises attenuated BVDV (types 1 and/or 2) as described herein and at least one antigen of IBR, PI-3, BRSV and H. somnus **[combo 041].** Preferably, all viral antigens are modified live viruses. According to a further embodiment of said combination vaccine, the IBR and PI-3 antigens are modified live viruses, whereas the BRSV antigen is a killed virus **[combo 038].** According to a further embodiment of said combination vaccine, the IBR, PI-3 and BRSV antigens are killed viruses **[combo 042].** According to a further embodiment, any of said combination vaccines, preferably the combination vaccine that comprises killed IBR, killed PI-3 and killed BRSV as antigens, contains neomycin and thimerosal as preservatives **[combo 043].**

According to a further embodiment, the present invention relates to a combination vaccine for the treatment and/or prophylaxis of cattle against viral infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3, BRSV, BHV** and **H. somnus [combo 044].** According to a more preferred embodiment, the combination vaccine comprises attenuated BVDV (types 1 and/or 2) as described herein and at least one antigen of IBR, PI-3, BHV and H. somnus **[combo 045].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by one or more pathogenic specie(s) **of Leptospira,** preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona. **[combo 046].** According to a more preferred embodiment, the combination vaccine comprises attenuated BVDV (types 1 and/or 2) as described herein and antigen of at least one or more pathogenic specie(s) of Leptospira, preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona **[combo 047].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR,** and one or more pathogenic **specie(s) of Leptospira,** preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona. **[combo 048].** According to a more preferred embodiment, the combination vaccine comprises attenuated BVDV (types 1 and/or 2) as described herein and at least one antigen of IBR and one or more pathogenic specie(s) of Leptospira, preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona **[combo 049].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR and Leptospira pomona [combo 050].** According to a more preferred embodiment, the combination vaccine comprises attenuated BVDV (types 1 and/or 2) as described herein and at least one antigen of IBR, preferably a live modified virus, and Leptospira pomona bacterin **[combo 51].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3,** and one or more pathogenic **specie(s) of Leptospira,** preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona **[combo 052].** According to a more preferred embodiment, the combination vaccine comprises attenuated BVDV (types 1 and/or 2) as described herein and at least one antigen of IBR, PI-3 and one or more pathogenic specie(s) of Leptospira, preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona **[combo 053].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3, Leptospira canicola, Leptospira grippotyphosa, Leptospira hardjo (Leptospira hardjoprajitno and/or Leptospira hardjo-bovis), Leptospira icterohaemorrhagiae, and Leptospira pomona [combo 054].** According to a more preferred embodiment, the combination vaccine comprises attenuated BVDV (types 1 and/or 2) as described herein and at least one antigen of IBR, PI-3, Leptospira canicola, Leptospira grippotyphosa, Leptospira hardjo, Leptospira icterohaemorrhagiae, and Leptospira pomona **[combo 055].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3, BRSV** and one or more pathogenic **specie(s) of Leptospira,** preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona **[combo 056].** According to a more preferred embodiment, the combination vaccine comprises attenuated BVDV (types 1 and/or 2) as described herein and at least one antigen of IBR, PI-3, BRSV and one or more pathogenic species of Leptospira, preferably selected from Leptospira canicola, Leptospira grippotyphosa, Leptospira borgpetersenii, Leptospira prajitno, Leptospira icterohaemmorrhagiae, Leptospira bovis, Leptospira interrogans and Leptospira pomona **[combo 057].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by IBR, PI-3, BRSV, **Leptospira canicola, Leptospira grippotyphosa, Leptospira hardjo (Leptospira hardjoprajitno and/or Leptospira hardjo-bovis), Leptospira icterohaemorrhagiae, and Leptospira pomona [combo 058].** According to a more preferred embodiment, the combination vaccine comprises attenuated BVDV (types 1 and/or 2) as described herein and at least one antigen of IBR, PI-3, BRSV, Leptospira canicola, Leptospira grippotyphosa, Leptospira hardjo (Leptospira hardjoprajitno and/or Leptospira hardjo-bovis), Leptospira icterohaemorrhagiae, and Leptospira pomona **[combo 059].** According to a preferred embodiment, **the viral antigens are killed viruses** and the bacterial antigens are bacterins **[combo 060].** Preferably, said combination vaccines as described in this paragraph further contain neomycin and thimerosal as preservatives **[combo 061].**

According to a further embodiment, the combination vaccine comprises attenuated BVDV (types 1 and/or 2) as described herein, **live modified viruses of IBR, PI-3, BRSV,** and bacterin of Leptospira canicola, Leptospira grippotyphosa, Leptospira hardjo (Leptospira hardjoprajitno and/or Leptospira hardjo-bovis), Leptospira icterohaemorrhagiae and Leptospira pomona **[combo 062].** According to a further preferred embodiment, the combination vaccine described in this paragraph comprises neomycin as a preservative **[combo 063].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3, BRSV, BHV** and one or more pathogenic **specie(s) of Leptospira,** preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona. **[combo 064].** According to a more preferred embodiment, the combination vaccine comprises attenuated BVDV (types 1 and/or 2) as described herein and at least one antigen of IBR, PI-3, BRSV, BHV and one or more pathogenic specie(s) of Leptospira, preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona **[combo 065].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by one or more pathogenic **specie(s) of Leptospira,** as mentioned above, and **H. somnus [combo 066].** According to a more preferred embodiment, the combination vaccine comprises attenuated BVDV (types 1 and/or 2) as described herein and at least antigen of one or more pathogenic species of Leptospira, as mentioned above, and H. somnus **[combo 067].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR** and one or more pathogenic **specie(s) of Leptospira,** as mentioned above, and **H. somnus [combo 068].** According to a more preferred embodiment, the combination vaccine comprises attenuated BVDV (types 1 and/or 2) as described herein and at least one antigen of IBR and one or more pathogenic species of Leptospira, as mentioned above, and H. somnus **[combo 069].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR, PI-3** and one or more pathogenic **specie(s) of Leptospira,** as mentioned above, and **H. somnus [combo 070].** According to a more preferred embodiment, the combination vaccine comprises attenuated BVDV (types 1 and/or 2) as described herein and at least one antigen of IBR, PI-3, and one or more pathogenic species of Leptospira, as mentioned above, and H. somnus **[combo 071].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3, Leptospira canicola, Leptospira grippotyphosa, Leptospira hardjo (Leptospira hardjoprajitno and/or Leptospira hardjo-bovis), Leptospira icterohaemorrhagiae, Leptospira pomona and H. somnus [combo 072].** According to a more preferred embodiment, the combination vaccine comprises attenuated BVDV (types 1 and/or 2) as described herein and at least one antigen of IBR, PI-3, Leptospira canicola, Leptospira grippotyphosa, Leptospira hardjo (Leptospira hardjoprajitno and/or Leptospira hardjo-bovis), Leptospira icterohaemorrhagiae, Leptospira pomona, and H. somnus **[combo 073].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR, PI-3, BRSV,** and one or more pathogenic **specie(s) of Leptospira,** as mentioned above, and **H. somnus [combo 074].** According to a preferred embodiment, the combination vaccine comprises attenuated BVDV (types 1 and/or 2) as described herein and at least one antigen of IBR, PI-3, BRSV, and one or more pathogenic species of Leptospira, as mentioned above, and H. somnus **[combo 075].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by **IBR, PI-3, BRSV, Leptospira canicola, Leptospira grippotyphosa, Leptospira hardjo (Leptospirahardjo prajitno and/or Leptospira hardjo-bovis), Leptospira icterohaemorrhagiae, Leptospira pomona and H. somnus [combo 076].** According to a more preferred embodiment, the combination vaccine comprises attenuated BVDV (types 1 and/or 2) as described herein and at least one antigen of IBR, PI-3, BRSV, Leptospira canicola, Leptospira grippotyphosa, Leptospira hardjo, Leptospira icterohaemorrhagiae, Leptospira pomona and H. somnus **[combo 077].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by IBR, PI-3, BRSV, **BHV** and one or more pathogenic **specie(s) of Leptospira,** as mentioned above, and **H. somnus [combo 078].** According to a more preferred embodiment, the combination vaccine comprises attenuated BVDV (types 1 and/or 2) as described herein and at least one antigen of IBR, PI-3, BRSV, BHV and one or more pathogenic species of Leptospira, as mentioned above, and H. somnus **[combo 079].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by one or more pathogenic **specie(s) of Leptospira,** as mentioned above, and **Campylobacter fetus [combo 080].** According to a more preferred embodiment, the combination vaccine comprises attenuated BVDV (types 1 and/or 2) as described herein and at least antigen of one or more pathogenic species of Leptospira, as mentioned above, and Campylobacter fetus **[combo 081].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **Leptospira canicola, Leptospira grippotyphosa, Leptospira hardjo (Leptospira hardjoprajitno and/or Leptospira hardjo-bovis), Leptospira lcterohaemorrhagiae, Leptospira pomona and Campylobacter fetus [combo 082].** According to a preferred embodiment, the combination vaccine comprises attenuated BVDV (types 1 and/or 2) as described herein and at least antigen of Leptospira canicola, Leptospira grippotyphosa, Leptospira hardjo (Leptospira hardjoprajitno and Leptospira hardjo-bovis), Leptospira lcterohaemorrhagiae, Leptospira pomona and Campylobacter fetus **[combo 083].** According to a more preferred embodiment, the bacterial antigens are chemically inactivated, aluminum hydroxide adsorbed, whole cultures of said bacteria **[combo 084].** According to a further preferred embodiment, said combination vaccine comprises gentamicin and Amphotericin B as preservatives **[combo 085].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR** and one or more pathogenic **specie(s) of Leptospira,** as mentioned above, and **Campylobacter fetus [combo 086].** According to a more preferred embodiment, the combination vaccine comprises attenuated BVDV (types 1 and/or 2) as described herein and at least one antigen of IBR and one or more pathogenic species of Leptospira, as mentioned above, and Campylobacter fetus**[combo 087].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR, PI-3** and one or more pathogenic **specie(s) of Leptospira,** as mentioned above, and **Campylobacter fetus [combo 088].** According to a more preferred embodiment, the combination vaccine comprises attenuated BVDV (types 1 and/or 2) as described herein and at least one antigen of IBR, PI-3, and one or more pathogenic species of Leptospira, as mentioned above, and Campylobacter fetus **[combo 089].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR, PI-3, BRSV,** and one or more pathogenic **specie(s) of Leptospira,** as mentioned above, and **Campylobacter fetus [combo 090].** According to a more preferred embodiment, the combination vaccine comprises attenuated BVDV (types 1 and/or 2) as described herein and at least one antigen of IBR, PI-3, BRSV, and one or more pathogenic species of Leptospira, as mentioned above, and Campylobacter fetus **[combo 091].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR, PI-3, BRSV, BHV,** one or more pathogenic **specie(s) of Leptospira,** as mentioned above, and **Campylobacter fetus [combo 092].** According to a more preferred embodiment, the combination vaccine comprises attenuated BVDV (types 1 and/or 2) as described herein and at least one antigen of IBR, PI-3, BRSV, BHV and one or more pathogenic species of Leptospira, as mentioned above, and Campylobacter fetus **[combo 093].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by one or more pathogenic **specie(s) of Leptospira,** as mentioned above, **H. somnus** and **Campylobacter fetus [combo 094].** According to a more preferred embodiment, the combination vaccine comprises attenuated BVDV (types 1 and/or 2) as described herein and at least antigen of one or more pathogenic species of Leptospira, as mentioned above, H. somnus and Campylobacter fetus **[combo 095].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR,** one or more pathogenic **specie(s) of Leptospira,** as mentioned above, **H. somnus** and **Campylobacter fetus [combo 096].** According to a more preferred embodiment, the combination vaccine comprises attenuated BVDV (types 1 and/or 2) as described herein and at least one antigen of IBR, one or more pathogenic species of Leptospira, as mentioned above, H. somnus and Campylobacter fetus **[combo 097].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR, PI-3,** and one or more pathogenic **specie(s) of Leptospira,** as mentioned above, **H. somnus** and **Campylobacter fetus [combo 098].** According to a preferred embodiment, the combination vaccine comprises attenuated BVDV (types 1 and/or 2) as described herein and at least one antigen of IBR, PI-3, and one or more pathogenic species of Leptospira, as mentioned above, H. somnus and Campylobacter fetus **[combo 099].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by IBR, PI-3, Leptospira canicola, Leptospira grippotyphosa, Leptospira hardjo, Leptospira icterohaemorrhagiae, Leptospira pomona, H. somnus and Campylobacter fetus **[combo 100].** According to a further embodiment, the combination vaccine comprises attenuated BVDV (types 1 and/or 2) as described herein and at least one antigen of IBR, PI-3, Leptospira canicola, Leptospira grippotyphosa, Leptospira hardjo (Leptospira hardjoprajitno and/or Leptospira hardjo-bovis), Leptospira icterohaemorrhagiae, Leptospira pomona, H. somnus and Campylobacter fetus **[combo 101].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR, PI-3, BRSV,** and one or more pathogenic **specie(s) of Leptospira,** as mentioned above, **H. somnus** and **Campylobacter fetus [combo 102].** According to a more preferred embodiment, the combination vaccine comprises attenuated BVDV (types 1 and/or 2) as described herein and at least one antigen of IBR, PI-3, BRSV, and one or more pathogenic species of Leptospira, as mentioned above, H. somnus and Campylobacter fetus **[combo 100].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by IBR, PI-3, BRSV, Leptospira canicola, Leptospira grippotyphosa, Leptospira hardjo (Leptospira hardjoprajitno and/or Leptospira hardjo-bovis), Leptospira icterohaemorrhagiae, Leptospira Pomona, H. somnus and Campylobacter fetus **[combo 103].** According to a further embodiment, the combination vaccine comprises attenuated BVDV (types 1 and/or 2) as described herein and at least one antigen of IBR, PI-3, BRSV, Leptospira canicola, Leptospira grippotyphosa, Leptospira hardjo (Leptospira hardjoprajitno and/or Leptospira hardjo-bovis), Leptospira icterohaemorrhagiae, Leptospira pomona, H. somnus and Campylobacter fetus **[combo 104].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **IBR, PI-3, BRSV, BHV** and one or more pathogenic **specie(s) of Leptospira,** as mentioned above, **H. somnus** and **Campylobacter fetus [combo 105].** According to a more preferred embodiment, the combination vaccine comprises attenuated BVDV (types 1 and/or 2) as described herein and at least one antigen of IBR, PI-3, BRSV, BHV and one or more pathogenic species of Leptospira, as mentioned above, H. somnus and Campylobacter fetus **[combo 106].**

According to a further embodiment, the present invention relates to a combination vaccine for the treatment and/or prophylaxis of cattle against infections of the respiratory and reproductive systems in cattle, wherein the combination vaccine comprises attenuated BVDV (types 1 and/or 2) as described herein and at least one further immunological active component effective for the treatment and/or prophylaxis of infections caused by BHV, BRSV, PI-3, IBR, Leptospira canicola, Leptospira grippotyphosa, Leptospira borgpetersenii Leptospira hardjo (Leptospira hardjoprajitno and/or Leptospira hardjo-bovis), Leptospira prajitno, Leptospira icterohaemmorrhagiae, Leptospira bovis, Leptospira interrogans and Campylobacter fetus **[combo 107].** According to a more preferred embodiment, the combination vaccine comprises attenuated BVDV as described herein and at least one antigen of BHV, BRSV, IBR, PI-3, Leptospira canicola, Leptospira grippotyphosa, Leptospira borgpetersenii Leptospira hardjo (Leptospira hardjoprajitno and/or Leptospira hardjo-bovis), Leptospira prajitno, Leptospira icterohaemmorrhagiae, Leptospira borgpetersenii, Leptospira bovis, Leptospira interrogans and Campylobacter fetus **[combo 108].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **Pasteurella haemolytica and Pasteurella multocida [combo 109].** According to a preferred embodiment, the combination vaccine comprises attenuated BVDV (types 1 and/or 2) as described herein and at least one antigen of Pasteurella haemolytica bacterin and Pasteurella multocida bacterin. **[combo 110]** According to a further preferred embodiment, said combination vaccine comprises neomycin and thimerosal as preservatives **[combo 111].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, Pasteurella haemolytica and Pasteurella multocida [combo 112].** According to a preferred embodiment, the combination vaccine comprises attenuated BVDV (types 1 and/or 2) as described herein and at least one antigen of IBR, preferably as live modified viruses, and Pasteurella haemolytica bacterin and Pasteurella multocida bacterin **[combo 113].** According to a further preferred embodiment, said combination vaccine comprises neomycin and thimerosal as preservatives **[combo 114].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, Pasteurella haemolytica and Pasteurella multocida [combo 115].** According to a preferred embodiment, the combination vaccine comprises attenuated BVDV (types 1 and/or 2) as described herein and at least one antigen of IBR, PI-3, preferably as live modified viruses, and Pasteurella haemolytica bacterin and Pasteurella multocida bacterin **[combo 116].** According to a further preferred embodiment, said combination vaccine comprises neomycin and thimerosal as preservatives **[combo 117].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, BRSV, Pasteurella haemolytica and Pasteurella multocida [combo 118].** According to a preferred embodiment, the combination vaccine comprises attenuated BVDV (types 1 and/or 2) as described herein and at least one antigen of IBR, PI-3, BRSV, preferably as live modified viruses, and Pasteurella haemolytica bacterin and Pasteurella multocida bacterin **[combo 119].** According to a further preferred embodiment, said combination vaccine comprises neomycin and thimerosal as preservatives **[combo 120].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, BRSV, BHV, Pasteurella haemolytica and Pasteurella multocida [combo 121].** According to a preferred embodiment, the combination vaccine comprises attenuated BVDV (types 1 and/or 2) as described herein and at least one antigen of IBR, PI-3, BRSV, BHV, preferably as live modified viruses, and Pasteurella haemolytica bacterin and Pasteurella multocida bacterin **[combo 122].** According to a further preferred embodiment, said combination vaccine comprises neomycin and thimerosal as preservatives **[combo 123].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **Mycoplasma bovis [combo 124].** According to a preferred embodiment, the combination vaccine comprises attenuated BVDV (types 1 and/or 2) as described herein and at least antigen of Mycoplasma bovis **[combo 125].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, and Mycoplasma bovis [combo 126].** According to a preferred embodiment, the combination vaccine comprises attenuated BVDV (types 1 and/or 2) as described herein and at least antigen of IBR, preferably as live modified viruses, and Mycoplasma bovis **[combo 127].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, and Mycoplasma bovis [combo 128].** According to a preferred embodiment, the combination vaccine comprises attenuated BVDV (types 1 and/or 2) as described herein and at least one antigen of IBR, PI-3, preferably as live modified viruses, and Mycoplasma bovis **[combo 129].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, BRSV, and Mycoplasma bovis [combo 130].** According to a preferred embodiment, the combination vaccine comprises attenuated BVDV (types 1 and/or 2) as described herein and at least one antigen of IBR, PI-3, BRSV, preferably as live modified viruses, and Mycoplasma bovis **[combo 131].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, BRSV, BHV, and Mycoplasma bovis [combo 132].** According to a preferred embodiment, the combination vaccine comprises attenuated BVDV (types 1 and/or 2) as described herein and at least one antigen of IBR, PI-3, BRSV, BHV, preferably as live modified viruses, and Mycoplasma bovis **[combo 133].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **Pasteurella haemolytica, Pasteurella multocida and Mycoplasma bovis [combo 134].** According to a preferred embodiment, the combination vaccine comprises attenuated BVDV (types 1 and/or 2) as described herein and at least one antigen of Pasteurella haemolytica, Pasteurella multocida and Mycoplasma bovis **[combo 135].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, Pasteurella haemolytica, Pasteurella multocida and Mycoplasma bovis [combo 136].** According to a preferred embodiment, the combination vaccine comprises attenuated BVDV (types 1 and/or 2) as described herein and at least one antigen of IBR, preferably as live modified viruses, Pasteurella haemolytica, Pasteurella multocida and Mycoplasma bovis **[combo 137].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, Pasteurella haemolytica, Pasteurella multocida and Mycoplasma bovis [combo 138].** According to a preferred embodiment, the combination vaccine comprises attenuated BVDV (types 1 and/or 2) as described herein and at least one antigen of IBR, PI-3, preferably as live modified viruses, Pasteurella haemolytica, Pasteurella multocida and Mycoplasma bovis **[combo 139].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, BRSV, Pasteurella haemolytica, Pasteurella multocida and Mycoplasma bovis [combo 140].** According to a preferred embodiment, the combination vaccine comprises attenuated BVDV (types 1 and/or 2) as described herein and at least one antigen of IBR, PI-3, BRSV, preferably as live modified viruses, Pasteurella haemolytica, Pasteurella multocida and Mycoplasma bovis **[combo 141].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, BRSV, BHV, Pasteurella haemolytica, Pasteurella multocida and Mycoplasma bovis [combo 140].** According to a preferred embodiment, the combination vaccine comprises attenuated BVDV (types 1 and/or 2) as described herein and at least one antigen of IBR, PI-3, BRSV, BHV, preferably as live modified viruses, Pasteurella haemolytica, Pasteurella multocida and Mycoplasma bovis **[combo 141].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **Pasteurella haemolytica, Pasteurella multocida and H. somnus [combo 142].** According to a preferred embodiment, the combination vaccine comprises attenuated BVDV (types 1 and/or 2) as described herein and at least one antigen of Pasteurella haemolytica, Pasteurella multocida and H. somnus **[combo 143].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, Pasteurella haemolytica, Pasteurella multocida and H. somnus [combo 144].** According to a preferred embodiment, the combination vaccine comprises attenuated BVDV (types 1 and/or 2) as described herein and at least one antigen of IBR, preferably as live modified virus, and Pasteurella haemolytica, Pasteurella multocida and H. somnus **[combo 145].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, Pasteurella haemolytica, Pasteurella multocida and H. somnus [combo 146].** According to a preferred embodiment, the combination vaccine comprises attenuated BVDV (types 1 and/or 2) as described herein and at least one antigen of IBR, PI-3, preferably as live modified viruses, and Pasteurella haemolytica, Pasteurella multocida and H. somnus **[combo 147].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, BRSV, Pasteurella haemolytica, Pasteurella multocida and H. somnus [combo 148].** According to a preferred embodiment, the combination vaccine comprises attenuated BVDV (types 1 and/or 2) as described herein and at least one antigen of IBR, PI-3, BRSV, preferably as live modified viruses, and Pasteurella haemolytica, Pasteurella multocida and H. somnus **[combo 149].**

According to a further embodiment, the present invention relates to a combination vaccine for the treatment and/or prophylaxis of cattle against infections in cattle, wherein the combination vaccine comprises attenuated BVDV (types 1 and/or 2) as described herein and at least one further immunological active component effective for the treatment and/or prophylaxis of infections caused by **IBR, PI-3, BRSV, BHV, Pasteurella haemolytica, Pasteurella multocida and H. somnus [combo 150].** According to a preferred embodiment, the combination vaccine comprises attenuated BVDV (types 1 and/or 2) as described herein and at least one antigen of IBR, PI-3, BRSV, BHV, preferably as live modified viruses, and Pasteurella haemolytica, Pasteurella multocida and H. somnus **[combo 151].**

According to a further embodiment, the present invention relates to a combination vaccine according to any one of **[combo 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150 and 151],** that further comprises an immunological active component effective for the treatment and/or prophylaxis of infections caused by one or more pathogenic **species of Leptospira,** preferably selected from Leptospira canicola, Leptospira grippotyphosa, Leptospira borgpetersenii, Leptospira hardjo, Leptospira prajitno, Leptospira icterohaemmorrhagiae, Leptospira bovis, Leptospira interrogans and Leptospira pomona **[combo 152].** According to a further embodiment, the present invention relates to a combination vaccine according to any one of [combo 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150 and 151], that further comprises antigen of one or more specie(s) of Leptospira, preferably selected from Leptospira canicola, Leptospira grippotyphosa, Leptospira borgpetersenii, Leptospira hardjo (Leptospira hardjoprajitno and Leptospira hardjo-bovis),, Leptospira prajitno, Leptospira icterohaemmorrhagiae, Leptospira bovis, Leptospira interrogans and Leptospira pomona. **[combo 153].**

According to a further embodiment, the present invention relates to a combination vaccine according to any one of **[combo 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150 and 151],** that further comprises an immunological active component effective for the treatment and/or prophylaxis of infections caused by **Campylobacter fetus [combo 154].** According to a further embodiment, the present invention relates to a combination vaccine according to any one of [combo 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150 and 151], that further comprises antigen of Campylobacter fetus **[combo 155].**

According to a further embodiment, the present invention relates to a combination vaccine according to any one of **[combo 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150 and 151],** that further comprises an immunological active component effective for the treatment and/or prophylaxis of infections caused by one or more pathogenic **specie(s) of Leptospira,** preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona, and **Campylobacter fetus [combo 156].** According to a further embodiment, the present invention relates to a combination vaccine according to any one **of [combo 110, 111, 112,113, 114,115, 116, 117, 118, 119, 120, 121, 122, 123, 124, 125, 126, 127, 128, 129, 130, 131,132, 133, 134, 135, 136, 137, 138, 139, 140, 141,142, 143, 144, 145, 146, 147, 148, 149, 150 and 151], that** further comprises antigen of Campylobacter fetus and of one or more **specie(s) of Leptospira,** preferably selected from Leptospira canicola, Leptospira grippotyphosa, Leptospira borgpetersenii, Leptospira hardjo (Leptospira hardjoprajitno and Leptospira hardjo-bovis), Leptospira prajitno, Leptospira icterohaemmorrhagiae, Leptospira bovis, Leptospira interrogans and Leptospira pomona, and. **[combo 157].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Clostridium perfringens, preferably Types A, C and/or D [combo 158].** According to a preferred embodiment, the combination vaccine comprises attenuated BVDV (types 1 and/or 2) as described herein and toxins of Clostridium perfringens Types C and D **[combo 254].** According to a more preferred embodiment, said vaccine comprises antigens, preferably toxins, of Clostridium perfringens, preferably Types A, B, C, and/or D **[combo 159].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156 and 157],** that further comprises an immunological active component effective for the treatment and/or prophylaxis of infections caused by infections caused by **Clostridium perfringens, preferably Types A, C and/or D [combo 160].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156 and 157], that further comprises antigen of Clostridium perfringens, preferably, Types A, C, and/or D **[combo 161].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156 and 157], that further comprises antigen of Clostridium perfringens Types, B, C, and/or D **[combo 162].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Clostridium perfringens Types A, C and/or D, and Clostridium tetani [combo 163].** According to a preferred embodiment, the combination vaccine comprises attenuated BVDV (types 1 and/or 2) as described herein and toxins of Clostridium perfringens Types A, C and/or D, and Clostridium tetani **[combo 164].** According to a more preferred embodiment, said vaccine comprises antigens, preferably toxins, of Clostridium perfringens Types A, B, C, and/or D, and Clostridium tetani **[combo 165].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156 and 157],** that further comprises an immunological active component effective for the treatment and/or prophylaxis of infections caused by infections caused by **Clostridium perfringens Types A, C and/or D, and Clostridium tetani [combo 166].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156 and 157], that further comprises antigen of Clostridium perfringens Types A, C, and/or D, and Clostridium tetani **[combo 167].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156 and 157], that further comprises antigen of Clostridium perfringens Types A, B, C, and/or D, and Clostridium tetani **[combo 168].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Clostridium chauvoei, Clostridium septicum, Clostridium novyi, Clostridium sordellii, and Clostridium perfringens Types A, C and/or D [combo 169].** According to a preferred embodiment, the combination vaccine comprises attenuated BVDV (types 1 and/or 2) as described herein and antigens, preferably toxins, of Clostridium chauvoei, Clostridium septicum, Clostridium novyi, Clostridium sordellii, and Clostridium perfringens Types A, C and/or D **[combo 170].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156 and 157],** that further comprises an immunological active component effective for the treatment and/or prophylaxis of infections caused by infections caused by **Clostridium chauvoei, Clostridium septicum, Clostridium novyi, Clostridium sordellii, and Clostridium perfringens Types A, C and/or D [combo 171].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156 and 157], that further comprises antigen of Clostridium chauvoei, Clostridium septicum, Clostridium novyi, Clostridium sordellii, and Clostridium perfringens Types A, C and/or D **[combo 172].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156 and 157], that further comprises antigen of Clostridium perfringens Types, A, B, C, and/or D, Clostridium chauvoei, Clostridium septicum, Clostridium novyi, Clostridium sordellii and Clostridium tetani **[combo 173].**

According to more preferred embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Clostridium chauvoei, Clostridium septicum, Clostridium novyi, Clostridium sordellii, Clostridium perfringens Types A, C and/or D and Mycoplasma bovis [combo 174].** According to a preferred embodiment, the combination vaccine comprises attenuated BVDV (types 1 and/or 2) as described herein and antigens, preferably toxins, of Clostridium chauvoei, Clostridium septicum, Clostridium novyi, Clostridium sordellii, and Clostridium perfringens Types A, C and/or D and Mycoplasma bovis **[combo 175].**

According to more preferred embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Clostridium chauvoei, Clostridium septicum, Clostridium novyi, Clostridium sordellii, Clostridium perfringens Types A, C and/or D, and H. somnus. [combo 176].** According to a preferred embodiment, the combination vaccine comprises attenuated BVDV (types 1 and/or 2) as described herein and antigens, preferably toxins, of Clostridium chauvoei, Clostridium septicum, Clostridium novyi, Clostridium sordellii, and Clostridium perfringens Types C and D and H. somnus. **[combo 177].**

According to more preferred embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Clostridium chauvoei, Clostridium septicum, Clostridium novyi, Clostridium sordellii, Clostridium perfringens Types A, C and/or D, Mycoplasma bovis, and H.somnus [combo 178].** According to a preferred embodiment, the combination vaccine comprises attenuated BVDV (types 1 and/or 2) as described herein and antigens, preferably toxins, of Clostridium chauvoei, Clostridium septicum, Clostridium novyi, Clostridium sordellii, and Clostridium perfringens Types A, C and/or D, Mycoplasma bovis, and H.somnus **[combo 179].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by Salmonella, preferably **Salmonella dublin, Salmonella newport and Salmonella typhimurium [combo 180].** According to a preferred embodiment, the combination vaccine comprises attenuated BVDV (types 1 and/or 2) as described herein and toxins of Salmonella, preferably Salmonella dublin, Salmonella newport, and Salmonella typhimurium **[combo 181].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178 and 179],** that further comprises an immunological active component effective for the treatment and/or prophylaxis of infections caused by infections caused by Salmonella, preferably **Salmonella dublin, Salmonella newport and Salmonella typhimurium [combo 182].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 254, 159, 160, 161, 162, 163, 164, 165, 166, 167, 168, 169, 170, 171, 172, 173, 174, 175, 176, 177, 178 and 179], that further comprises antigen, preferably a toxin, of Salmonella, preferably **Salmonella dublin, salmonella newport and Salmonella typhimurium [combo 183].**

According to a preferred embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Pasteurella haemolytica, Pasteurella multocida, Salmonella, preferably Salmonella dublin, Salmonella newport and Salmonella typhimurium [combo 184].** According to a preferred embodiment, the combination vaccine comprises attenuated BVDV (types 1 and/or 2) as described herein and **Pasteurella haemolytica, Pasteurella multocida, Salmonella, preferably Salmonella dublin, Salmonella newport, and Salmonella typhimurium** Bacterin-Toxoid **[combo 185].** According to more preferred embodiment, said combination vaccine comprises multiple isolates of Pasteurella haemolytica Type A1 and an associated toxoid fraction, and single isolates of P multocida, S dublin, and S typhimurium **[combo 186].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Moraxella bovis and/or Klebsiella spp.** , **preferably Klebsiella pneumoniae [combo 187].** According to a preferred embodiment, the combination vaccine comprises attenuated BVDV (types 1 and/or 2) as described herein and toxins of **Moraxella bovis and/or Klebsiella spp. preferably Klebsiella pneumoniae [combo 188].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184, 185, and 186],** that further comprises an immunological active component effective for the treatment and/or prophylaxis of infections caused by infections caused by **Moraxella bovis and/or Klebsiella spp., preferably Klebsiella pneumoniae [combo 189].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149,150, 151, 152, 153, 154, 155, 156, 157, 158, 254, 159, 163, 164, 165, 169, 170, 174, 175, 176, 177, 178, 179, 180, 181, 184, 185 and 186], that further comprises antigen, preferably a toxin, of **Moraxella bovis and/or Klebsiella spp., preferably, Klebsiella pneumoniae [combo 190].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Escherichia coli [combo 191].** According to a preferred embodiment, the combination vaccine comprises attenuated BVDV (types 1 and/or 2) as described herein and toxins of Escherichia coli **[combo 192].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189 and 190],** that further comprises an immunological active component effective for the treatment and/or prophylaxis of infections caused by infections caused by Eschericia coli **[combo 193].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 254, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189 and 190], that further comprises antigen, preferably a toxin, of Escherichia coli **[combo 194].**

According to a preferred embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Pasteurella haemolytica, Pasteurella multocida, Salmonella dublin ,Salmonella typhimurium and Eschericha coli [combo 195].** According to a preferred embodiment, the combination vaccine comprises attenuated BVDV (types 1 and/or 2) as described herein and **Pasteurella haemolytica, Pasteurella multocida, Salmonella dublin,Salmonella typhimurium and Escherichia coli** Bacterin-Toxoid **[combo 196].** According to more preferred embodiment, said combination vaccine comprises multiple isolates of Pasteurella haemolytica Type A1 and an associated toxoid fraction, and single isolates of P multocida, S dublin, and S typhimurium **[combo 197].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **bovine Rotavirus [combo 198].** According to a preferred embodiment, the combination vaccine comprises attenuated BVDV (types 1 and/or 2) as described herein and antigen of bovine Rotavirus **[combo 199].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184, 185,186, 187, 188, 189, 190, 191, 192, 195, 196, and 197],** that further comprises an immunological active component effective for the treatment and/or prophylaxis of infections caused by infections caused by **bovine Rotavirus [200].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 254, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196 and 197], that further comprises **antigen of bovine Rotavirus [combo 201].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **bovine Coronavirus [combo 202].** According to a preferred embodiment, the combination vaccine comprises attenuated BVDV (types 1 and/or 2) as described herein and antigen of bovine Coronavirus **[combo 203].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173,174,175,176,177,178,179,180,181,182,183,184,185,186,187,188,189,190,191,192, 193, 194, 195, 196 and 197],** that further comprises an immunological active component effective for the treatment and/or prophylaxis of infections caused by infections caused by **bovine Coronavirus [combo 204].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 254, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188,189, 190, 191, 192, 193, 194, 195, 196, and 197], that further comprises antigen of bovine Coronavirus **[combo 205].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **bovine Coronavirus and bovine Rotavirus [combo 206].** According to a preferred embodiment, the combination vaccine comprises attenuated BVDV (types 1 and/or 2) as described herein and antigen of bovine Coronavirus and bovine Rotavirus **[combo 207].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112,113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173,174,175,176,177,178,179,180,181,182,183,184,185,186,187,188,189,190,191,192, 193, 194, 195, 196 and 197],** that further comprises an immunological active component effective for the treatment and/or prophylaxis of infections caused by infections caused by **bovine Coronavirus and bovine Rotavirus [combo 208].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, and 197], that further comprises antigen of bovine Coronavirus and bovine Rotavirus **[combo 209].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Cryptosporidium parvum [combo 210].** According to a preferred embodiment, the combination vaccine comprises attenuated BVDV (types 1 and/or 2) as described herein and antigen of Cryptosporidium parvum **[combo 211].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184 184, 185, 186, 187, 188, 189, 190, 191, 192, 193, 194, 195, 196, 197, 198, 199, 200, 201, 202, 203, 204, 205, 206, 207, 208 and 209],** that further comprises an immunological active component effective for the treatment and/or prophylaxis of infections caused by infections caused by **Cryptosporidium parvum [combo 212].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 ,158, 254, 159, 160, 161, 162, 163, 164, 165, 166, 167, 168, 169, 170,171, 172, 173, 174, 175, 176, 177, 178, 179, 180, 181, 182, 183, 184, 185,186, 187, 188, 189, 190, 191, 192, 193, 194, 195, 196, 197 198, 199, 200, 201, 202, 203, 204 205, 206, 207, 208 and 209], that further comprises antigen of Cryptosporidium parvum **[combo 213].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Cryptosporidium hominis [combo 214].** According to a preferred embodiment, the combination vaccine comprises attenuated BVDV (types 1 and/or 2) as described herein and antigen of Cryptosporidium hominis **[combo 215].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184 184, 185, 186, 187, 188, 189, 190, 191, 192, 193, 194, 195, 196, 197, 198, 199, 200, 201, 202, 203, 204, 205, 206, 207, 208 and 209],** that further comprises an immunological active component effective for the treatment and/or prophylaxis of infections caused by infections caused by **Cryptosporidium hominis [combo 216].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 ,158, 254, 159, 160, 161, 162, 163, 164, 165, 166, 167, 168, 169, 170,171, 172, 173, 174, 175, 176, 177, 178, 179, 180, 181, 182, 183, 184, 185,186, 187, 188, 189, 190, 191, 192, 193, 194, 195, 196, 197 198, 199, 200, 201, 202, 203, 204 205, 206, 207, 208 and 209], that further comprises antigen of Cryptosporidium hominis **[combo 217].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Cryptosporidium parvum and Cryptosporidium hominis [combo 218].** According to a preferred embodiment, the combination vaccine comprises attenuated BVDV (types 1 and/or 2) as described herein and antigen of Cryptosporidium parvum and Cryptosporidium hominis **[combo 219].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184 184, 185, 186, 187, 188, 189, 190, 191, 192, 193, 194, 195, 196, 197, 198, 199, 200, 201, 202, 203, 204, 205, 206, 207, 208 and 209],** that further comprises an immunological active component effective for the treatment and/or prophylaxis of infections caused by infections caused by **Cryptosporidium parvum and Cryptosporidium hominis [combo 220].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157, 158, 254, 159, 160, 161, 162, 163, 164, 165, 166, 167, 168, 169, 170,171, 172, 173, 174, 175, 176, 177, 178, 179, 180, 181, 182, 183, 184, 185,186, 187, 188, 189, 190, 191, 192, 193, 194, 195, 196, 197 198, 199, 200, 201, 202, 203, 204 205, 206, 207, 208 and 209], that further comprises antigen of Cryptosporidium parvum and Cryptosporidium hominis **[combo 221].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Mycobacterium avium paratuberculosis [combo 222].** According to a preferred embodiment, the combination vaccine comprises attenuated BVDV (types 1 and/or 2) as described herein and antigen of Mycobacterium avium paratuberculosis **[combo 223].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157, 158, 254, 159, 160, 161, 162, 163, 164, 165, 166, 167, 168, 169, 170, 171, 172, 173, 174, 175, 176, 177, 178, 179, 180, 181, 182, 183, 184 184, 185, 186, 187, 188, 189, 190, 191, 192,193,194,195,196, 197, 198, 199, 200, 201, 202, 203, 204, 205, 206, 207, 208, 209, 210, 211, 212, 213, 214, 215, 216, 217, 218, 219, 220 and 221],** that further comprises an immunological active component effective for the treatment and/or prophylaxis of infections caused by infections caused by **Mycobacterium avium paratuberculosis [combo 224].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 ,158, 254, 159,160, 161, 162, 163, 164, 165, 166, 167, 168, 169, 170, 171, 172, 173, 174, 175, 176, 177, 178, 179, 180, 181, 182, 183, 184, 185,186, 187, 188, 189, 190, 191, 192, 193, 194, 195, 196, 197 198, 199, 200, 201, 202, 203, 204 205, 206, 207, 208, 209, 210, 211, 212, 213, 214, 215, 216, 217, 218, 219, 220 and 221], that further comprises antigen of Mycobacterium avium paratuberculosis **[combo 225].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Adenovirus [combo 226].** According to a preferred embodiment, the combination vaccine comprises attenuated BVDV (types 1 and/or 2) as described herein and antigen of Adenovirus **[combo 227].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184 184, 185, 186, 187, 188, 189, 190, 191, 192,193,194,195,196, 197, 198, 199, 200, 201, 202, 203, 204, 205, 206, 207, 208, 209, 210, 211, 212, 213, 214, 215, 216, 217, 218, 219, 220, 221, 222, 223, 224 and 225],** that further comprises an immunological active component effective for the treatment and/or prophylaxis of infections caused by infections caused by **Adenovirus [combo 228].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 ,158, 254, 159, 160, 161, 162, 163, 164, 165, 166, 167, 168, 169, 170,171, 172, 173, 174, 175, 176, 177, 178, 179, 180, 181, 182, 183, 184, 185,186, 187, 188, 189, 190, 191, 192, 193, 194, 195, 196, 197 198, 199, 200, 201, 202, 203, 204 205, 206, 207, 208, 209, 210, 211, 212, 213, 214, 215, 216, 217, 218, 219, 220, 221, 222, 223,224, and 225], that further comprises antigen of Adenovirus **[combo 229].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Astrovirus [combo 230].** According to a preferred embodiment, the combination vaccine comprises attenuated BVDV (types 1 and/or 2) as described herein and antigen of Astrovirus **[combo 231].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184 184, 185, 186, 187, 188, 189, 190, 191, 192,193,194,195,196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228 and 229],** that further comprises an immunological active component effective for the treatment and/or prophylaxis of infections caused by infections caused by **Astrovirus [combo 232].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 ,158, 254, 159, 160, 161, 162, 163, 164, 165, 166, 167, 168, 169, 170,171, 172, 173, 174, 175, 176, 177, 178, 179, 180, 181, 182, 183, 184, 185,186, 187, 188, 189, 190, 191, 192, 193, 194, 195, 196, 197 198, 199, 200, 201, 202, 203, 204 205, 206, 207, 208, 209, 210, 211, 212, 213, 214, 215, 216, 217, 218, 219, 220, 221, 222, 223, 224, 225, 226, 227, 228 and 229], that further comprises antigen of Astrovirus **[combo 233].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **bovine Parvovirus [combo 234].** According to a preferred embodiment, the combination vaccine comprises attenuated BVDV (types 1 and/or 2) as described herein and antigen of bovine Parvovirus **[combo 235].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184 184, 185, 186, 187, 188, 189, 190, 191,** **192,193,194,195,196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232 and 233],** that further comprises an immunological active component effective for the treatment and/or prophylaxis of infections caused by infections caused by **bovine Parvovirus [combo 236].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 ,158, 254, 159, 160, 161, 162, 163, 164, 165, 166, 167, 168, 169, 170,171, 172, 173, 174, 175, 176, 177, 178, 179, 180, 181, 182, 183, 184, 185,186, 187, 188, 189, 190, 191, 192, 193, 194, 195, 196, 197 198, 199, 200, 201, 202, 203, 204 205, 206, 207, 208, 209, 210, 211, 212, 213, 214, 215, 216, 217, 218, 219, 220, 221, 222, 223, 224, 225, 226, 227, 228, 229, 230, 231, 232, and 233], that further comprises antigen of bovine Parvovirus **[combo 237].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Cryptosporidium parvum, Adenovirus, Astrovirus, bovine Parvovirus and Mycobacterim avium paratuberculosis [combo 238].** According to a preferred embodiment, the combination vaccine comprises attenuated BVDV (types 1 and/or 2) as described herein and antigen of Cryptosporidium parvum, Adenovirus, Astrovirus, bovine Parvovirus and Mycobacterim avium paratuberculosis **[combo 239].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Escherichia coli, Salmonella spp., preferably Salmonella dublin, Salmonella typhimurium and Salmonella newport, bovine Rotavirus and bovine Coronavirus, Cryptosporidium parvum, Adenovirus, Astrovirus, bovine Parvovirus and Mycobacterim avium paratuberculosis [combo 240].** According to a preferred embodiment, the combination vaccine comprises attenuated BVDV (types 1 and/or 2) as described herein and antigen of Escherichia coli, Salmonella spp., preferably Salmonella dublin, Salmonella typhimurium and Salmonella newport, bovine rotavirus and bovine Coronavirus, Cryptosporidium parvum, Adenovirus, Astrovirus, bovine Parvovirus and Mycobacterim avium paratuberculosis **[combo 241].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Streptococcus spp., preferably Streptoccoccus uberus and** /or **Streptococcus dysgalactiae [combo 242].** According to a preferred embodiment, the combination vaccine comprises attenuated BVDV (types 1 and/or 2) as described herein and antigen of Streptococcus spp., preferably Streptoccoccus uberus and/or Streptococcus dysgalactiae, **[combo 243].** According to a more preferred embodiment, the combination vaccine comprises attenuated BVDV (types 1 and/or 2) as described herein and antigen of Streptococcus spp., preferably Streptoccoccus uberus and/or Streptococcus dysgalactiae [combo 244].

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Streptococcus spp., preferably Streptoccoccus uberus and /or Streptococcus dysgalactiae and/or Staphylococcus aureus [combo 245].** According to a more preferred embodiment, the combination vaccine comprises attenuated BVDV (types 1 and/or 2) as described herein and antigen of Streptococcus spp., preferably Streptoccoccus uberus and/or Streptococcus dysgalactiae, and/or **Staphylococcus aureus [combo 246].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033,** **034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184 184, 185, 186, 187, 188, 189, 190, 191, 192,193,194,195,196, 197, 198, 199, 200, 201, 202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, and 241],** that further comprises an immunological active component effective for the treatment and/or prophylaxis of infections caused by infections caused by **Streptococcus spp., preferably Streptoccoccus uberus and/or Streptococcus dysgalactiae, and/or Staphylococcus aureus [combo 247].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 ,158, 254, 159, 160, 161, 162, 163, 164, 165, 166, 167, 168, 169, 170,171, 172, 173, 174, 175, 176, 177, 178, 179, 180, 181, 182, 183, 184, 185,186, 187, 188, 189, 190, 191, 192, 193, 194, 195, 196, 197 198, 199, 200, 201, 202, 203, 204 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240 and 241], that further comprises antigen **of Streptococcus spp., preferably Streptoccoccus uberus and/or Streptococcus dysgalactiae, and/or Staphylococcus aureus [combo 248].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 ,158, 254, 159, 160, 161, 162, 163, 164, 165, 166, 167, 168, 169, 170,171, 172, 173, 174, 175, 176, 177, 178, 179, 180, 181, 182, 183, 184, 185,186, 187, 188, 189, 190, 191, 192, 193, 194, 195, 196, 197 198, 199, 200, 201, 202, 203, 204 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240 and 241], that further comprises antigen **of Streptococcus spp., preferably Streptoccoccus uberus and/or Streptococcus dysgalactiae, and/or Staphylococcus aureus [combo 249].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Streptococcus spp., preferably Streptoccoccus uberus and/or Streptococcus dysgalactiae, Staphylococcus aureus, Klebsiella spp. and Mycoplasma spp. [combo 250].** According to a preferred embodiment, the combination vaccine comprises attenuated BVDV (types 1 and/or 2) as described herein and antigen of **Streptococcus spp., preferably Streptoccoccus uberus, Streptococcus dysgalactiae, and/or Streptococcus aureus, Klebsiella spp. and Mycoplasma spp. [combo 251].** According to a more preferred embodiment, the combination vaccine comprises attenuated BVDV (types 1 and/or 2) as described herein and antigen of **Streptococcus spp., preferably Streptoccoccus uberus and/or Streptococcus dysgalactiae, Staphylococcus aureus, Klebsiella spp., Mycoplasma spp. and endotoxin [combo 252].**

According to a further embodiment, the present invention relates to a combination vaccine for the treatment and/or prophylaxis of cattle against microbiological infections in cattle, wherein the combination vaccine comprises attenuated BVDV (types 1 and/or 2) as described herein and at least one further immunological active component effective for the treatment and/or prophylaxis of infections caused by **Trichophyten and Microsporus,** preferably selected from **Trichophyton verrucosum, Trichophyton mentagrophytes, Trichophyton equinum, Trichophyton sarkisovii, Microsporum canis, Microsporum canis var. obesum, Microsporum canis var. distortum, and Microsporum gypseum [combo 253].** According to a preferred embodiment, the combination vaccine comprises attenuated BVDV (types 1 and/or 2) as described herein and antigen of Trichophyten, **and Microsporus,** preferably selected from Trichophyton verrucosum, Trichophyton mentagrophytes, Trichophyton equinum, Trichophyton sarkisovii, Microsporum canis, Microsporum canis var. obesum, Microsporum canis var. distortum, and Microsporum gypseum **[combo 254].**

According to a further embodiment, the present invention relates to a combination vaccine according to any one of **[combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118,119, 120, 121, 122, 123, 124, 125, 126, 127, 128,129, 130, 131, 132, 133, 134, 135, 136, 137, 138,139, 140, 141, 142, 143, 144, 145, 146, 147, 148,149, 150, 151, 152, 153, 154, 155, 156, 157 ,158, 254, 159, 160, 161, 162, 163, 164, 165, 166, 167, 168, 169, 170, 171, 172, 173,174,175,176,177,178,179,180,181,182,183,184,185,186,187,188,189,190,191,192, 193, 194, 195, 196, 197, 198, 199, 200, 201,202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, and 251],** that further comprises an immunological active component effective for the treatment and/or prophylaxis of infections caused by infections caused by **Trichophyten and Microsporus,** preferably selected from **Trichophyton verrucosum, Trichophyton mentagrophytes, Trichophyton equinum, Trichophyton sarkisovii, Microsporum canis, Microsporum canis var. obesum, Microsporum canis var. distortum, and Microsporum gypseum [combo 255].** According to a further embodiment, the present invention relates to a combination vaccine according to any one of [combo 001, 002, 003, 004, 005, 006, 007, 008, 009, 010, 011, 012, 013, 014, 015, 016, 017, 018, 019, 020, 021, 022, 023, 024, 025, 026, 027, 028, 029, 030, 031, 032, 033, 034, 035, 036, 037, 038, 039, 040, 041, 042, 043, 044, 045, 046, 047, 048, 049, 050, 051, 052, 053, 054, 055, 056, 057, 058, 059, 060, 061, 062, 063, 064, 065, 066, 067, 068, 069, 070, 071, 072, 073, 074, 075, 076, 077, 078, 079, 080, 081, 082, 083, 084, 085, 086, 087, 088, 089, 090, 091, 092, 093, 094, 095, 096, 097, 098, 099, 100, 101, 102, 103, 104, 105, 106, 107, 108, 109, 110, 111, 112, 113, 114, 115, 116, 117, 118, 119, 120, 121, 122, 123, 124, 125, 126, 127, 128, 129, 130, 131, 132, 133, 134, 135, 136, 137, 138, 139, 140, 141, 142, 143, 144, 145, 146, 147, 148, 149, 150, 151, 152, 153, 154, 155, 156, 157 ,158, 254, 159, 160, 161, 162, 163, 164, 165, 166, 167, 168, 169, 170, 171, 172, 173, 174, 175, 176, 177, 178, 179, 180, 181, 182, 183, 184, 185, 186, 187, 188, 189, 190, 191, 192, 193, 194, 195, 196, 197, 198, 199, 200, 201,202, 203, 204, 205, 206, 207, 208, 209, 210, 211, 212, 213, 214, 215, 216, 217, 218, 219, 220, 221, 222, 223, 224, 225, 226, 227, 228, 229, 230, 231, 232, 233, 234, 235, 236, 237, 238, 239, 240, 241, 242, 243, 244, 245, 246, 247, 248, 249, 250, 251, and 252], that further comprises antigen of Trichophyten and Microsporus, preferably selected from Trichophyton verrucosum, Trichophyton mentagrophytes, Trichophyton equinum, Trichophyton sarkisovii, Microsporum canis, Microsporum canis var. obesum, Microsporum canis var. distortum, and Microsporum gypseum **[combo 256].**

According to further embodiment, the source of the combination vaccine is, Alpha 7™, ALPHA-7/MB™, ALPHA-CD™, BAR-VAC® 7, BAR-VAC® 7/SOMNUS, BAR-VAC® 8, BAR-VAC® CD, BAR-VAC® C/DT, BREED-BACK™ FP 10, BREED-BACK™ FP 10 HS, BREED-BACK™ FP 5, BREED-BACK™ FP 5 HS, BREED-BACK-10™, CALIBER® 3, CALIBER® 7, ELITE 4™, ELITE 9™, ELITE 9-HS™, EXPRESS®10, EXPRESS®10-HS, EXPRESS® 3, EXPRESS® 3/Lp, EXPRESS 4®, EXPRESS® 5, EXPRESS® 5-HS, EXPRESS® 5-PHM, EXPRESS® I, EXPRESS® I/LP, OCU-GUARD® MB, PULMO-GUARD™ MpB, PULMO-GUARD™ PH-M, PULMO-GUARD™ PH-M/SDT, PULMO-GUARD™ PHM-1, TETGUARD™, VIBRIO-LEPTO-5™ (all of **Boehringer Ingelheim, St. Joseph, MO**); Cobalt™ 7, I-Site™, Lepto 5, Master Guard® Preg 5, Master Guard® 10, Master Guard® 10 + Vibrio, Master Guard® J5, P.H.M. Bac® 1, Prevent 6™, Respromune® 4, Respromune® 4 + Somnumune® (IM, SC), Respromune® 5 I-B-P+BRSV, Respromune® 5+L5, Respromune® 5+L5 Somnus, Respromune® 5+Somnumune, Respromune® 5+VL5, Respromune® 8, Respromune® 9, Respromune® 10, Scour Vac™ 4, Scour Vac™ 9, Scour Vac™ E coli + C, Somnumune®, Titanium™ 3, Titanium™ 4, Titanium™ 4 L5, Titanium™ 5, Titanium™ 5 L5, Titanium® 5+P.H.M. Bac®-1, Titanium™ BRSV 3, Titanium™ IBR, Titanium™ IBR-LP (all of **Agri Laboratories Inc., St. Joseph, MO**); Herd-Vac® 3, Herd-Vac® 3 S, Herd-Vac® 8, Herd-Vac® 9, Surround™ 4, Surround™ 4+HS, Surround™ 8, Surround™ 9, Surround™ 9+HS, Surround™ HS, Surround™ L5, Surround™ V-L5 (all of **BioCor, Omaha, NE (Pfizer));** Mycomune® (**Biomune Co., Lenexa, KS);** Bluetongue vaccine, Bovine Virus Diarrhea Vaccine, Campylobacter fetus bacterin-bovine, Essential 1, Essential 2, Essential 2+P, Essential 3, Essential 3+T, Essential 4, Lepto-5, Mannheimia haemolytica-Pasteurella multocida bacterin, Pre-breed 6, Pre-breed 8, Respira-1, Respira-3,Wart Vaccine (all of **Colorado Serum Company, Denver, CO);** Pyramid® 3, Pyramid® 4, Pyramid® 4 + Presponse® SQ, Pyramid® 5, Pyramid® 8, Pyramid® 9, Pyramid®IBR, Pyramid®IBR+Lepto, Triangle® 1 + Type II BVD, Triangle® 3 + VL5, Triangle® 4 + HS, Triangle® 4 + PH/HS, Triangle® 4 + PH-K, Triangle® 4 + Type II BVD, Triangle® 9 + HS, Triangle® 9 + PH-K, Triangle® + Type II BVD, Trichguard®, Trichguard® + V5L, TriVib 5L® (all of **Fort Dodge Animal Health, Overland Park, KS (Wyeth));** J-5 Escherichia coli Bacterin, Serpens Species Bacterin; Staphylococcus aureus bacterin-toxoid (all of **Hygieia Biological Laboratories, Woodland, CA);** Endovac-Bovi® with Immuneplus® **(Immvac, Inc., Columbia, MO);** 20/20 Vision® with Spur®, L5 SQ, Neoguard™, MasterGuard® Preg 5, Once PMH®, Once PMH® SQ, Vibralone™-L5, Vision® 7 Somnus with Spur®, Vision@ 7 with Spur®, Vision® 8 Somnus with Spur®, Vision® 8 with Spur®, Vision® CD-T with Spur®, Vision® CD with Spur®, Vista™ IBR SQ, Vista™ 3 SQ, Vista™ 5 SQ, Vista™ 5 L5 SQ, Vista™ Once SQ, VL5 SQ, Volar®, (all of **Intervet Inc., Millsboro, DE);** Vac®, Reliant® 3, Reliant® 4, Reliant® IBR, Reliant® IBR/BVD, Reliant® IBR/Lepto, Reliant® Plus BVD-K (Dual IBR™), Reliant® Plus (Dual IBR™), Respishield™ 4, Respishield™ 4 L5, Respishield™ HM (all of **Merial LTD, Duluth, GA);** Arsenal® 4.1, Arsenal® IBR, Arsenal® IBR BVD, Bovine Pili Shield™, Bovine Pili Shield™+C, Clostri Shield® 7, Clostri Shield® BCD, Fusogard®, Lepto Shield™ 5, Pinkeye Shield™ XT4, Salmo Shield® T, Salmo Shield® TD, Scour Bos™ 4, Scour Bos™ 9, Somnu Shield™, Trep Shield™ HW, Vib Shield® L5, Vib Shield® Plus, Vib Shield® Plus L5, Vira Shield® 2, Vira Shield® 2+BRSV, Vira Shield® 3, Vira Shield® 3+VL5, Vira Shield® 4, Vira Shield® 4+L5, Vira Shield® 5, Vira Shield® 5+L5, Vira Shield® 5+L5 Somnus, Vira Shield® 5+Somnus, Vira Shield® 5+VL5, Vira Shield® 5+VL5 Somnus, Vira Shield® 6, Vira Shield® 6+Somnus, Wart Shield™ (all of **Novartis Animal Health, Basel, Switzerland);** Bovi-K® 4, Bovi-Shield™ 3, Bovi-Shield™ 4, Bovi-Shield™ BRSV, Bovi-Shield® FP™ 4+L5, Bovi-Shield® GOLD 3, Bovi-Shield® GOLD 5, Bovi-Shield® GOLD FP™ 5 L5, Bovi-Shield® GOLD FP™ 5 VL5, Bovi-Shield® Gold IBR-BVD, Bovi-Shield® Gold IBR-BVD-BRSV-LP, Bovi-Shield™ IBR, Bovi-Shield™ IBR-BRSV-LP, Bovi-Shield™ IBR-BVD, Bovi-Shield™ IBR-BVD-BRSV-LP, Bovi-Shield™ IBR-PI3-BRSV, Calf-Guard®, CattleMaster® 4, CattleMaster® 4+L5, CattleMaster® 4+VL5, CattleMaster® BVD-K, CattleMaster® Gold FP™ 5, CattleMaster® Gold FP™ 5 L5, Defensor® 3, Fortress® 7, Fortress® 8, Fortress® CD, Leptoferm®-5, One Shot®, One Shot Ultra™ 7, One Shot Ultra™ 8, PregGuard™ FP 9, PregGuard® Gold FP™ 10, Resvac® BRSV/Somubac®, Resvac® 4/Somubac®, ScourGuard 3® (K), ScourGuard 3® (K)/C, Somubac®, Spirovac®, Spirovac® L5, Spirovac® VL5, StayBred™ VL5, TSV-2™, Ultrabac® 7, Ultrabac® 7/Somubac®, Ultrabac® 8, Ultrabac® CD, UltraChoice™ 7, UltraChoice™ 8, UltraChoice™ CD, Upjohn J-5 Bacterin™, Vibrin® (all of **Pfizer Inc., New York, NY);** Covexin® 8 Vaccine, Electroid® 7 Vaccine, Electroid® D, Guardian™, Jencine® 2, Jencine® 3, Jencine® 4, Nasalgen® IP Vaccine, Piliguard® Pinkeye-1 Trivalent, Piliguard® Pinkeye + 7, Piliguard® Pinkeye Triview®, Siteguard® G, Siteguard® MLG Vaccine (all of **Schering-Plough Animal Health Corporation, Kenilworth, NJ);** Myco-Bac™ B, Poly-Bac B® 3, Poly-Bac B® Somnus, Super Poly-Bac B® Somnus (all of **Texas Vet Lab, Inc., San Angelo, TX),** Virabos™-3 with Immunostim®, Virabos™-4 + H. somnus with Immunostim®, Virabos™-4 with Immunostim® (all of **Bioniche Animal Health, Athens, GA),** wherein attenuated BVDV, as described herein, is added. Alternatively, when BVDV antigen is present in any of those vaccines, attenuated BVDV, as described herein, is added, or BVDV present any of those vaccines is substituted by attenuated BVDV, as described herein.

### Formulations:

An important apect of the present dislcosure is the preparation of the combination vaccine(s). The skilled person knows additional components which may be comprised in said composition (see also Remington's Pharmaceutical Sciences. (1990). 18th ed. Mack Publ., Easton). The expert may use known injectable, physiologically acceptable sterile solutions. For preparing a ready-to-use solution for parenteral injection or infusion, aqueous isotonic solutions, such as e.g. saline or corresponding plasma protein solutions are readily available. The pharmaceutical compositions may be present as lyophylisates or dry preparations, which can be reconstituted with a known injectable solution directly before use under sterile conditions, e.g. as a kit of parts.

In addition, the immunogenic and vaccine compositions of the present invention can include one or more veterinary-acceptable carriers. As used herein,"a veterinary-acceptable carrier"includes any and all solvents, dispersion media, coatings, adjuvants, stabilizing agents, diluents, preservatives, antibacterial and antifungal agents, isotonic agents, adsorption delaying agents, and the like.

Diluents can include water, saline, dextrose, ethanol, glycerol, and the like. Isotonic agents can include sodium chloride, dextrose, mannitol, sorbitol, and lactose, among others. Stabilizers include albumin and alkalisalts of ethylendiamintetracetic acid, among others.

Adjuvants include, but are not limited to, the RIBI adjuvant system (Ribi Inc.), alum, aluminum hydroxide gel, Cholesterol, oil-in water emulsions, water-in-oil emulsions such as, e. g., Freund's complete and incomplete adjuvants, Block co-polymer (CytRx, Atlanta GA), SAF-M (Chiron, Emeryville CA), CARBOPOL®, AMPHIGENO adjuvant, saponin,Quil A, QS-21 (Cambridge Biotech Inc., Cambridge MA), GPI-0100 (Galenica Pharmaceuticals, Inc., Birmingham, AL) or other saponin fractions, monophosphoryl lipid A, Avridine lipid-amine adjuvant, heat-labile enterotoxin from E. coli (recombinant or otherwise), cholera toxin, or muramyl dipeptide, among many others.

The immunogenic compositions can further include one or more other immunomodulatory agents such as, e. g.,interleukins, interferons, or other cytokines. The immunogenic compositions can also include Gentamicin and Merthiolate. While the amounts and concentrations of adjuvants and additives useful in the context of the present invention can readily be determined by the skilled artisan, the present invention contemplates compositions comprising from about 50 ug to about 2000 ug of adjuvant and preferably about 250 ug/ ml dose of the vaccine composition. In another preferred embodiment, the present invention contemplates vaccine compositions comprising from about 1ug/ml to about 60 ug/ml of antibiotics, and more preferably less than about 30 ug/ml of antibiotics.

For example, in a composition according to the invention, 10⁴ to 10⁶ TCID₅₀ of attenutated BVDV may be solved in 25% (v/v) SGS (Sucrose 75 mg, Gelatine 20 mg, Potassium hydroxide 0.274 mg, L- glutamic acid 0.72 mg, Potassium dihydrogen phosphate 0.516 mg, Dipotassium phosphate 1.254 mg, and 2 ml with water for injection), and 5 % (v/v) cell culture medium, and 1 ml with water for injection. This is further mixed with at least one further antigen of a bovine pathogen, as listed above.

According to a further aspect the combination vaccine is first dehydrated. If the composition is first lyophilized or dehydrated by other methods, then, prior to vaccination, said composition is rehydrated in aqueous (e.g. saline, PBS (phosphate buffered saline)) or non-aqueous solutions (e.g. oil emulsion (mineral oil, or vegetable/metabolizable oil based/single or double emulsion based), aluminum-based, carbomer based adjuvant).

### Dose and administration:

According to the present disclosure, an effective amount of a combination vaccine administered to cattle, including pregnant cows and calves nursing pregnant cows, provides effective immunity against microbiological infections caused by BVDV and at least one further pathogen as listed above. Prefered combinations of antigens for the treatment and prophylaxis of microbiological diseases in cattle are listed above.

Acording to one aspect, the combination vaccine is administered to calves in two doses at an interval of about 3 to 4 weeks. For example, the first administration is performed when the animal is about 1 to about 3 months of age. The second administration is performed about 1 to about 4 weeks after the first administration of the combination vaccine. According to a further aspect, revaccination is performed in an interval of 6 to 12 month after administration of the second dose. In a preferred aspect, the first administration is performed about 5 weeks prior to animal breeding. The second administration is performed about 2 weeks prior to animal breeding. Administration of subsequent vaccine doses is preferably done on a 6 month to an annual basis. In another preferred embodiment, animals vaccinated before the age of about 6 months should be revaccinated after 6 months of age. Administration of subsequent vaccine doses is preferably done on an annual basis.

The amount of combination vaccine that is effective depends on the ingredients of the vaccine and the schedule of administration. Typically, when an inactivated virus or a modified live virus preparation is used in the combination vaccine, an amount of the vaccine containing about 10² to about 10⁹ TCID₅₀ per dose, preferably about 10³ to about 10⁸ TCID₅₀ per dose, more preferably, about 10⁴ to about 10⁸ TCID₅₀ per dose. For example, about 10⁵ to about 10⁸ TCID₅₀ per dose of attenuated BVDV (types 1 and 2) is effective when administered twice to the animal during a period of about 3 to 4 weeks. In general, inactivated antigen is normally used in higher amounts than live modivied viruses. Typically, when bacterial antigen is used in the combination vaccine, the vaccine contains an amount of about 10³ to about 10⁹ colony forming units (CFU) per dose, preferably, about 10⁴ to about 10⁸ (CFU) per dose, more preferably about 10⁵ to about 10⁶ (CFU) per dose.

In the event, the combination vaccine comprises live modified IBR, the amount of IBR antigen is preferably in a range of about 10⁵ to 10^{7.5} TCID₅₀ per dose. In the event, the combination vaccine comprises live modified PI3, the amount of PI3 antigen is preferably in a range of about 10⁷ to 10⁹ TCID₅₀ per dose. In the event, the combination vaccine comprises live modified BRSV, the amount of BRSV antigen is preferably in a range of about 10^{4.5} to 10^{6.5} TCID₅₀ per dose. In the event, the combination vaccine comprises killed IBR, the amount of IBR antigen is preferably in a range of about 10^{7.0} to 10^{9.0} TCID₅₀ per dose. In the event, the combination vaccine comprises killed PI3, the amount of PI3 antigen is preferably in a range of about 10^{7.2} to 10^{9.2} TCID₅₀ per dose. In the event, the combination vaccine comprises killed BRSV, the amount of BRSV antigen is preferably in a range of about 10^{5.0} to 10^{7.5} TCID₅₀ per dose. In the event, the combination vaccine comprises killed Leptospira spp. the amount of each Leptospira spp. antigen is preferably in a range of about 10^{7.0} to 10¹⁰ (CFU) per dose. In the event, the combination vaccine comprises killed H. somnus the amount of H. sommnus antigen is preferably in a range of about 10^{6.0} to 10⁹ (CFU) per dose.

The composition according to the invention may be applied intradermally, intratracheally, intravaginally, intramuscularly or intranasally, and preferably intramuscularly or intranasally. In an animal body, it can prove advantageous to apply the pharmaceutical compositions as described above via an intravenous or by direct injection into target tissues. For systemic application, the intravenous, intravascular, intramuscular, intranasal, intraarterial, intraperitoneal, oral, or intrathecal routes are preferred. A more local application can be effected subcutaneously, intradermally, intracutaneously, intracardially, intralobally, intramedullarly, intrapulmonarily or directly in or near the tissue to be treated (connective-, bone-, muscle-, nerve-, epithelial tissue). Depending on the desired duration and effectiveness of the treatment, the compositions according to the invention may be administered once or several times, as well as intermittently, for instance on a daily basis for several days, weeks or months and in different dosages.

### Methods for treatment:

Yet another important aspect of the dislcosure is a method for the prophylaxis or treatment of diseases caused by BVDV, and further bovine pathogenic microorganism(s), wherein an attenuated BVDV as described herein and further immunological active components effective for the treatment and/or prophylaxis of the infection caused by said further bovine pathogenic microorganism is administered to an animal in need thereof at a suitable dose, as known to the skilled person.

### FURTHER ASPECTS OF THE INVENTION:

Disclosed is also a combination vaccine for the treatment and/or prophylaxis of cattle against microbiological infections, wherein said combination vaccine comprises one or more attenuated BVDV, having at least one mutation in the coding sequence for glycoprotein E^{rns} and/or at least another mutation in the coding sequence for N^{pro}, wherein said mutation in the coding sequence for glycoprotein E^{rns} leads to inactivation of RNase activity residing in E^{rns} and/or said mutation in the coding sequence for N^{pro} leads to inactivation of said N^{pro}; and one or more immunological active component(s) effective for the treatment and/or prophylaxis of microbiological infection in cattle caused by a bovine pathogen other than BVDV **[combo 257].**

Moreover, disclosed is a vaccine as mentioned hereinabove as combo [257], wherein said combination comprises one or more attenuated BVDV, having at least one mutation in the coding sequence for glycoprotein Erns, wherein said mutation in the coding sequence for glycoprotein Erns leads to inactivation of RNase activity residing in E^{rns} and one or more immunological active component(s) effective for the treatment and/or prophylaxis of microbiological infection in cattle caused by a bovine pathogen other than BVDV.

Furthermore disclosed is a vaccine as mentioned hereinabove as combo [257], wherein said combination comprises one or more attenuated BVDV, having at least one mutation in the coding sequence for N^{pro}, said mutation in the coding sequence for N^{pro} leads to inactivation of said N^{pro}; and one or more immunological active component(s) effective for the treatment and/or prophylaxis of microbiological infection in cattle caused by a bovine pathogen other than BVDV.

Furthermore disclosed is a vaccine as mentioned hereinabove as combo [257], wherein said combination comprises one or more attenuated BVDV, having at least one mutation in the coding sequence for glycoprotein E^{rns} and at least another mutation in the coding sequence for Npro, wherein said mutation in the coding sequence for glycoprotein E^{rns} leads to inactivation of RNase activity residing in E^{rns} and/or said mutation in the coding sequence for N^{pro} leads to inactivation of said N^{pro}; and one or more immunological active component(s) effective for the treatment and/or prophylaxis of microbiological infection in cattle caused by a bovine pathogen other than BVDV.

Furthermore disclosed is a vaccine as mentioned hereinabove, wherein said combination vaccine comprises attenuated BVDV type 1 and attenuated BVDV type 2, both having at least one mutation in the coding sequence for glycoprotein E^{rns} and at least another mutation in the coding sequence for N^{pro}, wherein said mutation in the coding sequence for glycoprotein E^{rns} leads to inactivation of RNase activity residing in Erns and/or said mutation in the coding sequence for N^{pro} leads to inactivation of said N^{pro}.

Furthermore disclosed is a vaccine as mentioned hereinabove, wherein said infection in cattle other than BVDV is caused by at least one pathogen selected from the group consisting of: Parainfluenza-3 Virus (PI-3), Infectious Bovine Rhinotracheitis virus (IBR), Bovine Respiratory Syncytial Virus (BRSV), Bovine Herpesvirus (BHV), Bovine Rotavirus (BRV), Bovine Enterovirus (BEV), Bovine Coronovirus (BCV), Bovine Rabies (BR), Bovie Parvovirus (PPV), Adenovirus Astrovirus, Mannheimia haemolytica (formerly Pasteurella haemolytica), Pasteurella multocida, Haemophilus somnus (Histophilus ovis and Haemophilus agni), Actinomyces (Corynebacterium), Actinomyces pyogenes, Chlamydia psittaci, Campylobacter fetus venerealis and Campylobacter fetus fetus (formerly C fetus intestinalis), Leptospira interrogans, Leptospira hardjo, Leptospira pomona, and Leptospira grippotyphosa, Leptospira canicola, Leptospira grippotyphosa, Leptospira hardjo (Leptospira hardjoprajitno and Leptospira hardjo-bovis), Brucella abortus, Brucella suis and Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium; Salmonella newport, Mycobacterium avium paratuberculosis, Cryptsporidium parvum, Cryptsporidium hominis, Staphylococcus aureus, Streptococcus dysgalactiae, Streptococcus uberus, Mycoplasma spp, Mycoplasma dispar, Mycoplasma bovis, and Ureaplasma spp., Tritrichomonas foetus, Trichophyton verrucosum, Trichophyton mentagrophytes, Trichophyton sarkisovii, Neospora caninum (formerly Toxoplasma gondii), Babesia bigemina and Babesia bovis, and Dictyocaulus viviparous (Lungworm disease).

Furthermore disclosed is a vaccine as mentioned hereinabove, wherein said immunological active component is an antigen of one at least one pathogen selected from the group consisting of: Parainfluenza-3 Virus (PI-3), Infectious Bovine Rhinotracheitis virus (IBR), Bovine Respiratory Syncytial Virus (BRSV), Bovine Herpesvirus (BHV), Bovine Rotavirus (BRV), Bovine Enterovirus (BEV), Bovine Coronovirus (BCV), Bovine Rabies (BR), Bovie Parvovirus (PPV), Adenovirus Astrovirus, Mannheimia haemolytica (formerly Pasteurella haemolytica), Pasteurella multocida, Haemophilus somnus (Histophilus ovis and Haemophilus agni), Actinomyces (Corynebacterium), Actinomyces pyogenes, Chlamydia psittaci, Campylobacter fetus venerealis and Campylobacter fetus fetus (formerly C fetus intestinalis), Leptospira interrogans, Leptospira hardjo, Leptospira pomona, and Leptospira grippotyphosa, Leptospira canicola, Leptospira grippotyphosa, Leptospira hardjo (Leptospira hardjoprajitno and Leptospira hardjo-bovis), Brucella abortus, Brucella suis and Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium; Salmonella newport, Mycobacterium avium paratuberculosis, Cryptsporidium parvum, Cryptsporidium hominis, Staphylococcus aureus, Streptococcus dysgalactiae, Streptococcus uberus, Mycoplasma spp, Mycoplasma dispar, Mycoplasma bovis, and Ureaplasma spp., Tritrichomonas foetus, Trichophyton verrucosum, Trichophyton mentagrophytes, Trichophyton sarkisovii, Neospora caninum (formerly Toxoplasma gondii), Babesia bigemina and Babesia bovis, and Dictyocaulus viviparous (Lungworm disease).

### EXAMPLES

The following examples serve to further illustrate the present invention; but the same should not be construed as limiting the scope of the invention disclosed herein.

### EXAMPLE 1

### BVDVXIKE-B: foetopathogenicity assessment in pregnant heifers

BVDV XIKE-B, an RNase negative mutant of the highly pathogenic BVDV type 2 isolate NewYork'93/C was recovered from the infectious cDNA clone pKANE40B and showed wild type-like (wt-like) growth characteristics in tissue culture. In animal experiments, the mutant virus was found to be considerably attenuated so that it represented a promising candidate for development of a live attenuated vaccine virus (Meyer et al., 2002). To test whether this attenuated virus is still able to cross the placenta and infect the fetus, pregnant heifers were infected with XIKE-B. As a control, wild type BVDV recovered from cDNA clone pKANE40A was used. The respective virus named XIKE-A expresses an active E^{rns} RNase in the infected cell. The study aimed to assess the safety of XIKE-A and XIKE-B in pregnant animals.

### EXPERIMENTAL DESIGN

Ten pregnant heifers were selected from a BVDV negative herd. The following groups of 5 heifers were included in the trial:

| | No. | Inoculation | Virus |
|---|---|---|---|
| Group 1: | 5 | One i. n. administration, 3 ml in each nostril | XIKE-A |
| | | | |
| Group 2: | 5 | One i. n. administration, 3 ml in each nostril | XIKE- B |

Heifers were moved to the experimental facilities 8 days before inoculations. Pregnancy status was confirmed after transport into the experimental facility. Heifers were between days 60 and 90 of gestation on the day of inoculation. Inoculation took place for all animals at one point of time.

Heifers were monitored for the presence of clinical signs of BVDV infection including abortions during the observation period. Blood samples were collected from the animals for serology, antigen detection and white blood cells were counted. The experiment was terminated 9 weeks after infection. Non-aborted cows were slaughtered, the uterus examined and collected. Fetal organ samples were collected during routine necropsy and examined for BVDV infection.

The presence of fetal infection was the main evaluation parameter, composed from the number of BVDV-related cow mortalities, the number of BVDV-related abortions and the number of BVDV positive fetuses at termination. In addition to the main parameter, clinical signs characteristic for BVDV infection, viraemia and white blood cell counts in cows, and rectal temperature after challenge were evaluated.

### Animals

Heifers were purchased from a farm free of BVDV. Only animals, which met the following inclusion criteria, were used.

### Inclusion criteria

- Free of BVD antibodies; each individual was tested in the serum antibody test prior to transport and at the initiation of the study (at the animal test facility).
- Free of BVDV; plasma and/or buffy-coat preparation from each individual was tested by a suitable test.
- Clinically healthy at the initiation of the study judged upon physical examination. The health examination of the animals was accomplished in accordance with the current, generally accepted veterinary practice.
- Pregnancy confirmed by physical examination before inoculation. Pregnancy was between 60 - 90 days at the time of inoculation, proven by insemination records.

**Test Strain A**

| Description: | XIKE A, live virus BVDV strain |
|---|---|
| Composition: | Experimental material comprising of cell culture supernatant of low passaged XIKE-A |
| BVD components: | BVDV type II strain: XIKE-A |
| Supplied by: | Dr. Gregor Meyers, "Bundesforschungsanstalt für Viruskrankheiten der Tiere" (BFAV), Paul-Ehrlich-StraBe 28, 72076 Tübingen, Germany |
| Applied BVD virus dose: | Type 1 strain: 10⁵ TCID₅₀/6ml (TCID = **T**issue **C**ulture **I**nfective **D**ose) |
| Applied vaccine volume: | 3 ml per nostril |
| Application route: | Intranasal |
| Preparation of dosage form: | The inoculum was sent in a pre-diluted frozen form in a 50 ml vial on dry ice and was stored at -70 °C before inoculation. Immediately before inoculation of Group 1 heifers, the material was thawed avoiding local temperatures above 37 °C. After no ice was visible in the fluid, material was gently stirred and immediately used for inoculation of the animals. |
| Unused inoculum: | The volume of the unused material was measured and split on two aliquots before immediate freezing in dry ice or liquid nitrogen and stored for re-titration purposes. Virus and contaminated plastic or glassware were incubated with an appropriate volume of an 8 - 10 % formaldehyde solution for at least 24 hours at room temperature before discarding in order to inactivate viruses. |

**Test Strain B**

| Description: | XIKE B, live virus BVDV strain |
|---|---|
| Composition: | Experimental material comprising of cell culture supernatant of low passaged XIKE-B |
| BVD components: | BVDV type II strain: XIKE-B |
| Supplied by: | Dr. Gregor Meyers, "Bundesforschungsanstalt für Viruskrankheiten der Tiere" (BFAV), Paul-Ehrlich-StraBe 28, 72076 Tübingen, Germany |
| Applied BVD virus dose: | Type 1 strain: 10⁵ TCID₅₀/6ml (TCID = **T**issue **C**ulture **I**nfective **D**ose) |
| Applied vaccine volume: | 3 ml per nostril |
| Application route: | Intranasal |
| Preparation of dosage form: | The inoculum was sent in a pre-diluted frozen form in a 50 ml vial on dry ice and was stored at -70 °C before inoculation. Immediately before inoculation of Group 2 heifers, the material was thawed avoiding local temperatures above 37 °C. After no ice was visible in the fluid, material was gently stirred and immediately used for inoculation of the animals. |
| Unused vaccine: | The volume of the unused material was measured and split on two aliquots before immediate freezing in dry ice or liquid nitrogen and stored for re-titration purposes. Virus and contaminated plastic or glassware was incubated with an appropriate volume of an 8 - 10 % formaldehyde solution for at least 24 hours at room temperature before discarding in order to inactivate viruses. |

### Pregnancy Control

Pregnancy was confirmed immediately before inoculation.

### Inoculation of Heifers

The date of inoculation is Day 0 of the experiment.

In each nostril, 3 ml of the test material was administered intranasally by syringe without needle. Each time a new sterile syringe was taken. Administration was performed during the aspiration phase in order to minimize loss of fluid via expiration of material.

### Post-Inoculation Observations

### Collection and examination of blood samples

Blood was collected following standard, aseptic procedures (disinfecting the bleeding site). A new sterile syringe and needle was used for each animal.

### Blood collection to prepare serum

At least 10 ml blood was collected from the heifers immediately before inoculation, then weekly after infection and at the termination of the study. Serum was stored at -20 °C until required.

### Blood collection for leukocyte counts and buffy coat preparations

For leukocyte counting, 3 ml blood was transferred immediately after collection to suitable sterile vessels (Venoject, Terumo Europe N.V., Leuven, Belgium), pre-filled with 0.06 ml EDTA (0.235 MOL/L).

For buffy coat preparations, at least 15 ml blood was transferred immediately after collection to suitable sterile vessels, pre-filled with 0.1 ml Heparin solution (Na-heparin for inj., 5 000 IU/ml lot. A7B163A exp. date: 11/2000: Gedeon Richter RT, Budapest, Hungary) yielding at least 20 IU Heparin per ml blood in the blood sample. The content was carefully mixed thereafter.

For preparation of buffy coats and leukocyte counting, blood was collected from the heifers
- on every day, between Day 0 and Day 14 after infection;
- on every second day, between Day 15 and Day 40, or until all animals were negative for virus isolation for three consecutive sampling time points.

### Preparation of serum

Blood was allowed to clot at room temperature, and separated by centrifugation. Each serum sample was divided into two aliquots of at least 2 ml each. One set of aliquots was assayed for BVDV specific antibodies by ELISA. The rest of the sera was frozen and stored at -20 °C until required.

### Leukocyte counts

Leukocyte counts were determined with a coulter-counter semi-automated electronic device (Diatron Minicell-16, Messtechnik GmbH, Wien, Austria) with a claimed accuracy of 0.1 x 10⁹ / l, 100 / µl. The instrument was used (calibration and leukocyte-counts) according to the manufacturer's recommendations.

### Preparation of buffy coats

Heparin blood samples were transported to the laboratory as soon as possible. Buffy coat preparation procedure, following a standard laboratory procedure was performed under aseptic conditions (sterile pipettes, handling, clean bench etc.).

The obtained buffy coats were re-suspended in a small volume (2 ml) of RPMI 1640 and frozen at-70 °C in two aliquots of 0.5 ml. The residual 1ml buffy coats were immediately used for determination of blood cell associated BVDV by co-cultivation in a permissive cell culture.

### BVD serum antibody ELISA-test

Each serum sample was tested for the presence of BVDV-antibodies using a suitable and validated ELISA test (Svanovir™ BVDV antibody test Cat# 10-2200-10). Each test was validated and performed according to the manufacturer's recommendations. Positive samples were diluted according to the log₂ scale to determine BVDV antibody titers.

### BVD antigen assay(s)

Each buffy coat sample was assayed for the presence of BVDV by co-cultivation of the freshly prepared buffy-coats with susceptible cells or a cell-line. No freezing was allowed before co-cultivation.

Plasma was collected and provided to Man-Gene from each sample.

### Clinical observations

### Observation of heifers

Animals were examined daily from Day 0 - 42 post inoculation for the presence of clinical symptoms by a sufficiently trained veterinarian.

All clinical signs were recorded and described by their nature, consistence/touch, severity (mild, medium or severe) location, size of the area affected, and they were scored according to agreed and standard definitions. Special attention was paid to respiratory signs (respiration, its rate; nasal or ocular discharge; conjunctivitis, sneezing, coughing, etc.) and diarrhea.

### Rectal temperatures

Rectal temperatures were measured daily in each heifer, at the same hour of the day (preferably in the morning) from 5 days prior to the inoculation until 21 days post infection.

Daily measurement of rectal temperature was continued until each animal had rectal temperatures below or equal to 39 °C for at least 3 consecutive days.

### Detection of interrupted pregnancy

Pregnancy was confirmed and suspicion for abortion or resorption of the fetus was established by rectal examination. A trained veterinarian examined all animals at inoculation, 1, and 2 months post-inoculation. The examination was carried out according to the generally accepted veterinary practice.

Heifers were examined daily for any sign of abortion until termination of the study (8 - 12 weeks post-challenge).

### Termination of the Study

The study was terminated by slaughtering the heifers and extracting the fetuses. Fetuses and fetal material were transferred into closed transport containers marked with the number of the cow and the date/time. Containers were transported to a selected necropsy room.

Necropsy of the heifers was not required. Necropsy was performed on fetuses, findings recorded and a panel of samples collected as described below.

### Post-Mortem Examination

A detailed necropsy of the experimental animals was done in each case of death. Post-mortem examinations were carried out by an experienced veterinary surgeon and the data were recorded on appropriate data sheets. Further laboratory tests were performed according to the clinical signs and lesions observed. If the diagnosis of the necropsy referred to a disease caused by microbial agent the diagnosis was verified by an appropriate test, specific for the agent. Each tissue sample was collected in at least 2 separate, labeled containers and snap-frozen in liquid nitrogen. Samples were stored at -70 °C until required.

### Aborted fetuses and study termination

At least the following tissue samples were collected from the fetuses:
- exudate from the peritoneal cavity or thorax, if present,
- mesenteric lymph nodes,
- spleen,
- thymus,
- cerebellum,
- kidney,
- bone marrow from the sternum,
- sample from the placenta, if available.

### Dead or sacrificed heifers

At least the following tissue samples were collected:
- blood for buffy coat, if available,
- blood for serum, if available,
- Peyer's patches,
- mesenteric lymph nodes,
- spleen,
- kidney,
- uterus, including a sample from the placenta, if available.

**Storage and Transport of Samples**

| Samples: | Storage: |
|---|---|
| Serum | -20 °C |
| Buffy coat | -70 °C |
| Virus | -70 °C |
| Tissue from heifers | -70 °C |
| Tissue from fetuses | -70 °C |

Samples were sent for laboratory analysis as required by the sponsor. The choice of samples and the timing of transport were agreed with the study monitor or the project manager. As a matter of general principle, samples coming from aborted material or from new-born calves were investigated as soon as possible.

### RESULTS

### Summary of BVD related clinical and laboratory data

**Group 1**

| Animal No. | Conclusion | BVD |
|---|---|---|
| 526 | BVD abortion (uterus with placenta post-mortem) | NT (no sample found) |
| 598 | BVD abortion (foetus post-mortem) | + (foetus)* |
| 615 | Clinical BVD abortion | - (foetus)* |
| 618 | BVD abortion (foetus post-mortem) | - (foetus)* |
| 626 | Died due to BVD | + (foetus) / + (heifer) |

| | | |
|---|---|---|
| NT: not tested | | |

**Group 2**

| Animal No. | Conclusion | BVD |
|---|---|---|
| 469 | Clinical BVD abortion | - (foetus)* |
| 565 | Expected BVD abortion; non-viable foetus | + (foetus) |
| 588 | Normal | - (foetus) |
| 608 | Normal | + (foetus) |
| 619 | BVD abortion (foetus post-mortem) | - (foetus)* |

| | | |
|---|---|---|
| * Foetuses were autolysed at the time of sampling | | |

### Conclusion:

The study aimed to assess the safety of XIKE-A and XIKE-B in pregnant animals. Ten pregnant heifers were selected from a BVDV negative herd. Two groups of 5 heifers were included in the trial: one was inoculated with XIKE-A the other with XIKE-B virus strain. Heifers were between days 60 and 90 of gestation on the day of inoculation. Heifers were monitored for the presence of clinical signs of BVDV infection including abortions during the observation period. Blood samples were collected from the animals for serology, antigen detection and white blood cells were counted. The experiment was terminated 9 weeks after infection. Non-aborted cows were slaughtered, the uterus examined and collected. Fetal organ samples were collected during routine necropsy and examined for BVDV infection.

The XIKE-B virus proved to be less pathogenic than XIKE-A, nevertheless BVD related abortion and infection of the foetus was observed in the XIKE-B group, too. Therfore it can be concluded that the inactivation of the E^{rns} RNase does not prevent fetal infection.

### EXAMPLE 2

### BVDV XIKE-A-NdN: foetopathogenicity assessment in pregnant heifers

The N^{pro} gene has been shown to be nonessential for growth of CSFV in tissue culture (Tratschin et al., 1998). Even though a proof for BVDV attenuation in consequence of N^{pro} deletion is still missing, a role of this protein in the interaction between virus and host seemed to be possible and was actually indicated by recent experiments for CSFV (Mayer et al., 2004; Rüggli et al., 2003). We therefore wanted to investigate, whether the deletion of the major part of the N^{pro} coding sequence leads to a virus that no longer infects the fetus in pregnant heifers. The N^{pro} gene, except for the 5' terminal 4 codons, was deleted from the full length cDNA clone pKANE40A according to standard procedures. The resulting mutant full length clone was used as template for in vitro transcription and the resulting cRNA was transfected into MDBK cells as described (Meyer et al., 2002). The recovered virus was amplified in tissue culture and then used in the animal experiment described below. BVDV XIKE-B served as a control since it was shown before that it is able to cross the placenta (EXAMPLE 1).

### OBJECTIVE(S)/PURPOSE OF THE STUDY

The study aims to assess the safety of a live attenuated BVDV with a genomic deletion of most of the N^{pro} coding region in pregnant animals.

### Material and Methods applied are described in Example 1

### STUDY DESIGN

Eight pregnant heifers were assigned at random to two groups. They were treated and observed according to the following schedule:

| | Group 1 | Group 2 |
|---|---|---|
| N | 5 | 3 |
| Treatments | XIKE-A-NdN | XIKE-B / control |
| Route | Intramuscular | |
| Vaccination time | between days 60 and 90 of pregnancy (day 0 of the study) | |
| Observations Post-vaccination (in life) | • Clinical signs | |
| | • Serum at days 0, 14, 28, 42 and at termination | |
| | • WBC at day 0 and then daily for 14 days | |
| | • Buffy coat at day 0 and then daily for 14 days | |
| Post-mortem (day 60) | • Gross-pathology | |
| | • Organ panel for virus isolation | |

| Type of study: | open controlled clinical study |
|---|---|
| Experimental unit: | Individual animal |
| Method of blinding: | Partial blinding. No detailed procedures for blinding and access to treatment schedule were applied. The observing veterinarian at the study location and the pathologist were not be aware of the treatment; they only received a protocol extract relevant to their tasks. Vaccination was performed by the investigator or his assignee. Samples for virus isolation were coded by the investigator until all results are available. |

### RESULTS

All heifers were healthy and pregnant at study start. All animals proved to be free of BVDV and BVDV antibodies before the initiation of the study.

### Preparation and Control of the Virus used for the Infection

Samples were collected throughout the dilution steps and assayed on the day of preparation, i.e. without freezing by co-cultivation on suitable tissue culture. The results of virus titration are shown in the following table.

| Sample ID | Virus strain | Dilution / description | Log₁₀ titre/ml |
|---|---|---|---|
| VT1a | XIKE-A/NdN (S) | 1:2 (at 4 °C) | 4.4 |
| VT1b | | #2a on ice without opening | 4.0 |
| VT1c | | Return of #2b | 2.8 |
| VT2a | XIKE-B | 1:2.2 (at 4 °C) | 2.3 |
| VT2b | | #3a on ice without opening | 2.8 |
| VT2c | | Return of #3b | Negative |

### Clinical Symptoms of BVDV Infection

The table below gives a summary about the animals that had clinical signs during the observation period.

### Clinical signs and the days post inoculation (DPI) when they were observed

| Group 1 (XIKE-A NdN) | | | Group 2 (XIKE-B) | |
|---|---|---|---|---|
| Clinical sign | Animal ID | | Animal ID | |
| | 1583 | | 1438 | 1585 |
| | | | | |
| Loss of appetite | 8 | | - | 10 |
| Lachrymation | - | | - | - |
| Conjunctivitis | - | | - | - |
| Nasal discharge | - | | - | - |
| Oral erosion | - | | - | - |
| Oral haemorrhage | - | | - | - |
| Diarrhoea | - | | - | - |
| Coughing | - | | 12 | 10-13 |
| Abnormal breathing | - | | - | - |
| Elevated respiratory rate | - | | - | - |
| Hoof erosion | - | | - | - |

Only mild and transient clinical signs were observed in some of the animals in each group.

In Group 1, one out of the 5 heifers had loss of appetite on day 8 PI. In Group 2, two out of the 3 animals had clinical signs. Both heifers experienced coughing around day 21 PI that was accompanied with loss of appetite in one of the animals.

### Rectal Temperatures

No abnormal temperature changes were detected before the inoculation of the animals. The few cases of elevated temperatures measured after the inoculation are summarised in the table below.

| Group | Animal ID | Temperature (°C) | PI day |
|---|---|---|---|
| 1 | 1583 | 39.9 | 8 |
| | 1621 | 39.0 | 5 |
| 2 | 1438 | 39.0 | 2 |
| | 1585 | 40.8 | 9 |

One animal in each group had slightly elevated temperature, and also one animal in each group had fever. Fever was detected on day 8 or 9 PI. Temperature values always returned to normal values on the following day.

### Leukocyte Counts

Some leukopenia was observed in all groups between PI days 3 - 8. The number of animals with at least 40 % reduction in white blood cell count is noted below:

| Group | Number of animals having leukopenia / total |
|---|---|
| 1 | 3/5 (60%) |
| 2 | 1/3 (33%) |

### Serology (BVDV antibodies)

In compliance with the study protocol, all heifers were free of BVDV antibodies before vaccination. In Group 1 (inoculated with XIKE-A NdN) and Group 2 (inoculated with XIKE-B), complete seroconversion was detected only at study termination (2 months after inoculation).

### BVD virus isolation from buffy coats

No viremia was detected

### BVD virus isolation from fetal tissue samples

| | Group 1 | Group 2 |
|---|---|---|
| N | 5 | 3 |
| Treatments | XIKE-A-NdN | XIKE-B / control |
| Route | Intramuscular | Intramuscular |
| Number of fetuses in which fetal transmission was detected: | 4 out of 5 foetuses infected | 2 out of 3 foetuses infected |
| Conclusion of the virus used for treatment has the potential to be transmitted over the placenta: | Fetal transmission for XIKE-A-NdN observed | Fetal transmission for XIKE - B observed |

### Conclusion

The N^{pro} deletion resulted in a considerable attenuation of the BVDV in comparison to the parental virus XIKE-A that was shown to be highly pathogenic (Meyer et al., 2002). However, the N^{pro} deletion alone is not preventing transmission of a NY93 based virus recombinant to the foetus after inoculation of pregnant cows.

### EXAMPLE 3

### BVDV XIKE-B-NdN: foetopathogenicity assessment in pregnant heifers

To be able to test the potential of a combination of RNase inactivation and N^{pro} deletion with regard to BVDV attenuation and fetal transmission, different BVDV-2 mutants with deletions within the N^{pro} coding region were established based on the infectious cDNA clone pKANE40B, the RNase negative mutant of pKANE40A with a deletion of codon 349. The recovered viruses were analyzed with regard to presence of the desired mutations, the absence of second site mutations in the regions flanking the introduced changes and their growth characteristics in tissue culture. XIKE-B-NdN (V-pK88C), a variant containing a deletion of the complete N^{pro} coding region except for codons 1 to 4 in addition to the RNase inactivating deletion of codon 349 was chosen for an animal experiment since it combined the desired mutations with acceptable growth characteristics. The aim of the study was to assess the safety of a live attenuated BVDV isolate in pregnant animals.

Five BVDV-negative, pregnant heifers were inoculated intranasally with an infective dose of 10⁵ TCID₅₀/animal XIKE-B-NdN. Clinical data were recorded daily. Blood samples were collected for white blood cell counting, buffy-coat preparation, and serology. After termination of the study fetal tissues were collected for virus isolation.

### MATERIAL AND METHODS:

As detailed for example 1:

### RESULTS

No clinical data were observed (data not shown). Leukocyte counts remained virtually unchanged except for a significant decrease by approximately 40% below the baseline value (day0) in heifer no- 1015 on a single day (day 6 p.i.) (data not shown).

### a) Analysis of buffy coat preparations:

Approximately 10⁶ leukocytes were cultured in duplicates with MDBK-cells in 24-well tissue culture plates for 5 days. Samples were freeze-thawed twice. One hundred microliter aliquots of thawed samples were inoculated onto freshly seeded 24-well tissue culture plates and tested for virus by indirect immuno-fluorescence staining (mAb Code 4, directed against a conserved epitope in nonstructural protein NS3). No BVDV could be isolated from the buffy coat preparations of animals # 921, 1013, 1015, 1055 and 1075, whereas positive controls clearly showed the correct conduction of the test.

### b) Post-mortem examination of fetal tissues

After termination of the study the following fetal tissues were collected for virus isolation: spleen, kidney, thymus, sternum, cerebellum, placenta, intestine and abdominal fluid. Briefly, tissue suspensions were made in a mortar using sterile sea sand and ice-cold PBS without Ca²⁺ and Mg²⁺. Mortars were rinsed with 1 ml ice-cold PBS without Ca²⁺ and Mg²⁺ and suspensions were centrifuged for 10 minutes at 2000 x g (4°C). The supernatant was first passed through a disposable 0.45 µm filter holder, followed by a second filter passage (0.2 µm pore size). Virus isolation was carried out in duplicate (400 µl fetal tissue suspension or 100 µl fetal abdominal fluid) on a monolayer of MDBK-cells in a 24 wells tissue culture plate (37°C, 7% CO2). Tissue samples were controlled daily for cytopathic effects or bacterial contamination, and after an incubation time of 5 days, plates were frozen and thawed twice. 100 µl of samples were passaged to freshly seeded MDBK-cells. Virus was detected by indirect immuno-fluorescence staining (mAb Code 4). No BVDV could be detected in the tissue samples or fetal abdominal fluid.

### c) Serological Findings

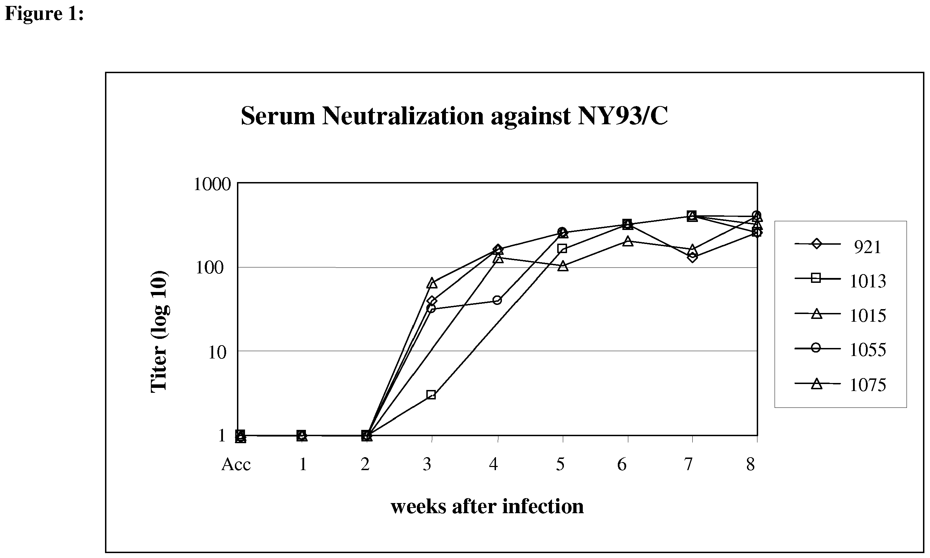

Serum neutralization titres were determined before inoculation, 1 month post-inoculation and at termination of the study. Sera from all animals were tested in triplicate for neutralizing antibodies against NY93/C, and the endpoint dilution was read by indirect immunofluorescence staining. Results were expressed as the endpoint dilution, which neutralized approximately 100 TCID₅₀ and calculated by the method of Kaerber. No definite data could be obtained for day 0, and 1 and 2 weeks post infection as the sera were toxic for MBDK-cells in dilutions up to 1:16 and no neutralization could be detected at higher dilutions. Starting with the third week post vaccination all animals developed neutralizing antibodies against the homologous BVDV-2 virus NY'93/C lasting till the end of the experiment (Fig. 1).

### d) Conclusions

The data obtained during the animal study clearly show that BVDV XIKE-B-NdN represents a highly attenuated virus. In contrast to wild type virus or the single mutants XIKE-B or XIKE-A-NdN that show fetal transmission in pregnant heifers at high rates the double mutant did not cross the placenta. BVDV XIKE-B-NdN as well as similar double mutants are extremely suitable for use in a live attenuated vaccine.

### Efficacy and crossprotection study

Two possible problems have to be faced with regard to vaccination with attenuated virus mutants BVDV XIKE-B or BVDV XIKE-B-NdN. First, there is a general problem concerning cross protection between BVDV-1 and BVDV-2. At least vaccination with inactivated BVDV-1 vaccines did not prevent the transmission of BVDV-2 to the foetus in pregnant animals. Since protection against fetal infection represents the major aim of anti BVDV vaccination, such vaccines cannot be regarded to induce a protective immunity on a broad range. The question therefore was, whether vaccination with live attenuated BVDV-2 can prevent virus transmission to the foetus. Second, the reduced growth rates of BVDV XIKE-B-NdN might result in only a low level of protection not able to prevent transplacental infection of the foetus in pregnant heifers. To address these problems, an animal study was started. The animals (2 groups of 10 animals each) were vaccinated either with BVDV XIKE-B or XIKE-B-NdN (intended dosage: 1ml of supernatant with 10⁵ TCID₅₀ of virus). None of the animals showed significant clinical signs after the vaccination except for one animal of the nonvaccinated control group with mild coughing for one day. Rectal temperature values were below 39 °C except for one animal of the nonvaccinated control group that was 39.1°C for one day. Buffy coat samples prepared after vaccination were analysed for the presence of virus as described above. The experiments showed that only 5 of the 20 animals contained virus in the blood for 1 or 2 days at 4 to 8 days post infection.

Four weeks after vaccination, insemination of the animals was carried out. Challenge infections were performed 60 to 90 days later using either a BVDV-1 strain (BVDV KE-9, heterologous challenge, animals vaccinated with XIKE-B) or a heterologous BVDV-2 strain (BVDV KE-13, homologous challenge, animals vaccinated with XIKE-B-NdN) (intended dosage: 10⁵ TCID50 in 6ml). From each group of vaccinated animals 5 pregnant heifers were randomly selected for the challenge infection. Animals vaccinated with BVDV XIKE-B were challenged with the BVDV-1 strain KE-9, whereas heifers vaccinated with BVDV XIKE-B/NdN were challenged with BVDV-2 KE-13. In addition, 2 nonvaccinated control animals were infected with each of the challenge viruses. The vaccinated animals did not show viremia or clinical symptoms upon challenge infection. The challenge was successful as all non-vaccinated controls were BVDV positive. Only mild signs of disease were observed in the control groups. The white blood cell counts were nearly normal (not shown).

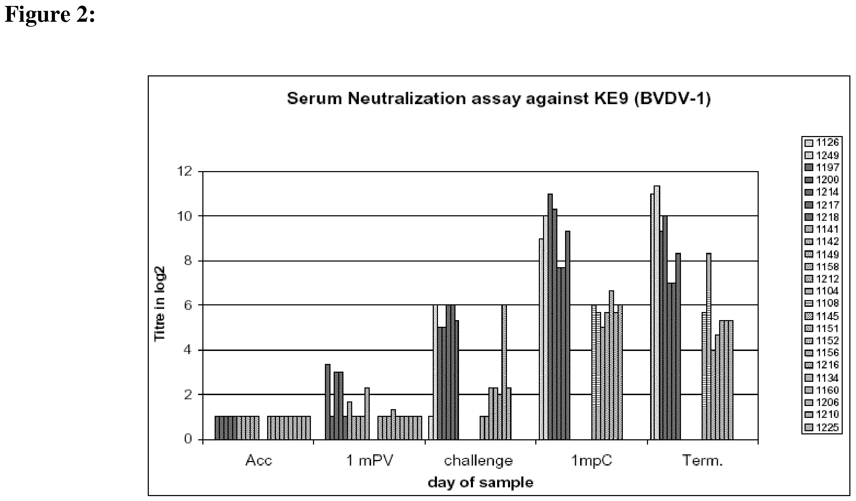

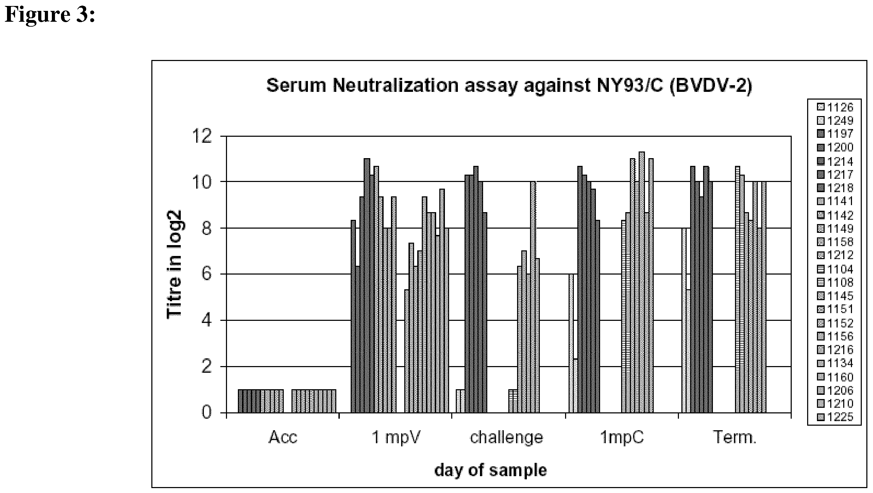

Serum neutralization titers were determined before inoculation, 1 month post-inoculation, before challenge, 1 month after challenge and at termination of the study. Sera from all animals were tested in triplicates for neutralizing antibodies against KE9 and NY93/C (1456Nase), and the endpoint dilution was read by indirect immunofluorescence staining. Results were expressed as the endpoint dilution, which neutralized approximately 100 TCID₅₀ and calculated by the method of Kaerber. At some of the higher antibody titres, the used endpoint dilution was not high enough. Against KE9, only animals vaccinated with XIKE-B developed low antibody titres starting about week 4. At challenge, all animals had antibody titres, which increased considerably starting around week 4 post challenge. XIKE-B vaccinated animals had higher antibody titres then those vaccinated with XIKE-B-NdN vaccinated. All animals developed about the same neutralization titre against NY93/C four weeks post vaccination, with marginally lower titres in XIKE-B-NdN vaccinated animals. After challenge, all animals had high antibody titres. Fig. 2 shows the serum neutralization assay against KE9 (BVDV-1) and Fig. 3 shows the serum neutralization assay against NY93/C (BVDV-2).

Analysis of tissue samples obtained after termination of the study from the foetuses revealed that the material obtained from the vaccinated animals gave negative results whereas transmission had occurred in all 4 control animals. Thus, it is clear that the established BVDV-2 mutants are well suited as efficient cross protective vaccine viruses .

### Conclusion

The challenge was successful as all non-vaccinated controls were BVDV viraemic and foetuses of all non-vaccinated controls were BVDV positive. Both isolates gave full protection under the present test and assay conditions. Isolate XIKE-B, with the single genetic marker was shown to cross-protect against type 1 BVDV challenge in terms of BVD viraemia and transmission to the foetus after challenge. Isolate XIKE-B-NdN with the double genetic marker was able to fully protect against a heterologue type 2 BVDV challenge strain in terms of BVD viraemia and transmission to the foetus after challenge:
1. Isolate XIKE-B (type 2 isolate) was shown to cross-protect against type 1 BVDV challenge in terms of BVD viraemia and transmission to the foetus after challenge under the present test and assay conditions (n=4).
2. Isolate XIKE-B-NdN (type 2 isolate) fully protected against a heterologues type 2 BVDV challenge strain in terms of BVD viraemia and transmission to the foetus after challenge under the present test and assay conditions (n=5).

### EXAMPLE 4

### Establishment of N^{pro} mutants

This Example further analyzes BVDV-2 mutants with N^{pro} deletions. Different mutants with deletions in the N^{pro}-coding region of the genome were established. Initially, only true deletions or a deletion accompanied by a point mutation were introduced.
A: [N^{pro}]₁-[C-term];
B: [N^{pro}]₃-[C-term];
C: [N^{pro}]₄-[C-term];
D: [N^{pro}]₆-[C-term];
E: [N^{pro}]₄-[C-term*]

In the formulas [N^{pro}]ₓ represents the number of residues of the aminoterminus of N^{pro} that are left in the mutated polyprotein amino acids, [C-term] is the complete polyprotein except for N^{pro} (starting with the C protein and ending with NS5B), and [C-term*] is the same as [C-term] but with a mutation at position 2 of the C protein (N instead of D). The growth rates of the recovered viruses were considerably lower than those of wild type XIKE-A or the RNase negative mutant XIKE-B. There are two possible explanations for this finding: (i) dependent on the virus strain, sequences of variable length of the N^{pro}-coding region are necessary for efficient translation initiation (Mayers et al., 2001; Tautz et al., 1999) and (ii) the fusion of additional sequences to the aminoterminus of the capsid protein interferes with capsid protein function. To obtain better growing N^{pro} deletion mutants, a second set of mutants was generated with either a bovine ubiquitin gene or a fragment of the bovine LC3-coding sequence replacing the major part of the N^{pro} gene. These constructs allow efficient translation and generate a capsid protein with the correct amino terminus.
[N^{pro}]₂₂-[PS]- [C-term]
wherein PS is ubiquitin or LC3, C-term is the complete polyprotein except for N^{pro} (starting with the C protein and ending with NS5B).

The growth rates of these mutants were more similar to what was determined for XIKE-A. It even seemed that the two RNase positive viruses according to the formula [N^{pro}]₂₂-[PS]- [C-term] named V-pK87F and V-pK87G showed no significant growth retardation at all, whereas the RNase negative counterpart V-pK88G once again was somewhat hampered in propagation but to a lesser extent than the formerly described mutants.

Further examples of N^{pro} deletion mutants may be:

| | |
|---|---|
| *ME*SDEGSK... | (SEQ ID NO 28) |
| *MELFS*SDEGSK... | (SEQ ID NO 29) |
| *MELFSNE*SDEGSK... | (SEQ ID NO 30) |
| *MELFSNEL*SDEGSK... | (SEQ ID NO 31) |
| *MELFSNELL*SDEGSK... | (SEQ ID NO 32) |
| *MELFSNELLY*SDEGSK... | (SEQ ID NO 33) |
| *MELFSNELLYK*SDEGSK... | (SEQ ID NO 34) |
| *MELFSNELLYKT*SDEGSK... | (SEQ ID NO 35) |

*MELFSNELLYKT* represents the aminoterminal sequence of N^{pro} of the BVDV isolate NewYork93/C.

It may also be possible to use variants of this sequence with one or several mutations. Especially the naturally occurring variations as found in other pestiviruses can be expected to be functional. Therefore, the complete list of the tested or proposed variants with the different parts of the aminoterminal end of N^{pro} can be enlarged by equivalent sets with amino acid exchanges. Below, typical examples of the respective sequences are given for several pestiviruses but the possible variations are not limited to these examples.

| | | |
|---|---|---|
| **BVDV NewYork93/C:** | *MELFSNELLYKT* | |
| **BVDV CP13:** | *MEL**I**SNELLYKT* | (SEQ ID NO 36) |
| **BVDV SD1:** | *MEL**IT**NELLYKT* | (SEQ ID NO 37) |
| **CSFV Brescia:** | *MEL**NHF**ELLYKT* | (SEQ ID NO 38) |
| **BDV X818:** | *MEL**NKF**ELLYKT* | (SEQ ID NO 39) |

Thus, these variants for example may include: *MELI*-[PS]₀-[C-term];
*MELIS*-[PS]₀*-*[C-term];
*MELISN*-[PS]₀-[C-term];
*MELISNE-*[PS]₀-[C-term];
*MELISNEL-*[PS]₀-[C-term];
*MELISNELL*-[PS]₀-[C-term];
*MELISNELLY-*[PS]₀-[C-term] ;
*MELISNELLYK*-[PS]₀-[C-term];
*MELISNELLYKT*-[PS]₀-[C-term];
*MELIT*-[PS]₀-[C-term];
*MELITN*-[PS]₀-[C-term];
*MELITNE-*[PS]₀-[C-term];
*MELITNEL*-[PS]₀-[C-term];
*MELITNELL-*[PS]₀-[C-term];
*MELITNELLY*-[PS]₀-[C-term];
*MELITNELLYK*-[PS]₀-[C-term];
*MELITNELLYKT-*[PS]₀-[C-term];
These formulas may also have [PS]₁, i.e. PS may also be one of the PS as described herein. Sequences belonging to the N^{pro} protein are in italics. Amino acid exchanges with regard to the sequence of BVDV NewYork93/C are in bold.

Further examples can be found e.g. by using the GenBank accession numbers given in Becher et al., 2003, Virology 311, 96-104) or by standard sequence data searches.

A further possibility could be the use of a processing signal (PS) inserted between the (residual) N^{pro} sequence and the aminoterminus of the capsid protein. The PS leads to a cleavage that generates a functional capsid protein. The configuration of such constructs could be as follows:

[N^{pro}]₂₂-**PS**[C-term]

PS: Processing signal. Can either be a target for a protease (e.g. ubiquitin, LC3 as defined herein or a protease or an unstable peptide leading to processing at its own carboxyterminus like e.g. intein (Chong et al. 1998 and references therein) or 3C of picornaviruses, 2A of cardioviruses or aphtoviruses, p15 of rabbit hemorrhagic disease virus or the corresponding protease of other caliciviruses (Proter 1993, and references therein; Meyers et al., 2000 and references therein). When using a PS, a large number of different variants are possible since the PS ensures the generation of the correct amino terminus of the capsid protein C. Thus, when using a PS construct, all kinds of deletions or mutations of the N^{pro} sequence are expected to result in viable mutants as long as the reading frame is not shifted or translation stopped by an in frame stop codon. As an example we established a viable CSFV N^{pro} deletion mutant according to the formula

[N^{pro}]₂₂-**PS**[C-term]

Especially interesting could be N^{pro} mutations blocking the proteolytic activity of the protein. Rümenapf et al. (1998) have published the identification of the active site residues of the protease for CSFV Alfort Tübingen. The respective amino acids (glutamic acid at position 22, histidine at position 49 and cysteine at postion 69) are conserved for other pestiviruses. Thus, exchanges of any amino acid expect for serine or threonine for the cysteine at position 69 will result in destruction of the protease activity. Similarly, changing the glutamic acid at position 22 will most likely result in inactivation of the protease unless the new amino acid is aspartic acid. Similarly, most if not all exchanges at position 49 will lead to an inactive protease.

### EXAMPLE 5

### Preparation of combination vaccines according to the invention

### Vaccine A

### IBR, BVDV types 1 and 2, BRSV

Attenuated BVDV type 1 and 2 strains, having at least one mutation in the coding sequence for glycoprotein E^{rns} and/or at least another mutation in the coding sequence for N^{pro}, wherein said mutation in the coding sequence for glycoprotein E^{rns} leads to inactivation of RNase activity residing in E^{rns} and/or said mutation in the coding sequence for N^{pro} leads to inactivation of said N^{pro}, are grown in MDBK-cells until a TCID₅₀ of about 10^{5.0} to 10^{8.1} per ml cell culture fluid. A live attenuated strain of IBR is grown in MDBK cells until a TCID₅₀ of about 10^{5.0} to 10^{8.6} per ml cell culture fluid. A live attenuated strain of BRSV is grown in MDBK cells until a TCID₅₀ of about 10^{5.0} to 10^{7.2} per ml cell culture fluid. Each virus containing culture fluid is collected and lyophilized. Equal amounts of the lyphilized antigens are mixed. For reconstitution, an aqueous solution containing 1 to 3% : 0.8 ml of Alhydrogel is used. One dose of the combinantion vaccine contains 4 ml of the reconstituted antigens. A final dose includes IBR (10^{5.0} to 10^{8.6} TCID₅₀), BVDV-1 (10^{5.0} to 10^{8.1} TCID₅₀), BVDV-2 (10^{5.0} to 10^{8.1} TCID₅₀), and BRSV (10^{5.0} to 10^{7.2} TCID₅₀).

### Vaccine B

### IBR, BVDV types 1 and 2, PI3 BRSV

The preparation of the IBR, BVDV 1 and 2 and BRSV antigens is performed as described for vaccine A. In addition, a live attenuated strain of PI3 is grown in MDBK cells until a TCID₅₀ of about 10^{4.2} to 10^{6.5} per ml cell culture fluid. Afterwards, the PI3 containing culture fluid is harvested and lyophilized. An amount of 10^{4.2} to 10^{6.5} (TCID₅₀) of the lyophlized antigen is mixed with the lyophilized IBR, BVDV types 1 and 2, and BRSV antigens. The mixture is then reconstituted in 4 ml as described for Vaccine A. A final dose includes IBR (10^{5.0} to 10^{8.6} TCID₅₀), BVDV-1 (10^{5.0} to 10^{8.1} TCID₅₀), BVDV-2 (10^{5.0} to 10^{8.1} TCID₅₀), BRSV (10^{5.0} to 10^{7.2} TCID₅₀), and PI3 (10^{4.2} to 10^{6.5} TCID₅₀).

### Vaccine C

### BVDV types 1 and 2, PI3, BRSV, Mannheimia (Pasteurella) haemolytica

BVDV 1 and 2, BRSV and PI3 viruses are grown as described for vaccines A and B. After the culture fluids are harvested, the viruses are inactivated and lyophilized. Mannheima (Pasteurella) haemalytica is grown until 10^{8.0} to 10^{11.0} cells per ml culture. The bacteria are inactivated and the culture fluid is lyophilized or freeze dried. An amount of 10^{8.0} to 10^{11.0} lyophlized or freeze dried bacteria cells are mixed with the lyophilized BVDV types 1 and 2 antigen (each in an amount of 10^{5.0} to 10^{8.1} TCID₅₀), PI3 antigen (10^{7.3} to 10^{8.3} TCID₅₀) and BRSV antigen (10^{5.0} to 10^{7.2} TCID₅₀). The reconstituted suspension (5 ml per dose) further contains 30 to 50 mg Aluminium hydroxide, 0.4 to 0.8 mg Quil A (Saponin), 0.04 to 0.06 mg sodium timerfonate and traces of neomycin. Final antigen amounts per dose are BVDV-1 (10^{5.0} to 10^{8.1} TCID₅₀), BVDV-2 (10^{5.0} to 10^{8.1} TCID₅₀), PI3 (10^{7.3} to 10^{8.3} TCID₅₀) BRSV (10^{5.0} to 10^{7.2} TCID₅₀) and Mannheima (Pasteurella) haemalytica (10^{8.0} to 10^{11.0} cells).

### Vaccine D

### BVDV types 1 and 2, IBR, BRSV, PI3, Leptospira canicola, Leptospira grippo, Leptospira hardjo, Leptospira ponoma, Leptospora borgpetersenii hardjo-bovis

Modified live viruses of BVDV 1 and 2, BRSV, IBR, and PI3 are grown as described for vaccines A and B. After the culture fluids are harvested, the viruses are lyophilized. Leptospira canicola, Leptospira grippo, Leptospira hardjo, Leptospira ponoma, Leptospora borgpetersenii hardjo-bovis are separately cultivated until reaching 10^{8.0} to 10^{11.0} cells per ml culture. The bacteria cultures are inactivated and the culture fluids are lyophilized or freeze dried. Each of the 10^{8.0} to 10^{11.0} of the lyophilised or freeze dried bacteria cells are reconstituted with the lyophilized modified BVDV types 1 and 2 (each in an amount of 10^{5.0} to 10^{7.0} TCID₅₀), modified live PI3 (10^{7.3} to 10^{8.3} TCID₅₀), modified live BRSV (10^{5.0} to 10^{7.0} TCID₅₀) and modified live IBR (10^{6.1} to 10^{7.7} TCID₅₀). The reconstituted suspension (2 ml per dose) contains traces of neomycin as preservative. Final antigen amounts per dose are BVDV-1 (10^{5.0} to 10^{7.0} TCID₅₀), BVDV-2 (10^{5.0} to 10^{7.0} TCID₅₀), PI3 (10^{7.3} to 10^{8.3} TCID₅₀) BRSV (10^{5.0} to 10^{7.0} TCID₅₀), PI3 (10^{7.3} to 10^{8.3} TCID₅₀), and Leptospira canicola, Leptospira grippo, Leptospira hardjo, Leptospira ponoma, and Leptospora borgpetersenii hardjo-bovis (each 10^{8.0} to 10^{11.0} cells).

### Vaccine E

### BVDV types 1 and 2, IBR, BRSV, PI3, and H.somnus

Modified live viruses of BVDV 1 and 2, BRSV, IBR, and PI3 are grown as described for vaccines A and B. After the culture fluids are harvested, the viruses are lyophilized. H.somnus is cultivated until achieving 10^{7.1} to 10^{9.2} cells per ml culture. The bacteria culture is inactivated and the culture fluid is lyophilized or freeze dried. 10^{7.1} to 10^{9.2} of the loyphilized or freeze dried bacteria are reconstituted with the lyophilized modified BVDV types 1 and 2 (each in an amount of 10^{5.0} to 10^{7.0} TCID₅₀), modified live PI3 (10^{7.3} to 10^{8.3} TCID₅₀), modified live BRSV (10^{5.0} to 10^{7.0} TCID₅₀) and modified live IBR (10^{6.1} to 10^{7.7} TCID₅₀). The reconstituted suspension (2 ml per dose) contains traces of neomycin as preservative. Final antigen amounts per dose are BVDV-1 (10^{5.0} to 10^{7.0} TCID₅₀), BVDV-2 (10^{5.0} to 10^{7.0} TCID₅₀), PI3 (10^{7.3} to 10^{8.3} TCID₅₀) BRSV (10^{5.0} to 10^{7.0} TCID₅₀), PI3 (10^{7.3} to 10^{8.3} TCID₅₀), and H.somnus (10^{7.1} to 10^{9.2} cells).

### REFERENCES:

Ausubel, F.M. et al., Current Protocols in molecular biology. New York : Greene Publishing Associates and Wiley-Interscience. 1994 (updated)
Baker, J.C. 1987. Bovine viral diarrhea virus: a review. J. Am. Vet. Med.Assoc. 190: 1449-1458.
Becher, P., Konig, M., Paton, D.J., Thiel, H.J., 1995, Further characterization of border disease virus isolates: evidence for the presence of more than three species within the genus pesivirus. Virology 209 (1), 200-206.
Chong, S., Williams, K.S., Wotkowicz, C., and Xu, M.Q.1998. Modulation of Protein Splicing of the Saccharomyces cerevisiae Vacuolar Membrane ATPase Intein. J. Biol. Chem. 273: 10567 - 10577.Donis, R.O., Corapi, W., and Dubovi, E.J. 1988. Neutralizing monoclonal antibodies to bovine viral diarrhea virus bind to the 56K to 58K glycoprotein. J. Gen. Virol. 69: 77-86.
Fuerst T.R. et al. 1986. Eukaryotic transient expression system based on recombinant vaccinia virus that synthesizes bacteriophage T7 RNA polymerase. Proc. Natl. Acad. Sci. 83: 8122-8126.
Heinz, F.X., Collett, M.S., Purcell., R.H., Cold, E.A., Howard, C.R., Houghton, M., Moormann, R.J.M., Rice, C.M., and Thiel, H.-J. 2000. Familiy Flaviviridae. PP 859-878. In: Virus Taxonomy (van Regenmortel., H.H.V., Fauquet, C.M., and Bishop, D.H.L, Eds.). Academic Press, San Diego.
Hulst, M.M., Himes, G., Newbigin, E., Moormann, R.J.M. 1994. Glycoprotein E2 of classical swine fever virus: expression in insect cells and identification as a ribonuclease. Virology 200: 558-565.
Hulst, M.M., F.E. Panoto, A. Hooekmann, H.G.P. van Gennip., and Moormann, R.J.M. 1998. Inactivation of the RNase activity of glycoprotein Erns of classical swine fever virus results in a cytopathogenic virus. J. Virol. 72: 151-157.
Kit, M. and S. Kit. 1991. Sensitive glycoprotein gIII blocking ELISA to distinguish between pseudorabies (Aujeszky's disease) -infected and vaccinated pigs. Veterinary Microbiology 28:141-155.
Kunkel, T. A., J. D. Roberts, and R. A. Zakour. 1987. Rapid and efficient site-specific mutagenesis without phenotypic selection. Methods Enzymol. 154:367-392.
König, Matthias, 1994, Virus der klassischen Schweinepest: Untersuchungen zur Pathogenese und zur Induktion einer protektiven Immunantwort. Dissertation, Tierärztliche Hochschule Hannover, Germany.
Lindenbach, B.D., and Rice, C. M. 2001. The pestiviruses. In Fields Virology, eds. Knipe, D.M., & Howley, P.M. (Lippincott-Raven, Philadelphia), pp. 991-1042.Mayer, D., Hofmann, M.A., and Tratschin, J.D. 2004. Attenuation of classical swine fever virus by deletion of the viral N(pro) gene. Vaccine. 22:317-328.
Meyers, G., Rümenapf, T. and Thiel, H.-J. 1989. Molecular cloning and nucleotide sequence of the genome of hog cholera virus.Virology 171: 555-567.
Meyers,G., Saalmüller,A., and Büttner,M. (1999). Mutations abrogating the RNase activity in glycoprotein e(rns) of the pestivirus classical swine fever virus lead to virus attenuation. J Virol 73: 10224-10235.
Meyers, G., Tautz, N., Becher, P., Thiel, H.-J., & Kümmerer, B.M. 1996b. Recovery of cytopathogenic and noncytopathogenic bovine viral diarrhea viruses from cDNA constructs. J. Virol., 70: 8606-8613.
Meyers, G., Thiel, H.-J., and Rümenapf, T. 1996a. Classical swine fever virus: Recovery of infectious viruses from cDNA constructs and generation of recombinant cytopathogenic swine fever virus. J. Virol. 67:7088-709526.
Meyers, G., Wirblich, C., Thiel. H.-J. and Thumfart, J.O. 2000. Rabbit hemorrhagic disease Virus: genome organization and polyprotein processing of a calicivirus studied after transient expression of cDNA constructs. Virology 276: 349-363.
Moennig, V. and Plagemann, J. 1992. The pestiviruses. Adv. Virus Res. 41: 53-91.
Paton, D.J., Lowings, J.P., Barrett, A.D. 1992. Epitope mapping of the gp53 envelope protein of bovine viral diarrhea virus. Virology 190: 763-772.
Pellerin, C. et. al. Identification of a new group of bovine viral diarrhea virus strains associated with severe outbreaks and high mortalities, Virology 203, 1994:260-268.
Porter, A.G. (1993). Picornavirus nonstructural proteins: emerging roles in virus replication and inhibition of host cell functions. J. Virol. 67, 6917-6921.
Rüggli, N., Tratschin, J.D., Schweizer, M., McCullough, K.C., Hofmann, M.A., Summerfield, A. 2003. Classical swine fever virus interferes with cellular antiviral defense: evidence for a novel function of N(pro). J. Virol. 77:7645-7654.
Rümenapf, T., Stark, R., Heimann, M., and Thiel, H.-J. 1998. N-terminal protease of pestiviruses: identification of putative catalytic residues by site directed mutagenesis. J. Virol. 72: 2544-2547.
Rümenapf, T., Unger, G., Strauss, J.H., and Thiel, H.-J. 1993. Processing of the evelope glycoproteins of pestiviruses. J. Virol. 67: 3288-3294.Schneider, R., G. Unger, R. Stark, E. Schneider-Scherzer, and H.-J. Thiel. 1993. Identification of a structural glycoprotein of an RNA virus as a ribonuclease. Science 261: 1169-1171.
Sambrook, J., Fritsch, E.F. & Maniatis, T.,Molecular Cloning: A Laboratory Manual Cold Spring Harbor Laboratory, Cold Spring Harbor, New York, 1989
Stark, R., Meyers, G., Rümenapf, T., and Thiel, H.-J. (1993): Processing of pestivirus polyprotein: Cleavage site between autoprotease and nucleocapsid protein of classical swine fever virus. J. Virol., 67, 7088-7095.Thiel, H.-J., Plagemann, G.W., & Moennig, V. 1996. The pestiviruses. In Fields Virology, eds. Fields, B.N., Knipe, D.M., & Howley, P.M. (Lippincott-Raven, Philadelphia), pp.1059-1073.
Thiel, H.-J., Stark, R., Weiland, E., Rümenapf, T. & Meyers, G. 1991. Hog cholera virus: molecular composition of virions from a pestivirus. J. Virol. 65: 4705-4712.31.
Tratschin, J.-D., Moser, C., Ruggli, N., and Hofmann, M.A. 1998. Classical swine fever virus leader proteinase Npro is not required for viral replication in cell culture. J. Virol. 72, 7681-7684.van Rijn, P.A., van Gennip, H.G., de Meijer, E.J., Moormann, R.J. 1993. Epitope mapping of envelope glycoprotein E1 of hog cholera virus strain Brescia. J. Gen. Virol. 74: 2053-2060.
Weiland, E., Thiel, H.-J., Hess, G., and Weiland, F. (1989). Development of monoclonal neutralizing antibodies agaist bovine viral diarrhea virus after pretreatment of mice with normal bovine cells and cyclophosphamide. J. Virol. Methods 24: 237-244.
Weiland, E., Stark, R., Haas, B., Rümenapf, T., Meyers, G. and Thiel, H.-J. (1990). Pestivirus glycoprotein which induces neutralizing antibodies forms part of a disulfide-linked heterodimer. J. Virology 64, 3563-3569.
Weiland, E., Ahl, R., Stark, R., Weiland, F. and Thiel, H.-J. (1992). A second envelope glycoprotein mediates neutralization of a pestivirus, hog cholera virus. J. Virology 66, 3677-3682.
Windisch, J.M., Schneider, R., Stark, R., Weiland, E., Meyers, G., and Thiel, H.-J. 1996. RNase of classical swine fever virus: biochemical characterization and inhibition by virus-neutralizing monoclonal antibodies. J. Virol. 70: 352-358
Wiskerchen, M., Belzer, S.K., and Collett, M. S. 1991. Pestivirus gene expression: the first protein product of the bovine viral diarrhea virus large open reading frame, p20, possesses proteolytic activity J. Virol. 65:4508-4514.

## Claims

1. A combination vaccine for the prophylaxis of cattle against microbiological infections, wherein said combination vaccine comprises
a. one or more attenuated BVDV, having at least one mutation in the coding sequence for glycoprotein E^{rns} and at least another mutation in the coding sequence for N^{pro}, wherein said mutation(s) in the coding sequence for glycoprotein E^{rns} lead(s) to inactivation of RNase activity residing in E^{rns} and said mutation(s) in the coding sequence for N^{pro} lead(s) to inactivation of said N^{pro}; and
b. one or more immunological active component(s) effective for the prophylaxis of microbiological infection in cattle caused by a bovine pathogen other than BVDV, wherein the immunological active component is an attenuated microorganism, a killed microorganism, or at least an immunological part of a microorganism.

2. The vaccine according to claim 1, wherein the attenuated microorganism is a modified live virus (MLV).

3. The vaccine according to claim 1 or 2, wherein said mutation(s) in the coding sequence for glycoprotein E^{rns} are located in the encoding nucleotide sequence corresponding to amino acids at position 298 to 310 and/or position 341 to 360.

4. The vaccine according to any one of claims 1 to 3, wherein said mutation(s) in the coding sequence for N^{pro} lead(s) to an encoded polyprotein as **characterized by** the following formula:
[N^{pro}]ₓ-[C-term]
wherein **[N^{pro}]** relates to the N^{pro} portion of said polyprotein;
wherein **"x"** represents the number of amino acids of the N^{pro} present in the polyprotein
wherein **"x"** is 0 to 12, which means that no N^{pro} specific amino acid or 1 to 12 amino acids of N^{pro}, preferably of the N-terminus of N^{pro} are present; and
wherein **[C-term]** relates to the complete BVDV polyprotein except for N^{pro}, but including the capsid (C)-protein and any other protein present in the BVDV polyprotein including the carboxyterminal NS5B.

5. The combination vaccine according to any one of claims 1 to 4, wherein said mutation in the coding sequence for glycoprotein E^{rns} is selected from the group consisting of S298G, H300K, H300L, H300R, H300del, W303G, P304del, E305A, C308G, R343G, E345del, W346G, K348A, H349K, H349L, H349del, H349Q, H349SV, K348R, W351P, W351G, W351L, W351K, W351H, H300L/H349L, K348del/H349del, H349del/G350del, E345del/H349del, W303G/E305A, H300K/H349K, H300K/H349L, L299del/H300del/G301del and K348del/H349del/G350del.

6. The combination vaccine according to any one of claims 1 to 5, wherein said mutation(s) in the coding sequence for N^{pro} leads to an N^{pro} having the amino acid sequence ME, MELFS, MELFSNE, MELFSNEL, MELFSNELL, MELFSNELLY, MELFSNELLYK, MELFSNELLYKT.

7. The combination vaccine according to any one of claims 1 to 5, wherein said mutation(s) in the coding sequence for N^{pro} leads to an N^{pro} having the amino acid sequence MESDEGSK, MELFSSDEGSK, MELFSNESDEGSK, MELFSNELSDEGSK, MELFSNELLSDEGSK, MELFSNELLYSDEGSK, MELFSNELLYKSDEGSK or MELFSNELLYKTSDEGSK.

8. The combination vaccine according to any one of claims 1 to 5, wherein said mutation(s) in the coding sequence for N^{pro} leads to an N^{pro} having the amino acid sequence MEL.

9. The combination vaccine according to any one of claims 1 to 5, wherein said mutation(s) in the coding sequence for N^{pro} leads to an N^{pro} having the amino acid sequence MELF.

10. The combination vaccine according to any one of claims 1 to 5, wherein said mutation(s) in the coding sequence for N^{pro} leads to an N^{pro} having the amino acid sequence MELFSN.

11. The vaccine according to any one of claim 1 to 10, wherein said combination vaccine comprises attenuated BVDV type 1 and attenuated BVDV type 2, both having at least one mutation in the coding sequence for glycoprotein E^{rns} and at least another mutation in the coding sequence for N^{pro}, wherein said mutation(s) in the coding sequence for glycoprotein E^{rns} lead(s) to inactivation of RNase activity residing in E^{rns} and wherein said mutation(s) in the coding sequence for N^{pro} lead(s) to inactivation of said N^{pro}.

12. The combination vaccine according to any one of claims 1 to 11, wherein said infection in cattle other than BVDV is caused by at least one pathogen selected from the group consisting of: Parainfluenza-3 Virus (PI-3), Infectious Bovine Rhinotracheitis virus (IBR), Bovine Respiratory Syncytial Virus (BRSV), Bovine Herpesvirus (BHV), Bovine Rotavirus (BRV), Bovine Enterovirus (BEV), Bovine Coronovirus (BCV), Bovine Rabies (BR), Bovie Parvovirus (PPV), Adenovirus Astrovirus, Mannheimia haemolytica (formerly Pasteurella haemolytica), Pasteurella multocida, Haemophilus somnus (Histophilus ovis and Haemophilus agni), Actinomyces (Corynebacterium), Actinomyces pyogenes, Chlamydia psittaci, Campylobacter fetus venerealis and Campylobacter fetus fetus (formerly C fetus intestinalis), Leptospira interrogans, Leptospira hardjo, Leptospira pomona, and Leptospira grippotyphosa, Leptospira canicola, Leptospira grippotyphosa, Leptospira hardjo (Leptospira hardjoprajitno and Leptospira hardjo-bovis), Brucella abortus, Brucella suis and Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium; Salmonella newport, Mycobacterium avium paratuberculosis, Cryptsporidium parvum, Cryptsporidium hominis, Staphylococcus aureus, Streptococcus dysgalactiae, Streptococcus uberus, Mycoplasma spp, Mycoplasma dispar, Mycoplasma bovis, and Ureaplasma spp., Tritrichomonas foetus, Trichophyton verrucosum, Trichophyton mentagrophytes, Trichophyton sarkisovii, Neospora caninum (formerly Toxoplasma gondii), Babesia bigemina and Babesia bovis, and Dictyocaulus viviparous (Lungworm disease).

13. The combination vaccine according to any one of claims 1 to 12, wherein said immunological active component is an antigen of at least one pathogen selected from the group consisting of: Parainfluenza-3 Virus (PI-3), Infectious Bovine Rhinotracheitis virus (IBR), Bovine Respiratory Syncytial Virus (BRSV), Bovine Herpesvirus (BHV), Bovine Rotavirus (BRV), Bovine Enterovirus (BEV), Bovine Coronovirus (BCV), Bovine Rabies (BR), Bovie Parvovirus (PPV), Adenovirus Astrovirus, Mannheimia haemolytica (formerly Pasteurella haemolytica), Pasteurella multocida, Haemophilus somnus (Histophilus ovis and Haemophilus agni), Actinomyces (Corynebacterium), Actinomyces pyogenes, Chlamydia psittaci, Campylobacter fetus venerealis and Campylobacter fetus fetus (formerly C fetus intestinalis), Leptospira interrogans, Leptospira hardjo, Leptospira pomona, and Leptospira grippotyphosa, Leptospira canicola, Leptospira grippotyphosa, Leptospira hardjo (Leptospira hardjoprajitno and Leptospira hardjo-bovis), Brucella abortus, Brucella suis and Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium; Salmonella newport, Mycobacterium avium paratuberculosis, Cryptsporidium parvum, Cryptsporidium hominis, Staphylococcus aureus, Streptococcus dysgalactiae, Streptococcus uberus, Mycoplasma spp, Mycoplasma dispar, Mycoplasma bovis, and Ureaplasma spp., Tritrichomonas foetus, Trichophyton verrucosum, Trichophyton mentagrophytes, Trichophyton sarkisovii, Neospora caninum (formerly Toxoplasma gondii), Babesia bigemina and Babesia bovis, and Dictyocaulus viviparous (Lungworm disease).

14. The combination vaccine according to claim 13, wherein said immunological active component is an antigen of at least one pathogen of viral origin selected from the group consisting of:
such as Parainfluenza-3 Virus, Infectious Bovine Rhinotracheitis virus, Bovine Respiratory Syncytial Virus, Bovine Herpesvirus, Bovine Rotavirus (BRV), Bovine Enterovirus, Bovine Coronovirus, Bovine Rabies, Bovine Parvovirus, Adenovirus and Astrovirus.

## Patentansprüche

1. Kombinationsimpfstoff für die Prophylaxe von Rindern gegen mikrobiologische Infektionen, wobei besagter Kombinationsimpfstoff umfasst
a. ein oder mehrere abgeschwächte(s) BVDV, welche(s) mindestens eine Mutation in der kodierenden Sequenz für Glykoprotein E^{rns} und mindestens eine weitere Mutation in der kodierenden Sequenz für N^{pro} hat, wobei besagte Mutation(en) in der kodierenden Sequenz für Glykoprotein E^{rns} zur Inaktivierung von RNase-Aktivität, die in E^{rns} gegeben ist, führt (führen) und besagte Mutation(en) in der kodierenden Sequenz für N^{pro} zur Inaktivierung des besagten N^{pro} führt (führen); und
b. ein oder mehrere immunologisch aktive(r) Bestandteil(e), welche(r) für die Prophylaxe mikrobiologischer Infektion in Rindern, ausgelöst von einem anderen Rinderpathogen als BVDV, wirksam ist (sind), wobei der immunologisch aktive Bestandteil ein abgeschwächter Mikroorganismus, ein abgetöteter Mikroorganismus oder zumindest ein immunologischer Teil eines Mikroorganismus ist.

2. Impfstoff gemäß Anspruch 1, wobei der abgeschwächte Mikroorganismus ein modifiziertes lebendes Virus ("modified live virus", MLV) ist.

3. Impfstoff gemäß Anspruch 1 oder 2, wobei besagte Mutation(en) in der Kodierungssequenz für Glykoprotein E^{rns} in der kodierenden Nukleotidsequenz lokalisiert ist (sind), die den Aminosäuren an Position 298 bis 310 und/oder Position 341 bis 360 entspricht.

4. Impfstoff gemäß irgendeinem der Ansprüche 1 bis 3, wobei besagte Mutation(en) in der kodierenden Sequenz für N^{pro} zu einem kodierten Polyprotein führt (führen), welches durch die folgende Formel charakterisiert ist:
[N^{pro}]ₓ-[C-term]
wobei **[N^{pro}]** sich auf den N^{pro}-Anteil des besagten Polyproteins bezieht;
wobei **"x"** die Anzahl von Aminosäuren des N^{pro} repräsentiert, die in dem Polyprotein vorhanden sind,
wobei **"x"** 0 bis 12 ist, was bedeutet, dass keine N^{pro}-spezifische Aminosäure oder 1 bis 12 Aminosäuren von N^{pro}, bevorzugt vom N-Terminus von N^{pro}, vorhanden sind; und wobei **[C-term]** sich auf das komplette BVDV-Polyprotein abgesehen von N^{pro} bezieht, aber einschließlich des Kapsid (C)-Proteins und irgendeines anderen Proteins das in dem BVDV-Polyprotein vorhanden ist, einschließlich des carboxyterminalen NS5B.

5. Kombinationsimpfstoff gemäß irgendeinem der Ansprüche 1 bis 4, wobei besagte Mutation in der Kodierungssequenz für Glykoprotein E^{rns} ausgewählt ist aus der Gruppe bestehend aus S298G, H300K, H300L, H300R, H300del, W303G, P304del, E305A, C308G, R343G, E345del, W346G, K348A, H349K, H349L, H349del, H349Q, H349SV, K348R, W351P, W351G, W351L, W351K, W351H, H300L/H349L, K348del/H349del, H349del/G350del, E345del/H349del, W303G/E305A, H300K/H349K, H300K/H349L, L299del/H300del/G301del und K348del/H349del/G350del.

6. Kombinationsimpfstoff gemäß irgendeinem der Ansprüche 1 bis 5, wobei besagte Mutation(en) in der Kodierungssequenz für N^{pro} zu einem N^{pro} führt, welches die Aminosäuresequenz ME, MELFS, MELFSNE, MELFSNEL, MELFSNELL, MELFSNELLY, MELFSNELLYK, MELFSNELLYKT hat.

7. Kombinationsimpfstoff gemäß irgendeinem der Ansprüche 1 bis 5, wobei besagte Mutation(en) in der Kodierungssequenz für N^{pro} zu einem N^{pro} führt, welches die Aminosäuresequenz MESDEGSK, MELFSSDEGSK, MELFSNESDEGSK, MELFSNELSDEGSK, MELFSNELLSDEGSK, MELFSNELLYSDEGSK, MELFSNELLYKSDEGSK oder MELFSNELLYKTSDEGSK hat.

8. Kombinationsimpfstoff gemäß irgendeinem der Ansprüche 1 bis 5, wobei besagte Mutation(en) in der Kodierungssequenz für N^{pro} zu einem N^{pro} führt, welches die Aminosäuresequenz MEL hat.

9. Kombinationsimpfstoff gemäß irgendeinem der Ansprüche 1 bis 5, wobei besagte Mutation(en) in der Kodierungssequenz für N^{pro} zu einem N^{pro} führt, welches die Aminosäuresequenz MELF hat.

10. Kombinationsimpfstoff gemäß irgendeinem der Ansprüche 1 bis 5, wobei besagte Mutation(en) in der Kodierungssequenz für N^{pro} zu einem N^{pro} führt, welches die Aminosäuresequenz MELFSN hat.

11. Impfstoff gemäß irgendeinem der Ansprüche 1 bis 10, wobei besagter Kombinationsimpfstoff abgeschwächtes BVDV Typ 1 und abgeschwächtes BVDV Typ 2 umfasst, wobei beide mindestens eine Mutation in der Kodierungssequenz für Glykoprotein E^{rns}, und mindestens eine weitere Mutation in der Kodierungssequenz für N^{pro} haben, wobei besagte Mutation(en) in der Kodierungssequenz für Glykoprotein E^{rns} zur Inaktivierung von RNase-Aktivität, die in E^{rns} gegeben ist, führt (führen) und wobei besagte Mutation(en) in der Kodierungssequenz für N^{pro} zur Inaktivierung des besagten N^{pro} führt (führen).

12. Kombinationsimpfstoff gemäß irgendeinem der Ansprüche 1 bis 11, wobei besagte andere Infektion als BVDV in Rindern von mindestens einem Pathogen hervorgerufen wird, das ausgewählt ist aus der Gruppe bestehend aus: Parainfluenza-3 Virus (PI-3), Infektiösem Bovinem Rhinotracheitis-Virus (IBR), Bovinem Respiratorischem Syncytial-Virus (BRSV), Bovinem Herpesvirus (BHV), Bovinem Rotavirus (BRV), Bovinem Enterovirus (BEV), Bovinem Coronavirus (BCV), Boviner Tollwut ("Bovine Rabies") (BR), Bovinem Parvovirus (PPV), Adenovirus Astrovirus, Mannheimia haemolytica (früher Pasteurella haemolytica), Pasteurella multocida, Haemophilus somnus (Histophilus ovis und Haemophilus agni), Actinomyces (Corynebakterium), Actinomyces pyogenes, Chlamydia psittaci, Campylobacter fetus venerealis und Campylobacter fetus fetus (früher C fetus intestinalis), Leptospira interrogans, Leptospira hardjo, Leptospira pomona, und Leptospira grippotyphosa, Leptospira canicola, Leptospira grippotyphosa, Leptospira hardjo (Leptospira hardjoprajitno und Leptospira hardjo-bovis), Brucella abortus, Brucella suis und Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium; Salmonella newport, Mycobacterium avium paratuberculosis, Cryptsporidium parvum, Cryptsporidium hominis, Staphylococcus aureus, Streptococcus dysgalactiae, Streptococcus uberus, Mycoplasma spp, Mycoplasma dispar, Mycoplasma bovis, und Ureaplasma spp., Tritrichomonas foetus, Trichophyton verrucosum, Trichophyton mentagrophytes, Trichophyton sarkisovii, Neospora caninum (früher Toxoplasma gondii), Babesia bigemina und Babesia bovis, und Dictyocaulus viviparous (Lungenwurm-Erkrankung).

13. Kombinationsimpfstoff gemäß irgendeinem der Ansprüche 1 bis 12, wobei besagter immunologisch aktiver Bestandteil ein Antigen von mindestens einem Pathogen ist, das ausgewählt ist aus der Gruppe bestehend aus: Parainfluenza-3 Virus (PI-3), Infektiösem Bovinem Rhinotracheitis-Virus (IBR), Bovinem Respiratorischem Syncytial-Virus (BRSV), Bovinem Herpesvirus (BHV), Bovinem Rotavirus (BRV), Bovinem Enterovirus (BEV), Bovinem Coronavirus (BCV), Boviner Tollwut ("Bovine Rabies") (BR), Bovinem Parvovirus (PPV), Adenovirus Astrovirus, Mannheimia haemolytica (früher Pasteurella haemolytica), Pasteurella multocida, Haemophilus somnus (Histophilus ovis und Haemophilus agni), Actinomyces (Corynebakterium), Actinomyces pyogenes,
Chlamydia psittaci, Campylobacter fetus venerealis und Campylobacter fetus fetus (früher C fetus intestinalis), Leptospira interrogans, Leptospira hardjo, Leptospira pomona, und Leptospira grippotyphosa, Leptospira canicola, Leptospira grippotyphosa, Leptospira hardjo (Leptospira hardjoprajitno und Leptospira hardjo-bovis), Brucella abortus, Brucella suis und Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium; Salmonella newport, Mycobacterium avium paratuberculosis, Cryptsporidium parvum, Cryptsporidium hominis, Staphylococcus aureus, Streptococcus dysgalactiae, Streptococcus uberus, Mycoplasma spp, Mycoplasma dispar, Mycoplasma bovis, und Ureaplasma spp., Tritrichomonas foetus, Trichophyton verrucosum, Trichophyton mentagrophytes, Trichophyton sarkisovii, Neospora caninum (früher Toxoplasma gondii), Babesia bigemina und Babesia bovis, und Dictyocaulus viviparous (Lungenwurm-Erkrankung).

14. Kombinationsimpfstoff gemäß Anspruch 13, wobei besagter immunologisch aktiver Bestandteil ein Antigen von mindestens einem Pathogen viralen Ursprungs ist, ausgewählt aus der Gruppe bestehen aus:
wie beispielsweise Parainfluenza-3 Virus, Infektiösem Bovinem Rhinotracheitis-Virus, Bovinem Respiratorischem Syncytial-Virus, Bovinem Herpesvirus, Bovinem Rotavirus (BRV), Bovinem Enterovirus, Bovinem Coronavirus, Boviner Tollwut, Bovinem Parvovirus, Adenovirus und Astrovirus.

## Revendications

1. Vaccin combiné pour la prophylaxie des bovins contre les infections microbiologiques, dans lequel ledit vaccin combiné comprend
a. un ou plusieurs BVDV atténués, ayant au moins une mutation dans la séquence codante pour la glycoprotéine E^{rns} et au moins une autre mutation dans la séquence codante pour N^{pro}, dans lequel ladite/lesdites mutation(s) dans la séquence codante pour la glycoprotéine E^{rns} conduit/conduisent à l'inactivation de l'activité d'ARNase résidant dans E^{rns} et ladite/lesdites mutation(s) dans la séquence codante pour N^{pro} conduit/conduisent à l'inactivation dudit N^{pro} ; et
b. un ou plusieurs composants actifs immunologiques efficaces pour la prophylaxie d'une infection microbiologique chez les bovins provoquée par un pathogène bovin autre que le BVDV, dans lequel le composant actif immunologique est un microorganisme atténué, un microorganisme tué, ou au moins une partie immunologique d'un microorganisme.

2. Vaccin selon la revendication 1, dans lequel le microorganisme atténué est un virus vivant modifié (MLV).

3. Vaccin selon la revendication 1 ou 2, dans lequel ladite/lesdites mutation(s) dans la séquence codante pour la glycoprotéine E^{rns} est/sont située(s) dans la séquence nucléotidique codante correspondant aux acides aminés en position 298 à 310 et/ou en position 341 à 360.

4. Vaccin selon l'une quelconque des revendications 1 à 3, dans lequel ladite/lesdites mutation(s) dans la séquence codante pour N^{pro} conduit/conduisent à une polyprotéine codée telle que **caractérisée par** la formule suivante :
[N^{pro}]ₓ-[C-term]
dans laquelle [N^{pro}] concerne la partie N^{pro} de ladite polyprotéine ;
dans laquelle « x » représente le nombre d'acides aminés du N^{pro} présent dans la polyprotéine,
dans laquelle « x » est 0 à 12, ce qui signifie qu'aucun acide aminé spécifique de N^{pro} ou 1 à 12 acides aminés de N^{pro}, de préférence de l'extrémité N-terminale de N^{pro} sont présents ; et
dans laquelle [**C-term**] concerne la polyprotéine complète du BVDV à l'exception de N^{pro}, mais incluant la protéine de capside (C) et n'importe quelle autre protéine présente dans la polyprotéine du BVDV incluant le NS5B carboxy terminal.

5. Vaccin combiné selon l'une quelconque des revendications 1 à 4, dans lequel ladite mutation dans la séquence codante pour la glycoprotéine E^{rns} est sélectionnée à partir du groupe constitué par S298G, H300K, H300L, H300R, H300del, W303G, P304del, E305A, C308G, R343G, E345del, W346G, K348A, H349K, H349L, H349del, H349Q, H349SV, K348R, W351P, W351G, W351L, W351K, W351H, H300L/H349L, K348del/H349del, H349del/G350del, E345del/H349del, W303G/E305A, H300K/H349K, H300K/H349L, L299del/H300del/G301del et K348del/H349del/G350del.

6. Vaccin combiné selon l'une quelconque des revendications 1 à 5, dans lequel ladite/lesdites mutation(s) dans la séquence codante pour N^{pro} conduit/conduisent à un N^{pro} ayant la séquence d'acides aminés ME, MELFS, MELFSNE, MELFSNEL, MELFSNELL, MELFSNELLY, MELFSNELLYK, MELFSNELLYKT.

7. Vaccin combiné selon l'une quelconque des revendications 1 à 5, dans lequel ladite/lesdites mutation(s) dans la séquence codante pour N^{pro} conduit/conduisent à un N^{pro} ayant la séquence d'acides aminés MESDEGSK, MELFSSDEGSK, MELFSNESDEGSK, MELFSNELSDEGSK, MELFSNELLSDEGSK, MELFSNELLYSDEGSK, MELFSNELLYKSDEGSK ou MELFSNELLYKTSDEGSK.

8. Vaccin combiné selon l'une quelconque des revendications 1 à 5, dans lequel ladite/lesdites mutation(s) dans la séquence codante pour N^{pro} conduit/conduisent à un N^{pro} ayant la séquence d'acides aminés MEL.

9. Vaccin combiné selon l'une quelconque des revendications 1 à 5, dans lequel ladite/lesdites mutation(s) dans la séquence codante pour N^{pro} conduit/conduisent à un N^{pro} ayant la séquence d'acides aminés MELF.

10. Vaccin combiné selon l'une quelconque des revendications 1 à 5, dans lequel ladite/lesdites mutation(s) dans la séquence codante pour N^{pro} conduit/conduisent à un N^{pro} ayant la séquence d'acides aminés MELFSN.

11. Vaccin selon l'une quelconque des revendications 1 à 10, dans lequel ledit vaccin combiné comprend un BVDV de type 1 atténué et un BVDV de type 2 atténué, tous les deux ayant au moins une mutation dans la séquence codante de la glycoprotéine E^{rns} et au moins une autre mutation dans la séquence codante de N^{pro}, dans lequel ladite/lesdites mutation(s) dans la séquence codante de la glycoprotéine E^{rns} conduit/conduisent à l'inactivation de l'activité d'ARNase résidant dans E^{rns} et dans lequel ladite/lesdites mutation(s) dans la séquence codante pour N^{pro} conduit/conduisent à l'inactivation dudit N^{pro}.

12. Vaccin combiné selon l'une quelconque des revendications 1 à 11, dans lequel ladite infection chez les bovins autre que le BVDV est provoquée par au moins un pathogène sélectionné à partir du groupe constitué par : le virus parainfluenza-3 (PI-3), le virus de la rhinotrachéite infectieuse bovine (IBR), le virus respiratoire syncytial bovin (BRSV), l'herpèsvirus bovin (BHV), le rotavirus bovin (BRV), l'entérovirus bovin (BEV), le coronovirus bovin (BCV), la rage bovine (BR), le parvovirus bovin (PPV), un adénovirus astrovirus, Mannheimia haemolytica (anciennement Pasteurella haemolytica), Pasteurella multocida, Haemophilus somnus (Histophilus ovis et Haemophilus agni), Actinomyces (Corynebacterium), Actinomyces pyogenes, Chlamydia psittaci, Campylobacter fetus venerealis et Campylobacter fetus fetus (anciennement C fetus intestinalis), Leptospira interrogans, Leptospira hardjo, Leptospira pomona, et Leptospira grippotyphosa, Leptospira canicola, Leptospira grippotyphosa, Leptospira hardjo (Leptospira hardjoprajitno et Leptospira hardjo-bovis), Brucella abortus, Brucella suis et Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium ; Salmonella newport, Mycobacterium avium paratuberculosis, Cryptsporidium parvum, Cryptsporidium hominis, Staphylococcus aureus, Streptococcus dysgalactiae, Streptococcus uberus,
Mycoplasma spp, Mycoplasma dispar, Mycoplasma bovis, et Ureaplasma spp., Tritrichomonas foetus, Trichophyton verrucosum, Trichophyton mentagrophytes, Trichophyton sarkisovii, Neospora caninum (anciennement Toxoplasma gondii), Babesia bigemina et Babesia bovis, et Dictyocaulus viviparous (bronchite vermineuse).

13. Vaccin combiné selon l'une quelconque des revendications 1 à 12, dans lequel ledit composant actif immunologique est un antigène d'au moins un pathogène sélectionné à partir du groupe constitué par : le virus parainfluenza-3 (PI-3), le virus de la rhinotrachéite infectieuse bovine (IBR), le virus respiratoire syncytial bovin (BRSV), l'herpèsvirus bovin (BHV), le rotavirus bovin (BRV), l'entérovirus bovin (BEV), le coronovirus bovin (BCV), la rage bovine (BR), le parvovirus bovin (PPV), un adénovirus astrovirus, Mannheimia haemolytica (anciennement Pasteurella haemolytica), Pasteurella multocida, Haemophilus somnus (Histophilus ovis et Haemophilus agni), Actinomyces (Corynebacterium), Actinomyces pyogenes, Chlamydia psittaci, Campylobacter fetus venerealis et Campylobacter fetus fetus (anciennement C fetus intestinalis), Leptospira interrogans, Leptospira hardjo, Leptospira pomona, et Leptospira grippotyphosa, Leptospira canicola, Leptospira grippotyphosa, Leptospira hardjo (Leptospira hardjoprajitno et Leptospira hardjo-bovis), Brucella abortus, Brucella suis et Brucella melitensis, Listeria monocytogenes, Chlamydia psittaci, Clostridium chauvoei, Clostridium septicum, Clostridium haemolyticum, Clostridium novyi, Clostridium sordellii, Clostridium perfringens, Clostridium tetani, Moraxella bovis, Klebsiella spp, Klebsiella pneumoniae, Salmonella typhimurium ; Salmonella newport, Mycobacterium avium paratuberculosis, Cryptsporidium parvum, Cryptsporidium hominis, Staphylococcus aureus, Streptococcus dysgalactiae, Streptococcus uberus, Mycoplasma spp, Mycoplasma dispar, Mycoplasma bovis, et Ureaplasma spp., Tritrichomonas foetus, Trichophyton verrucosum, Trichophyton mentagrophytes, Trichophyton sarkisovii, Neospora caninum (anciennement Toxoplasma gondii), Babesia bigemina et Babesia bovis, et Dictyocaulus viviparous (bronchite vermineuse).

14. Vaccin combiné selon la revendication 13, dans lequel ledit composant actif immunologique est un antigène d'au moins un pathogène d'origine virale sélectionné à partir du groupe constitué par :
tel que le virus parainfluenza-3, le virus de la rhinotrachéite infectieuse bovine, le virus respiratoire syncytial bovin, l'herpèsvirus bovin, le rotavirus bovin (BRV), l'entérovirus bovin, le coronovirus bovin, la rage bovine, le parvovirus bovin, un adénovirus et un astrovirus.
